# EUROPEAN PATENT SPECIFICATION

(11) **EP 2 678 432 B1**
(45) Date of publication and mention of the grant of the patent: **01.08.2018**
(21) Application number: 12749472.2
(22) Date of filing: 24.02.2012
(51) Int. Cl.: C12P 7/04

(54) **RECOMBINANT MICROORGANISMS AND USES THEREFOR**
REKOMBINANTE MIKROORGANISMEN UND VERWENDUNGEN DAFÜR
MICRO-ORGANISMES RECOMBINANTS ET UTILISATIONS DE CEUX-CI

(30) Priority: 25.02.2011 US 201161446832 P
(43) Date of publication of application: 01.01.2014
(60) Divisional application: 18179128.6
(73) Proprietor: Lanzatech New Zealand Limited, Auckland 1052 (NZ)
(72) Inventor: SIMPSON, Sean, Dennis, Auckland 1052 (NZ); KOEPKE, Michael, Auckland 1052 (NZ); LIEW, Fungmin, Auckland 1052 (NZ); CHEN, Wendy, Yiting, Auckland 1052 (NZ)
(74) Representative: J A Kemp
(86) International application number: PCT/NZ2012/000022
(87) International publication number: WO 2012/115527

(56) References cited:
- WO-A1-2009/113878
- WO-A1-2010/121849
- WO-A1-2010/121849
- US-A1- 2010 304 453
- DUERRE P: "NEW INSIGHTS AND NOVEL DEVELOPMENTS IN CLOSTRIDIAL ACETONE/BUTANOL/ISOPROPANOL FERMENTATION", APPLIED MICROBIOLOGY AND BIOTECHNOLOGY, SPRINGER, DE, vol. 49, no. 6, 1 January 1998 (1998-01-01), pages 639-648, XP001181082, ISSN: 0175-7598, DOI: 10.1007/S002530051226
- KOPKE, M. ET AL.: 'Clostridium ljungdahlii represents a microbial production platform based on syngas' PROCEEDINGS OF THE NATIONAL ACADEMY OF SCIENCES OF USA. vol. 107, no. 29, July 2010, pages 13087 - 13092, XP055086327
- RAMACHANDRIYA, K.D. ET AL.: 'Reduction of acetone to isopropanol using producer gas fermenting microbes' BIOTECHNOLOGY AND BIOENGINEERING. vol. 108, no. 10, October 2011, pages 2330 - 2338, XP055115054
- SCHIEL-BENGELSDORF, B. ET AL.: 'Pathway engineering and synthetic biology using acetogens' FEBS LETTERS. vol. 586, no. 15, July 2012, pages 2191 - 2198, XP028400698
- MEHTA M D ET AL: "Enzyme activities in clostridia producing ethanol from carbon monoxide", ABSTRACTS OF THE GENERAL MEETING OF THE AMERICAN SOCIETY FOR MICROBIOLOGY, AMERICAN SOCIETY FOR MICROBIOLOGY, US, vol. 104, 1 January 2004 (2004-01-01), page 474, XP008126075, ISSN: 1060-2011

### SEQUENCE LISTING

<110> Lanzatech New Zealand Limited
   Koepke, Michael
   Chen, wendy
   Liew, Fungmin
   Simpson, Sean
<120> Recombinant microorganisms and methods of use thereof
<130> 509018PCTPR
<150> US 61/446,832
   <151> 2011-02-25
<160> 101
<170> PatentIn version 3.5
<210> 1
   <211> 351
   <212> PRT
   <213> clostridium autoethanogenum
<400> 1
   Met Lys Gly Phe Ala Met Leu Gly Ile Asn Lys Leu Gly Trp Ile Glu
   1               5                   10                  15
   Lys Lys Asn Pro Val Pro Gly Pro Tyr Asp Ala Ile Val His Pro Leu
               20                  25                  30
   Ala Val Ser Pro Cys Thr Ser Asp Ile His Thr Val Phe Glu Gly Ala
           35                  40                  45
   Leu Gly Asn Arg Glu Asn Met Ile Leu Gly His Glu Ala Val Gly Glu
       50                  55                  60
   Ile Ala Glu Val Gly Ser Glu Val Lys Asp Phe Lys Val Gly Asp Arg
   65                  70                  75                  80
   Val Ile Val Pro Cys Thr Thr Pro Asp Trp Arg Ser Leu Glu Val Gln
                   85                  90                  95
   Ala Gly Phe Gln Gln His Ser Asn Gly Met Leu Ala Gly Trp Lys Phe
               100                 105                 110
   Ser Asn Phe Lys Asp Gly Val Phe Ala Asp Tyr Phe His Val Asn Asp
           115                 120                 125
   Ala Asp Met Asn Leu Ala Ile Leu Pro Asp Glu Ile Pro Leu Glu Ser
       130                 135                 140
   Ala Val Met Met Thr Asp Met Met Thr Thr Gly Phe His Gly Ala Glu
   145                 150                 155                 160
   Leu Ala Asp Ile Lys Met Gly Ser Ser Val Val Val Ile Gly Ile Gly
                   165                 170                 175
   Ala Val Gly Leu Met Gly Ile Ala Gly Ser Lys Leu Arg Gly Ala Gly
               180                 185                 190
   Arg Ile Ile Gly Val Gly Ser Arg Pro Val Cys Val Glu Thr Ala Lys
           195                 200                 205
   Phe Tyr Gly Ala Thr Asp Ile Val Asn Tyr Lys Asn Gly Asp Ile Val
       210                 215                 220
   Glu Gln Ile Met Asp Leu Thr His Gly Lys Gly Val Asp Arg Val Ile
   225                 230                 235                 240
   Met Ala Gly Gly Gly Ala Glu Thr Leu Ala Gln Ala Val Thr Met Val
                   245                 250                 255
   Lys Pro Gly Gly Val Ile Ser Asn Ile Asn Tyr His Gly Ser Gly Asp
               260                 265                 270
   Thr Leu Pro Ile Pro Arg Val Gln Trp Gly Cys Gly Met Ala His Lys
           275                 280                 285
   Thr Ile Arg Gly Gly Leu Cys Pro Gly Gly Arg Leu Arg Met Glu Met
       290                 295                 300
   Leu Arg Asp Leu Val Leu Tyr Lys Arg Val Asp Leu Ser Lys Leu Val
   305                 310                 315                 320
   Thr His Val Phe Asp Gly Ala Glu Asn Ile Glu Lys Ala Leu Leu Leu
                   325                 330                 335
   Met Lys Asn Lys Pro Lys Asp Leu Ile Lys Ser Val Val Thr Phe
               340                 345                 350
<210> 2
   <211> 1056
   <212> DNA
   <213> clostridium autoethanogenum
<400> 2
   atgaaaggtt ttgcaatgtt aggtattaac aaattaggat ggattgaaaa gaaaaaccca      60
   gtgccaggtc cttatgatgc gattgtacat cctctagctg tatccccatg tacatcagat     120
   atacatacgg tttttgaagg agcacttggt aatagggaaa atatgatttt aggccatgaa     180
   gctgtaggtg aaatagccga agttggcagc gaagttaaag attttaaagt tggcgataga     240
   gttatcgtac catgcacaac acctgactgg agatctttag aagtccaagc tggttttcag     300
   cagcattcaa acggtatgct tgcaggatgg aagttttcca attttaaaga cggtgtattt     360
   gcagattact ttcatgtaaa cgatgcagat atgaatcttg ccatactccc agatgaaata     420
   cctttagaaa gtgcagttat gatgacagac atgatgacta ctggttttca tggagcagaa     480
   cttgcagaca taaaaatggg ctccagcgtt gtagtaattg gtataggagc tgttggatta    540
   atgggaatag ccggttccaa acttcgagga gcaggcagaa ttatcggtgt tggaagcaga    600
   cctgtttgtg ttgaaacagc taaattttat ggagcaactg atattgtaaa ttataaaaat    660
   ggtgatatag ttgaacaaat catggactta actcatggta aaggtgtaga ccgtgtaatc    720
   atggcaggcg gtggtgctga aacactagca caagcagtaa ctatggttaa acctggcggc    780
   gtaatttcta acatcaacta ccatggaagc ggtgatactt taccaatacc tcgtgttcaa    840
   tggggctgcg gcatggctca caaaactata agaggaggat tatgccccgg cggacgtctt    900
   agaatggaaa tgctaagaga tcttgttcta tataaacgtg ttgatttgag taaacttgtt    960
   actcatgtat ttgatggtgc agaaaatatt gaaaaggccc ttttgcttat gaaaaataag   1020
   ccaaaagatt taattaaatc agtagttaca ttctaa                              1056
<210> 3
   <211> 1056
   <212> DNA
   <213> clostridium ljundahlii
<400> 3
   atgaaaggtt ttgcaatgtt aggtattaac aaattaggat ggattgaaaa gaaaaaccca      60
   gtgccaggtc cttatgatgc gattgtacat cctctagctg tatccccatg tacatcagat    120
   atacatacgg tttttgaagg agcacttggt aatagggaaa atatgatttt aggccatgaa    180
   gctgtaggtg aaatagccga agttggcagc gaagttaaag attttaaagt tggcgataga    240
   gttatcgtac catgcacaac acctgactgg agatctttag aagtccaagc tggttttcag    300
   cagcattcaa acggtatgct tgcaggatgg aagttttcca attttaaaga tggtgtattt    360
   gcagattact ttcatgtaaa cgatgcagat atgaatcttg ccatactccc agatgaaata    420
   cctttagaaa gtgcagttat gatgacagac atgatgacta ctggttttca tggagcagaa    480
   cttgcagaca taaaaatggg ctccagcgtt gtagtaattg gtataggagc tgttggatta    540
   atgggaatag ccggttccaa acttcgagga gcaggcagaa ttatcggtgt tggaagcaga    600
   cctgtttgtg ttgaaacagc taaattttat ggagcaactg atattgtaaa ttataaaaat    660
   ggtgatatag ttgaacaaat catggactta actcatggta aaggtgtaga ccgtgtaatc    720
   atggcaggcg gtggtgctga aacactagca caagcagtaa ctatggttaa acctggcggc    780
   gtaatttcta acatcaacta ccatggaagc ggtgatactt taccaatacc tcgtgttcaa    840
   tggggctgcg gcatggctca caaaactata agaggaggat tatgccccgg cggacgtctt    900
   agaatggaaa tgctaagaga tcttgttcta tataaacgtg ttgatttgag taaacttgtt    960
   actcatgtat ttgatggtgc agaaaatatt gaaaaggccc ttttgcttat gaaaaataag   1020
   ccaaaagatt taattaaatc agtagttaca ttctaa                              1056
<210> 4
   <211> 1056
   <212> DNA
   <213> clostridium ragsdalei
<400> 4
   atgaaaggtt ttgcaatgtt aggtattaac aagttaggat ggattgaaaa gaaaaaccca 60
   gtaccaggtc cttatgatgc gattgtacat cctctagctg tatccccatg tacatcagat 120
   atacatacgg tttttgaagg agcacttggt aatagggaaa atatgatttt aggtcacgaa 180
   gctgtaggtg aaatagctga agttggcagt gaagttaaag attttaaagt tggcgataga 240
   gttatcgtac catgcacaac acctgactgg agatccttag aagtccaagc tggttttcaa 300
   cagcattcaa acggtatgct tgcaggatgg aagttttcca attttaaaga cggtgtattt 360
   gcagattact ttcatgtaaa cgatgcagat atgaatcttg caatacttcc agatgaaata 420
   cctttagaaa gtgcagttat gatgacagac atgatgacta ctggttttca tggggcagaa 480
   cttgctgaca taaaaatggg ttccagtgtt gtcgtaattg gtataggagc tgttggatta 540
   atgggaatag ccggttccaa acttcgagga gcaggtagaa ttatcggtgt tggaagcaga 600
   cccgtttgtg ttgaaacagc taaattttat ggagcaactg atattgtaaa ttataaaaat 660
   ggtgatatag ttgaacaaat aatggactta actcatggta aaggtgtaga ccgtgtaatc 720
   atggcaggcg gtggtgctga aacactagca caagcagtaa ctatggttaa acctggcggc 780
   gtaatttcta acatcaacta ccatggaagc ggtgatactt tgccaatacc tcgtgttcaa 840
   tggggctgcg gcatggctca caaaactata agaggagggt tatgtcccgg cggacgtctt 900
   agaatggaaa tgctaagaga ccttgttcta tataaacgtg ttgatttgag caaacttgtt 960
   actcatgtat ttgatggtgc agaaaatatt gaaaaggccc ttttgcttat gaaaaataag 1020
   ccaaaagatt taattaaatc agtagttaca ttctaa 1056
<210> 5
   <211> 20
   <212> DNA
   <213> Synthetic
<400> 5
   tcaggacctt ctggaactgg 20
<210> 6
   <211> 20
   <212> DNA
   <213> synthetic primer
<400> 6
   acctcccctt ttcttggaga 20
<210> 7
   <211> 20
   <212> DNA
   <213> synthetic primer
<400> 7
   caggtttcgg tgctgaccta 20
<210> 8
   <211> 20
   <212> DNA
   <213> synthetic primer
<400> 8
   aactccgccg ttgtatttca 20
<210> 9
   <211> 25
   <212> DNA
   <213> synthetic primer
<400> 9
   acaagatggg gtcgaaacag tttgg 25
<210> 10
   <211> 25
   <212> DNA
   <213> synthetic primer
<400> 10
   tggcactgga cttactctac atggg 25
<210> 11
   <211> 30
   <212> DNA
   <213> synthetic primer
<400> 11
   tatttccgaa gatgatattg aattgtatgg 30
<210> 12
   <211> 24
   <212> DNA
   <213> synthetic primer
<400> 12
   tccagcaggt gttgggttta tagc 24
<210> 13
   <211> 22
   <212> DNA
   <213> synthetic primer
<400> 13
   agctgcaact cctggtggag gc 22
<210> 14
   <211> 27
   <212> DNA
   <213> synthetic primer
<400> 14
   gccttttacc ttttcgtcat actgtgc 27
<210> 15
   <211> 29
   <212> DNA
   <213> synthetic primer
<400> 15
   gcttacatta gtaagagttg gagcaaacg 29
<210> 16
   <211> 29
   <212> DNA
   <213> synthetic primer
<400> 16
   acttgtcctg tgatatatct gctggtagc 29
<210> 17
   <211> 26
   <212> DNA
   <213> synthetic primer
<400> 17
   ggtccttatg atgcgattgt acatcc 26
<210> 18
   <211> 26
   <212> DNA
   <213> synthetic primer
<400> 18
   gctatttcac ctacagcttc atggcc 26
<210> 19
   <211> 654
   <212> DNA
   <213> Clostridium beijerinckii
<400> 19
   atgaataaat tagtaaaatt aacagattta aagcgcattt tcaaagatgg catgacaatt 60
   atggttgggg gttttttaga ttgtggaact cctgaaaata ttatagatat gctagttgat 120
   ttaaatataa aaaatctgac tattataagc aatgatacag cttttcctaa taaaggaata 180
   ggaaaactta ttgtaaatgg tcaagtttct aaagtaattg cttcacatat tggaactaat 240
   cctgaaactg gaaaaaaaat gagctctgga gaacttaaag ttgagctttc cccacaagga 300
   acactgattg aaagaattcg tgcagctgga tctggactcg gaggtgtatt aactccaact 360
   ggacttggaa ctatcgttga agaaggtaag aaaaaagtta ctatcgatgg caaagaatat 420
   ctattagaac ttcctttatc tgctgatgtt tcattaataa aaggtagcat tgtagatgaa 480
   tttggaaata ccttctatag ggctgctact aaaaatttca atccatatat ggcaatggct 540
   gcaaaaacag ttatagttga agcagaaaat ttagttaaat gtgaagattt aaaaagagat 600
   gccataatga ctcctggcgt attagtagat tatatcgtta aggaggcggc ttaa 654
<210> 20
   <211> 666
   <212> DNA
   <213> Clostridium beijerinckii
<400> 20
   ttgattgtag ataaagtttt agcaaaagag ataattgcca aaagagttgc aaaagaacta 60
   aaaaaagacc aactcgtaaa ccttggaata ggacttccaa ctttagtagc aaattatgta 120
   ccaaaagaaa tgaacattac ttttgaatca gaaaatggca tggttggtat ggcacaaatg 180
   gcatcatcag gtgaaaatga cccagatata ataaatgctg gcggggaata tgtaacatta 240
   ttacctcaag gttcattttt tgatagttca atgtctttcg cactaatacg aggaggacat 300
   gttgatgttg ctgttcttgg tgctctagaa gttgatgaaa aaggtaattt agctaactgg 360
   attgttccaa ataaaattgt cccaggtatg ggtggcgcta tggatttagc aataggcgca 420
   aaaaaaataa tagtggcaat gcaacataca ggaaaaagta aacctaaaat cgttaaaaaa 480
   tgtactctcc cacttactgc taaggctcaa gtggatttaa ttgtcacaga actttgtgta 540
   attgatgtaa caaatgacgg cttactttta aaagaaattc ataaagatac aactattgat 600
   gaaattaaat ttttaacaga tgcagattta attattccag ataacttaaa gattatggat 660
   atatga 666
<210> 21
   <211> 741
   <212> DNA
   <213> clostridium beijerinckii
<400> 21
   atgttagaaa gtgaagtatc taaacaaatt acaactccac ttgctgctcc agcgtttcct 60
   agaggaccat ataggtttca caatagagaa tatctaaaca ttatttatcg aactgattta 120
   gatgctcttc gaaaaatagt accagagcca cttgaattag atagagcata tgttagattt 180
   gaaatgatgg ctatgcctga tacaaccgga ctaggctcat atacagaatg tggtcaagct 240
   attccagtaa aatataatgg tgttaagggt gactacttgc atatgatgta tctagataat 300
   gaacctgcta ttgctgttgg aagagaaagt agcgcttatc caaaaaagct tggctatcca 360
   aagctatttg ttgattcaga tactttagtt gggacactta aatatggtac attaccagta 420
   gctactgcaa caatgggata taagcacgag cctctagatc ttaaagaagc ctatgctcaa 480
   attgcaagac ccaattttat gctaaaaatc attcaaggtt acgatggtaa gccaagaatt 540
   tgtgaactaa tatgtgcaga aaatactgat ataactattc acggtgcttg gactggaagt 600
   gcacgtctac aattatttag ccatgcacta gctcctcttg ctgatttacc tgtattagag 660
   attgtatcag catctcatat cctcacagat ttaactcttg gaacacctaa ggttgtacat 720
   gattatcttt cagtaaaata a 741
<210> 22
   <211> 558
   <212> DNA
   <213> Clostridium autoethanogenum
<400> 22
   agatagtcat aatagttcca gaatagttca atttagaaat tagactaaac ttcaaaatgt 60
   ttgttaaata tataccaaac tagtatagat attttttaaa tactggactt aaacagtagt 120
   aatttgccta aaaaattttt tcaatttttt ttaaaaaatc cttttcaagt tgtacattgt 180
   tatggtaata tgtaattgaa gaagttatgt agtaatattg taaacgtttc ttgatttttt 240
   tacatccatg tagtgcttaa aaaaccaaaa tatgtcacat gcaattgtat atttcaaata 300
   acaatattta ttttctcgtt aaattcacaa ataatttatt aataatatca ataaccaaga 360
   ttatacttaa atggatgttt attttttaac acttttatag taaatatatt tattttatgt 420
   agtaaaaagg ttataattat aattgtattt attacaatta attaaaataa aaaatagggt 480
   tttaggtaaa attaagttat tttaagaagt aattacaata aaaattgaag ttatttcttt 540
   aaggagggaa ttattaaa 558
<210> 23
   <211> 26
   <212> DNA
   <213> synthetic primer
<400> 23
   gttcatatga aagaagttgt aatagc 26
<210> 24
   <211> 25
   <212> DNA
   <213> synthetic primer
<400> 24
   caagaattcc tagcactttt ctagc 25
<210> 25
   <211> 26
   <212> DNA
   <213> synthetic primer
<400> 25
   ctaggtacca gggagatatt aaaatg 26
<210> 26
   <211> 26
   <212> DNA
   <213> synthetic primer
<400> 26
   cgtggatcct ctatattgct tttatt 26
<210> 27
   <211> 29
   <212> DNA
   <213> synthetic primer
<400> 27
   aagcggccgc agatagtcat aatagttcc 29
<210> 28
   <211> 29
   <212> DNA
   <213> synthetic primer
<400> 28
   ttccatatga ataattccct ccttaaagc 29
<210> 29
   <211> 20
   <212> DNA
   <213> synthetic primer
<400> 29
   cagaggatgt taatgaagtc 20
<210> 30
   <211> 18
   <212> DNA
   <213> synthetic primer
<400> 30
   ctgtgcagca gtacttgt 18
<210> 31
   <211> 15
   <212> DNA
   <213> synthetic primer
<400> 31
   gcaatgatac agctt 15
<210> 32
   <211> 19
   <212> DNA
   <213> synthetic primer
<400> 32
   aaccttggaa taggacttc 19
<210> 33
   <211> 20
   <212> DNA
   <213> synthetic primer
<400> 33
   tgtgaactaa tatgtgcaga 20
<210> 34
   <211> 601
   <212> PRT
   <213> synthetic protein
<400> 34
   Met Phe Pro Cys Asn Ala Tyr Ile Glu Tyr Gly Asp Lys Asn Met Asn
   1               5                   10                  15
   Ser Phe Ile Glu Asp Val Glu Gln Ile Tyr Asn Phe Ile Lys Lys Asn
               20                  25                  30
   Ile Asp Val Glu Glu Lys Met His Phe Ile Glu Thr Tyr Lys Gln Lys
           35                  40                  45
   Ser Asn Met Lys Lys Glu Ile Ser Phe Ser Glu Glu Tyr Tyr Lys Gln
       50                  55                  60
   Lys Ile Met Asn Gly Lys Asn Gly Val Val Tyr Thr Pro Pro Glu Met
   65 70 75 80
   Ala Ala Phe Met Val Lys Asn Leu Ile Asn Val Asn Asp Val Ile Gly
   85 90 95
   Asn Pro Phe Ile Lys Ile Ile Asp Pro Ser Cys Gly Ser Gly Asn Leu
   100 105 110
   Ile Cys Lys Cys Phe Leu Tyr Leu Asn Arg Ile Phe Ile Lys Asn Ile
   115 120 125
   Glu Val Ile Asn Ser Lys Asn Asn Leu Asn Leu Lys Leu Glu Asp Ile
   130 135 140
   Ser Tyr His Ile Val Arg Asn Asn Leu Phe Gly Phe Asp Ile Asp Glu
   145 150 155 160
   Thr Ala Ile Lys Val Leu Lys Ile Asp Leu Phe Leu Ile Ser Asn Gln
   165 170 175
   Phe Ser Glu Lys Asn Phe Gln Val Lys Asp Phe Leu Val Glu Asn Ile
   180 185 190
   Asp Arg Lys Tyr Asp Val Phe Ile Gly Asn Pro Pro Tyr Ile Gly His
   195 200 205
   Lys Ser Val Asp Ser Ser Tyr Ser Tyr Val Leu Arg Lys Ile Tyr Gly
   210 215 220
   Ser Ile Tyr Arg Asp Lys Gly Asp Ile Ser Tyr Cys Phe Phe Gln Lys
   225 230 235 240
   Ser Leu Lys Cys Leu Lys Glu Gly Gly Lys Leu Val Phe Val Thr Ser
   245 250 255
   Arg Tyr Phe Cys Glu Ser Cys Ser Gly Lys Glu Leu Arg Lys Phe Leu
   260 265 270
   Ile Glu Asn Thr Ser Ile Tyr Lys Ile Ile Asp Phe Tyr Gly Ile Arg
   275 280 285
   Pro Phe Lys Arg Val Gly Ile Asp Pro Met Ile Ile Phe Leu Val Arg
   290 295 300
   Thr Lys Asn Trp Asn Asn Asn Ile Glu Ile Ile Arg Pro Asn Lys Ile
   305 310 315 320
   Glu Lys Asn Glu Lys Asn Lys Phe Leu Asp Ser Leu Phe Leu Asp Lys
   325 330 335
   Ser Glu Lys Cys Lys Lys Phe Ser Ile Ser Gln Lys Ser Ile Asn Asn
   340 345 350
   Asp Gly Trp Val Phe Val Asp Glu Val Glu Lys Asn Ile Ile Asp Lys
   355 360 365
   Ile Lys Glu Lys Ser Lys Phe Ile Leu Lys Asp Ile Cys His Ser Cys
   370 375 380
   Gln Gly Ile Ile Thr Gly Cys Asp Arg Ala Phe Ile Val Asp Arg Asp
   385 390 395 400
   Ile Ile Asn Ser Arg Lys Ile Glu Leu Arg Leu Ile Lys Pro Trp Ile
   405 410 415
   Lys Ser Ser His Ile Arg Lys Asn Glu Val Ile Lys Gly Glu Lys Phe
   420 425 430
   Ile Ile Tyr Ser Asn Leu Ile Glu Asn Glu Thr Glu Cys Pro Asn Ala
   435 440 445
   Ile Lys Tyr Ile Glu Gln Tyr Lys Lys Arg Leu Met Glu Arg Arg Glu
   450 455 460
   Cys Lys Lys Gly Thr Arg Lys Trp Tyr Glu Leu Gln Trp Gly Arg Lys
   465 470 475 480
   Pro Glu Ile Phe Glu Glu Lys Lys Ile Val Phe Pro Tyr Lys Ser Cys
   485 490 495
   Asp Asn Arg Phe Ala Leu Asp Lys Gly Ser Tyr Phe Ser Ala Asp Ile
   500 505 510
   Tyr Ser Leu Val Leu Lys Lys Asn Val Pro Phe Thr Tyr Glu Ile Leu
   515 520 525
   Leu Asn Ile Leu Asn Ser Pro Leu Tyr Glu Phe Tyr Phe Lys Thr Phe
   530 535 540
   Ala Lys Lys Leu Gly Glu Asn Leu Tyr Glu Tyr Tyr Pro Asn Asn Leu
   545 550 555 560
   Met Lys Leu Cys Ile Pro Ser Ile Asp Phe Gly Gly Glu Asn Asn Ile
   565 570 575
   Glu Lys Lys Leu Tyr Asp Phe Phe Gly Leu Thr Asp Lys Glu Ile Glu
   580 585 590
   Ile Val Glu Lys Ile Lys Asp Asn Cys
   595 600
<210> 35
   <211> 1806
   <212> DNA
   <213> synthetic gene
<400> 35
   atgtttccgt gcaatgccta tatcgaatat ggtgataaaa atatgaacag ctttatcgaa 60
   gatgtggaac agatctacaa cttcattaaa aagaacattg atgtggaaga aaagatgcat 120
   ttcattgaaa cctataaaca gaaaagcaac atgaagaaag agattagctt tagcgaagaa 180
   tactataaac agaagattat gaacggcaaa aatggcgttg tgtacacccc gccggaaatg 240
   gcggccttta tggttaaaaa tctgatcaac gttaacgatg ttattggcaa tccgtttatt 300
   aaaatcattg acccgagctg cggtagcggc aatctgattt gcaaatgttt tctgtatctg 360
   aatcgcatct ttattaagaa cattgaggtg attaacagca aaaataacct gaatctgaaa 420
   ctggaagaca tcagctacca catcgttcgc aacaatctgt ttggcttcga tattgacgaa 480
   accgcgatca aagtgctgaa aattgatctg tttctgatca gcaaccaatt tagcgagaaa 540
   aatttccagg ttaaagactt tctggtggaa aatattgatc gcaaatatga cgtgttcatt 600
   ggtaatccgc cgtatatcgg tcacaaaagc gtggacagca gctacagcta cgtgctgcgc 660
   aaaatctacg gcagcatcta ccgcgacaaa ggcgatatca gctattgttt ctttcagaag 720
   agcctgaaat gtctgaagga aggtggcaaa ctggtgtttg tgaccagccg ctacttctgc 780
   gagagctgca gcggtaaaga actgcgtaaa ttcctgatcg aaaacacgag catttacaag 840
   atcattgatt tttacggcat ccgcccgttc aaacgcgtgg gtatcgatcc gatgattatt 900
   tttctggttc gtacgaagaa ctggaacaat aacattgaaa ttattcgccc gaacaagatt 960
   gaaaagaacg aaaagaacaa attcctggat agcctgttcc tggacaaaag cgaaaagtgt 1020
   aaaaagttta gcattagcca gaaaagcatt aataacgatg gctgggtttt cgtggacgaa 1080
   gtggagaaaa acattatcga caaaatcaaa gagaaaagca agttcattct gaaagatatt 1140
   tgccatagct gtcaaggcat tatcaccggt tgtgatcgcg cctttattgt ggaccgtgat 1200
   atcatcaata gccgtaagat cgaactgcgt ctgattaaac cgtggattaa aagcagccat 1260
   atccgtaaga atgaagttat taagggcgaa aaattcatca tctatagcaa cctgattgag 1320
   aatgaaaccg agtgtccgaa tgcgattaaa tatatcgaac agtacaagaa acgtctgatg 1380
   gagcgccgcg aatgcaaaaa gggcacgcgt aagtggtatg aactgcaatg gggccgtaaa 1440
   ccggaaatct tcgaagaaaa gaaaattgtt ttcccgtata aaagctgtga caatcgtttt 1500
   gcactggata agggtagcta ttttagcgca gacatttata gcctggttct gaagaaaaat 1560
   gtgccgttca cctatgagat cctgctgaat atcctgaata gcccgctgta cgagttttac 1620
   tttaagacct tcgcgaaaaa gctgggcgag aatctgtacg agtactatcc gaacaacctg 1680
   atgaagctgt gcatcccgag catcgatttc ggcggtgaga acaatattga gaaaaagctg 1740
   tatgatttct ttggtctgac ggataaagaa attgagattg tggagaagat caaagataac 1800
   tgctaa 1806
<210> 36
   <211> 37
   <212> DNA
   <213> synthetic primer
<400> 36
   ccgaattcgt cgacaacaga gtttgatcct ggctcag 37
<210> 37
   <211> 37
   <212> DNA
   <213> synthetic primer
<400> 37
   cccgggatcc aagcttacgg ctaccttgtt acgactt 37
<210> 38
   <211> 351
   <212> PRT
   <213> Clostridium beijerinckii
<400> 38
   Met Lys Gly Phe Ala Met Leu Gly Ile Asn Lys Leu Gly Trp Ile Glu
   1               5                   10                  15
   Lys Glu Arg Pro Val Ala Gly Ser Tyr Asp Ala Ile Val Arg Pro Leu
               20                  25                  30
   Ala Val Ser Pro Cys Thr Ser Asp Ile His Thr Val Phe Glu Gly Ala
           35                  40                  45
   Leu Gly Asp Arg Lys Asn Met Ile Leu Gly His Glu Ala Val Gly Glu
       50                  55                  60
   Val Val Glu Val Gly Ser Glu Val Lys Asp Phe Lys Pro Gly Asp Arg
   65                  70                  75                  80
   Val Ile Val Pro Cys Thr Thr Pro Asp Trp Arg Ser Leu Glu Val Gln
                   85                  90                  95
   Ala Gly Phe Gln Gln His Ser Asn Gly Met Leu Ala Gly Trp Lys Phe
               100                 105                 110
   Ser Asn Phe Lys Asp Gly Val Phe Gly Glu Tyr Phe His Val Asn Asp
           115                 120                 125
   Ala Asp Met Asn Leu Ala Ile Leu Pro Lys Asp Met Pro Leu Glu Asn
       130                 135                 140
   Ala Val Met Ile Thr Asp Met Met Thr Thr Gly Phe His Gly Ala Glu
   145                 150                 155                 160
   Leu Ala Asp Ile Gln Met Gly Ser Ser Val Val Val Ile Gly Ile Gly
                   165                 170                 175
   Ala Val Gly Leu Met Gly Ile Ala Gly Ala Lys Leu Arg Gly Ala Gly
   180 185 190
   Arg Ile Ile Gly Val Gly Ser Arg Pro Ile Cys Val Glu Ala Ala Lys
   195 200 205
   Phe Tyr Gly Ala Thr Asp Ile Leu Asn Tyr Lys Asn Gly His Ile Val
   210 215 220
   Asp Gln Val Met Lys Leu Thr Asn Gly Lys Gly Val Asp Arg Val Ile
   225 230 235 240
   Met Ala Gly Gly Gly Ser Glu Thr Leu Ser Gln Ala Val Ser Met Val
   245 250 255
   Lys Pro Gly Gly Ile Ile Ser Asn Ile Asn Tyr His Gly Ser Gly Asp
   260 265 270
   Ala Leu Leu Ile Pro Arg Val Glu Trp Gly Cys Gly Met Ala His Lys
   275 280 285
   Thr Ile Lys Gly Gly Leu Cys Pro Gly Gly Arg Leu Arg Ala Glu Met
   290 295 300
   Leu Arg Asp Met Val Val Tyr Asn Arg Val Asp Leu Ser Lys Leu Val
   305 310 315 320
   Thr His Val Tyr His Gly Phe Asp His Ile Glu Glu Ala Leu Leu Leu
   325 330 335
   Met Lys Asp Lys Pro Lys Asp Leu Ile Lys Ala Val Val Ile Leu
   340 345 350
<210> 39
   <211> 1056
   <212> DNA
   <213> Clostridium beijerinckii
<400> 39
   atgaaaggtt ttgcaatgct aggtattaat aagttaggat ggatcgaaaa agaaaggcca 60
   gttgcgggtt catatgatgc tattgtacgc ccattagcag tatctccgtg tacatcagat 120
   atacatactg tttttgaggg agctcttgga gataggaaga atatgatttt agggcatgaa 180
   gctgtaggtg aagttgttga agtaggaagt gaagtgaagg attttaaacc tggtgacaga 240
   gttatagttc cttgtacaac tccagattgg agatctttgg aagttcaagc tggttttcaa 300
   cagcactcaa acggtatgct cgcaggatgg aaattttcaa atttcaagga tggagttttt 360
   ggtgaatatt ttcatgtaaa tgatgcggat atgaatcttg cgattctacc taaagacatg 420
   ccattagaaa atgctgttat gataacagat atgatgacta ctggatttca tggagcagaa 480
   cttgcagata ttcaaatggg ttcaagtgtt gtggtaattg gcattggagc tgttggctta 540
   atgggaatag caggtgctaa attacgtgga gcaggtagaa taattggagt ggggagcagg 600
   ccgatttgtg ttgaggctgc aaaattttat ggagcaacag atattctaaa ttataaaaat 660
   ggtcatatag ttgatcaagt tatgaaatta acgaatggaa aaggcgttga ccgcgtaatt 720
   atggcaggcg gtggttctga aacattatcc caagcagtat ctatggttaa accaggagga 780
   ataatttcta atataaatta tcatggaagt ggagatgctt tactaatacc acgtgtagaa 840
   tggggatgtg gaatggctca caagactata aaaggaggtc tttgtcctgg gggacgtttg 900
   agagcagaaa tgttaagaga tatggtagta tataatcgtg ttgatctaag taaattagtt 960
   acacatgtat atcatggatt tgatcacata gaagaagcac tgttattaat gaaagacaag 1020
   ccaaaagact taattaaagc agtagttata ttataa 1056
<210> 40
   <211> 352
   <212> PRT
   <213> Thermoanaerobacter brockii
<400> 40
   Met Lys Gly Phe Ala Met Leu Ser Ile Gly Lys Val Gly Trp Ile Glu
   1               5                   10                  15
   Lys Glu Lys Pro Ala Pro Gly Pro Phe Asp Ala Ile Val Arg Pro Leu
               20                  25                  30
   Ala Val Ala Pro Cys Thr Ser Asp Ile His Thr Val Phe Glu Gly Ala
           35                  40                  45
   Ile Gly Glu Arg His Asn Met Ile Leu Gly His Glu Ala Val Gly Glu
       50                  55                  60
   Val Val Glu Val Gly Ser Glu Val Lys Asp Phe Lys Pro Gly Asp Arg
   65                  70                  75                  80
   Val Val Val Pro Ala Ile Thr Pro Asp Trp Arg Thr Ser Glu Val Gln
                   85                  90                  95
   Arg Gly Tyr His Gln His Ser Gly Gly Met Leu Ala Gly Trp Lys Phe
               100                 105                 110
   Ser Asn Val Lys Asp Gly Val Phe Gly Glu Phe Phe His Val Asn Asp
           115                 120                 125
   Ala Asp Met Asn Leu Ala His Leu Pro Lys Glu Ile Pro Leu Glu Ala
       130                 135                 140
   Ala Val Met Ile Pro Asp Met Met Thr Thr Gly Phe His Gly Ala Glu
   145                 150                 155                 160
   Leu Ala Asp Ile Glu Leu Gly Ala Thr Val Ala Val Leu Gly Ile Gly
   165 170 175
   Pro Val Gly Leu Met Ala Val Ala Gly Ala Lys Leu Arg Gly Ala Gly
   180 185 190
   Arg Ile Ile Ala Val Gly Ser Arg Pro Val Cys Val Asp Ala Ala Lys
   195 200 205
   Tyr Tyr Gly Ala Thr Asp Ile Val Asn Tyr Lys Asp Gly Pro Ile Glu
   210 215 220
   Ser Gln Ile Met Asn Leu Thr Glu Gly Lys Gly Val Asp Ala Ala Ile
   225 230 235 240
   Ile Ala Gly Gly Asn Ala Asp Ile Met Ala Thr Ala Val Lys Ile Val
   245 250 255
   Lys Pro Gly Gly Thr Ile Ala Asn Val Asn Tyr Phe Gly Glu Gly Glu
   260 265 270
   Val Leu Pro Val Pro Arg Leu Glu Trp Gly Cys Gly Met Ala His Lys
   275 280 285
   Thr Ile Lys Gly Gly Leu Cys Pro Gly Gly Arg Leu Arg Met Glu Arg
   290 295 300
   Leu Ile Asp Leu Val Phe Tyr Lys Arg Val Asp Pro Ser Lys Leu Val
   305 310 315 320
   Thr His Val Phe Arg Gly Phe Asp Asn Ile Glu Lys Ala Phe Met Leu
   325 330 335
   Met Lys Asp Lys Pro Lys Asp Leu Ile Lys Pro Val Val Ile Leu Ala
   340 345 350
<210> 41
   <211> 1056
   <212> DNA
   <213> Thermoanaerobacter brockii
<400> 41
   atgaaaggtt ttgcaatgct aggtattaat aagttaggat ggatcgaaaa agaaaggcca 60
   gttgcgggtt catatgatgc tattgtacgc ccattagcag tatctccgtg tacatcagat 120
   atacatactg tttttgaggg agctcttgga gataggaaga atatgatttt agggcatgaa 180
   gctgtaggtg aagttgttga agtaggaagt gaagtgaagg attttaaacc tggtgacaga 240
   gttatagttc cttgtacaac tccagattgg agatctttgg aagttcaagc tggttttcaa 300
   cagcactcaa acggtatgct cgcaggatgg aaattttcaa atttcaagga tggagttttt 360
   ggtgaatatt ttcatgtaaa tgatgcggat atgaatcttg cgattctacc taaagacatg 420
   ccattagaaa atgctgttat gataacagat atgatgacta ctggatttca tggagcagaa 480
   cttgcagata ttcaaatggg ttcaagtgtt gtggtaattg gcattggagc tgttggctta 540
   atgggaatag caggtgctaa attacgtgga gcaggtagaa taattggagt ggggagcagg 600
   ccgatttgtg ttgaggctgc aaaattttat ggagcaacag atattctaaa ttataaaaat 660
   ggtcatatag ttgatcaagt tatgaaatta acgaatggaa aaggcgttga ccgcgtaatt 720
   atggcaggcg gtggttctga aacattatcc caagcagtat ctatggttaa accaggagga 780
   ataatttcta atataaatta tcatggaagt ggagatgctt tactaatacc acgtgtagaa 840
   tggggatgtg gaatggctca caagactata aaaggaggtc tttgtcctgg gggacgtttg 900
   agagcagaaa tgttaagaga tatggtagta tataatcgtg ttgatctaag taaattagtt 960
   acacatgtat atcatggatt tgatcacata gaagaagcac tgttattaat gaaagacaag 1020
   ccaaaagact taattaaagc agtagttata ttataa 1056
<210> 42
   <211> 392
   <212> PRT
   <213> Clostiridum acetobutylicum
<400> 42
   Met Lys Glu Val Val Ile Ala Ser Ala Val Arg Thr Ala Ile Gly Ser
   1 5 10 15
   Tyr Gly Lys Ser Leu Lys Asp Val Pro Ala Val Asp Leu Gly Ala Thr
   20 25 30
   Ala Ile Lys Glu Ala Val Lys Lys Ala Gly Ile Lys Pro Glu Asp Val
   35 40 45
   Asn Glu Val Ile Leu Gly Asn Val Leu Gln Ala Gly Leu Gly Gln Asn
   50 55 60
   Pro Ala Arg Gln Ala Ser Phe Lys Ala Gly Leu Pro Val Glu Ile Pro
   65 70 75 80
   Ala Met Thr Ile Asn Lys Val Cys Gly Ser Gly Leu Arg Thr Val Ser
   85 90 95
   Leu Ala Ala Gln Ile Ile Lys Ala Gly Asp Ala Asp Val Ile Ile Ala
   100 105 110
   Gly Gly Met Glu Asn Met Ser Arg Ala Pro Tyr Leu Ala Asn Asn Ala
   115 120 125
   Arg Trp Gly Tyr Arg Met Gly Asn Ala Lys Phe Val Asp Glu Met Ile
   130 135 140
   Thr Asp Gly Leu Trp Asp Ala Phe Asn Asp Tyr His Met Gly Ile Thr
   145 150 155 160
   Ala Glu Asn Ile Ala Glu Arg Trp Asn Ile Ser Arg Glu Glu Gln Asp
   165 170 175
   Glu Phe Ala Leu Ala Ser Gln Lys Lys Ala Glu Glu Ala Ile Lys Ser
   180 185 190
   Gly Gln Phe Lys Asp Glu Ile Val Pro Val Val Ile Lys Gly Arg Lys
   195 200 205
   Gly Glu Thr Val Val Asp Thr Asp Glu His Pro Arg Phe Gly Ser Thr
   210 215 220
   Ile Glu Gly Leu Ala Lys Leu Lys Pro Ala Phe Lys Lys Asp Gly Thr
   225 230 235 240
   Val Thr Ala Gly Asn Ala Ser Gly Leu Asn Asp Cys Ala Ala Val Leu
   245 250 255
   Val Ile Met Ser Ala Glu Lys Ala Lys Glu Leu Gly Val Lys Pro Leu
   260 265 270
   Ala Lys Ile Val Ser Tyr Gly Ser Ala Gly Val Asp Pro Ala Ile Met
   275 280 285
   Gly Tyr Gly Pro Phe Tyr Ala Thr Lys Ala Ala Ile Glu Lys Ala Gly
   290 295 300
   Trp Thr Val Asp Glu Leu Asp Leu Ile Glu Ser Asn Glu Ala Phe Ala
   305 310 315 320
   Ala Gln Ser Leu Ala Val Ala Lys Asp Leu Lys Phe Asp Met Asn Lys
   325 330 335
   Val Asn Val Asn Gly Gly Ala Ile Ala Leu Gly His Pro Ile Gly Ala
   340 345 350
   Ser Gly Ala Arg Ile Leu Val Thr Leu Val His Ala Met Gln Lys Arg
   355 360 365
   Asp Ala Lys Lys Gly Leu Ala Thr Leu Cys Ile Gly Gly Gly Gln Gly
   370 375 380
   Thr Ala Ile Leu Leu Glu Lys Cys
   385 390
<210> 43
   <211> 217
   <212> PRT
   <213> Clostridium beijerinckii
<400> 43
   Met Asn Lys Leu Val Lys Leu Thr Asp Leu Lys Arg Ile Phe Lys Asp
   1               5                   10                  15
   Gly Met Thr Ile Met Val Gly Gly Phe Leu Asp Cys Gly Thr Pro Glu
               20                  25                  30
   Asn Ile Ile Asp Met Leu Val Asp Leu Asn Ile Lys Asn Leu Thr Ile
           35                  40                  45
   Ile Ser Asn Asp Thr Ala Phe Pro Asn Lys Gly Ile Gly Lys Leu Ile
       50                  55                  60
   Val Asn Gly Gln Val Ser Lys Val Ile Ala Ser His Ile Gly Thr Asn
   65                  70                  75                  80
   Pro Glu Thr Gly Lys Lys Met Ser Ser Gly Glu Leu Lys Val Glu Leu
                   85                  90                  95
   Ser Pro Gln Gly Thr Leu Ile Glu Arg Ile Arg Ala Ala Gly Ser Gly
               100                 105                 110
   Leu Gly Gly Val Leu Thr Pro Thr Gly Leu Gly Thr Ile Val Glu Glu
           115                 120                 125
   Gly Lys Lys Lys Val Thr Ile Asp Gly Lys Glu Tyr Leu Leu Glu Leu
       130                 135                 140
   Pro Leu Ser Ala Asp Val Ser Leu Ile Lys Gly Ser Ile Val Asp Glu
   145                 150                 155                 160
   Phe Gly Asn Thr Phe Tyr Arg Ala Ala Thr Lys Asn Phe Asn Pro Tyr
                   165                 170                 175
   Met Ala Met Ala Ala Lys Thr Val Ile Val Glu Ala Glu Asn Leu Val
               180                 185                 190
   Lys Cys Glu Asp Leu Lys Arg Asp Ala Ile Met Thr Pro Gly Val Leu
           195                 200                 205
   Val Asp Tyr Ile Val Lys Glu Ala Ala
       210                 215
<210> 44
   <211> 221
   <212> PRT
   <213> clostridium beijerinckii
<400> 44
   Leu Ile Val Asp Lys Val Leu Ala Lys Glu Ile Ile Ala Lys Arg Val
   1               5                   10                  15
   Ala Lys Glu Leu Lys Lys Asp Gln Leu Val Asn Leu Gly Ile Gly Leu
               20                  25                  30
   Pro Thr Leu Val Ala Asn Tyr Val Pro Lys Glu Met Asn Ile Thr Phe
           35                  40                  45
   Glu Ser Glu Asn Gly Met Val Gly Met Ala Gln Met Ala Ser Ser Gly
       50                  55                  60
   Glu Asn Asp Pro Asp Ile Ile Asn Ala Gly Gly Glu Tyr Val Thr Leu
   65                  70                  75                  80
   Leu Pro Gln Gly Ser Phe Phe Asp Ser Ser Met Ser Phe Ala Leu Ile
                   85                  90                  95
   Arg Gly Gly His Val Asp Val Ala Val Leu Gly Ala Leu Glu Val Asp
               100                 105                 110
   Glu Lys Gly Asn Leu Ala Asn Trp Ile Val Pro Asn Lys Ile Val Pro
           115                 120                 125
   Gly Met Gly Gly Ala Met Asp Leu Ala Ile Gly Ala Lys Lys Ile Ile
       130                 135                 140
   Val Ala Met Gln His Thr Gly Lys Ser Lys Pro Lys Ile Val Lys Lys
   145                 150                 155                 160
   Cys Thr Leu Pro Leu Thr Ala Lys Ala Gln Val Asp Leu Ile Val Thr
                   165                 170                 175
   Glu Leu Cys Val Ile Asp Val Thr Asn Asp Gly Leu Leu Leu Lys Glu
               180                 185                 190
   Ile His Lys Asp Thr Thr Ile Asp Glu Ile Lys Phe Leu Thr Asp Ala
           195                 200                 205
   Asp Leu Ile Ile Pro Asp Asn Leu Lys Ile Met Asp Ile
       210                 215                 220
<210> 45
   <211> 246
   <212> PRT
   <213> clostridium beijerinckii
<400> 45
   Met Leu Glu Ser Glu Val Ser Lys Gln Ile Thr Thr Pro Leu Ala Ala
   1               5                   10                  15
   Pro Ala Phe Pro Arg Gly Pro Tyr Arg Phe His Asn Arg Glu Tyr Leu
               20                  25                  30
   Asn Ile Ile Tyr Arg Thr Asp Leu Asp Ala Leu Arg Lys Ile Val Pro
           35                  40                  45
   Glu Pro Leu Glu Leu Asp Arg Ala Tyr Val Arg Phe Glu Met Met Ala
       50                  55                  60
   Met Pro Asp Thr Thr Gly Leu Gly Ser Tyr Thr Glu Cys Gly Gln Ala
   65                  70                  75                  80
   Ile Pro Val Lys Tyr Asn Gly Val Lys Gly Asp Tyr Leu His Met Met
                   85                  90                  95
   Tyr Leu Asp Asn Glu Pro Ala Ile Ala Val Gly Arg Glu Ser Ser Ala
               100                 105                 110
   Tyr Pro Lys Lys Leu Gly Tyr Pro Lys Leu Phe Val Asp Ser Asp Thr
           115                 120                 125
   Leu Val Gly Thr Leu Lys Tyr Gly Thr Leu Pro Val Ala Thr Ala Thr
       130                 135                 140
   Met Gly Tyr Lys His Glu Pro Leu Asp Leu Lys Glu Ala Tyr Ala Gln
   145                 150                 155                 160
   Ile Ala Arg Pro Asn Phe Met Leu Lys Ile Ile Gln Gly Tyr Asp Gly
                   165                 170                 175
   Lys Pro Arg Ile Cys Glu Leu Ile Cys Ala Glu Asn Thr Asp Ile Thr
               180                 185                 190
   Ile His Gly Ala Trp Thr Gly Ser Ala Arg Leu Gln Leu Phe Ser His
           195                 200                 205
   Ala Leu Ala Pro Leu Ala Asp Leu Pro Val Leu Glu Ile Val Ser Ala
       210                 215                 220
   Ser His Ile Leu Thr Asp Leu Thr Leu Gly Thr Pro Lys Val Val His
   225                 230                 235                 240
   Asp Tyr Leu Ser Val Lys
                   245
<210> 46
   <211> 6832
   <212> DNA
   <213> synthetic plasmid
<400> 46
   tgaaagaaag atatggaaca gtctataaag gctctcagag gctcatagac gaagaaagtg       60
   gagaagtcat agaggtagac aagttatacc gtaaacaaac gtctggtaac ttcgtaaagg      120
   catatatagt gcaattaata agtatgttag atatgattgg cggaaaaaaa cttaaaatcg      180
   ttaactatat cctagataat gtccacttaa gtaacaatac aatgatagct acaacaagag      240
   aaatagcaaa agctacagga acaagtctac aaacagtaat aacaacactt aaaatcttag 300
   aagaaggaaa tattataaaa agaaaaactg gagtattaat gttaaaccct gaactactaa 360
   tgagaggcga cgaccaaaaa caaaaatacc tcttactcga atttgggaac tttgagcaag 420
   aggcaaatga aatagattga cctcccaata acaccacgta gttattggga ggtcaatcta 480
   tgaaatgcga ttaagggccg gccagtgggc aagttgaaaa attcacaaaa atgtggtata 540
   atatctttgt tcattagagc gataaacttg aatttgagag ggaacttaga tggtatttga 600
   aaaaattgat aaaaatagtt ggaacagaaa agagtatttt gaccactact ttgcaagtgt 660
   accttgtacc tacagcatga ccgttaaagt ggatatcaca caaataaagg aaaagggaat 720
   gaaactatat cctgcaatgc tttattatat tgcaatgatt gtaaaccgcc attcagagtt 780
   taggacggca atcaatcaag atggtgaatt ggggatatat gatgagatga taccaagcta 840
   tacaatattt cacaatgata ctgaaacatt ttccagcctt tggactgagt gtaagtctga 900
   ctttaaatca tttttagcag attatgaaag tgatacgcaa cggtatggaa acaatcatag 960
   aatggaagga aagccaaatg ctccggaaaa catttttaat gtatctatga taccgtggtc 1020
   aaccttcgat ggctttaatc tgaatttgca gaaaggatat gattatttga ttcctatttt 1080
   tactatgggg aaatattata aagaagataa caaaattata cttcctttgg caattcaagt 1140
   tcatcacgca gtatgtgacg gatttcacat ttgccgtttt gtaaacgaat tgcaggaatt 1200
   gataaatagt taacttcagg tttgtctgta actaaaaaca agtatttaag caaaaacatc 1260
   gtagaaatac ggtgtttttt gttaccctaa gtttaaactc ctttttgata atctcatgac 1320
   caaaatccct taacgtgagt tttcgttcca ctgagcgtca gaccccgtag aaaagatcaa 1380
   aggatcttct tgagatcctt tttttctgcg cgtaatctgc tgcttgcaaa caaaaaaacc 1440
   accgctacca gcggtggttt gtttgccgga tcaagagcta ccaactcttt ttccgaaggt 1500
   aactggcttc agcagagcgc agataccaaa tactgttctt ctagtgtagc cgtagttagg 1560
   ccaccacttc aagaactctg tagcaccgcc tacatacctc gctctgctaa tcctgttacc 1620
   agtggctgct gccagtggcg ataagtcgtg tcttaccggg ttggactcaa gacgatagtt 1680
   accggataag gcgcagcggt cgggctgaac ggggggttcg tgcacacagc ccagcttgga 1740
   gcgaacgacc tacaccgaac tgagatacct acagcgtgag ctatgagaaa gcgccacgct 1800
   tcccgaaggg agaaaggcgg acaggtatcc ggtaagcggc agggtcggaa caggagagcg 1860
   cacgagggag cttccagggg gaaacgcctg gtatctttat agtcctgtcg ggtttcgcca 1920
   cctctgactt gagcgtcgat ttttgtgatg ctcgtcaggg gggcggagcc tatggaaaaa 1980
   cgccagcaac gcggcctttt tacggttcct ggccttttgc tggccttttg ctcacatgtt 2040
   ctttcctgcg ttatcccctg attctgtgga taaccgtatt accgcctttg agtgagctga 2100
   taccgctcgc cgcagccgaa cgaccgagcg cagcgagtca gtgagcgagg aagcggaaga 2160
   gcgcccaata cgcagggccc cctgcaggat aaaaaaattg tagataaatt ttataaaata 2220
   gttttatcta caattttttt atcaggaaac agctatgacc gcggccgcag atagtcataa 2280
   tagttccaga atagttcaat ttagaaatta gactaaactt caaaatgttt gttaaatata 2340
   taccaaacta gtatagatat tttttaaata ctggacttaa acagtagtaa tttgcctaaa 2400
   aaattttttc aatttttttt aaaaaatcct tttcaagttg tacattgtta tggtaatatg 2460
   taattgaaga agttatgtag taatattgta aacgtttctt gattttttta catccatgta 2520
   gtgcttaaaa aaccaaaata tgtcacatgc aattgtatat ttcaaataac aatatttatt 2580
   ttctcgttaa attcacaaat aatttattaa taatatcaat aaccaagatt atacttaaat 2640
   ggatgtttat tttttaacac ttttatagta aatatattta ttttatgtag taaaaaggtt 2700
   ataattataa ttgtatttat tacaattaat taaaataaaa aatagggttt taggtaaaat 2760
   taagttattt taagaagtaa ttacaataaa aattgaagtt atttctttaa ggagggaatt 2820
   attcatatga aagaagttgt aatagctagt gcagtaagaa cagcgattgg atcttatgga 2880
   aagtctctta aggatgtacc agcagtagat ttaggagcta cagctataaa ggaagcagtt 2940
   aaaaaagcag gaataaaacc agaggatgtt aatgaagtca ttttaggaaa tgttcttcaa 3000
   gcaggtttag gacagaatcc agcaagacag gcatctttta aagcaggatt accagttgaa 3060
   attccagcta tgactattaa taaggtttgt ggttcaggac ttagaacagt tagcttagca 3120
   gcacaaatta taaaagcagg agatgctgac gtaataatag caggtggtat ggaaaatatg 3180
   tctagagctc cttacttagc gaataacgct agatggggat atagaatggg aaacgctaaa 3240
   tttgttgatg aaatgatcac tgacggattg tgggatgcat ttaatgatta ccacatggga 3300
   ataacagcag aaaacatagc tgagagatgg aacatttcaa gagaagaaca agatgagttt 3360
   gctcttgcat cacaaaaaaa agctgaagaa gctataaaat caggtcaatt taaagatgaa 3420
   atagttcctg tagtaattaa aggcagaaag ggagaaactg tagttgatac agatgagcac 3480
   cctagatttg gatcaactat agaaggactt gcaaaattaa aacctgcctt caaaaaagat 3540
   ggaacagtta cagctggtaa tgcatcagga ttaaatgact gtgcagcagt acttgtaatc 3600
   atgagtgcag aaaaagctaa agagcttgga gtaaaaccac ttgctaagat agtttcttat 3660
   ggttcagcag gagttgaccc agcaataatg ggatatggac ctttctatgc aacaaaagca 3720
   gctattgaaa aagcaggttg gacagttgat gaattagatt taatagaatc aaatgaagct 3780
   tttgcagctc aaagtttagc agtagcaaaa gatttaaaat ttgatatgaa taaagtaaat 3840
   gtaaatggag gagctattgc ccttggtcat ccaattggag catcaggtgc aagaatactc 3900
   gttactcttg tacacgcaat gcaaaaaaga gatgcaaaaa aaggcttagc aactttatgt 3960
   ataggtggcg gacaaggaac agcaatattg ctagaaaagt gctaggaatt cgagctcggt 4020
   accagggaga tattaaaatg aataaattag taaaattaac agatttaaag cgcattttca 4080
   aagatggcat gacaattatg gttgggggtt ttttagattg tggaactcct gaaaatatta 4140
   tagatatgct agttgattta aatataaaaa atctgactat tataagcaat gatacagctt 4200
   ttcctaataa aggaatagga aaacttattg taaatggtca agtttctaaa gtaattgctt 4260
   cacatattgg aactaatcct gaaactggaa aaaaaatgag ctctggagaa cttaaagttg 4320
   agctttcccc acaaggaaca ctgattgaaa gaattcgtgc agctggatct ggactcggag 4380
   gtgtattaac tccaactgga cttggaacta tcgttgaaga aggtaagaaa aaagttacta 4440
   tcgatggcaa agaatatcta ttagaacttc ctttatctgc tgatgtttca ttaataaaag 4500
   gtagcattgt agatgaattt ggaaatacct tctatagggc tgctactaaa aatttcaatc 4560
   catatatggc aatggctgca aaaacagtta tagttgaagc agaaaattta gttaaatgtg 4620
   aagatttaaa aagagatgcc ataatgactc ctggcgtatt agtagattat atcgttaagg 4680
   aggcggctta attgattgta gataaagttt tagcaaaaga gataattgcc aaaagagttg 4740
   caaaagaact aaaaaaagac caactcgtaa accttggaat aggacttcca actttagtag 4800
   caaattatgt accaaaagaa atgaacatta cttttgaatc agaaaatggc atggttggta 4860
   tggcacaaat ggcatcatca ggtgaaaatg acccagatat aataaatgct ggcggggaat 4920
   atgtaacatt attacctcaa ggttcatttt ttgatagttc aatgtctttc gcactaatac 4980
   gaggaggaca tgttgatgtt gctgttcttg gtgctctaga agttgatgaa aaaggtaatt 5040
   tagctaactg gattgttcca aataaaattg tcccaggtat gggtggcgct atggatttag 5100
   caataggcgc aaaaaaaata atagtggcaa tgcaacatac aggaaaaagt aaacctaaaa 5160
   tcgttaaaaa atgtactctc ccacttactg ctaaggctca agtggattta attgtcacag 5220
   aactttgtgt aattgatgta acaaatgacg gcttactttt aaaagaaatt cataaagata 5280
   caactattga tgaaattaaa tttttaacag atgcagattt aattattcca gataacttaa 5340
   agattatgga tatatgaatc attctatttt aaatatataa ctttaaaaat cttatgtatt 5400
   aaaaactaag aaaagaggtt gattgtttta tgttagaaag tgaagtatct aaacaaatta 5460
   caactccact tgctgctcca gcgtttccta gaggaccata taggtttcac aatagagaat 5520
   atctaaacat tatttatcga actgatttag atgctcttcg aaaaatagta ccagagccac 5580
   ttgaattaga tagagcatat gttagatttg aaatgatggc tatgcctgat acaaccggac 5640
   taggctcata tacagaatgt ggtcaagcta ttccagtaaa atataatggt gttaagggtg 5700
   actacttgca tatgatgtat ctagataatg aacctgctat tgctgttgga agagaaagta 5760
   gcgcttatcc aaaaaagctt ggctatccaa agctatttgt tgattcagat actttagttg 5820
   ggacacttaa atatggtaca ttaccagtag ctactgcaac aatgggatat aagcacgagc 5880
   ctctagatct taaagaagcc tatgctcaaa ttgcaagacc caattttatg ctaaaaatca 5940
   ttcaaggtta cgatggtaag ccaagaattt gtgaactaat atgtgcagaa aatactgata 6000
   taactattca cggtgcttgg actggaagtg cacgtctaca attatttagc catgcactag 6060
   ctcctcttgc tgatttacct gtattagaga ttgtatcagc atctcatatc ctcacagatt 6120
   taactcttgg aacacctaag gttgtacatg attatctttc agtaaaataa aagcaatata 6180
   gaggatcctc tagagtcgac gtcacgcgtc catggagatc tcgaggcctg cagacatgca 6240
   agcttggcac tggccgtcgt tttacaacgt cgtgactggg aaaaccctgg cgttacccaa 6300
   cttaatcgcc ttgcagcaca tccccctttc gccagctggc gtaatagcga agaggcccgc 6360
   accgatcgcc cttcccaaca gttgcgcagc ctgaatggcg aatggcgcta gcataaaaat 6420
   aagaagcctg catttgcagg cttcttattt ttatggcgcg ccgcattcac ttcttttcta 6480
   tataaatatg agcgaagcga ataagcgtcg gaaaagcagc aaaaagtttc ctttttgctg 6540
   ttggagcatg ggggttcagg gggtgcagta tctgacgtca atgccgagcg aaagcgagcc 6600
   gaagggtagc atttacgtta gataaccccc tgatatgctc cgacgcttta tatagaaaag 6660
   aagattcaac taggtaaaat cttaatatag gttgagatga taaggtttat aaggaatttg 6720
   tttgttctaa tttttcactc attttgttct aatttctttt aacaaatgtt cttttttttt 6780
   tagaacagtt atgatatagt tagaatagtt taaaataagg agtgagaaaa ag 6832
<210> 47
   <211> 2133
   <212> DNA
   <213> clostridium beijerinckii
<400> 47
   atgaataaat tagtaaaatt aacagattta aagcgcattt tcaaagatgg catgacaatt      60
   atggttgggg gttttttaga ttgtggaact cctgaaaata ttatagatat gctagttgat      120
   ttaaatataa aaaatctgac tattataagc aatgatacag cttttcctaa taaaggaata      180
   ggaaaactta ttgtaaatgg tcaagtttct aaagtaattg cttcacatat tggaactaat      240
   cctgaaactg gaaaaaaaat gagctctgga gaacttaaag ttgagctttc cccacaagga      300
   acactgattg aaagaattcg tgcagctgga tctggactcg gaggtgtatt aactccaact      360
   ggacttggaa ctatcgttga agaaggtaag aaaaaagtta ctatcgatgg caaagaatat      420
   ctattagaac ttcctttatc tgctgatgtt tcattaataa aaggtagcat tgtagatgaa      480
   tttggaaata ccttctatag ggctgctact aaaaatttca atccatatat ggcaatggct      540
   gcaaaaacag ttatagttga agcagaaaat ttagttaaat gtgaagattt aaaaagagat      600
   gccataatga ctcctggcgt attagtagat tatatcgtta aggaggcggc ttaattgatt      660
   gtagataaag ttttagcaaa agagataatt gccaaaagag ttgcaaaaga actaaaaaaa      720
   gaccaactcg taaaccttgg aataggactt ccaactttag tagcaaatta tgtaccaaaa      780
   gaaatgaaca ttacttttga atcagaaaat ggcatggttg gtatggcaca aatggcatca      840
   tcaggtgaaa atgacccaga tataataaat gctggcgggg aatatgtaac attattacct      900
   caaggttcat tttttgatag ttcaatgtct ttcgcactaa tacgaggagg acatgttgat      960
   gttgctgttc ttggtgctct agaagttgat gaaaaaggta atttagctaa ctggattgtt     1020
   ccaaataaaa ttgtcccagg tatgggtggc gctatggatt tagcaatagg cgcaaaaaaa     1080
   ataatagtgg caatgcaaca tacaggaaaa agtaaaccta aaatcgttaa aaaatgtact     1140
   ctcccactta ctgctaaggc tcaagtggat ttaattgtca cagaactttg tgtaattgat     1200
   gtaacaaatg acggcttact tttaaaagaa attcataaag atacaactat tgatgaaatt     1260
   aaatttttaa cagatgcaga tttaattatt ccagataact taaagattat ggatatatga     1320
   atcattctat tttaaatata taactttaaa aatcttatgt attaaaaact aagaaaagag     1380
   gttgattgtt ttatgttaga aagtgaagta tctaaacaaa ttacaactcc acttgctgct 1440
   ccagcgtttc ctagaggacc atataggttt cacaatagag aatatctaaa cattatttat 1500
   cgaactgatt tagatgctct tcgaaaaata gtaccagagc cacttgaatt agatagagca 1560
   tatgttagat ttgaaatgat ggctatgcct gatacaaccg gactaggctc atatacagaa 1620
   tgtggtcaag ctattccagt aaaatataat ggtgttaagg gtgactactt gcatatgatg 1680
   tatctagata atgaacctgc tattgctgtt ggaagagaaa gtagcgctta tccaaaaaag 1740
   cttggctatc caaagctatt tgttgattca gatactttag ttgggacact taaatatggt 1800
   acattaccag tagctactgc aacaatggga tataagcacg agcctctaga tcttaaagaa 1860
   gcctatgctc aaattgcaag acccaatttt atgctaaaaa tcattcaagg ttacgatggt 1920
   aagccaagaa tttgtgaact aatatgtgca gaaaatactg atataactat tcacggtgct 1980
   tggactggaa gtgcacgtct acaattattt agccatgcac tagctcctct tgctgattta 2040
   cctgtattag agattgtatc agcatctcat atcctcacag atttaactct tggaacacct 2100
   aaggttgtac atgattatct ttcagtaaaa taa 2133
<210> 48
   <211> 7922
   <212> DNA
   <213> synthetic plasmid
<400> 48
   atgaaagaaa gatatggaac agtctataaa ggctctcaga ggctcataga cgaagaaagt     60
   ggagaagtca tagaggtaga caagttatac cgtaaacaaa cgtctggtaa cttcgtaaag    120
   gcatatatag tgcaattaat aagtatgtta gatatgattg gcggaaaaaa acttaaaatc    180
   gttaactata tcctagataa tgtccactta agtaacaata caatgatagc tacaacaaga    240
   gaaatagcaa aagctacagg aacaagtcta caaacagtaa taacaacact taaaatctta    300
   gaagaaggaa atattataaa aagaaaaact ggagtattaa tgttaaaccc tgaactacta    360
   atgagaggcg acgaccaaaa acaaaaatac ctcttactcg aatttgggaa ctttgagcaa    420
   gaggcaaatg aaatagattg acctcccaat aacaccacgt agttattggg aggtcaatct    480
   atgaaatgcg attaagggcc ggccagtggg caagttgaaa aattcacaaa aatgtggtat    540
   aatatctttg ttcattagag cgataaactt gaatttgaga gggaacttag atggtatttg    600
   aaaaaattga taaaaatagt tggaacagaa aagagtattt tgaccactac tttgcaagtg    660
   taccttgtac ctacagcatg accgttaaag tggatatcac acaaataaag gaaaagggaa    720
   tgaaactata tcctgcaatg ctttattata ttgcaatgat tgtaaaccgc cattcagagt    780
   ttaggacggc aatcaatcaa gatggtgaat tggggatata tgatgagatg ataccaagct    840
   atacaatatt tcacaatgat actgaaacat tttccagcct ttggactgag tgtaagtctg    900
   actttaaatc atttttagca gattatgaaa gtgatacgca acggtatgga aacaatcata    960
   gaatggaagg aaagccaaat gctccggaaa acatttttaa tgtatctatg ataccgtggt   1020
   caaccttcga tggctttaat ctgaatttgc agaaaggata tgattatttg attcctattt 1080
   ttactatggg gaaatattat aaagaagata acaaaattat acttcctttg gcaattcaag 1140
   ttcatcacgc agtatgtgac ggatttcaca tttgccgttt tgtaaacgaa ttgcaggaat 1200
   tgataaatag ttaacttcag gtttgtctgt aactaaaaac aagtatttaa gcaaaaacat 1260
   cgtagaaata cggtgttttt tgttacccta agtttaaact cctttttgat aatctcatga 1320
   ccaaaatccc ttaacgtgag ttttcgttcc actgagcgtc agaccccgta gaaaagatca 1380
   aaggatcttc ttgagatcct ttttttctgc gcgtaatctg ctgcttgcaa acaaaaaaac 1440
   caccgctacc agcggtggtt tgtttgccgg atcaagagct accaactctt tttccgaagg 1500
   taactggctt cagcagagcg cagataccaa atactgttct tctagtgtag ccgtagttag 1560
   gccaccactt caagaactct gtagcaccgc ctacatacct cgctctgcta atcctgttac 1620
   cagtggctgc tgccagtggc gataagtcgt gtcttaccgg gttggactca agacgatagt 1680
   taccggataa ggcgcagcgg tcgggctgaa cggggggttc gtgcacacag cccagcttgg 1740
   agcgaacgac ctacaccgaa ctgagatacc tacagcgtga gctatgagaa agcgccacgc 1800
   ttcccgaagg gagaaaggcg gacaggtatc cggtaagcgg cagggtcgga acaggagagc 1860
   gcacgaggga gcttccaggg ggaaacgcct ggtatcttta tagtcctgtc gggtttcgcc 1920
   acctctgact tgagcgtcga tttttgtgat gctcgtcagg ggggcggagc ctatggaaaa 1980
   acgccagcaa cgcggccttt ttacggttcc tggccttttg ctggcctttt gctcacatgt 2040
   tctttcctgc gttatcccct gattctgtgg ataaccgtat taccgccttt gagtgagctg 2100
   ataccgctcg ccgcagccga acgaccgagc gcagcgagtc agtgagcgag gaagcggaag 2160
   agcgcccaat acgcagggcc ccctgcagga taaaaaaatt gtagataaat tttataaaat 2220
   agttttatct acaatttttt tatcaggaaa cagctatgac cgcggccgca gatagtcata 2280
   atagttccag aatagttcaa tttagaaatt agactaaact tcaaaatgtt tgttaaatat 2340
   ataccaaact agtatagata ttttttaaat actggactta aacagtagta atttgcctaa 2400
   aaaatttttt caattttttt taaaaaatcc ttttcaagtt gtacattgtt atggtaatat 2460
   gtaattgaag aagttatgta gtaatattgt aaacgtttct tgattttttt acatccatgt 2520
   agtgcttaaa aaaccaaaat atgtcacatg caattgtata tttcaaataa caatatttat 2580
   tttctcgtta aattcacaaa taatttatta ataatatcaa taaccaagat tatacttaaa 2640
   tggatgttta ttttttaaca cttttatagt aaatatattt attttatgta gtaaaaaggt 2700
   tataattata attgtattta ttacaattaa ttaaaataaa aaatagggtt ttaggtaaaa 2760
   ttaagttatt ttaagaagta attacaataa aaattgaagt tatttcttta aggagggaat 2820
   tattcatatg aaagaagttg taatagctag tgcagtaaga acagcgattg gatcttatgg 2880
   aaagtctctt aaggatgtac cagcagtaga tttaggagct acagctataa aggaagcagt 2940
   taaaaaagca ggaataaaac cagaggatgt taatgaagtc attttaggaa atgttcttca 3000
   agcaggttta ggacagaatc cagcaagaca ggcatctttt aaagcaggat taccagttga 3060
   aattccagct atgactatta ataaggtttg tggttcagga cttagaacag ttagcttagc 3120
   agcacaaatt ataaaagcag gagatgctga cgtaataata gcaggtggta tggaaaatat 3180
   gtctagagct ccttacttag cgaataacgc tagatgggga tatagaatgg gaaacgctaa 3240
   atttgttgat gaaatgatca ctgacggatt gtgggatgca tttaatgatt accacatggg 3300
   aataacagca gaaaacatag ctgagagatg gaacatttca agagaagaac aagatgagtt 3360
   tgctcttgca tcacaaaaaa aagctgaaga agctataaaa tcaggtcaat ttaaagatga 3420
   aatagttcct gtagtaatta aaggcagaaa gggagaaact gtagttgata cagatgagca 3480
   ccctagattt ggatcaacta tagaaggact tgcaaaatta aaacctgcct tcaaaaaaga 3540
   tggaacagtt acagctggta atgcatcagg attaaatgac tgtgcagcag tacttgtaat 3600
   catgagtgca gaaaaagcta aagagcttgg agtaaaacca cttgctaaga tagtttctta 3660
   tggttcagca ggagttgacc cagcaataat gggatatgga cctttctatg caacaaaagc 3720
   agctattgaa aaagcaggtt ggacagttga tgaattagat ttaatagaat caaatgaagc 3780
   ttttgcagct caaagtttag cagtagcaaa agatttaaaa tttgatatga ataaagtaaa 3840
   tgtaaatgga ggagctattg cccttggtca tccaattgga gcatcaggtg caagaatact 3900
   cgttactctt gtacacgcaa tgcaaaaaag agatgcaaaa aaaggcttag caactttatg 3960
   tataggtggc ggacaaggaa cagcaatatt gctagaaaag tgctaggaat tcgagctcgg 4020
   taccagggag atattaaaat gaataaatta gtaaaattaa cagatttaaa gcgcattttc 4080
   aaagatggca tgacaattat ggttgggggt tttttagatt gtggaactcc tgaaaatatt 4140
   atagatatgc tagttgattt aaatataaaa aatctgacta ttataagcaa tgatacagct 4200
   tttcctaata aaggaatagg aaaacttatt gtaaatggtc aagtttctaa agtaattgct 4260
   tcacatattg gaactaatcc tgaaactgga aaaaaaatga gctctggaga acttaaagtt 4320
   gagctttccc cacaaggaac actgattgaa agaattcgtg cagctggatc tggactcgga 4380
   ggtgtattaa ctccaactgg acttggaact atcgttgaag aaggtaagaa aaaagttact 4440
   atcgatggca aagaatatct attagaactt cctttatctg ctgatgtttc attaataaaa 4500
   ggtagcattg tagatgaatt tggaaatacc ttctataggg ctgctactaa aaatttcaat 4560
   ccatatatgg caatggctgc aaaaacagtt atagttgaag cagaaaattt agttaaatgt 4620
   gaagatttaa aaagagatgc cataatgact cctggcgtat tagtagatta tatcgttaag 4680
   gaggcggctt aattgattgt agataaagtt ttagcaaaag agataattgc caaaagagtt 4740
   gcaaaagaac taaaaaaaga ccaactcgta aaccttggaa taggacttcc aactttagta 4800
   gcaaattatg taccaaaaga aatgaacatt acttttgaat cagaaaatgg catggttggt 4860
   atggcacaaa tggcatcatc aggtgaaaat gacccagata taataaatgc tggcggggaa 4920
   tatgtaacat tattacctca aggttcattt tttgatagtt caatgtcttt cgcactaata 4980
   cgaggaggac atgttgatgt tgctgttctt ggtgctctag aagttgatga aaaaggtaat 5040
   ttagctaact ggattgttcc aaataaaatt gtcccaggta tgggtggcgc tatggattta 5100
   gcaataggcg caaaaaaaat aatagtggca atgcaacata caggaaaaag taaacctaaa 5160
   atcgttaaaa aatgtactct cccacttact gctaaggctc aagtggattt aattgtcaca 5220
   gaactttgtg taattgatgt aacaaatgac ggcttacttt taaaagaaat tcataaagat 5280
   acaactattg atgaaattaa atttttaaca gatgcagatt taattattcc agataactta 5340
   aagattatgg atatatgaat cattctattt taaatatata actttaaaaa tcttatgtat 5400
   taaaaactaa gaaaagaggt tgattgtttt atgttagaaa gtgaagtatc taaacaaatt 5460
   acaactccac ttgctgctcc agcgtttcct agaggaccat ataggtttca caatagagaa 5520
   tatctaaaca ttatttatcg aactgattta gatgctcttc gaaaaatagt accagagcca 5580
   cttgaattag atagagcata tgttagattt gaaatgatgg ctatgcctga tacaaccgga 5640
   ctaggctcat atacagaatg tggtcaagct attccagtaa aatataatgg tgttaagggt 5700
   gactacttgc atatgatgta tctagataat gaacctgcta ttgctgttgg aagagaaagt 5760
   agcgcttatc caaaaaagct tggctatcca aagctatttg ttgattcaga tactttagtt 5820
   gggacactta aatatggtac attaccagta gctactgcaa caatgggata taagcacgag 5880
   cctctagatc ttaaagaagc ctatgctcaa attgcaagac ccaattttat gctaaaaatc 5940
   attcaaggtt acgatggtaa gccaagaatt tgtgaactaa tatgtgcaga aaatactgat 6000
   ataactattc acggtgcttg gactggaagt gcacgtctac aattatttag ccatgcacta 6060
   gctcctcttg ctgatttacc tgtattagag attgtatcag catctcatat cctcacagat 6120
   ttaactcttg gaacacctaa ggttgtacat gattatcttt cagtaaaata aaagcaatat 6180
   agaggatcct ctagagtcga cttaggaggt tctattatga aaggttttgc aatgttaggt 6240
   attaacaaat taggatggat tgaaaagaaa aacccagtgc caggtcctta tgatgcgatt 6300
   gtacatcctc tagctgtatc cccatgtaca tcagatatac atacggtttt tgaaggagca 6360
   cttggtaata gggaaaatat gattttaggc catgaagctg taggtgaaat agccgaagtt 6420
   ggcagcgaag ttaaagattt taaagttggc gatagagtta tcgtaccatg cacaacacct 6480
   gactggagat ctttagaagt ccaagctggt tttcagcagc attcaaacgg tatgcttgca 6540
   ggatggaagt tttccaattt taaagatggt gtatttgcag attactttca tgtaaacgat 6600
   gcagatatga atcttgccat actcccagat gaaatacctt tagaaagtgc agttatgatg 6660
   acagacatga tgactactgg ttttcatgga gcagaacttg cagacataaa aatgggctcc 6720
   agcgttgtag taattggtat aggagctgtt ggattaatgg gaatagccgg ttccaaactt 6780
   cgaggagcag gcagaattat cggtgttgga agcagacctg tttgtgttga aacagctaaa 6840
   ttttatggag caactgatat tgtaaattat aaaaatggtg atatagttga acaaatcatg 6900
   gacttaactc atggtaaagg tgtagaccgt gtaatcatgg caggcggtgg tgctgaaaca 6960
   ctagcacaag cagtaactat ggttaaacct ggcggcgtaa tttctaacat caactaccat 7020
   ggaagcggtg atactttacc aatacctcgt gttcaatggg gctgcggcat ggctcacaaa 7080
   actataagag gaggattatg ccccggcgga cgtcttagaa tggaaatgct aagagatctt 7140
   gttctatata aacgtgttga tttgagtaaa cttgttactc atgtatttga tggtgcagaa 7200
   aatattgaaa aggccctttt gcttatgaaa aataagccaa aagatttaat taaatcagta 7260
   gttacattct aaaaattcat ataaaaaaac tgtcgcatta aaaaaatgtc tcgaggcctg 7320
   cagacatgca agcttggcac tggccgtcgt tttacaacgt cgtgactggg aaaaccctgg 7380
   cgttacccaa cttaatcgcc ttgcagcaca tccccctttc gccagctggc gtaatagcga 7440
   agaggcccgc accgatcgcc cttcccaaca gttgcgcagc ctgaatggcg aatggcgcta 7500
   gcataaaaat aagaagcctg catttgcagg cttcttattt ttatggcgcg ccgcattcac 7560
   ttcttttcta tataaatatg agcgaagcga ataagcgtcg gaaaagcagc aaaaagtttc 7620
   ctttttgctg ttggagcatg ggggttcagg gggtgcagta tctgacgtca atgccgagcg 7680
   aaagcgagcc gaagggtagc atttacgtta gataaccccc tgatatgctc cgacgcttta 7740
   tatagaaaag aagattcaac taggtaaaat cttaatatag gttgagatga taaggtttat 7800
   aaggaatttg tttgttctaa tttttcactc attttgttct aatttctttt aacaaatgtt 7860
   cttttttttt tagaacagtt atgatatagt tagaatagtt taaaataagg agtgagaaaa 7920
   ag 7922
<210> 49
   <211> 4709
   <212> DNA
   <213> synthetic plasmid
<400> 49
   gtttgccacc tgacgtctaa gaaaaggaat attcagcaat ttgcccgtgc cgaagaaagg      60
   cccacccgtg aaggtgagcc agtgagttga ttgctacgta attagttagt tagcccttag     120
   tgactcgtaa tacgactcac tatagggctc gaggcggccg cgcaacgcaa ttaatgtgag     180
   ttagctcact cattaggcac cccaggcttt acactttatg cttccggctc gtatgttgtg     240
   tggaattgtg agcggataac aatttcacac aggaaacaca tatgtttccg tgcaatgcct     300
   atatcgaata tggtgataaa aatatgaaca gctttatcga agatgtggaa cagatctaca     360
   acttcattaa aaagaacatt gatgtggaag aaaagatgca tttcattgaa acctataaac     420
   agaaaagcaa catgaagaaa gagattagct ttagcgaaga atactataaa cagaagatta     480
   tgaacggcaa aaatggcgtt gtgtacaccc cgccggaaat ggcggccttt atggttaaaa     540
   atctgatcaa cgttaacgat gttattggca atccgtttat taaaatcatt gacccgagct     600
   gcggtagcgg caatctgatt tgcaaatgtt ttctgtatct gaatcgcatc tttattaaga     660
   acattgaggt gattaacagc aaaaataacc tgaatctgaa actggaagac atcagctacc     720
   acatcgttcg caacaatctg tttggcttcg atattgacga aaccgcgatc aaagtgctga     780
   aaattgatct gtttctgatc agcaaccaat ttagcgagaa aaatttccag gttaaagact     840
   ttctggtgga aaatattgat cgcaaatatg acgtgttcat tggtaatccg ccgtatatcg     900
   gtcacaaaag cgtggacagc agctacagct acgtgctgcg caaaatctac ggcagcatct     960
   accgcgacaa aggcgatatc agctattgtt tctttcagaa gagcctgaaa tgtctgaagg    1020
   aaggtggcaa actggtgttt gtgaccagcc gctacttctg cgagagctgc agcggtaaag 1080
   aactgcgtaa attcctgatc gaaaacacga gcatttacaa gatcattgat ttttacggca 1140
   tccgcccgtt caaacgcgtg ggtatcgatc cgatgattat ttttctggtt cgtacgaaga 1200
   actggaacaa taacattgaa attattcgcc cgaacaagat tgaaaagaac gaaaagaaca 1260
   aattcctgga tagcctgttc ctggacaaaa gcgaaaagtg taaaaagttt agcattagcc 1320
   agaaaagcat taataacgat ggctgggttt tcgtggacga agtggagaaa aacattatcg 1380
   acaaaatcaa agagaaaagc aagttcattc tgaaagatat ttgccatagc tgtcaaggca 1440
   ttatcaccgg ttgtgatcgc gcctttattg tggaccgtga tatcatcaat agccgtaaga 1500
   tcgaactgcg tctgattaaa ccgtggatta aaagcagcca tatccgtaag aatgaagtta 1560
   ttaagggcga aaaattcatc atctatagca acctgattga gaatgaaacc gagtgtccga 1620
   atgcgattaa atatatcgaa cagtacaaga aacgtctgat ggagcgccgc gaatgcaaaa 1680
   agggcacgcg taagtggtat gaactgcaat ggggccgtaa accggaaatc ttcgaagaaa 1740
   agaaaattgt tttcccgtat aaaagctgtg acaatcgttt tgcactggat aagggtagct 1800
   attttagcgc agacatttat agcctggttc tgaagaaaaa tgtgccgttc acctatgaga 1860
   tcctgctgaa tatcctgaat agcccgctgt acgagtttta ctttaagacc ttcgcgaaaa 1920
   agctgggcga gaatctgtac gagtactatc cgaacaacct gatgaagctg tgcatcccga 1980
   gcatcgattt cggcggtgag aacaatattg agaaaaagct gtatgatttc tttggtctga 2040
   cggataaaga aattgagatt gtggagaaga tcaaagataa ctgctaagaa ttcgatatca 2100
   cccgggaact agtctgcagc cctttagtga gggttaattg gagtcactaa gggttagtta 2160
   gttagattag cagaaagtca aaagcctccg accggaggct tttgactaaa acttcccttg 2220
   gggttatcat tggggctcac tcaaaggcgg taatcagata aaaaaaatcc ttagctttcg 2280
   ctaaggatga tttctgctag agatggaata gactggatgg aggcggataa agttgcagga 2340
   ccacttctgc gctcggccct tccggctggc tggtttattg ctgataaatc tggagccggt 2400
   gagcgtgggt ctcgcggtat cattgcagca ctggggccag atggtaagcc ctcccgtatc 2460
   gtagttatct acacgacggg gagtcaggca actatggatg aacgaaatag acagatcgct 2520
   gagataggtg cctcactgat taagcattgg taactgtcag accaagttta ctcatatata 2580
   ctttagattg atttaaaact tcatttttaa tttaaaagga tctaggtgaa gatccttttt 2640
   gataatctca tgaccaaaat cccttaacgt gagttttcgt tccactgagc gtcagacccc 2700
   ttaataagat gatcttcttg agatcgtttt ggtctgcgcg taatctcttg ctctgaaaac 2760
   gaaaaaaccg ccttgcaggg cggtttttcg aaggttctct gagctaccaa ctctttgaac 2820
   cgaggtaact ggcttggagg agcgcagtca ccaaaacttg tcctttcagt ttagccttaa 2880
   ccggcgcatg acttcaagac taactcctct aaatcaatta ccagtggctg ctgccagtgg 2940
   tgcttttgca tgtctttccg ggttggactc aagacgatag ttaccggata aggcgcagcg 3000
   gtcggactga acggggggtt cgtgcataca gtccagcttg gagcgaactg cctacccgga 3060
   actgagtgtc aggcgtggaa tgagacaaac gcggccataa cagcggaatg acaccggtaa 3120
   accgaaaggc aggaacagga gagcgcacga gggagccgcc aggggaaacg cctggtatct 3180
   ttatagtcct gtcgggtttc gccaccactg atttgagcgt cagatttcgt gatgcttgtc 3240
   aggggggcgg agcctatgga aaaacggctt tgccgcggcc ctctcacttc cctgttaagt 3300
   atcttcctgg catcttccag gaaatctccg ccccgttcgt aagccatttc cgctcgccgc 3360
   agtcgaacga ccgagcgtag cgagtcagtg agcgaggaag cggaatatat cctgtatcac 3420
   atattctgct gacgcaccgg tgcagccttt tttctcctgc cacatgaagc acttcactga 3480
   caccctcatc agtgccaaca tagtaagcca gtatacactc cgctagcgct gaggtctgcc 3540
   tcgtgaagaa ggtgttgctg actcatacca ggcctgaatc gccccatcat ccagccagaa 3600
   agtgagggag ccacggttga tgagagcttt gttgtaggtg gaccagttgg tgattttgaa 3660
   cttttgcttt gccacggaac ggtctgcgtt gtcgggaaga tgcgtgatct gatccttcaa 3720
   ctcagcaaaa gttcgattta ttcaacaaag ccacgttgtg tctcaaaatc tctgatgtta 3780
   cattgcacaa gataaaaata tatcatcatg aacaataaaa ctgtctgctt acataaacag 3840
   taatacaagg ggtgtttact agaggttgat cgggcacgta agaggttcca actttcacca 3900
   taatgaaata agatcactac cgggcgtatt ttttgagtta tcgagatttt caggagctaa 3960
   ggaagctaaa atggagaaaa aaatcacggg atataccacc gttgatatat cccaatggca 4020
   tcgtaaagaa cattttgagg catttcagtc agttgctcaa tgtacctata accagaccgt 4080
   tcagctggat attacggcct ttttaaagac cgtaaagaaa aataagcaca agttttatcc 4140
   ggcctttatt cacattcttg cccgcctgat gaacgctcac ccggagtttc gtatggccat 4200
   gaaagacggt gagctggtga tctgggatag tgttcaccct tgttacaccg ttttccatga 4260
   gcaaactgaa acgttttcgt ccctctggag tgaataccac gacgatttcc ggcagtttct 4320
   ccacatatat tcgcaagatg tggcgtgtta cggtgaaaac ctggcctatt tccctaaagg 4380
   gtttattgag aatatgtttt ttgtctcagc caatccctgg gtgagtttca ccagttttga 4440
   tttaaacgtg gccaatatgg acaacttctt cgcccccgtt ttcacgatgg gcaaatatta 4500
   tacgcaaggc gacaaggtgc tgatgccgct ggcgatccag gttcatcatg ccgtttgtga 4560
   tggcttccat gtcggccgca tgcttaatga attacaacag tactgtgatg agtggcaggg 4620
   cggggcgtaa taatactagc tccggcaaaa aaacgggcaa ggtgtcacca ccctgccctt 4680
   tttctttaaa accgaaaaga ttacttcgc 4709
<210> 50
   <211> 128
   <212> DNA
   <213> e.coli
<400> 50
   gcggccgcgc aacgcaatta atgtgagtta gctcactcat taggcacccc aggctttaca 60
   ctttatgctt ccggctcgta tgttgtgtgg aattgtgagc ggataacaat ttcacacagg 120
   aaacacat 128
<210> 51
   <211> 560
   <212> DNA
   <213> Clostridium autoethanogenum
<400> 51
   ttataagaaa tcctaaggaa tatgatgtaa tgagtaaagc tataaatcct tatggagatg 60
   gcaaggcagc ttatagaata acagaagcta ttttacaata ttttgattta gcaaaaggta 120
   catatagtga gtttaaatca aattaaaaag ttataatttt caattttcat tctttttaaa 180
   ggagattagc atacatttta tcataattat acagacaata tagtaatata tgatgttaaa 240
   atatcaatat atggttaaaa atctgtatat tttttcccat tttaattatt tgtactataa 300
   tattacactg agtgtattgt atatttaaaa aatatttggt acaattagtt agttaaataa 360
   attctaaatt gtaaattatc agaatcctta ttaaggaaat acatagattt aaggagaaat 420
   cataaaaagg tgtaatataa actggctaaa attgagcaaa aattgagcaa ttaagacttt 480
   ttgattgtat ctttttatat atttaaggta tataatctta tttatattgg gggaacttga 540
   tgaataaaca tattctagac 560
<210> 52
   <211> 297
   <212> DNA
   <213> Clostridium autoethanogenum
<400> 52
   taattttttg tgtcaataat ttttgttata ttattttaat taaatttttc acatgtataa 60
   ttaaaagtaa gatagatatt ctaatgtact tacttaggta gaaaaacatg tatacaaaat 120
   taaaaaacta ttataacaca tagtatcaat attgaaggta atactgttca atatcgatac 180
   agataaaaaa atatataata cagaagaaaa aattataaat ttgtggtata atataaagta 240
   tagtaattta agtttaaacc tcgtgaaaac gctaacaaat aataggaggt gtattat 297
<210> 53
   <211> 599
   <212> DNA
   <213> Clostridium autoethanogenum
<400> 53
   atagtataac tttaaaaaac tgtcttaaaa agttgttata taaaaaatgt tgacaattaa 60
   acagctattt agtgcaaaac aaccataaaa atttaaaaaa taccataaat tacttgaaaa 120
   atagttgata ataatgtaga gttataaaca aaggtgaaaa gcattacttg tattcttttt 180
   tatatattat tataaattaa aatgaagctg tattagaaaa aatacacacc tgtaatataa 240
   aattttaaat taatttttaa ttttttcaaa atgtatttta catgtttaga attttgatgt 300
   atattaaaat agtagaatac ataagatact taatttaatt aaagatagtt aagtactttt 360
   caatgtgctt ttttagatgt ttaatacaaa tctttaattg taaaagaaat gctgtactat 420
   ttactgtact agtgacggga ttaaactgta ttaattataa ataaaaaata agtacagttg 480
   tttaaaatta tattttgtat taaatctaat agtacgatgt aagttatttt atactattgc 540
   tagtttaata aaaagattta attatatact tgaaaaggag aggaattttt atgcgtaaa 599
<210> 54
   <211> 34
   <212> DNA
   <213> clostridium autoethanogenum
<400> 54
   tatttgtcga cttaggaggt tctattatga aagg 34
<210> 55
   <211> 30
   <212> DNA
   <213> Clostridium autoethanogenum
<400> 55
   aaaactcgag acattttttt aatgcgacag 30
<210> 56
   <211> 560
   <212> DNA
   <213> clostridium ljungdahlii
<400> 56
   agatagtcat aatagttcca gaatagttta atttagcatt tggattaaat tcccatatgt 60
   ttgttaaata tataccaaac tagtatagat atttttaaaa tactgtactt aaacagtagt 120
   aatttacgta aaaaaatttt ttgatttttt taaaaaagtc cttttcaagt tgtacattat 180
   tatggtaata tgtaattgaa gaagttgtgt agtaatattg taaacgtttc ttaatttatt 240
   ttcatccatg tagtgcttaa aaaaccaaaa tatgtcacac gcaattgcat atttcaaaca 300
   ataatattta ttttctcgtt aaattcacaa ataatttatt aataatatca ataaccaaga 360
   ttatacttaa atggatgttt attttttaac attttttata gtaaatatat ttattttatg 420
   tagtaaaaag gttataatta taattgtatt tattacaatt aattaaaata aaaaaatagg 480
   gttttaggta aaattaagtt attttaagaa gtaattacaa caaaaattga agttatttct 540
   ttaaggaggg aattattaaa 560
<210> 57
   <211> 557
   <212> DNA
   <213> clostridium ragsdalei
<400> 57
   agatagtcat aatagttcca gaatagttta attttgaaat tggagtaaac ttccaaatgt 60
   ttgttaaata tataccaaac tagtatagat attttttaaa tactagactt aaacagtaga 120
   aatttgccta aaaaattttt tagtttttta aaaaaatcct tttcaagttg tacgttatta 180
   tggtaatatg taattgaaga agttatgtaa taatattgta aacgtttctt aattttttta 240
   catccatgta atgcttaaaa gaccaaaata tgtcacatgt aattgtatat ttcacataat 300
   aatatttatt ttcttattaa attcacaaat aatttattaa taatatcaat aaccaagatt 360
   atacttaaat ggatgtttat tttttaacat tttttatggt aaatatattt attttatgta 420
   gtaaaaaggt tataattata attgtattta ttacaattaa ttaaaataaa aaatagggtt 480
   ttaggtaaaa ttaagttatt ttaagaagta attacaacaa aaattgaagt tatttcttta 540
   aggagggaat tattaaa 557
<210> 58
   <211> 536
   <212> DNA
   <213> Clostridium ljungdahlii
<400> 58
   ttataagaaa tcctaaggaa tatgatgtaa tgagtaaagc tataaatcct tatggagatg 60
   gcaaggcagc ttatagaata acagaagcta ttttacaata ttttgattta gcaaaaggta 120
   catatagtga gtttaaatca aattaaaaag ttataatttt caattttcat tctttttaaa 180
   ggagattagc atacatttta tcataattat acagacaata tagtaatata tgatgttaaa 240
   atatcaatat atggttaaaa atctgtatat tttttcccat tttaattatt tgtactataa 300
   tattacactg agtgtattgt atatttaaaa aatatttggt acaattagtt agttaaataa 360
   attctaaatt gtaaattatc agaatcctta ttaaggaaat acatagattt aaggagaaat 420
   cataaaaagg tgtaatataa actggctaaa attgagcaaa aattgagcaa ttaagacttt 480
   ttgattgtat ctttttatat atttaaggta tataatctta tttatattgg gggaac 536
<210> 59
   <211> 549
   <212> DNA
   <213> clostridium ragsdalei
<400> 59
   ttataagaaa tcctaaggaa tatgatgtaa tgagtaaagc tataaatcct tatggagatg 60
   gcaaggcagc ttatagaata acagaagcta ttttacaata ttttgattta gcaaaaggta 120
   catatagtga gtttaaatca aattaaaaag ttataatttt gaattttcat tctttttaaa 180
   ggagattagc atacatttta tcataattat acagacaata tagtaatata tgatgttaaa 240
   atatcaatat atggttaaaa aactgtatat tttttcccat tttaattatt tgtactataa 300
   tattacactg agtgtattgt atatttaaaa aatatttggt acaattagtt agttaaataa 360
   attctaaatt ataaattatc agaaacctta ttaaggaaat acatagattt agggagaaat 420
   aataaaaagg tgtaatataa actggctaaa gttgagtaat taagactttt aggttgtatc 480
   tttttatata tttaaggtat ataatcttag ttatataggg ggaacttgat gaataaacat 540
   attctagac 549
<210> 60
   <211> 298
   <212> DNA
   <213> Clostridium ljungdahlii
<400> 60
   taattttttg tgtcaataat ttttgttata ttattttaat taaatttttc acatgtataa 60
   ttaaaagtaa gatagatatt ctaatgtact tacttaggta gaaaaacatg tatacaaaat 120
   taaaaaacta ttataacaca tagtatcaat attgaaggta atactgttca atatcgatac 180
   agataaaaaa aatatataat acagaagaaa aaattataaa tttgtggtat aatataaagt 240
   atagtaattt aagtttaaac ctcgtgaaaa cgctaacaaa taataggagg tgtattat 298
<210> 61
   <211> 300
   <212> DNA
   <213> clostridium ragsdalei
<400> 61
   taatttttta tatcaataat ttttattata ttattttaat taaatttttc acatgtataa      60
   ttaaaagtaa gatagagata gttaggatat tttagtgcat ttatttagat aaaaaatatg     120
   tatacaagat tagaaaaaaa ttataacaca taatagttgc attgaaggta atactgttca     180
   atatcgatac agataaaaaa atttataata cagaagaaaa aaatataaat ttgtggtata     240
   atataaaata taataattta gatttacacc ccgtgaaaac gctaacaaat aaatagggag     300
<210> 62
   <211> 590
   <212> DNA
   <213> Clostridium ljungdahlii
<400> 62
   atagtataac tttaaaaaac tgtcttaaaa agttgttata taaaaaatgt tgacaattaa      60
   acagctattt agtgcaaaac aaccataaaa atttaaaaaa taccataaat tacttgaaaa     120
   atagttgata ataatgtaga gttataaaca aaggtgaaaa gcattacttg tattcttttt     180
   tatatattat tataaattaa aatgaagctg tattagaaaa aatacacacc tgtaatataa     240
   aattttaaat taatttttaa ttttttcaaa atgtatttta catgtttaga attttgatgt    300
   atattaaaat agtagaatac ataagatact taatttaatt aaagatagtt aagtactttt     360
   caatgtgctt ttttagatgt ttaatacaaa tctttaattg taaaagaaat gctgtactat     420
   ttactgtact agtgacggga ttaaactgta ttaattataa ataaaaaata agtacagttg     480
   tttaaaatta tattttgtat taaatctaat agtacgatgt aagttatttt atactattgc     540
   tagtttaata aaaagattta attatatact tgaaaaggag aggaattttt                590
<210> 63
   <211> 600
   <212> DNA
   <213> Clostridium ragsdalei
<400> 63
   atagaataac ttaaaaaaac tgtcttaaaa agctgttata taaaaaaatg ttaacaatta      60
   aacagctatt tagtgcaaaa caaccataaa aatttaaaaa ataccataaa ttacttgaaa     120
   aatagtagag aataatgtag agttataaac gaaggtgaaa agcattactt gtattccttt     180
   ttacagacta ttataaatta agataaagct gtattaggaa aaatgcacac ctgtaatata     240
   aggttttaaa ttaattttta attttcccaa aatgtatttt acatgtttag aattttgatg 300
   tatattaaaa tagtagaata cataagatac ttaatttaat aaagatagtt aagtactttt 360
   caatgtactt ttttagatat ttaatacaag tttttaattg taaaaaaatg ctgtgctatt 420
   tactgtacta atggtagtac tatatctgta ttaattgtat gtaaaaagta agtatagtta 480
   tttaagatta tgttttgtat taaatctaaa tagtacaatg taggttatgt tatactattg 540
   ctagtttaat aaaaagattt aattatatac ttgaaaagga gaggaatttt tatgcgtaaa 600
<210> 64
   <211> 25
   <212> DNA
   <213> synthetic primer
<400> 64
   ttgatgaaat gatcactgac ggatt 25
<210> 65
   <211> 25
   <212> DNA
   <213> synthetic primer
<400> 65
   gaaatgttcc atctctcagc tatgt 25
<210> 66
   <211> 25
   <212> DNA
   <213> synthetic primer
<400> 66
   ctaatacgag gaggacatgt tgatg 25
<210> 67
   <211> 25
   <212> DNA
   <213> synthetic primer
<400> 67
   cacccatacc tgggacaatt ttatt 25
<210> 68
   <211> 25
   <212> DNA
   <213> synthetic primer
<400> 68
   gggctgctac taaaaatttc aatcc 25
<210> 69
   <211> 25
   <212> DNA
   <213> synthetic primer
<400> 69
   caggagtcat tatggcatct ctttt 25
<210> 70
   <211> 25
   <212> DNA
   <213> synthetic primer
<400> 70
   tagtaccaga gccacttgaa ttaga 25
<210> 71
   <211> 25
   <212> DNA
   <213> synthetic primer
<400> 71
   ggaatagctt gaccacattc tgtat 25
<210> 72
   <211> 1647
   <212> DNA
   <213> Lactococcus lactis
<400> 72
   atgtatacag taggagatta cctattagac cgattacacg agttaggaat tgaagaaatt     60
   tttggagtcc ctggagacta taacttacaa tttttagatc aaattatttc ccacaaggat    120
   atgaaatggg tcggaaatgc taatgaatta aatgcttcat atatggctga tggctatgct    180
   cgtactaaaa aagctgccgc atttcttaca acctttggag taggtgaatt gagtgcagtt    240
   aatggattag caggaagtta cgccgaaaat ttaccagtag tagaaatagt gggatcacct    300
   acatcaaaag ttcaaaatga aggaaaattt gttcatcata cgctggctga cggtgatttt    360
   aaacacttta tgaaaatgca cgaacctgtt acagcagctc gaactttact gacagcagaa    420
   aatgcaaccg ttgaaattga ccgagtactt tctgcactat taaaagaaag aaaacctgtc    480
   tatatcaact taccagttga tgttgctgct gcaaaagcag agaaaccctc actccctttg    540
   aaaaaggaaa actcaacttc aaatacaagt gaccaagaaa ttttgaacaa aattcaagaa    600
   agcttgaaaa atgccaaaaa accaatcgtg attacaggac atgaaataat tagttttggc    660
   ttagaaaaaa cagtcactca atttatttca aagacaaaac tacctattac gacattaaac    720
   tttggtaaaa gttcagttga tgaagccctc ccttcatttt taggaatcta taatggtaca    780
   ctctcagagc ctaatcttaa agaattcgtg gaatcagccg acttcatctt gatgcttgga    840
   gttaaactca cagactcttc aacaggagcc ttcactcatc atttaaatga aaataaaatg    900
   atttcactga atatagatga aggaaaaata tttaacgaaa gaatccaaaa ttttgatttt    960
   gaatccctca tctcctctct cttagaccta agcgaaatag aatacaaagg aaaatatatc   1020
   gataaaaagc aagaagactt tgttccatca aatgcgcttt tatcacaaga ccgcctatgg   1080
   caagcagttg aaaacctaac tcaaagcaat gaaacaatcg ttgctgaaca agggacatca   1140
   ttctttggcg cttcatcaat tttcttaaaa tcaaagagtc attttattgg tcaaccctta   1200
   tggggatcaa ttggatatac attcccagca gcattaggaa gccaaattgc agataaagaa   1260
   agcagacacc ttttatttat tggtgatggt tcacttcaac ttacagtgca agaattagga   1320
   ttagcaatca gagaaaaaat taatccaatt tgctttatta tcaataatga tggttataca   1380
   gtcgaaagag aaattcatgg accaaatcaa agctacaatg atattccaat gtggaattac   1440
   tcaaaattac cagaatcgtt tggagcaaca gaagatcgag tagtctcaaa aatcgttaga   1500
   actgaaaatg aatttgtgtc tgtcatgaaa gaagctcaag cagatccaaa tagaatgtac   1560
   tggattgagt taattttggc aaaagaaggt gcaccaaaag tactgaaaaa aatgggcaaa   1620
   ctatttgctg aacaaaataa atcataa                                       1647
<210> 73
   <211> 548
   <212> PRT
   <213> Lactococcus lactis
<400> 73
   Met Tyr Thr Val Gly Asp Tyr Leu Leu Asp Arg Leu His Glu Leu Gly
   1               5                   10                  15
   Ile Glu Glu Ile Phe Gly Val Pro Gly Asp Tyr Asn Leu Gln Phe Leu
               20                  25                  30
   Asp Gln Ile Ile Ser His Lys Asp Met Lys Trp Val Gly Asn Ala Asn
           35                  40                  45
   Glu Leu Asn Ala Ser Tyr Met Ala Asp Gly Tyr Ala Arg Thr Lys Lys
       50                  55                  60
   Ala Ala Ala Phe Leu Thr Thr Phe Gly Val Gly Glu Leu Ser Ala Val
   65                  70                  75                      80
   Asn Gly Leu Ala Gly Ser Tyr Ala Glu Asn Leu Pro Val Val Glu Ile
                   85                  90                  95
   Val Gly Ser Pro Thr Ser Lys Val Gln Asn Glu Gly Lys Phe Val His
               100                 105                 110
   His Thr Leu Ala Asp Gly Asp Phe Lys His Phe Met Lys Met His Glu
           115                 120                 125
   Pro Val Thr Ala Ala Arg Thr Leu Leu Thr Ala Glu Asn Ala Thr Val
       130                 135                 140
   Glu Ile Asp Arg Val Leu Ser Ala Leu Leu Lys Glu Arg Lys Pro Val
   145                 150                 155                 160
   Tyr Ile Asn Leu Pro Val Asp Val Ala Ala Ala Lys Ala Glu Lys Pro
                   165                 170                 175
   Ser Leu Pro Leu Lys Lys Glu Asn Ser Thr Ser Asn Thr Ser Asp Gln
               180                 185                 190
   Glu Ile Leu Asn Lys Ile Gln Glu Ser Leu Lys Asn Ala Lys Lys Pro
   195 200 205
   Ile Val Ile Thr Gly His Glu Ile Ile Ser Phe Gly Leu Glu Lys Thr
   210 215 220
   Val Thr Gln Phe Ile Ser Lys Thr Lys Leu Pro Ile Thr Thr Leu Asn
   225 230 235 240
   Phe Gly Lys Ser Ser Val Asp Glu Ala Leu Pro Ser Phe Leu Gly Ile
   245 250 255
   Tyr Asn Gly Thr Leu Ser Glu Pro Asn Leu Lys Glu Phe Val Glu Ser
   260 265 270
   Ala Asp Phe Ile Leu Met Leu Gly Val Lys Leu Thr Asp Ser Ser Thr
   275 280 285
   Gly Ala Phe Thr His His Leu Asn Glu Asn Lys Met Ile Ser Leu Asn
   290 295 300
   Ile Asp Glu Gly Lys Ile Phe Asn Glu Arg Ile Gln Asn Phe Asp Phe
   305 310 315 320
   Glu Ser Leu Ile Ser Ser Leu Leu Asp Leu Ser Glu Ile Glu Tyr Lys
   325 330 335
   Gly Lys Tyr Ile Asp Lys Lys Gln Glu Asp Phe Val Pro Ser Asn Ala
   340 345 350
   Leu Leu Ser Gln Asp Arg Leu Trp Gln Ala Val Glu Asn Leu Thr Gln
   355 360 365
   Ser Asn Glu Thr Ile Val Ala Glu Gln Gly Thr Ser Phe Phe Gly Ala
   370 375 380
   Ser Ser Ile Phe Leu Lys Ser Lys Ser His Phe Ile Gly Gln Pro Leu
   385 390 395 400
   Trp Gly Ser Ile Gly Tyr Thr Phe Pro Ala Ala Leu Gly Ser Gln Ile
   405 410 415
   Ala Asp Lys Glu Ser Arg His Leu Leu Phe Ile Gly Asp Gly Ser Leu
   420 425 430
   Gln Leu Thr Val Gln Glu Leu Gly Leu Ala Ile Arg Glu Lys Ile Asn
   435 440 445
   Pro Ile Cys Phe Ile Ile Asn Asn Asp Gly Tyr Thr Val Glu Arg Glu
   450 455 460
   Ile His Gly Pro Asn Gln Ser Tyr Asn Asp Ile Pro Met Trp Asn Tyr
   465 470 475 480
   Ser Lys Leu Pro Glu Ser Phe Gly Ala Thr Glu Asp Arg Val Val Ser
   485 490 495
   Lys Ile Val Arg Thr Glu Asn Glu Phe Val Ser Val Met Lys Glu Ala
   500 505 510
   Gln Ala Asp Pro Asn Arg Met Tyr Trp Ile Glu Leu Ile Leu Ala Lys
   515 520 525
   Glu Gly Ala Pro Lys Val Leu Lys Lys Met Gly Lys Leu Phe Ala Glu
   530 535 540
   Gln Asn Lys Ser
   545
<210> 74
   <211> 2094
   <212> DNA
   <213> Sacchromyces cerevisiae
<400> 74
   atgtctattc cagaaactca aaaagccatt atcttctacg aatccaacgg caagttggag 60
   cataaggata tcccagttcc aaagccaaag cccaacgaat tgttaatcaa cgtcaagtac 120
   tctggtgtct gccacaccga tttgcacgct tggcatggtg actggccatt gccaactaag 180
   ttaccattag ttggtggtca cgaaggtgcc ggtgtcgttg tcggcatggg tgaaaacgtt 240
   aagggctgga agatcggtga ctacgccggt atcaaatggt tgaacggttc ttgtatggcc 300
   tgtgaatact gtgaattggg taacgaatcc aactgtcctc acgctgactt gtctggttac 360
   acccacgacg gttctttcca agaatacgct accgctgacg ctgttcaagc cgctcacatt 420
   cctcaaggta ctgacttggc tgaagtcgcg ccaatcttgt gtgctggtat caccgtatac 480
   aaggctttga agtctgccaa cttgagagca ggccactggg cggccatttc tggtgctgct 540
   ggtggtctag gttctttggc tgttcaatat gctaaggcga tgggttacag agtcttaggt 600
   attgatggtg gtccaggaaa ggaagaattg tttacctcgc tcggtggtga agtattcatc 660
   gacttcacca aagagaagga cattgttagc gcagtcgtta aggctaccaa cggcggtgcc 720
   cacggtatca tcaatgtttc cgtttccgaa gccgctatcg aagcttctac cagatactgt 780
   agggcgaacg gtactgttgt cttggttggt ttgccagccg gtgcaaagtg ctcctctgat 840
   gtcttcaacc acgttgtcaa gtctatctcc attgtcggct cttacgtggg gaacagagct 900
   gataccagag aagccttaga tttctttgcc agaggtctag tcaagtctcc aataaaggta 960
   gttggcttat ccagtttacc agaaatttac gaaaagatgg agaagggcca aattgctggt 1020
   agatacgttg ttgacacttc taaataaatg tctattccag aaactcaaaa agccattatc 1080
   ttctacgaat ccaacggcaa gttggagcat aaggatatcc cagttccaaa gccaaagccc 1140
   aacgaattgt taatcaacgt caagtactct ggtgtctgcc acaccgattt gcacgcttgg 1200
   catggtgact ggccattgcc aactaagtta ccattagttg gtggtcacga aggtgccggt 1260
   gtcgttgtcg gcatgggtga aaacgttaag ggctggaaga tcggtgacta cgccggtatc 1320
   aaatggttga acggttcttg tatggcctgt gaatactgtg aattgggtaa cgaatccaac 1380
   tgtcctcacg ctgacttgtc tggttacacc cacgacggtt ctttccaaga atacgctacc 1440
   gctgacgctg ttcaagccgc tcacattcct caaggtactg acttggctga agtcgcgcca 1500
   atcttgtgtg ctggtatcac cgtatacaag gctttgaagt ctgccaactt gagagcaggc 1560
   cactgggcgg ccatttctgg tgctgctggt ggtctaggtt ctttggctgt tcaatatgct 1620
   aaggcgatgg gttacagagt cttaggtatt gatggtggtc caggaaagga agaattgttt 1680
   acctcgctcg gtggtgaagt attcatcgac ttcaccaaag agaaggacat tgttagcgca 1740
   gtcgttaagg ctaccaacgg cggtgcccac ggtatcatca atgtttccgt ttccgaagcc 1800
   gctatcgaag cttctaccag atactgtagg gcgaacggta ctgttgtctt ggttggtttg 1860
   ccagccggtg caaagtgctc ctctgatgtc ttcaaccacg ttgtcaagtc tatctccatt 1920
   gtcggctctt acgtggggaa cagagctgat accagagaag ccttagattt ctttgccaga 1980
   ggtctagtca agtctccaat aaaggtagtt ggcttatcca gtttaccaga aatttacgaa 2040
   aagatggaga agggccaaat tgctggtaga tacgttgttg acacttctaa ataa 2094
<210> 75
   <211> 348
   <212> PRT
   <213> Saccharomyces cerevisiae
<400> 75
   Met Ser Ile Pro Glu Thr Gln Lys Ala Ile Ile Phe Tyr Glu Ser Asn
   1 5 10 15
   Gly Lys Leu Glu His Lys Asp Ile Pro Val Pro Lys Pro Lys Pro Asn
   20 25 30
   Glu Leu Leu Ile Asn Val Lys Tyr Ser Gly Val Cys His Thr Asp Leu
   35 40 45
   His Ala Trp His Gly Asp Trp Pro Leu Pro Thr Lys Leu Pro Leu Val
   50 55 60
   Gly Gly His Glu Gly Ala Gly Val Val Val Gly Met Gly Glu Asn Val
   65 70 75 80
   Lys Gly Trp Lys Ile Gly Asp Tyr Ala Gly Ile Lys Trp Leu Asn Gly
   85 90 95
   Ser Cys Met Ala Cys Glu Tyr Cys Glu Leu Gly Asn Glu Ser Asn Cys
   100 105 110
   Pro His Ala Asp Leu Ser Gly Tyr Thr His Asp Gly Ser Phe Gln Glu
           115                 120                 125
   Tyr Ala Thr Ala Asp Ala Val Gln Ala Ala His Ile Pro Gln Gly Thr
       130                 135                 140
   Asp Leu Ala Glu Val Ala Pro Ile Leu Cys Ala Gly Ile Thr Val Tyr
   145                 150                 155                 160
   Lys Ala Leu Lys Ser Ala Asn Leu Arg Ala Gly His Trp Ala Ala Ile
                   165                 170                 175
   Ser Gly Ala Ala Gly Gly Leu Gly Ser Leu Ala Val Gln Tyr Ala Lys
               180                 185                 190
   Ala Met Gly Tyr Arg Val Leu Gly Ile Asp Gly Gly Pro Gly Lys Glu
           195                 200                 205
   Glu Leu Phe Thr Ser Leu Gly Gly Glu Val Phe Ile Asp Phe Thr Lys
       210                 215                 220
   Glu Lys Asp Ile Val Ser Ala Val Val Lys Ala Thr Asn Gly Gly Ala
   225                 230                 235                 240
   His Gly Ile Ile Asn Val Ser Val Ser Glu Ala Ala Ile Glu Ala Ser
                   245                 250                 255
   Thr Arg Tyr Cys Arg Ala Asn Gly Thr Val Val Leu Val Gly Leu Pro
               260                 265                 270
   Ala Gly Ala Lys Cys Ser Ser Asp Val Phe Asn His Val Val Lys Ser
           275                 280                 285
   Ile Ser Ile Val Gly Ser Tyr Val Gly Asn Arg Ala Asp Thr Arg Glu
       290                 295                 300
   Ala Leu Asp Phe Phe Ala Arg Gly Leu Val Lys Ser Pro Ile Lys Val
   305                 310                 315                 320
   Val Gly Leu Ser Ser Leu Pro Glu Ile Tyr Glu Lys Met Glu Lys Gly
                   325                 330                 335
   Gln Ile Ala Gly Arg Tyr Val Val Asp Thr Ser Lys
               340                 345
<210> 76
   <211> 1650
   <212> DNA
   <213> Lactococcus lactis
<400> 76
   catatgtata cagtaggaga ttacctatta gaccgattac acgagttagg aattgaagaa 60
   atttttggag tccctggaga ctataactta caatttttag atcaaattat ttcccacaag 120
   gatatgaaat gggtcggaaa tgctaatgaa ttaaatgctt catatatggc tgatggctat 180
   gctcgtacta aaaaagctgc cgcatttctt acaacctttg gagtaggtga attgagtgca 240
   gttaatggat tagcaggaag ttacgccgaa aatttaccag tagtagaaat agtgggatca 300
   cctacatcaa aagttcaaaa tgaaggaaaa tttgttcatc atacgctggc tgacggtgat 360
   tttaaacact ttatgaaaat gcacgaacct gttacagcag ctcgaacttt actgacagca 420
   gaaaatgcaa ccgttgaaat tgaccgagta ctttctgcac tattaaaaga aagaaaacct 480
   gtctatatca acttaccagt tgatgttgct gctgcaaaag cagagaaacc ctcactccct 540
   ttgaaaaagg aaaactcaac ttcaaataca agtgaccaag aaattttgaa caaaattcaa 600
   gaaagcttga aaaatgccaa aaaaccaatc gtgattacag gacatgaaat aattagtttt 660
   ggcttagaaa aaacagtcac tcaatttatt tcaaagacaa aactacctat tacgacatta 720
   aactttggta aaagttcagt tgatgaagcc ctcccttcat ttttaggaat ctataatggt 780
   acactctcag agcctaatct taaagaattc gtggaatcag ccgacttcat cttgatgctt 840
   ggagttaaac tcacagactc ttcaacagga gccttcactc atcatttaaa tgaaaataaa 900
   atgatttcac tgaatataga tgaaggaaaa atatttaacg aaagaatcca aaattttgat 960
   tttgaatccc tcatctcctc tctcttagac ctaagcgaaa tagaatacaa aggaaaatat 1020
   atcgataaaa agcaagaaga ctttgttcca tcaaatgcgc ttttatcaca agaccgccta 1080
   tggcaagcag ttgaaaacct aactcaaagc aatgaaacaa tcgttgctga acaagggaca 1140
   tcattctttg gcgcttcatc aattttctta aaatcaaaga gtcattttat tggtcaaccc 1200
   ttatggggat caattggata tacattccca gcagcattag gaagccaaat tgcagataaa 1260
   gaaagcagac accttttatt tattggtgat ggttcacttc aacttacagt gcaagaatta 1320
   ggattagcaa tcagagaaaa aattaatcca atttgcttta ttatcaataa tgatggttat 1380
   acagtcgaaa gagaaattca tggaccaaat caaagctaca atgatattcc aatgtggaat 1440
   tactcaaaat taccagaatc gtttggagca acagaagatc gagtagtctc aaaaatcgtt 1500
   agaactgaaa atgaatttgt gtctgtcatg aaagaagctc aagcagatcc aaatagaatg 1560
   tactggattg agttaatttt ggcaaaagaa ggtgcaccaa aagtactgaa aaaaatgggc 1620
   aaactatttg ctgaacaaaa taaatcataa 1650
<210> 77
   <211> 1047
   <212> DNA
   <213> Saccharomyces cerevisiae
<400> 77
   atgtctattc cagaaactca aaaagccatt atcttctacg aatccaacgg caagttggag 60
   cataaggata tcccagttcc aaagccaaag cccaacgaat tgttaatcaa cgtcaagtac 120
   tctggtgtct gccacaccga tttgcacgct tggcatggtg actggccatt gccaactaag 180
   ttaccattag ttggtggtca cgaaggtgcc ggtgtcgttg tcggcatggg tgaaaacgtt 240
   aagggctgga agatcggtga ctacgccggt atcaaatggt tgaacggttc ttgtatggcc 300
   tgtgaatact gtgaattggg taacgaatcc aactgtcctc acgctgactt gtctggttac 360
   acccacgacg gttctttcca agaatacgct accgctgacg ctgttcaagc cgctcacatt 420
   cctcaaggta ctgacttggc tgaagtcgcg ccaatcttgt gtgctggtat caccgtatac 480
   aaggctttga agtctgccaa cttgagagca ggccactggg cggccatttc tggtgctgct 540
   ggtggtctag gttctttggc tgttcaatat gctaaggcga tgggttacag agtcttaggt 600
   attgatggtg gtccaggaaa ggaagaattg tttacctcgc tcggtggtga agtattcatc 660
   gacttcacca aagagaagga cattgttagc gcagtcgtta aggctaccaa cggcggtgcc 720
   cacggtatca tcaatgtttc cgtttccgaa gccgctatcg aagcttctac cagatactgt 780
   agggcgaacg gtactgttgt cttggttggt ttgccagccg gtgcaaagtg ctcctctgat 840
   gtcttcaacc acgttgtcaa gtctatctcc attgtcggct cttacgtggg gaacagagct 900
   gataccagag aagccttaga tttctttgcc agaggtctag tcaagtctcc aataaaggta 960
   gttggcttat ccagtttacc agaaatttac gaaaagatgg agaagggcca aattgctggt 1020
   agatacgttg ttgacacttc taaataa 1047
<210> 78
   <211> 2744
   <212> DNA
   <213> Synthetic nucleic acid
<400> 78
   catatgtata cagtaggaga ttacctatta gaccgattac acgagttagg aattgaagaa    60
   atttttggag tccctggaga ctataactta caatttttag atcaaattat ttcccacaag   120
   gatatgaaat gggtcggaaa tgctaatgaa ttaaatgctt catatatggc tgatggctat   180
   gctcgtacta aaaaagctgc cgcatttctt acaacctttg gagtaggtga attgagtgca   240
   gttaatggat tagcaggaag ttacgccgaa aatttaccag tagtagaaat agtgggatca   300
   cctacatcaa aagttcaaaa tgaaggaaaa tttgttcatc atacgctggc tgacggtgat   360
   tttaaacact ttatgaaaat gcacgaacct gttacagcag ctcgaacttt actgacagca   420
   gaaaatgcaa ccgttgaaat tgaccgagta ctttctgcac tattaaaaga aagaaaacct   480
   gtctatatca acttaccagt tgatgttgct gctgcaaaag cagagaaacc ctcactccct   540
   ttgaaaaagg aaaactcaac ttcaaataca agtgaccaag aaattttgaa caaaattcaa   600
   gaaagcttga aaaatgccaa aaaaccaatc gtgattacag gacatgaaat aattagtttt   660
   ggcttagaaa aaacagtcac tcaatttatt tcaaagacaa aactacctat tacgacatta   720
   aactttggta aaagttcagt tgatgaagcc ctcccttcat ttttaggaat ctataatggt   780
   acactctcag agcctaatct taaagaattc gtggaatcag ccgacttcat cttgatgctt   840
   ggagttaaac tcacagactc ttcaacagga gccttcactc atcatttaaa tgaaaataaa   900
   atgatttcac tgaatataga tgaaggaaaa atatttaacg aaagaatcca aaattttgat   960
   tttgaatccc tcatctcctc tctcttagac ctaagcgaaa tagaatacaa aggaaaatat 1020
   atcgataaaa agcaagaaga ctttgttcca tcaaatgcgc ttttatcaca agaccgccta 1080
   tggcaagcag ttgaaaacct aactcaaagc aatgaaacaa tcgttgctga acaagggaca 1140
   tcattctttg gcgcttcatc aattttctta aaatcaaaga gtcattttat tggtcaaccc 1200
   ttatggggat caattggata tacattccca gcagcattag gaagccaaat tgcagataaa 1260
   gaaagcagac accttttatt tattggtgat ggttcacttc aacttacagt gcaagaatta 1320
   ggattagcaa tcagagaaaa aattaatcca atttgcttta ttatcaataa tgatggttat 1380
   acagtcgaaa gagaaattca tggaccaaat caaagctaca atgatattcc aatgtggaat 1440
   tactcaaaat taccagaatc gtttggagca acagaagatc gagtagtctc aaaaatcgtt 1500
   agaactgaaa atgaatttgt gtctgtcatg aaagaagctc aagcagatcc aaatagaatg 1560
   tactggattg agttaatttt ggcaaaagaa ggtgcaccaa aagtactgaa aaaaatgggc 1620
   aaactatttg ctgaacaaaa taaatcataa gaattcaaaa aaaaaaaaaa aaaaaaaaaa 1680
   aaaaaaaaaa aatgtctatt ccagaaactc aaaaagccat tatcttctac gaatccaacg 1740
   gcaagttgga gcataaggat atcccagttc caaagccaaa gcccaacgaa ttgttaatca 1800
   acgtcaagta ctctggtgtc tgccacaccg atttgcacgc ttggcatggt gactggccat 1860
   tgccaactaa gttaccatta gttggtggtc acgaaggtgc cggtgtcgtt gtcggcatgg 1920
   gtgaaaacgt taagggctgg aagatcggtg actacgccgg tatcaaatgg ttgaacggtt 1980
   cttgtatggc ctgtgaatac tgtgaattgg gtaacgaatc caactgtcct cacgctgact 2040
   tgtctggtta cacccacgac ggttctttcc aagaatacgc taccgctgac gctgttcaag 2100
   ccgctcacat tcctcaaggt actgacttgg ctgaagtcgc gccaatcttg tgtgctggta 2160
   tcaccgtata caaggctttg aagtctgcca acttgagagc aggccactgg gcggccattt 2220
   ctggtgctgc tggtggtcta ggttctttgg ctgttcaata tgctaaggcg atgggttaca 2280
   gagtcttagg tattgatggt ggtccaggaa aggaagaatt gtttacctcg ctcggtggtg 2340
   aagtattcat cgacttcacc aaagagaagg acattgttag cgcagtcgtt aaggctacca 2400
   acggcggtgc ccacggtatc atcaatgttt ccgtttccga agccgctatc gaagcttcta 2460
   ccagatactg tagggcgaac ggtactgttg tcttggttgg tttgccagcc ggtgcaaagt 2520
   gctcctctga tgtcttcaac cacgttgtca agtctatctc cattgtcggc tcttacgtgg 2580
   ggaacagagc tgataccaga gaagccttag atttctttgc cagaggtcta gtcaagtctc 2640
   caataaaggt agttggctta tccagtttac cagaaattta cgaaaagatg gagaagggcc 2700
   aaattgctgg tagatacgtt gttgacactt ctaaataagg tacc 2744
<210> 79
   <211> 479
   <212> DNA
   <213> Clostridium autoethanogenum
<400> 79
   aatatgatat ttatgtccat tgtgaaaggg attatattca actattattc cagttacgtt 60
   catagaaatt ttcctttcta aaatatttta ttccatgtca agaactctgt ttatttcatt 120
   aaagaactat aagtacaaag tataaggcat ttgaaaaaat aggctagtat attgattgat 180
   tatttatttt aaaatgccta agtgaaatat atacatatta taacaataaa ataagtatta 240
   gtgtaggatt tttaaataga gtatctattt tcagattaaa tttttgatta tttgatttac 300
   attatataat attgagtaaa gtattgacta gcaaaatttt ttgatacttt aatttgtgaa 360
   atttcttatc aaaagttata tttttgaata atttttattg aaaaatacaa ctaaaaagga 420
   ttatagtata agtgtgtgta attttgtgtt aaatttaaag ggaggaaatg aacatgaaa 479
<210> 80
   <211> 28
   <212> DNA
   <213> Synthetic primer
<400> 80
   gagcggccgc aatatgatat ttatgtcc 28
<210> 81
   <211> 28
   <212> DNA
   <213> Synthetic primer
<400> 81
   ttccatatgt ttcatgttca tttcctcc 28
<210> 82
   <211> 3552
   <212> DNA
   <213> synthetic nucleic acid
<400> 82
   aaactccttt ttgataatct catgaccaaa atcccttaac gtgagttttc gttccactga     60
   gcgtcagacc ccgtagaaaa gatcaaagga tcttcttgag atcctttttt tctgcgcgta    120
   atctgctgct tgcaaacaaa aaaaccaccg ctaccagcgg tggtttgttt gccggatcaa    180
   gagctaccaa ctctttttcc gaaggtaact ggcttcagca gagcgcagat accaaatact    240
   gttcttctag tgtagccgta gttaggccac cacttcaaga actctgtagc accgcctaca    300
   tacctcgctc tgctaatcct gttaccagtg gctgctgcca gtggcgataa gtcgtgtctt    360
   accgggttgg actcaagacg atagttaccg gataaggcgc agcggtcggg ctgaacgggg    420
   ggttcgtgca cacagcccag cttggagcga acgacctaca ccgaactgag atacctacag    480
   cgtgagctat gagaaagcgc cacgcttccc gaagggagaa aggcggacag gtatccggta    540
   agcggcaggg tcggaacagg agagcgcacg agggagcttc cagggggaaa cgcctggtat    600
   ctttatagtc ctgtcgggtt tcgccacctc tgacttgagc gtcgattttt gtgatgctcg    660
   tcaggggggc ggagcctatg gaaaaacgcc agcaacgcgg cctttttacg gttcctggcc    720
   ttttgctggc cttttgctca catgttcttt cctgcgttat cccctgattc tgtggataac    780
   cgtattaccg cctttgagtg agctgatacc gctcgccgca gccgaacgac cgagcgcagc    840
   gagtcagtga gcgaggaagc ggaagagcgc ccaatacgca gggccccctg caggataaaa    900
   aaattgtaga taaattttat aaaatagttt tatctacaat ttttttatca ggaaacagct    960
   atgaccgcgg ccgcaatatg atatttatgt ccattgtgaa agggattata ttcaactatt   1020
   attccagtta cgttcataga aattttcctt tctaaaatat tttattccat gtcaagaact   1080
   ctgtttattt cattaaagaa ctataagtac aaagtataag gcatttgaaa aaataggcta   1140
   gtatattgat tgattattta ttttaaaatg cctaagtgaa atatatacat attataacaa   1200
   taaaataagt attagtgtag gatttttaaa tagagtatct attttcagat taaatttttg   1260
   attatttgat ttacattata taatattgag taaagtattg actagcaaaa ttttttgata   1320
   ctttaatttg tgaaatttct tatcaaaagt tatatttttg aataattttt attgaaaaat   1380
   acaactaaaa aggattatag tataagtgtg tgtaattttg tgttaaattt aaagggagga   1440
   aatgaacatg aaacatatgg tgaccatgat tacgaattcg agctcggtac ccggggatcc   1500
   tctagagtcg acgtcacgcg tccatggaga tctcgaggcc tgcagacatg caagcttggc   1560
   actggccgtc gttttacaac gtcgtgactg ggaaaaccct ggcgttaccc aacttaatcg   1620
   ccttgcagca catccccctt tcgccagctg gcgtaatagc gaagaggccc gcaccgatcg   1680
   cccttcccaa cagttgcgca gcctgaatgg cgaatggcgc tagcataaaa ataagaagcc   1740
   tgcatttgca ggcttcttat ttttatggcg cgccgcattc acttcttttc tatataaata   1800
   tgagcgaagc gaataagcgt cggaaaagca gcaaaaagtt tcctttttgc tgttggagca   1860
   tgggggttca gggggtgcag tatctgacgt caatgccgag cgaaagcgag ccgaagggta   1920
   gcatttacgt tagataaccc cctgatatgc tccgacgctt tatatagaaa agaagattca   1980
   actaggtaaa atcttaatat aggttgagat gataaggttt ataaggaatt tgtttgttct   2040
   aatttttcac tcattttgtt ctaatttctt ttaacaaatg ttcttttttt tttagaacag   2100
   ttatgatata gttagaatag tttaaaataa ggagtgagaa aaagatgaaa gaaagatatg   2160
   gaacagtcta taaaggctct cagaggctca tagacgaaga aagtggagaa gtcatagagg   2220
   tagacaagtt ataccgtaaa caaacgtctg gtaacttcgt aaaggcatat atagtgcaat   2280
   taataagtat gttagatatg attggcggaa aaaaacttaa aatcgttaac tatatcctag   2340
   ataatgtcca cttaagtaac aatacaatga tagctacaac aagagaaata gcaaaagcta   2400
   caggaacaag tctacaaaca gtaataacaa cacttaaaat cttagaagaa ggaaatatta   2460
   taaaaagaaa aactggagta ttaatgttaa accctgaact actaatgaga ggcgacgacc   2520
   aaaaacaaaa atacctctta ctcgaatttg ggaactttga gcaagaggca aatgaaatag   2580
   attgacctcc caataacacc acgtagttat tgggaggtca atctatgaaa tgcgattaag   2640
   ggccggccga agcaaactta agagtgtgtt gatagtgcag tatcttaaaa ttttgtataa   2700
   taggaattga agttaaatta gatgctaaaa atttgtaatt aagaaggagt gattacatga   2760
   acaaaaatat aaaatattct caaaactttt taacgagtga aaaagtactc aaccaaataa   2820
   taaaacaatt gaatttaaaa gaaaccgata ccgtttacga aattggaaca ggtaaagggc   2880
   atttaacgac gaaactggct aaaataagta aacaggtaac gtctattgaa ttagacagtc 2940
   atctattcaa cttatcgtca gaaaaattaa aactgaatac tcgtgtcact ttaattcacc 3000
   aagatattct acagtttcaa ttccctaaca aacagaggta taaaattgtt gggagtattc 3060
   cttaccattt aagcacacaa attattaaaa aagtggtttt tgaaagccat gcgtctgaca 3120
   tctatctgat tgttgaagaa ggattctaca agcgtacctt ggatattcac cgaacactag 3180
   ggttgctctt gcacactcaa gtctcgattc agcaattgct taagctgcca gcggaatgct 3240
   ttcatcctaa accaaaagta aacagtgtct taataaaact tacccgccat accacagatg 3300
   ttccagataa atattggaag ctatatacgt actttgtttc aaaatgggtc aatcgagaat 3360
   atcgtcaact gtttactaaa aatcagtttc atcaagcaat gaaacacgcc aaagtaaaca 3420
   atttaagtac cgttacttat gagcaagtat tgtctatttt taatagttat ctattattta 3480
   acgggaggaa ataattctat gagtcgcttt tgtaaatttg gaaagttaca cgttactaaa 3540
   gggaatgtgt tt 3552
<210> 83
   <211> 6263
   <212> DNA
   <213> Synthetic nucleic acid
<400> 83
   aaactccttt ttgataatct catgaccaaa atcccttaac gtgagttttc gttccactga 60
   gcgtcagacc ccgtagaaaa gatcaaagga tcttcttgag atcctttttt tctgcgcgta 120
   atctgctgct tgcaaacaaa aaaaccaccg ctaccagcgg tggtttgttt gccggatcaa 180
   gagctaccaa ctctttttcc gaaggtaact ggcttcagca gagcgcagat accaaatact 240
   gttcttctag tgtagccgta gttaggccac cacttcaaga actctgtagc accgcctaca 300
   tacctcgctc tgctaatcct gttaccagtg gctgctgcca gtggcgataa gtcgtgtctt 360
   accgggttgg actcaagacg atagttaccg gataaggcgc agcggtcggg ctgaacgggg 420
   ggttcgtgca cacagcccag cttggagcga acgacctaca ccgaactgag atacctacag 480
   cgtgagctat gagaaagcgc cacgcttccc gaagggagaa aggcggacag gtatccggta 540
   agcggcaggg tcggaacagg agagcgcacg agggagcttc cagggggaaa cgcctggtat 600
   ctttatagtc ctgtcgggtt tcgccacctc tgacttgagc gtcgattttt gtgatgctcg 660
   tcaggggggc ggagcctatg gaaaaacgcc agcaacgcgg cctttttacg gttcctggcc 720
   ttttgctggc cttttgctca catgttcttt cctgcgttat cccctgattc tgtggataac 780
   cgtattaccg cctttgagtg agctgatacc gctcgccgca gccgaacgac cgagcgcagc 840
   gagtcagtga gcgaggaagc ggaagagcgc ccaatacgca gggccccctg caggataaaa 900
   aaattgtaga taaattttat aaaatagttt tatctacaat ttttttatca ggaaacagct 960
   atgaccgcgg ccgcaatatg atatttatgt ccattgtgaa agggattata ttcaactatt 1020
   attccagtta cgttcataga aattttcctt tctaaaatat tttattccat gtcaagaact 1080
   ctgtttattt cattaaagaa ctataagtac aaagtataag gcatttgaaa aaataggcta 1140
   gtatattgat tgattattta ttttaaaatg cctaagtgaa atatatacat attataacaa 1200
   taaaataagt attagtgtag gatttttaaa tagagtatct attttcagat taaatttttg 1260
   attatttgat ttacattata taatattgag taaagtattg actagcaaaa ttttttgata 1320
   ctttaatttg tgaaatttct tatcaaaagt tatatttttg aataattttt attgaaaaat 1380
   acaactaaaa aggattatag tataagtgtg tgtaattttg tgttaaattt aaagggagga 1440
   aatgaacatg aaacatatgt atacagttgg tgattattta cttgatagat tacatgaact 1500
   tggaatagaa gaaatttttg gtgtaccagg tgattacaat cttcaattct tagatcaaat 1560
   aatatcacat aaggatatga aatgggttgg taatgctaat gaattaaatg catcatatat 1620
   ggcagacgga tatgcaagaa ctaaaaaggc agcagcattt cttactacat ttggtgttgg 1680
   tgaattaagt gcagtaaatg gattagctgg aagttacgca gaaaacttac cagttgttga 1740
   aatagttgga tctcctacta gtaaagtaca aaatgaaggt aaatttgtac atcacactct 1800
   tgcagatggt gattttaagc attttatgaa aatgcatgaa cctgttacag ctgcaagaac 1860
   acttcttaca gctgaaaacg ctactgtaga aattgataga gttttatctg ctttacttaa 1920
   agaaagaaag ccagtatata ttaaccttcc agtagatgta gcagcagcaa aagctgagaa 1980
   accttcatta ccacttaaaa aggaaaattc aacatcaaat acatctgatc aagagatatt 2040
   aaataaaatt caggaaagtc ttaaaaatgc aaagaaacct atagtaataa ctggacatga 2100
   aataattagt tttggattag aaaagacagt tacacagttt ataagtaaaa ctaagcttcc 2160
   aattacaact ttaaattttg gaaagagttc agtagatgag gcacttccat cattcttagg 2220
   aatttataat ggaacattat ctgaacctaa tcttaaagaa tttgtagaga gtgctgattt 2280
   tatattaatg ttaggtgtaa aacttactga tagtagtact ggtgcattta ctcatcatct 2340
   taacgaaaat aagatgatat cattaaatat agacgaaggt aaaatattca atgaaagaat 2400
   acagaacttt gattttgaat cacttatatc atcattactt gatttatcag agatagaata 2460
   caaaggaaaa tatatagata aaaagcaaga agattttgtt ccatctaatg ctcttctttc 2520
   tcaagataga ctttggcaag cagttgagaa tcttacacag tctaatgaaa ctatagttgc 2580
   tgagcaagga acatcatttt tcggtgcatc aagtatattt ttaaaatcta aaagtcactt 2640
   tattggacaa cctctttggg gttctattgg atatactttt ccagcagctt taggaagtca 2700
   aatagctgat aaagaaagta gacatttatt atttattggt gacggttcac ttcagcttac 2760
   agtacaagaa ttaggattag ctataagaga gaagataaat cctatttgtt tcataataaa 2820
   caatgatgga tatactgtag aaagagaaat tcacggacca aatcagtcat ataatgatat 2880
   tccaatgtgg aattattcaa agttacctga atctttcggt gctactgaag atagagtagt 2940
   ttctaaaatt gttagaacag agaacgaatt tgtatctgtt atgaaagaag ctcaggctga 3000
   ccctaataga atgtattgga ttgaattaat tttagcaaaa gaaggtgctc ctaaagtact 3060
   taagaaaatg ggaaaattat ttgcagaaca aaataagtca taagaattcc cataataaag 3120
   aaagaatttt aaataaagga ggaacaaaga tgagtatacc agaaacacaa aaagcaatta 3180
   tattttatga gtcaaatgga aaattagagc ataaagatat acctgtacca aaaccaaaac 3240
   caaacgaact tcttataaat gttaagtatt ctggtgtttg tcatactgat cttcatgcat 3300
   ggcatggtga ttggcctctt ccaactaaat tacctcttgt aggtggtcat gaaggtgctg 3360
   gtgtagttgt aggtatgggt gaaaatgtta aaggttggaa aataggtgat tatgctggaa 3420
   ttaaatggct taatggatct tgtatggcat gcgagtattg tgaattagga aatgaaagta 3480
   attgtccaca tgctgactta agtggttata ctcatgatgg atcttttcaa gaatatgcta 3540
   ctgcagatgc agttcaggct gcacacattc cacagggaac tgatcttgct gaagtagctc 3600
   ctatattatg cgctggaatt acagtataca aagcattaaa aagtgctaat cttagagcag 3660
   gacactgggc agctataagt ggtgctgcag gtggtttagg atctttagca gttcaatatg 3720
   ctaaagctat gggatataga gtattaggaa tagacggtgg tccaggaaaa gaagagttat 3780
   ttacatcatt aggtggtgaa gtttttatag atttcacaaa ggaaaaagat attgtttcag 3840
   ctgtagtaaa ggcaactaat ggtggtgcac acggaattat aaatgtttca gtatctgaag 3900
   cagcaataga agcaagtact agatattgta gagcaaacgg aacagtagtt ttagttggac 3960
   ttccagctgg tgcaaagtgt tcatctgacg tatttaacca tgtagtaaag agtatttcaa 4020
   tagttggatc ttacgtaggt aatagagctg atacaagaga agctttagat ttctttgcaa 4080
   gaggtttagt taagagtcct ataaaagtag taggactttc atcacttcct gaaatttatg 4140
   aaaagatgga aaagggacaa atagctggta gatatgttgt agatacaagt aaataaggta 4200
   cccggggatc ctctagagtc gacgtcacgc gtccatggag atctcgaggc ctgcagacat 4260
   gcaagcttgg cactggccgt cgttttacaa cgtcgtgact gggaaaaccc tggcgttacc 4320
   caacttaatc gccttgcagc acatccccct ttcgccagct ggcgtaatag cgaagaggcc 4380
   cgcaccgatc gcccttccca acagttgcgc agcctgaatg gcgaatggcg ctagcataaa 4440
   aataagaagc ctgcatttgc aggcttctta tttttatggc gcgccgcatt cacttctttt 4500
   ctatataaat atgagcgaag cgaataagcg tcggaaaagc agcaaaaagt ttcctttttg 4560
   ctgttggagc atgggggttc agggggtgca gtatctgacg tcaatgccga gcgaaagcga 4620
   gccgaagggt agcatttacg ttagataacc ccctgatatg ctccgacgct ttatatagaa 4680
   aagaagattc aactaggtaa aatcttaata taggttgaga tgataaggtt tataaggaat 4740
   ttgtttgttc taatttttca ctcattttgt tctaatttct tttaacaaat gttctttttt 4800
   ttttagaaca gttatgatat agttagaata gtttaaaata aggagtgaga aaaagatgaa 4860
   agaaagatat ggaacagtct ataaaggctc tcagaggctc atagacgaag aaagtggaga 4920
   agtcatagag gtagacaagt tataccgtaa acaaacgtct ggtaacttcg taaaggcata 4980
   tatagtgcaa ttaataagta tgttagatat gattggcgga aaaaaactta aaatcgttaa 5040
   ctatatccta gataatgtcc acttaagtaa caatacaatg atagctacaa caagagaaat 5100
   agcaaaagct acaggaacaa gtctacaaac agtaataaca acacttaaaa tcttagaaga 5160
   aggaaatatt ataaaaagaa aaactggagt attaatgtta aaccctgaac tactaatgag 5220
   aggcgacgac caaaaacaaa aatacctctt actcgaattt gggaactttg agcaagaggc 5280
   aaatgaaata gattgacctc ccaataacac cacgtagtta ttgggaggtc aatctatgaa 5340
   atgcgattaa gggccggccg aagcaaactt aagagtgtgt tgatagtgca gtatcttaaa 5400
   attttgtata ataggaattg aagttaaatt agatgctaaa aatttgtaat taagaaggag 5460
   tgattacatg aacaaaaata taaaatattc tcaaaacttt ttaacgagtg aaaaagtact 5520
   caaccaaata ataaaacaat tgaatttaaa agaaaccgat accgtttacg aaattggaac 5580
   aggtaaaggg catttaacga cgaaactggc taaaataagt aaacaggtaa cgtctattga 5640
   attagacagt catctattca acttatcgtc agaaaaatta aaactgaata ctcgtgtcac 5700
   tttaattcac caagatattc tacagtttca attccctaac aaacagaggt ataaaattgt 5760
   tgggagtatt ccttaccatt taagcacaca aattattaaa aaagtggttt ttgaaagcca 5820
   tgcgtctgac atctatctga ttgttgaaga aggattctac aagcgtacct tggatattca 5880
   ccgaacacta gggttgctct tgcacactca agtctcgatt cagcaattgc ttaagctgcc 5940
   agcggaatgc tttcatccta aaccaaaagt aaacagtgtc ttaataaaac ttacccgcca 6000
   taccacagat gttccagata aatattggaa gctatatacg tactttgttt caaaatgggt 6060
   caatcgagaa tatcgtcaac tgtttactaa aaatcagttt catcaagcaa tgaaacacgc 6120
   caaagtaaac aatttaagta ccgttactta tgagcaagta ttgtctattt ttaatagtta 6180
   tctattattt aacgggagga aataattcta tgagtcgctt ttgtaaattt ggaaagttac 6240
   acgttactaa agggaatgtg ttt 6263
<210> 84
   <211> 4630
   <212> DNA
   <213> Synthetic nucleic acid
<400> 84
   aaactccttt ttgataatct catgaccaaa atcccttaac gtgagttttc gttccactga 60
   gcgtcagacc ccgtagaaaa gatcaaagga tcttcttgag atcctttttt tctgcgcgta 120
   atctgctgct tgcaaacaaa aaaaccaccg ctaccagcgg tggtttgttt gccggatcaa 180
   gagctaccaa ctctttttcc gaaggtaact ggcttcagca gagcgcagat accaaatact 240
   gttcttctag tgtagccgta gttaggccac cacttcaaga actctgtagc accgcctaca 300
   tacctcgctc tgctaatcct gttaccagtg gctgctgcca gtggcgataa gtcgtgtctt 360
   accgggttgg actcaagacg atagttaccg gataaggcgc agcggtcggg ctgaacgggg 420
   ggttcgtgca cacagcccag cttggagcga acgacctaca ccgaactgag atacctacag 480
   cgtgagctat gagaaagcgc cacgcttccc gaagggagaa aggcggacag gtatccggta 540
   agcggcaggg tcggaacagg agagcgcacg agggagcttc cagggggaaa cgcctggtat 600
   ctttatagtc ctgtcgggtt tcgccacctc tgacttgagc gtcgattttt gtgatgctcg 660
   tcaggggggc ggagcctatg gaaaaacgcc agcaacgcgg cctttttacg gttcctggcc 720
   ttttgctggc cttttgctca catgttcttt cctgcgttat cccctgattc tgtggataac     780
   cgtattaccg cctttgagtg agctgatacc gctcgccgca gccgaacgac cgagcgcagc     840
   gagtcagtga gcgaggaagc ggaagagcgc ccaatacgca gggccccctg caggataaaa     900
   aaattgtaga taaattttat aaaatagttt tatctacaat ttttttatca ggaaacagct     960
   atgaccgcgg ccgcaatatg atatttatgt ccattgtgaa agggattata ttcaactatt    1020
   attccagtta cgttcataga aattttcctt tctaaaatat tttattccat gtcaagaact    1080
   ctgtttattt cattaaagaa ctataagtac aaagtataag gcatttgaaa aaataggcta    1140
   gtatattgat tgattattta ttttaaaatg cctaagtgaa atatatacat attataacaa    1200
   taaaataagt attagtgtag gatttttaaa tagagtatct attttcagat taaatttttg    1260
   attatttgat ttacattata taatattgag taaagtattg actagcaaaa ttttttgata     1320
   ctttaatttg tgaaatttct tatcaaaagt tatatttttg aataattttt attgaaaaat     1380
   acaactaaaa aggattatag tataagtgtg tgtaattttg tgttaaattt aaagggagga    1440
   aatgaacatg aaacatatgt atacagttgg tgattattta cttgatagat tacatgaact    1500
   tggaatagaa gaaatttttg gtgtaccagg tgattacaat cttcaattct tagatcaaat     1560
   aatatcacat aaggatatga aatgggttgg taatgctaat gaattaaatg catcatatat    1620
   ggcagacgga tatgcaagaa ctaaaaaggc agcagcattt cttactacat ttggtgttgg    1680
   tgaattaagt gcagtaaatg gattagctgg aagttacgca gaaaacttac cagttgttga    1740
   aatagttgga tctcctacta gtaaagtaca aaatgaaggt aaatttgtac atcacactct    1800
   tgcagatggt gattttaagc attttatgaa aatgcatgaa cctgttacag ctgcaagaac    1860
   acttcttaca gctgaaaacg ctactgtaga aattgataga gttttatctg ctttacttaa    1920
   agaaagaaag ccagtatata ttaaccttcc agtagatgta gcagcagcaa aagctgagaa    1980
   accttcatta ccacttaaaa aggaaaattc aacatcaaat acatctgatc aagagatatt    2040
   aaataaaatt caggaaagtc ttaaaaatgc aaagaaacct atagtaataa ctggacatga    2100
   aataattagt tttggattag aaaagacagt tacacagttt ataagtaaaa ctaagcttcc    2160
   aattacaact ttaaattttg gaaagagttc agtagatgag gcacttccat cattcttagg    2220
   aatttataat ggaacattat ctgaacctaa tcttaaagaa tttgtagaga gtgctgattt    2280
   tatattaatg ttaggtgtaa aacttactga tagtagtact ggtgcattta ctcatcatct    2340
   taacgaaaat aagatgatat cattaaatat agacgaaggt aaaatattca atgaaagaat    2400
   acagaacttt gattttgaat cacttatatc atcattactt gatttatcag agatagaata    2460
   caaaggaaaa tatatagata aaaagcaaga agattttgtt ccatctaatg ctcttctttc    2520
   tcaagataga ctttggcaag cagttgagaa tcttacacag tctaatgaaa ctatagttgc    2580
   tgagcaagga acatcatttt tcggtgcatc aagtatattt ttaaaatcta aaagtcactt    2640
   tattggacaa cctctttggg gttctattgg atatactttt ccagcagctt taggaagtca    2700
   aatagctgat aaagaaagta gacatttatt atttattggt gacggttcac ttcagcttac    2760
   agtacaagaa ttaggattag ctataagaga gaagataaat cctatttgtt tcataataaa 2820
   caatgatgga tatactgtag aaagagaaat tcacggacca aatcagtcat ataatgatat 2880
   tccaatgtgg aattattcaa agttacctga atctttcggt gctactgaag atagagtagt 2940
   ttctaaaatt gttagaacag agaacgaatt tgtatctgtt atgaaagaag ctcaggctga 3000
   ccctaataga atgtattgga ttgaattaat tttagcaaaa gaaggtgctc ctaaagtact 3060
   taagaaaatg ggaaaattat ttgcagaaca aaataagtca taagaatttg tttgttctaa 3120
   tttttcactc attttgttct aatttctttt aacaaatgtt cttttttttt tagaacagtt 3180
   atgatatagt tagaatagtt taaaataagg agtgagaaaa agatgaaaga aagatatgga 3240
   acagtctata aaggctctca gaggctcata gacgaagaaa gtggagaagt catagaggta 3300
   gacaagttat accgtaaaca aacgtctggt aacttcgtaa aggcatatat agtgcaatta 3360
   ataagtatgt tagatatgat tggcggaaaa aaacttaaaa tcgttaacta tatcctagat 3420
   aatgtccact taagtaacaa tacaatgata gctacaacaa gagaaatagc aaaagctaca 3480
   ggaacaagtc tacaaacagt aataacaaca cttaaaatct tagaagaagg aaatattata 3540
   aaaagaaaaa ctggagtatt aatgttaaac cctgaactac taatgagagg cgacgaccaa 3600
   aaacaaaaat acctcttact cgaatttggg aactttgagc aagaggcaaa tgaaatagat 3660
   tgacctccca ataacaccac gtagttattg ggaggtcaat ctatgaaatg cgattaaggg 3720
   ccggccgaag caaacttaag agtgtgttga tagtgcagta tcttaaaatt ttgtataata 3780
   ggaattgaag ttaaattaga tgctaaaaat ttgtaattaa gaaggagtga ttacatgaac 3840
   aaaaatataa aatattctca aaacttttta acgagtgaaa aagtactcaa ccaaataata 3900
   aaacaattga atttaaaaga aaccgatacc gtttacgaaa ttggaacagg taaagggcat 3960
   ttaacgacga aactggctaa aataagtaaa caggtaacgt ctattgaatt agacagtcat 4020
   ctattcaact tatcgtcaga aaaattaaaa ctgaatactc gtgtcacttt aattcaccaa 4080
   gatattctac agtttcaatt ccctaacaaa cagaggtata aaattgttgg gagtattcct 4140
   taccatttaa gcacacaaat tattaaaaaa gtggtttttg aaagccatgc gtctgacatc 4200
   tatctgattg ttgaagaagg attctacaag cgtaccttgg atattcaccg aacactaggg 4260
   ttgctcttgc acactcaagt ctcgattcag caattgctta agctgccagc ggaatgcttt 4320
   catcctaaac caaaagtaaa cagtgtctta ataaaactta cccgccatac cacagatgtt 4380
   ccagataaat attggaagct atatacgtac tttgtttcaa aatgggtcaa tcgagaatat 4440
   cgtcaactgt ttactaaaaa tcagtttcat caagcaatga aacacgccaa agtaaacaat 4500
   ttaagtaccg ttacttatga gcaagtattg tctattttta atagttatct attatttaac 4560
   gggaggaaat aattctatga gtcgcttttg taaatttgga aagttacacg ttactaaagg 4620
   gaatgtgttt 4630
<210> 85
   <211> 23
   <212> DNA
   <213> Synthetic primer
<400> 85
   tcagttccct gtggaatgtg tgc 23
<210> 86
   <211> 22
   <212> DNA
   <213> Synthetic primer
<400> 86
   tcagtagcac cgaaagattc ag 22
<210> 87
   <211> 21
   <212> DNA
   <213> Synthetic primer
<400> 87
   agtgcctcat ctactgaact c 21
<210> 88
   <211> 36
   <212> DNA
   <213> Synthetic primer
<400> 88
   attagtttaa acacgccagc aacgcggcct ttttac 36
<210> 89
   <211> 27
   <212> DNA
   <213> Synthetic primer
<400> 89
   tcctattcca aggtttacga gttggtc 27
<210> 90
   <211> 27
   <212> DNA
   <213> Synthetic primer
<400> 90
   acccccaacc ataattgtca tgccatc 27
<210> 91
   <211> 27
   <212> DNA
   <213> Synthetic primer
<400> 91
   tgcaagagca aactcatctt gttcttc 27
<210> 92
   <211> 43
   <212> DNA
   <213> Synthetic primer
<400> 92
   agggtgcggc cgcgattcat atatccataa tctttaagtt atc 43
<210> 93
   <211> 38
   <212> DNA
   <213> Synthetic primer
<400> 93
   atcttctgca gggccgcaga tagtcataat agttccag 38
<210> 94
   <211> 43
   <212> DNA
   <213> Synthetic primer
<400> 94
   agggtgcggc cgcgattcat atatccataa tctttaagtt atc 43
<210> 95
   <211> 7655
   <212> DNA
   <213> Synthetic nucleic acid
<400> 95
   aaactccttt ttgataatct catgaccaaa atcccttaac gtgagttttc gttccactga      60
   gcgtcagacc ccgtagaaaa gatcaaagga tcttcttgag atcctttttt tctgcgcgta     120
   atctgctgct tgcaaacaaa aaaaccaccg ctaccagcgg tggtttgttt gccggatcaa     180
   gagctaccaa ctctttttcc gaaggtaact ggcttcagca gagcgcagat accaaatact     240
   gttcttctag tgtagccgta gttaggccac cacttcaaga actctgtagc accgcctaca     300
   tacctcgctc tgctaatcct gttaccagtg gctgctgcca gtggcgataa gtcgtgtctt     360
   accgggttgg actcaagacg atagttaccg gataaggcgc agcggtcggg ctgaacgggg     420
   ggttcgtgca cacagcccag cttggagcga acgacctaca ccgaactgag atacctacag     480
   cgtgagctat gagaaagcgc cacgcttccc gaagggagaa aggcggacag gtatccggta     540
   agcggcaggg tcggaacagg agagcgcacg agggagcttc cagggggaaa cgcctggtat     600
   ctttatagtc ctgtcgggtt tcgccacctc tgacttgagc gtcgattttt gtgatgctcg     660
   tcaggggggc ggagcctatg gaaaaacgcc agcaacgcgg cctttttacg gttcctggcc     720
   ttttgctggc cttttgctca catgttcttt cctgcgttat cccctgattc tgtggataac     780
   cgtattaccg cctttgagtg agctgatacc gctcgccgca gccgaacgac cgagcgcagc     840
   gagtcagtga gcgaggaagc ggaagagcgc ccaatacgca gggccccctg cagggccgca     900
   gatagtcata atagttccag aatagttcaa tttagaaatt agactaaact tcaaaatgtt     960
   tgttaaatat ataccaaact agtatagata ttttttaaat actggactta aacagtagta    1020
   atttgcctaa aaaatttttt caattttttt taaaaaatcc ttttcaagtt gtacattgtt    1080
   atggtaatat gtaattgaag aagttatgta gtaatattgt aaacgtttct tgattttttt    1140
   acatccatgt agtgcttaaa aaaccaaaat atgtcacatg caattgtata tttcaaataa    1200
   caatatttat tttctcgtta aattcacaaa taatttatta ataatatcaa taaccaagat    1260
   tatacttaaa tggatgttta ttttttaaca cttttatagt aaatatattt attttatgta    1320
   gtaaaaaggt tataattata attgtattta ttacaattaa ttaaaataaa aaatagggtt 1380
   ttaggtaaaa ttaagttatt ttaagaagta attacaataa aaattgaagt tatttcttta 1440
   aggagggaat tattcatatg aaagaagttg taatagctag tgcagtaaga acagcgattg 1500
   gatcttatgg aaagtctctt aaggatgtac cagcagtaga tttaggagct acagctataa 1560
   aggaagcagt taaaaaagca ggaataaaac cagaggatgt taatgaagtc attttaggaa 1620
   atgttcttca agcaggttta ggacagaatc cagcaagaca ggcatctttt aaagcaggat 1680
   taccagttga aattccagct atgactatta ataaggtttg tggttcagga cttagaacag 1740
   ttagcttagc agcacaaatt ataaaagcag gagatgctga cgtaataata gcaggtggta 1800
   tggaaaatat gtctagagct ccttacttag cgaataacgc tagatgggga tatagaatgg 1860
   gaaacgctaa atttgttgat gaaatgatca ctgacggatt gtgggatgca tttaatgatt 1920
   accacatggg aataacagca gaaaacatag ctgagagatg gaacatttca agagaagaac 1980
   aagatgagtt tgctcttgca tcacaaaaaa aagctgaaga agctataaaa tcaggtcaat 2040
   ttaaagatga aatagttcct gtagtaatta aaggcagaaa gggagaaact gtagttgata 2100
   cagatgagca ccctagattt ggatcaacta tagaaggact tgcaaaatta aaacctgcct 2160
   tcaaaaaaga tggaacagtt acagctggta atgcatcagg attaaatgac tgtgcagcag 2220
   tacttgtaat catgagtgca gaaaaagcta aagagcttgg agtaaaacca cttgctaaga 2280
   tagtttctta tggttcagca ggagttgacc cagcaataat gggatatgga cctttctatg 2340
   caacaaaagc agctattgaa aaagcaggtt ggacagttga tgaattagat ttaatagaat 2400
   caaatgaagc ttttgcagct caaagtttag cagtagcaaa agatttaaaa tttgatatga 2460
   ataaagtaaa tgtaaatgga ggagctattg cccttggtca tccaattgga gcatcaggtg 2520
   caagaatact cgttactctt gtacacgcaa tgcaaaaaag agatgcaaaa aaaggcttag 2580
   caactttatg tataggtggc ggacaaggaa cagcaatatt gctagaaaag tgctaggaat 2640
   tcgagctcgg taccagggag atattaaaat gaataaatta gtaaaattaa cagatttaaa 2700
   gcgcattttc aaagatggca tgacaattat ggttgggggt tttttagatt gtggaactcc 2760
   tgaaaatatt atagatatgc tagttgattt aaatataaaa aatctgacta ttataagcaa 2820
   tgatacagct tttcctaata aaggaatagg aaaacttatt gtaaatggtc aagtttctaa 2880
   agtaattgct tcacatattg gaactaatcc tgaaactgga aaaaaaatga gctctggaga 2940
   acttaaagtt gagctttccc cacaaggaac actgattgaa agaattcgtg cagctggatc 3000
   tggactcgga ggtgtattaa ctccaactgg acttggaact atcgttgaag aaggtaagaa 3060
   aaaagttact atcgatggca aagaatatct attagaactt cctttatctg ctgatgtttc 3120
   attaataaaa ggtagcattg tagatgaatt tggaaatacc ttctataggg ctgctactaa 3180
   aaatttcaat ccatatatgg caatggctgc aaaaacagtt atagttgaag cagaaaattt 3240
   agttaaatgt gaagatttaa aaagagatgc cataatgact cctggcgtat tagtagatta 3300
   tatcgttaag gaggcggctt aattgattgt agataaagtt ttagcaaaag agataattgc 3360
   caaaagagtt gcaaaagaac taaaaaaaga ccaactcgta aaccttggaa taggacttcc 3420
   aactttagta gcaaattatg taccaaaaga aatgaacatt acttttgaat cagaaaatgg 3480
   catggttggt atggcacaaa tggcatcatc aggtgaaaat gacccagata taataaatgc 3540
   tggcggggaa tatgtaacat tattacctca aggttcattt tttgatagtt caatgtcttt 3600
   cgcactaata cgaggaggac atgttgatgt tgctgttctt ggtgctctag aagttgatga 3660
   aaaaggtaat ttagctaact ggattgttcc aaataaaatt gtcccaggta tgggtggcgc 3720
   tatggattta gcaataggcg caaaaaaaat aatagtggca atgcaacata caggaaaaag 3780
   taaacctaaa atcgttaaaa aatgtactct cccacttact gctaaggctc aagtggattt 3840
   aattgtcaca gaactttgtg taattgatgt aacaaatgac ggcttacttt taaaagaaat 3900
   tcataaagat acaactattg atgaaattaa atttttaaca gatgcagatt taattattcc 3960
   agataactta aagattatgg atatatgaat cgcggccgca atatgatatt tatgtccatt 4020
   gtgaaaggga ttatattcaa ctattattcc agttacgttc atagaaattt tcctttctaa 4080
   aatattttat tccatgtcaa gaactctgtt tatttcatta aagaactata agtacaaagt 4140
   ataaggcatt tgaaaaaata ggctagtata ttgattgatt atttatttta aaatgcctaa 4200
   gtgaaatata tacatattat aacaataaaa taagtattag tgtaggattt ttaaatagag 4260
   tatctatttt cagattaaat ttttgattat ttgatttaca ttatataata ttgagtaaag 4320
   tattgactag caaaattttt tgatacttta atttgtgaaa tttcttatca aaagttatat 4380
   ttttgaataa tttttattga aaaatacaac taaaaaggat tatagtataa gtgtgtgtaa 4440
   ttttgtgtta aatttaaagg gaggaaatga acatgaaaca tatgtataca gttggtgatt 4500
   atttacttga tagattacat gaacttggaa tagaagaaat ttttggtgta ccaggtgatt 4560
   acaatcttca attcttagat caaataatat cacataagga tatgaaatgg gttggtaatg 4620
   ctaatgaatt aaatgcatca tatatggcag acggatatgc aagaactaaa aaggcagcag 4680
   catttcttac tacatttggt gttggtgaat taagtgcagt aaatggatta gctggaagtt 4740
   acgcagaaaa cttaccagtt gttgaaatag ttggatctcc tactagtaaa gtacaaaatg 4800
   aaggtaaatt tgtacatcac actcttgcag atggtgattt taagcatttt atgaaaatgc 4860
   atgaacctgt tacagctgca agaacacttc ttacagctga aaacgctact gtagaaattg 4920
   atagagtttt atctgcttta cttaaagaaa gaaagccagt atatattaac cttccagtag 4980
   atgtagcagc agcaaaagct gagaaacctt cattaccact taaaaaggaa aattcaacat 5040
   caaatacatc tgatcaagag atattaaata aaattcagga aagtcttaaa aatgcaaaga 5100
   aacctatagt aataactgga catgaaataa ttagttttgg attagaaaag acagttacac 5160
   agtttataag taaaactaag cttccaatta caactttaaa ttttggaaag agttcagtag 5220
   atgaggcact tccatcattc ttaggaattt ataatggaac attatctgaa cctaatctta 5280
   aagaatttgt agagagtgct gattttatat taatgttagg tgtaaaactt actgatagta 5340
   gtactggtgc atttactcat catcttaacg aaaataagat gatatcatta aatatagacg 5400
   aaggtaaaat attcaatgaa agaatacaga actttgattt tgaatcactt atatcatcat 5460
   tacttgattt atcagagata gaatacaaag gaaaatatat agataaaaag caagaagatt 5520
   ttgttccatc taatgctctt ctttctcaag atagactttg gcaagcagtt gagaatctta 5580
   cacagtctaa tgaaactata gttgctgagc aaggaacatc atttttcggt gcatcaagta 5640
   tatttttaaa atctaaaagt cactttattg gacaacctct ttggggttct attggatata 5700
   cttttccagc agctttagga agtcaaatag ctgataaaga aagtagacat ttattattta 5760
   ttggtgacgg ttcacttcag cttacagtac aagaattagg attagctata agagagaaga 5820
   taaatcctat ttgtttcata ataaacaatg atggatatac tgtagaaaga gaaattcacg 5880
   gaccaaatca gtcatataat gatattccaa tgtggaatta ttcaaagtta cctgaatctt 5940
   tcggtgctac tgaagataga gtagtttcta aaattgttag aacagagaac gaatttgtat 6000
   ctgttatgaa agaagctcag gctgacccta atagaatgta ttggattgaa ttaattttag 6060
   caaaagaagg tgctcctaaa gtacttaaga aaatgggaaa attatttgca gaacaaaata 6120
   agtcataaga atttgtttgt tctaattttt cactcatttt gttctaattt cttttaacaa 6180
   atgttctttt ttttttagaa cagttatgat atagttagaa tagtttaaaa taaggagtga 6240
   gaaaaagatg aaagaaagat atggaacagt ctataaaggc tctcagaggc tcatagacga 6300
   agaaagtgga gaagtcatag aggtagacaa gttataccgt aaacaaacgt ctggtaactt 6360
   cgtaaaggca tatatagtgc aattaataag tatgttagat atgattggcg gaaaaaaact 6420
   taaaatcgtt aactatatcc tagataatgt ccacttaagt aacaatacaa tgatagctac 6480
   aacaagagaa atagcaaaag ctacaggaac aagtctacaa acagtaataa caacacttaa 6540
   aatcttagaa gaaggaaata ttataaaaag aaaaactgga gtattaatgt taaaccctga 6600
   actactaatg agaggcgacg accaaaaaca aaaatacctc ttactcgaat ttgggaactt 6660
   tgagcaagag gcaaatgaaa tagattgacc tcccaataac accacgtagt tattgggagg 6720
   tcaatctatg aaatgcgatt aagggccggc cgaagcaaac ttaagagtgt gttgatagtg 6780
   cagtatctta aaattttgta taataggaat tgaagttaaa ttagatgcta aaaatttgta 6840
   attaagaagg agtgattaca tgaacaaaaa tataaaatat tctcaaaact ttttaacgag 6900
   tgaaaaagta ctcaaccaaa taataaaaca attgaattta aaagaaaccg ataccgttta 6960
   cgaaattgga acaggtaaag ggcatttaac gacgaaactg gctaaaataa gtaaacaggt 7020
   aacgtctatt gaattagaca gtcatctatt caacttatcg tcagaaaaat taaaactgaa 7080
   tactcgtgtc actttaattc accaagatat tctacagttt caattcccta acaaacagag 7140
   gtataaaatt gttgggagta ttccttacca tttaagcaca caaattatta aaaaagtggt 7200
   ttttgaaagc catgcgtctg acatctatct gattgttgaa gaaggattct acaagcgtac 7260
   cttggatatt caccgaacac tagggttgct cttgcacact caagtctcga ttcagcaatt 7320
   gcttaagctg ccagcggaat gctttcatcc taaaccaaaa gtaaacagtg tcttaataaa 7380
   acttacccgc cataccacag atgttccaga taaatattgg aagctatata cgtactttgt 7440
   ttcaaaatgg gtcaatcgag aatatcgtca actgtttact aaaaatcagt ttcatcaagc 7500
   aatgaaacac gccaaagtaa acaatttaag taccgttact tatgagcaag tattgtctat 7560
   ttttaatagt tatctattat ttaacgggag gaaataattc tatgagtcgc ttttgtaaat 7620
   ttggaaagtt acacgttact aaagggaatg tgttt 7655
<210> 96
   <211> 35
   <212> DNA
   <213> Synthetic primer
<400> 96
   acgttggatc caggaggaac aaagatgagt atacc 35
<210> 97
   <211> 39
   <212> DNA
   <213> Synthetic primer
<400> 97
   agcgtccatg gccttattta cttgtatcta caacatatc 39
<210> 98
   <211> 8621
   <212> DNA
   <213> Synthetic nucleic acid
<400> 98
   ccagtgggca agttgaaaaa ttcacaaaaa tgtggtataa tatctttgtt cattagagcg 60
   ataaacttga atttgagagg gaacttagat ggtatttgaa aaaattgata aaaatagttg 120
   gaacagaaaa gagtattttg accactactt tgcaagtgta ccttgtacct acagcatgac 180
   cgttaaagtg gatatcacac aaataaagga aaagggaatg aaactatatc ctgcaatgct 240
   ttattatatt gcaatgattg taaaccgcca ttcagagttt aggacggcaa tcaatcaaga 300
   tggtgaattg gggatatatg atgagatgat accaagctat acaatatttc acaatgatac 360
   tgaaacattt tccagccttt ggactgagtg taagtctgac tttaaatcat ttttagcaga 420
   ttatgaaagt gatacgcaac ggtatggaaa caatcataga atggaaggaa agccaaatgc 480
   tccggaaaac atttttaatg tatctatgat accgtggtca accttcgatg gctttaatct 540
   gaatttgcag aaaggatatg attatttgat tcctattttt actatgggga aatattataa 600
   agaagataac aaaattatac ttcctttggc aattcaagtt catcacgcag tatgtgacgg 660
   atttcacatt tgccgttttg taaacgaatt gcaggaattg ataaatagtt aacttcaggt 720
   ttgtctgtaa ctaaaaacaa gtatttaagc aaaaacatcg tagaaatacg gtgttttttg 780
   ttaccctaag tttaaactcc tttttgataa tctcatgacc aaaatccctt aacgtgagtt 840
   ttcgttccac tgagcgtcag accccgtaga aaagatcaaa ggatcttctt gagatccttt 900
   ttttctgcgc gtaatctgct gcttgcaaac aaaaaaacca ccgctaccag cggtggtttg 960
   tttgccggat caagagctac caactctttt tccgaaggta actggcttca gcagagcgca 1020
   gataccaaat actgttcttc tagtgtagcc gtagttaggc caccacttca agaactctgt 1080
   agcaccgcct acatacctcg ctctgctaat cctgttacca gtggctgctg ccagtggcga 1140
   taagtcgtgt cttaccgggt tggactcaag acgatagtta ccggataagg cgcagcggtc 1200
   gggctgaacg gggggttcgt gcacacagcc cagcttggag cgaacgacct acaccgaact 1260
   gagataccta cagcgtgagc tatgagaaag cgccacgctt cccgaaggga gaaaggcgga 1320
   caggtatccg gtaagcggca gggtcggaac aggagagcgc acgagggagc ttccaggggg 1380
   aaacgcctgg tatctttata gtcctgtcgg gtttcgccac ctctgacttg agcgtcgatt 1440
   tttgtgatgc tcgtcagggg ggcggagcct atggaaaaac gccagcaacg cggccttttt 1500
   acggttcctg gccttttgct ggccttttgc tcacatgttc tttcctgcgt tatcccctga 1560
   ttctgtggat aaccgtatta ccgcctttga gtgagctgat accgctcgcc gcagccgaac 1620
   gaccgagcgc agcgagtcag tgagcgagga agcggaagag cgcccaatac gcagggcccc 1680
   ctgcaggata aaaaaattgt agataaattt tataaaatag ttttatctac aattttttta 1740
   tcaggaaaca gctatgaccg cggccgcaga tagtcataat agttccagaa tagttcaatt 1800
   tagaaattag actaaacttc aaaatgtttg ttaaatatat accaaactag tatagatatt 1860
   ttttaaatac tggacttaaa cagtagtaat ttgcctaaaa aattttttca atttttttta 1920
   aaaaatcctt ttcaagttgt acattgttat ggtaatatgt aattgaagaa gttatgtagt 1980
   aatattgtaa acgtttcttg atttttttac atccatgtag tgcttaaaaa accaaaatat 2040
   gtcacatgca attgtatatt tcaaataaca atatttattt tctcgttaaa ttcacaaata 2100
   atttattaat aatatcaata accaagatta tacttaaatg gatgtttatt ttttaacact 2160
   tttatagtaa atatatttat tttatgtagt aaaaaggtta taattataat tgtatttatt 2220
   acaattaatt aaaataaaaa atagggtttt aggtaaaatt aagttatttt aagaagtaat 2280
   tacaataaaa attgaagtta tttctttaag gagggaatta ttcatatgaa agaagttgta 2340
   atagctagtg cagtaagaac agcgattgga tcttatggaa agtctcttaa ggatgtacca 2400
   gcagtagatt taggagctac agctataaag gaagcagtta aaaaagcagg aataaaacca 2460
   gaggatgtta atgaagtcat tttaggaaat gttcttcaag caggtttagg acagaatcca 2520
   gcaagacagg catcttttaa agcaggatta ccagttgaaa ttccagctat gactattaat 2580
   aaggtttgtg gttcaggact tagaacagtt agcttagcag cacaaattat aaaagcagga 2640
   gatgctgacg taataatagc aggtggtatg gaaaatatgt ctagagctcc ttacttagcg 2700
   aataacgcta gatggggata tagaatggga aacgctaaat ttgttgatga aatgatcact 2760
   gacggattgt gggatgcatt taatgattac cacatgggaa taacagcaga aaacatagct 2820
   gagagatgga acatttcaag agaagaacaa gatgagtttg ctcttgcatc acaaaaaaaa 2880
   gctgaagaag ctataaaatc aggtcaattt aaagatgaaa tagttcctgt agtaattaaa 2940
   ggcagaaagg gagaaactgt agttgataca gatgagcacc ctagatttgg atcaactata 3000
   gaaggacttg caaaattaaa acctgccttc aaaaaagatg gaacagttac agctggtaat 3060
   gcatcaggat taaatgactg tgcagcagta cttgtaatca tgagtgcaga aaaagctaaa 3120
   gagcttggag taaaaccact tgctaagata gtttcttatg gttcagcagg agttgaccca 3180
   gcaataatgg gatatggacc tttctatgca acaaaagcag ctattgaaaa agcaggttgg 3240
   acagttgatg aattagattt aatagaatca aatgaagctt ttgcagctca aagtttagca 3300
   gtagcaaaag atttaaaatt tgatatgaat aaagtaaatg taaatggagg agctattgcc 3360
   cttggtcatc caattggagc atcaggtgca agaatactcg ttactcttgt acacgcaatg 3420
   caaaaaagag atgcaaaaaa aggcttagca actttatgta taggtggcgg acaaggaaca 3480
   gcaatattgc tagaaaagtg ctaggaattc gagctcggta ccagggagat attaaaatga 3540
   ataaattagt aaaattaaca gatttaaagc gcattttcaa agatggcatg acaattatgg 3600
   ttgggggttt tttagattgt ggaactcctg aaaatattat agatatgcta gttgatttaa 3660
   atataaaaaa tctgactatt ataagcaatg atacagcttt tcctaataaa ggaataggaa 3720
   aacttattgt aaatggtcaa gtttctaaag taattgcttc acatattgga actaatcctg 3780
   aaactggaaa aaaaatgagc tctggagaac ttaaagttga gctttcccca caaggaacac 3840
   tgattgaaag aattcgtgca gctggatctg gactcggagg tgtattaact ccaactggac 3900
   ttggaactat cgttgaagaa ggtaagaaaa aagttactat cgatggcaaa gaatatctat 3960
   tagaacttcc tttatctgct gatgtttcat taataaaagg tagcattgta gatgaatttg 4020
   gaaatacctt ctatagggct gctactaaaa atttcaatcc atatatggca atggctgcaa 4080
   aaacagttat agttgaagca gaaaatttag ttaaatgtga agatttaaaa agagatgcca 4140
   taatgactcc tggcgtatta gtagattata tcgttaagga ggcggcttaa ttgattgtag 4200
   ataaagtttt agcaaaagag ataattgcca aaagagttgc aaaagaacta aaaaaagacc 4260
   aactcgtaaa ccttggaata ggacttccaa ctttagtagc aaattatgta ccaaaagaaa 4320
   tgaacattac ttttgaatca gaaaatggca tggttggtat ggcacaaatg gcatcatcag 4380
   gtgaaaatga cccagatata ataaatgctg gcggggaata tgtaacatta ttacctcaag 4440
   gttcattttt tgatagttca atgtctttcg cactaatacg aggaggacat gttgatgttg 4500
   ctgttcttgg tgctctagaa gttgatgaaa aaggtaattt agctaactgg attgttccaa 4560
   ataaaattgt cccaggtatg ggtggcgcta tggatttagc aataggcgca aaaaaaataa 4620
   tagtggcaat gcaacataca ggaaaaagta aacctaaaat cgttaaaaaa tgtactctcc 4680
   cacttactgc taaggctcaa gtggatttaa ttgtcacaga actttgtgta attgatgtaa 4740
   caaatgacgg cttactttta aaagaaattc ataaagatac aactattgat gaaattaaat 4800
   ttttaacaga tgcagattta attattccag ataacttaaa gattatggat atatgaatca 4860
   ttctatttta aatatataac tttaaaaatc ttatgtatta aaaactaaga aaagaggttg 4920
   attgttttat gttagaaagt gaagtatcta aacaaattac aactccactt gctgctccag 4980
   cgtttcctag aggaccatat aggtttcaca atagagaata tctaaacatt atttatcgaa 5040
   ctgatttaga tgctcttcga aaaatagtac cagagccact tgaattagat agagcatatg 5100
   ttagatttga aatgatggct atgcctgata caaccggact aggctcatat acagaatgtg 5160
   gtcaagctat tccagtaaaa tataatggtg ttaagggtga ctacttgcat atgatgtatc 5220
   tagataatga acctgctatt gctgttggaa gagaaagtag cgcttatcca aaaaagcttg 5280
   gctatccaaa gctatttgtt gattcagata ctttagttgg gacacttaaa tatggtacat 5340
   taccagtagc tactgcaaca atgggatata agcacgagcc tctagatctt aaagaagcct 5400
   atgctcaaat tgcaagaccc aattttatgc taaaaatcat tcaaggttac gatggtaagc 5460
   caagaatttg tgaactaata tgtgcagaaa atactgatat aactattcac ggtgcttgga 5520
   ctggaagtgc acgtctacaa ttatttagcc atgcactagc tcctcttgct gatttacctg 5580
   tattagagat tgtatcagca tctcatatcc tcacagattt aactcttgga acacctaagg 5640
   ttgtacatga ttatctttca gtaaaataaa agcaatatag aggatccagg aggaacaaag 5700
   atgagtatac cagaaacaca aaaagcaatt atattttatg agtcaaatgg aaaattagag 5760
   cataaagata tacctgtacc aaaaccaaaa ccaaacgaac ttcttataaa tgttaagtat 5820
   tctggtgttt gtcatactga tcttcatgca tggcatggtg attggcctct tccaactaaa 5880
   ttacctcttg taggtggtca tgaaggtgct ggtgtagttg taggtatggg tgaaaatgtt 5940
   aaaggttgga aaataggtga ttatgctgga attaaatggc ttaatggatc ttgtatggca 6000
   tgcgagtatt gtgaattagg aaatgaaagt aattgtccac atgctgactt aagtggttat 6060
   actcatgatg gatcttttca agaatatgct actgcagatg cagttcaggc tgcacacatt 6120
   ccacagggaa ctgatcttgc tgaagtagct cctatattat gcgctggaat tacagtatac 6180
   aaagcattaa aaagtgctaa tcttagagca ggacactggg cagctataag tggtgctgca 6240
   ggtggtttag gatctttagc agttcaatat gctaaagcta tgggatatag agtattagga 6300
   atagacggtg gtccaggaaa agaagagtta tttacatcat taggtggtga agtttttata 6360
   gatttcacaa aggaaaaaga tattgtttca gctgtagtaa aggcaactaa tggtggtgca 6420
   cacggaatta taaatgtttc agtatctgaa gcagcaatag aagcaagtac tagatattgt 6480
   agagcaaacg gaacagtagt tttagttgga cttccagctg gtgcaaagtg ttcatctgac 6540
   gtatttaacc atgtagtaaa gagtatttca atagttggat cttacgtagg taatagagct 6600
   gatacaagag aagctttaga tttctttgca agaggtttag ttaagagtcc tataaaagta 6660
   gtaggacttt catcacttcc tgaaatttat gaaaagatgg aaaagggaca aatagctggt 6720
   agatatgttg tagatacaag taaataaggc catggagatc tcgaggcctg cagacatgca 6780
   agcttggcac tggccgtcgt tttacaacgt cgtgactggg aaaaccctgg cgttacccaa 6840
   cttaatcgcc ttgcagcaca tccccctttc gccagctggc gtaatagcga agaggcccgc 6900
   accgatcgcc cttcccaaca gttgcgcagc ctgaatggcg aatggcgcta gcataaaaat 6960
   aagaagcctg catttgcagg cttcttattt ttatggcgcg ccgccattat ttttttgaac 7020
   aattgacaat tcatttctta ttttttatta agtgatagtc aaaaggcata acagtgctga 7080
   atagaaagaa atttacagaa aagaaaatta tagaatttag tatgattaat tatactcatt 7140
   tatgaatgtt taattgaata caaaaaaaaa tacttgttat gtattcaatt acgggttaaa 7200
   atatagacaa gttgaaaaat ttaataaaaa aataagtcct cagctcttat atattaagct 7260
   accaacttag tatataagcc aaaacttaaa tgtgctacca acacatcaag ccgttagaga 7320
   actctatcta tagcaatatt tcaaatgtac cgacatacaa gagaaacatt aactatatat 7380
   attcaattta tgagattatc ttaacagata taaatgtaaa ttgcaataag taagatttag 7440
   aagtttatag cctttgtgta ttggaagcag tacgcaaagg cttttttatt tgataaaaat 7500
   tagaagtata tttatttttt cataattaat ttatgaaaat gaaagggggt gagcaaagtg 7560
   acagaggaaa gcagtatctt atcaaataac aaggtattag caatatcatt attgacttta 7620
   gcagtaaaca ttatgacttt tatagtgctt gtagctaagt agtacgaaag ggggagcttt 7680
   aaaaagctcc ttggaataca tagaattcat aaattaattt atgaaaagaa gggcgtatat 7740
   gaaaacttgt aaaaattgca aagagtttat taaagatact gaaatatgca aaatacattc 7800
   gttgatgatt catgataaaa cagtagcaac ctattgcagt aaatacaatg agtcaagatg 7860
   tttacataaa gggaaagtcc aatgtattaa ttgttcaaag atgaaccgat atggatggtg 7920
   tgccataaaa atgagatgtt ttacagagga agaacagaaa aaagaacgta catgcattaa 7980
   atattatgca aggagcttta aaaaagctca tgtaaagaag agtaaaaaga aaaaataatt 8040
   tatttattaa tttaatattg agagtgccga cacagtatgc actaaaaaat atatctgtgg 8100
   tgtagtgagc cgatacaaaa ggatagtcac tcgcattttc ataatacatc ttatgttatg 8160
   attatgtgtc ggtgggactt cacgacgaaa acccacaata aaaaaagagt tcggggtagg 8220
   gttaagcata gttgaggcaa ctaaacaatc aagctaggat atgcagtagc agaccgtaag 8280
   gtcgttgttt aggtgtgttg taatacatac gctattaaga tgtaaaaata cggataccaa 8340
   tgaagggaaa agtataattt ttggatgtag tttgtttgtt catctatggg caaactacgt 8400
   ccaaagccgt ttccaaatct gctaaaaagt atatcctttc taaaatcaaa gtcaagtatg 8460
   aaatcataaa taaagtttaa ttttgaagtt attatgatat tatgtttttc tattaaaata 8520
   aattaagtat atagaatagt ttaataatag tatatactta atgtgataag tgtctgacag 8580
   tgtcacagaa aggatgattg ttatggatta taagcggccg g 8621
<210> 99
   <211> 37
   <212> DNA
   <213> Synthetic primer
<400> 99
   atattggatc cacagctatg accgcggccg caatatg 37
<210> 100
   <211> 39
   <212> DNA
   <213> Synthetic primer
<400> 100
   agcgtccatg gccttattta cttgtatcta caacatatc 39
<210> 101
   <211> 10803
   <212> DNA
   <213> Synthetic nucleic acid
<400> 101
   ccagtgggca agttgaaaaa ttcacaaaaa tgtggtataa tatctttgtt cattagagcg 60
   ataaacttga atttgagagg gaacttagat ggtatttgaa aaaattgata aaaatagttg 120
   gaacagaaaa gagtattttg accactactt tgcaagtgta ccttgtacct acagcatgac 180
   cgttaaagtg gatatcacac aaataaagga aaagggaatg aaactatatc ctgcaatgct 240
   ttattatatt gcaatgattg taaaccgcca ttcagagttt aggacggcaa tcaatcaaga 300
   tggtgaattg gggatatatg atgagatgat accaagctat acaatatttc acaatgatac 360
   tgaaacattt tccagccttt ggactgagtg taagtctgac tttaaatcat ttttagcaga 420
   ttatgaaagt gatacgcaac ggtatggaaa caatcataga atggaaggaa agccaaatgc 480
   tccggaaaac atttttaatg tatctatgat accgtggtca accttcgatg gctttaatct 540
   gaatttgcag aaaggatatg attatttgat tcctattttt actatgggga aatattataa 600
   agaagataac aaaattatac ttcctttggc aattcaagtt catcacgcag tatgtgacgg 660
   atttcacatt tgccgttttg taaacgaatt gcaggaattg ataaatagtt aacttcaggt 720
   ttgtctgtaa ctaaaaacaa gtatttaagc aaaaacatcg tagaaatacg gtgttttttg 780
   ttaccctaag tttaaactcc tttttgataa tctcatgacc aaaatccctt aacgtgagtt 840
   ttcgttccac tgagcgtcag accccgtaga aaagatcaaa ggatcttctt gagatccttt 900
   ttttctgcgc gtaatctgct gcttgcaaac aaaaaaacca ccgctaccag cggtggtttg 960
   tttgccggat caagagctac caactctttt tccgaaggta actggcttca gcagagcgca 1020
   gataccaaat actgttcttc tagtgtagcc gtagttaggc caccacttca agaactctgt 1080
   agcaccgcct acatacctcg ctctgctaat cctgttacca gtggctgctg ccagtggcga 1140
   taagtcgtgt cttaccgggt tggactcaag acgatagtta ccggataagg cgcagcggtc 1200
   gggctgaacg gggggttcgt gcacacagcc cagcttggag cgaacgacct acaccgaact 1260
   gagataccta cagcgtgagc tatgagaaag cgccacgctt cccgaaggga gaaaggcgga 1320
   caggtatccg gtaagcggca gggtcggaac aggagagcgc acgagggagc ttccaggggg 1380
   aaacgcctgg tatctttata gtcctgtcgg gtttcgccac ctctgacttg agcgtcgatt 1440
   tttgtgatgc tcgtcagggg ggcggagcct atggaaaaac gccagcaacg cggccttttt 1500
   acggttcctg gccttttgct ggccttttgc tcacatgttc tttcctgcgt tatcccctga 1560
   ttctgtggat aaccgtatta ccgcctttga gtgagctgat accgctcgcc gcagccgaac 1620
   gaccgagcgc agcgagtcag tgagcgagga agcggaagag cgcccaatac gcagggcccc 1680
   ctgcaggata aaaaaattgt agataaattt tataaaatag ttttatctac aattttttta 1740
   tcaggaaaca gctatgaccg cggccgcaga tagtcataat agttccagaa tagttcaatt 1800
   tagaaattag actaaacttc aaaatgtttg ttaaatatat accaaactag tatagatatt 1860
   ttttaaatac tggacttaaa cagtagtaat ttgcctaaaa aattttttca atttttttta 1920
   aaaaatcctt ttcaagttgt acattgttat ggtaatatgt aattgaagaa gttatgtagt 1980
   aatattgtaa acgtttcttg atttttttac atccatgtag tgcttaaaaa accaaaatat 2040
   gtcacatgca attgtatatt tcaaataaca atatttattt tctcgttaaa ttcacaaata 2100
   atttattaat aatatcaata accaagatta tacttaaatg gatgtttatt ttttaacact 2160
   tttatagtaa atatatttat tttatgtagt aaaaaggtta taattataat tgtatttatt 2220
   acaattaatt aaaataaaaa atagggtttt aggtaaaatt aagttatttt aagaagtaat 2280
   tacaataaaa attgaagtta tttctttaag gagggaatta ttcatatgaa agaagttgta 2340
   atagctagtg cagtaagaac agcgattgga tcttatggaa agtctcttaa ggatgtacca 2400
   gcagtagatt taggagctac agctataaag gaagcagtta aaaaagcagg aataaaacca 2460
   gaggatgtta atgaagtcat tttaggaaat gttcttcaag caggtttagg acagaatcca 2520
   gcaagacagg catcttttaa agcaggatta ccagttgaaa ttccagctat gactattaat 2580
   aaggtttgtg gttcaggact tagaacagtt agcttagcag cacaaattat aaaagcagga 2640
   gatgctgacg taataatagc aggtggtatg gaaaatatgt ctagagctcc ttacttagcg 2700
   aataacgcta gatggggata tagaatggga aacgctaaat ttgttgatga aatgatcact 2760
   gacggattgt gggatgcatt taatgattac cacatgggaa taacagcaga aaacatagct 2820
   gagagatgga acatttcaag agaagaacaa gatgagtttg ctcttgcatc acaaaaaaaa 2880
   gctgaagaag ctataaaatc aggtcaattt aaagatgaaa tagttcctgt agtaattaaa 2940
   ggcagaaagg gagaaactgt agttgataca gatgagcacc ctagatttgg atcaactata 3000
   gaaggacttg caaaattaaa acctgccttc aaaaaagatg gaacagttac agctggtaat 3060
   gcatcaggat taaatgactg tgcagcagta cttgtaatca tgagtgcaga aaaagctaaa 3120
   gagcttggag taaaaccact tgctaagata gtttcttatg gttcagcagg agttgaccca 3180
   gcaataatgg gatatggacc tttctatgca acaaaagcag ctattgaaaa agcaggttgg 3240
   acagttgatg aattagattt aatagaatca aatgaagctt ttgcagctca aagtttagca 3300
   gtagcaaaag atttaaaatt tgatatgaat aaagtaaatg taaatggagg agctattgcc 3360
   cttggtcatc caattggagc atcaggtgca agaatactcg ttactcttgt acacgcaatg 3420
   caaaaaagag atgcaaaaaa aggcttagca actttatgta taggtggcgg acaaggaaca 3480
   gcaatattgc tagaaaagtg ctaggaattc gagctcggta ccagggagat attaaaatga 3540
   ataaattagt aaaattaaca gatttaaagc gcattttcaa agatggcatg acaattatgg 3600
   ttgggggttt tttagattgt ggaactcctg aaaatattat agatatgcta gttgatttaa 3660
   atataaaaaa tctgactatt ataagcaatg atacagcttt tcctaataaa ggaataggaa 3720
   aacttattgt aaatggtcaa gtttctaaag taattgcttc acatattgga actaatcctg 3780
   aaactggaaa aaaaatgagc tctggagaac ttaaagttga gctttcccca caaggaacac 3840
   tgattgaaag aattcgtgca gctggatctg gactcggagg tgtattaact ccaactggac 3900
   ttggaactat cgttgaagaa ggtaagaaaa aagttactat cgatggcaaa gaatatctat    3960
   tagaacttcc tttatctgct gatgtttcat taataaaagg tagcattgta gatgaatttg    4020
   gaaatacctt ctatagggct gctactaaaa atttcaatcc atatatggca atggctgcaa    4080
   aaacagttat agttgaagca gaaaatttag ttaaatgtga agatttaaaa agagatgcca    4140
   taatgactcc tggcgtatta gtagattata tcgttaagga ggcggcttaa ttgattgtag    4200
   ataaagtttt agcaaaagag ataattgcca aaagagttgc aaaagaacta aaaaaagacc    4260
   aactcgtaaa ccttggaata ggacttccaa ctttagtagc aaattatgta ccaaaagaaa    4320
   tgaacattac ttttgaatca gaaaatggca tggttggtat ggcacaaatg gcatcatcag    4380
   gtgaaaatga cccagatata ataaatgctg gcggggaata tgtaacatta ttacctcaag    4440
   gttcattttt tgatagttca atgtctttcg cactaatacg aggaggacat gttgatgttg    4500
   ctgttcttgg tgctctagaa gttgatgaaa aaggtaattt agctaactgg attgttccaa    4560
   ataaaattgt cccaggtatg ggtggcgcta tggatttagc aataggcgca aaaaaaataa    4620
   tagtggcaat gcaacataca ggaaaaagta aacctaaaat cgttaaaaaa tgtactctcc    4680
   cacttactgc taaggctcaa gtggatttaa ttgtcacaga actttgtgta attgatgtaa    4740
   caaatgacgg cttactttta aaagaaattc ataaagatac aactattgat gaaattaaat    4800
   ttttaacaga tgcagattta attattccag ataacttaaa gattatggat atatgaatca    4860
   ttctatttta aatatataac tttaaaaatc ttatgtatta aaaactaaga aaagaggttg    4920
   attgttttat gttagaaagt gaagtatcta aacaaattac aactccactt gctgctccag    4980
   cgtttcctag aggaccatat aggtttcaca atagagaata tctaaacatt atttatcgaa    5040
   ctgatttaga tgctcttcga aaaatagtac cagagccact tgaattagat agagcatatg    5100
   ttagatttga aatgatggct atgcctgata caaccggact aggctcatat acagaatgtg    5160
   gtcaagctat tccagtaaaa tataatggtg ttaagggtga ctacttgcat atgatgtatc    5220
   tagataatga acctgctatt gctgttggaa gagaaagtag cgcttatcca aaaaagcttg    5280
   gctatccaaa gctatttgtt gattcagata ctttagttgg gacacttaaa tatggtacat    5340
   taccagtagc tactgcaaca atgggatata agcacgagcc tctagatctt aaagaagcct    5400
   atgctcaaat tgcaagaccc aattttatgc taaaaatcat tcaaggttac gatggtaagc    5460
   caagaatttg tgaactaata tgtgcagaaa atactgatat aactattcac ggtgcttgga    5520
   ctggaagtgc acgtctacaa ttatttagcc atgcactagc tcctcttgct gatttacctg    5580
   tattagagat tgtatcagca tctcatatcc tcacagattt aactcttgga acacctaagg    5640
   ttgtacatga ttatctttca gtaaaataaa agcaatatag aggatccaca gctatgaccg    5700
   cggccgcaat atgatattta tgtccattgt gaaagggatt atattcaact attattccag    5760
   ttacgttcat agaaattttc ctttctaaaa tattttattc catgtcaaga actctgttta    5820
   tttcattaaa gaactataag tacaaagtat aaggcatttg aaaaaatagg ctagtatatt    5880
   gattgattat ttattttaaa atgcctaagt gaaatatata catattataa caataaaata    5940
   agtattagtg taggattttt aaatagagta tctattttca gattaaattt ttgattattt 6000
   gatttacatt atataatatt gagtaaagta ttgactagca aaattttttg atactttaat 6060
   ttgtgaaatt tcttatcaaa agttatattt ttgaataatt tttattgaaa aatacaacta 6120
   aaaaggatta tagtataagt gtgtgtaatt ttgtgttaaa tttaaaggga ggaaatgaac 6180
   atgaaacata tgtatacagt tggtgattat ttacttgata gattacatga acttggaata 6240
   gaagaaattt ttggtgtacc aggtgattac aatcttcaat tcttagatca aataatatca 6300
   cataaggata tgaaatgggt tggtaatgct aatgaattaa atgcatcata tatggcagac 6360
   ggatatgcaa gaactaaaaa ggcagcagca tttcttacta catttggtgt tggtgaatta 6420
   agtgcagtaa atggattagc tggaagttac gcagaaaact taccagttgt tgaaatagtt 6480
   ggatctccta ctagtaaagt acaaaatgaa ggtaaatttg tacatcacac tcttgcagat 6540
   ggtgatttta agcattttat gaaaatgcat gaacctgtta cagctgcaag aacacttctt 6600
   acagctgaaa acgctactgt agaaattgat agagttttat ctgctttact taaagaaaga 6660
   aagccagtat atattaacct tccagtagat gtagcagcag caaaagctga gaaaccttca 6720
   ttaccactta aaaaggaaaa ttcaacatca aatacatctg atcaagagat attaaataaa 6780
   attcaggaaa gtcttaaaaa tgcaaagaaa cctatagtaa taactggaca tgaaataatt 6840
   agttttggat tagaaaagac agttacacag tttataagta aaactaagct tccaattaca 6900
   actttaaatt ttggaaagag ttcagtagat gaggcacttc catcattctt aggaatttat 6960
   aatggaacat tatctgaacc taatcttaaa gaatttgtag agagtgctga ttttatatta 7020
   atgttaggtg taaaacttac tgatagtagt actggtgcat ttactcatca tcttaacgaa 7080
   aataagatga tatcattaaa tatagacgaa ggtaaaatat tcaatgaaag aatacagaac 7140
   tttgattttg aatcacttat atcatcatta cttgatttat cagagataga atacaaagga 7200
   aaatatatag ataaaaagca agaagatttt gttccatcta atgctcttct ttctcaagat 7260
   agactttggc aagcagttga gaatcttaca cagtctaatg aaactatagt tgctgagcaa 7320
   ggaacatcat ttttcggtgc atcaagtata tttttaaaat ctaaaagtca ctttattgga 7380
   caacctcttt ggggttctat tggatatact tttccagcag ctttaggaag tcaaatagct 7440
   gataaagaaa gtagacattt attatttatt ggtgacggtt cacttcagct tacagtacaa 7500
   gaattaggat tagctataag agagaagata aatcctattt gtttcataat aaacaatgat 7560
   ggatatactg tagaaagaga aattcacgga ccaaatcagt catataatga tattccaatg 7620
   tggaattatt caaagttacc tgaatctttc ggtgctactg aagatagagt agtttctaaa 7680
   attgttagaa cagagaacga atttgtatct gttatgaaag aagctcaggc tgaccctaat 7740
   agaatgtatt ggattgaatt aattttagca aaagaaggtg ctcctaaagt acttaagaaa 7800
   atgggaaaat tatttgcaga acaaaataag tcataagaat tcccataata aagaaagaat 7860
   tttaaataaa ggaggaacaa agatgagtat accagaaaca caaaaagcaa ttatatttta 7920
   tgagtcaaat ggaaaattag agcataaaga tatacctgta ccaaaaccaa aaccaaacga 7980
   acttcttata aatgttaagt attctggtgt ttgtcatact gatcttcatg catggcatgg 8040
   tgattggcct cttccaacta aattacctct tgtaggtggt catgaaggtg ctggtgtagt 8100
   tgtaggtatg ggtgaaaatg ttaaaggttg gaaaataggt gattatgctg gaattaaatg 8160
   gcttaatgga tcttgtatgg catgcgagta ttgtgaatta ggaaatgaaa gtaattgtcc 8220
   acatgctgac ttaagtggtt atactcatga tggatctttt caagaatatg ctactgcaga 8280
   tgcagttcag gctgcacaca ttccacaggg aactgatctt gctgaagtag ctcctatatt 8340
   atgcgctgga attacagtat acaaagcatt aaaaagtgct aatcttagag caggacactg 8400
   ggcagctata agtggtgctg caggtggttt aggatcttta gcagttcaat atgctaaagc 8460
   tatgggatat agagtattag gaatagacgg tggtccagga aaagaagagt tatttacatc 8520
   attaggtggt gaagttttta tagatttcac aaaggaaaaa gatattgttt cagctgtagt 8580
   aaaggcaact aatggtggtg cacacggaat tataaatgtt tcagtatctg aagcagcaat 8640
   agaagcaagt actagatatt gtagagcaaa cggaacagta gttttagttg gacttccagc 8700
   tggtgcaaag tgttcatctg acgtatttaa ccatgtagta aagagtattt caatagttgg 8760
   atcttacgta ggtaatagag ctgatacaag agaagcttta gatttctttg caagaggttt 8820
   agttaagagt cctataaaag tagtaggact ttcatcactt cctgaaattt atgaaaagat 8880
   ggaaaaggga caaatagctg gtagatatgt tgtagataca agtaaataag gccatggaga 8940
   tctcgaggcc tgcagacatg caagcttggc actggccgtc gttttacaac gtcgtgactg 9000
   ggaaaaccct ggcgttaccc aacttaatcg ccttgcagca catccccctt tcgccagctg 9060
   gcgtaatagc gaagaggccc gcaccgatcg cccttcccaa cagttgcgca gcctgaatgg 9120
   cgaatggcgc tagcataaaa ataagaagcc tgcatttgca ggcttcttat ttttatggcg 9180
   cgccgccatt atttttttga acaattgaca attcatttct tattttttat taagtgatag 9240
   tcaaaaggca taacagtgct gaatagaaag aaatttacag aaaagaaaat tatagaattt 9300
   agtatgatta attatactca tttatgaatg tttaattgaa tacaaaaaaa aatacttgtt 9360
   atgtattcaa ttacgggtta aaatatagac aagttgaaaa atttaataaa aaaataagtc 9420
   ctcagctctt atatattaag ctaccaactt agtatataag ccaaaactta aatgtgctac 9480
   caacacatca agccgttaga gaactctatc tatagcaata tttcaaatgt accgacatac 9540
   aagagaaaca ttaactatat atattcaatt tatgagatta tcttaacaga tataaatgta 9600
   aattgcaata agtaagattt agaagtttat agcctttgtg tattggaagc agtacgcaaa 9660
   ggctttttta tttgataaaa attagaagta tatttatttt ttcataatta atttatgaaa 9720
   atgaaagggg gtgagcaaag tgacagagga aagcagtatc ttatcaaata acaaggtatt 9780
   agcaatatca ttattgactt tagcagtaaa cattatgact tttatagtgc ttgtagctaa 9840
   gtagtacgaa agggggagct ttaaaaagct ccttggaata catagaattc ataaattaat 9900
   ttatgaaaag aagggcgtat atgaaaactt gtaaaaattg caaagagttt attaaagata 9960
   ctgaaatatg caaaatacat tcgttgatga ttcatgataa aacagtagca acctattgca 10020
   gtaaatacaa tgagtcaaga tgtttacata aagggaaagt ccaatgtatt aattgttcaa    10080
   agatgaaccg atatggatgg tgtgccataa aaatgagatg ttttacagag gaagaacaga    10140
   aaaaagaacg tacatgcatt aaatattatg caaggagctt taaaaaagct catgtaaaga    10200
   agagtaaaaa gaaaaaataa tttatttatt aatttaatat tgagagtgcc gacacagtat    10260
   gcactaaaaa atatatctgt ggtgtagtga gccgatacaa aaggatagtc actcgcattt    10320
   tcataataca tcttatgtta tgattatgtg tcggtgggac ttcacgacga aaacccacaa    10380
   taaaaaaaga gttcggggta gggttaagca tagttgaggc aactaaacaa tcaagctagg    10440
   atatgcagta gcagaccgta aggtcgttgt ttaggtgtgt tgtaatacat acgctattaa    10500
   gatgtaaaaa tacggatacc aatgaaggga aaagtataat ttttggatgt agtttgtttg    10560
   ttcatctatg ggcaaactac gtccaaagcc gtttccaaat ctgctaaaaa gtatatcctt    10620
   tctaaaatca aagtcaagta tgaaatcata aataaagttt aattttgaag ttattatgat    10680
   attatgtttt tctattaaaa taaattaagt atatagaata gtttaataat agtatatact    10740
   taatgtgata agtgtctgac agtgtcacag aaaggatgat tgttatggat tataagcggc    10800
   cgg                                                                   10803

## Description

### FIELD

The present invention relates to methods for the production of acetone, isopropanol and/or a precursor of acetone and/or isopropanol by microbial fermentation of substrates comprising carbon monoxide and genetically modified micro-organisms of use in such methods.

### BACKGROUND

Some microorganisms such as *Clostridium acetobutylicum* or *Clostridium beijerinckii* are known to produce acetone or isopropanol as major by-products during butanol fermentation (ABE or IBE fermentation) [George HA, Johnson JL, Moore WEC, Holdeman LV, Chen JS: Acetone, isopropanol, and butanol production by Clostridium beijerinckii (syn. Clostridium butylicum) and Clostridium aurantibutyricum. Appl Environ Microbiol 45: 1160-1163]. However, all these organisms rely on sugar or starch based substrates. Acetogenic organisms such as the closely related microorganisms *Clostridium autoethanogenum, C. Ijungdahlii, and C. ragsdalei* are able to grow chemoautotrophically on CO or CO₂/H₂ containing gases as sole energy and carbon source and synthesize products such as acetate,ethanol, or 2,3-butanediol, but neither acetone nor isopropanol [Munasinghe PC, Khanal SK: Biomass-derived syngas fermentation into biofuels: Opportunities and challenges. Bioresource Technol 2010, 5013-22].

Recently, production of isopropanol was reported in a study on *Clostridium ragsdalei (Clostridium* strain P11) in a 100-L pilot scale fermentor from switchgrass derived syngas [Kundiyana DK, Huhnke RL, Wilkins MR: Syngas fermentation in a 100-L pilot scale fermentor: Design and process considerations. J Biosci Bioeng 2010, 109: 492-498]. However, a related study from the same lab showed that this was due to a contamination in the used syngas since it was passed through a scrubbing mixture containing 20 % acetone [Ramachandriya KD: Effect of biomass generated producer gas, methane and physical parameters on producer gas fermentations by Clostridium strain P11. Masters thesis, Oklahoma State University 2009]. The authors also noted that the production of isopropanol may be the result of reduction of propionic acid rather than acetone. Experiments carried out by the inventors of the present invention with *Clostridium ragsdalei* (*Clostridium* strain P11) and also *C*. *autoethanogenum* and *C*. *Ijungdahlii* have never shown the production of acetone, isopropanol, or propionic acid.

The cost of many carbohydrate feed stocks suitable for the production of chemical products such as acetone and isoproanol is influenced by their value as human food or animal feed, and the cultivation of starch or sucrose-producing crops for such production is not economically sustainable in all geographies. Therefore, it is of interest to develop technologies to convert lower cost and/or more abundant carbon resources into useful chemical products such as acetone and isopropanol.

CO is a major free energy-rich by-product of the incomplete combustion of organic materials such as coal or oil and oil derived products. For example, the steel industry in Australia is reported to produce and release into the atmosphere over 500,000 tonnes of CO annually.

WO 2010/121849 A1 discloses cells and methods for the producing acetone. This document also makes reference to: a document summarising the microbial production of acetone, butanol and isopropanol (Biotechnology 6:229-268); developments in clostridial acetone, butanol and isopropanol fermentation (Appl Microbiol Biotechnol .49: 639-648); and specific Clostridium Ijungdahlii strains PETC, ERI-2 and O-52 (WO 98/00558 and WO 00/68407).

It is an object of the invention to overcome one or more of the disadvantages of the prior art, or to at least to provide the public with a useful choice.

### SUMMARY OF INVENTION

The invention generally provides methods for the production of isopropanol by microbial fermentation of substrates comprising CO.

The scope of the invention is defined by the claims.

In a first aspect, the invention provides a method for the production of isopropanol by microbial fermentation, comprising fermenting a substrate comprising CO using a recombinant carboxydotrophic acetogenic microorganism capable of producing isopropanol by fermentation of CO,
wherein the microorganism expresses the enzymes: acetyl-coenzyme A acetyltransferase (thiolase; ThIA; E.C. 2.3.1.9); acetoacetyl-CoA:acetate coenzyme A transferase A (CoA transferase; CtfA; EC 2.8.3.9); and acetoacetyl-CoA:acetate coenzyme A transferase B (CoA transferase; CtfB; EC 2.8.3.9); and
wherein the microorganism further expresses the enzyme:
a. acetoacetate decarboxylase (Adc; EC 4.1.1.4); or
b. alpha-ketoisovalerate decarboxylase (decarboxylase; KivD; EC4.1.1.74);
wherein the enzymes are not naturally present in a parental microorganism from which the recombinant microorganism is derived.

In a second aspect, the invention provides the method of part (a) of the first aspect, wherein the microorganism further expresses the enzyme alcohol dehydrogenase (Adh; EC 1.1.1.2).

In a third aspect, the invention provides the method of the second aspect, wherein Adh is exogenously expressed.

In a fourth aspect, the invention provides the method of part (a) of the first aspect, wherein the microorganism further expresses the enzyme alcohol dehydrogenase (Adh2; EC 1.1.1.1).

In a fifth aspect, the invention provides the method of the fourth aspect, wherein Adh2 is exogenously expressed.

In a sixth aspect, the invention provides the method of part (a) of the first aspect, wherein the microorganism further expresses the enzymes alpha-ketoisovalerate decarboxylase (decarboxylase; KivD; EC4.1.1.74) and alcohol dehydrogenase (Adh2; EC 1.1.1.1).

In a seventh aspect, the invention provides the method of the sixth aspect, wherein Adh2 is exogenously expressed.

In an eighth aspect, the invention provides the method of the first aspect, wherein the parental microorganism is selected from the group of carboxydotrophic acetogenic bacteria comprising: *Clostridium autoethanogenum, Clostridium Ijungdahlii, Clostridium ragsdalei, Clostridium carboxidivorans, Clostridium drakei, Clostridium scatologenes, Butyribacterium limosum, Butyribacterium methylotrophicum, Acetobacterium woodii, Alkalibaculum bacchii, Blautia producta, Eubacterium limosum, Moorella thermoacetica, Moorella thermautotrophica, Oxobacter pfennigii,* and *Thermoanaerobacter kiuvi.*

In a ninth aspect, the invention provides the method of the sixth aspect, wherein the parental microorganism is Clostridium autoethanogenum DSM23693 or Clostridium Ijungdahlii DSM13528 (or ATCC55383).

In a tenth aspect, the invention provides the method of any one of the previous aspect, wherein the enzyme:
a. ThIA is encoded by a nucleotide comprising the sequence of SEQ ID NO. 18; and
b. CtfA is encoded by a nucleotide comprising the sequence of SEQ ID NO. 19; and
c. CtfB is encoded by a nucleotide comprising the sequence of SEQ ID NO. 20 and/or
d. Adc is encoded by a nucleotide comprising the sequence of SEQ ID NO. 21; and/or
e. KivD is encoded by a nucleotide comprising the sequence of SEQ ID NO. 71; and/or
f. Adh is encoded by a nucleotide comprising the sequence of SEQ ID NO. 2 3 or 4; and/or
g. Adh2 is encoded by a nucleotide comprising the sequence of SEQ ID NO. 75.

In an eleventh aspect, the invention provides the method of any one of the previous aspects, wherein:
a. the gaseous substrate comprising CO is produced by an industrial process selected from the group consisting of ferrous metal products manufacturing, non-ferrous products manufacturing, petroleum refining processes, gasification of coal, gasification of biomass, electric power production, carbon black production, and coke manufacturing; or
b. the gaseous substrate comprises 20% to 70% CO by volume.

The invention may also be said broadly to consist in the parts, elements and features referred to or indicated in the specification of the application, individually or collectively, in any or all combinations of two or more of said parts, elements or features.

### BRIEF DESCRIPTION OF THE FIGURES

These and other aspects of the present invention, which should be considered in all its novel aspects, will become apparent from the following description, which is given by way of example only, with reference to the accompanying figures, in which:
Fig. 1 shows amino acid alignment of novel alcohol dehydrogenase of *C*. *autoethanogenum* (CAU), *C. ljungdahlii* (CU), and *C. ragsdalei* (CRA) with the secondary alcohol dehydrogenase of *C. beijerinckii* strain NRRL-B593.
Fig. 2 show expression of novel alcohol dehydrogenase gene of *Clostridium autoethanogenum* DSM23693 during a typical fermentation run, as well as expression of genes controlled by Wood-Ljungdahl operon promoter, F₁F_{O} ATPase operon promoter, Rnf complex operon promoter, and Pyruvate:ferredoxin oxidoreductase promoter. mRNA levels of more than 200 genes of interest were compared.
Fig. 3 shows acetone expression plasmid pMTL85147-thlA-ctfA-ctfB-adc.
Fig. 4 shows the pathway for production of acetone and isopropanol from CO or CO/H₂ containing gases in engineered *C. autoethanogenum* and *C. ljungdahlii* carrying plasmid pMTL85147-thlA-ctfA-ctfB-adc.
Fig. 5 shows the sequencing results of acetone expression plasmid pMTL85147-thlA-ctfA-ctfB-adc.
Fig. 6 illustrates the designed methylation plasmid.
Fig. 7 shows detection of *ctfAB-adc* (2.2kb) from PCR of plasmids isolated from transformed C. *autoethanogenum* DSM23693 and *C. ljungdahlii* DSM13528. Ladder = 1 KB Plus DNA ladder (Invitrogen); 1= non-template control; 2= plasmid isolated from *C. autoethanogenum*; 3= plasmid isolated from *C. ljungdahlii*; 4= original pMTL85147-thlA-ctfAB-adc (positive control).
Fig. 8 shows the result of growth experiments with *C. autoethanogenum* DSM23693 + pMTL85147-thlA-ctfAB-adc on steel mill gas.
Fig. 9 shows the result of growth experiments with *C. ljungdahlii* DSM13528 + pMTL85147-thlA-ctfAB-adc on steel mill gas.
Fig. 10 shows the GC result confirming acetone production with *C. autoethanogenum* DSM13528 + pMTL85147-thlA-ctfAB-adc (top) and *C. ljungdahlii* DSM13528 + pMTL85147-thlA-ctfAB-adc (bottom) from steel mill gas.
Fig. 11 shows the GC result confirming acetone production with *C. autoethanogenum* DSM23693 + pMTL85147-thlA-ctfAB-adc from syngas.
Fig. 12 shows the toxicity of acetone on cultures of *C*. *autoethanogenum* DSM23693.
Fig. 13 shows the toxicity of isopropanol on cultures of *C*. *autoethanogenum* DSM23693.

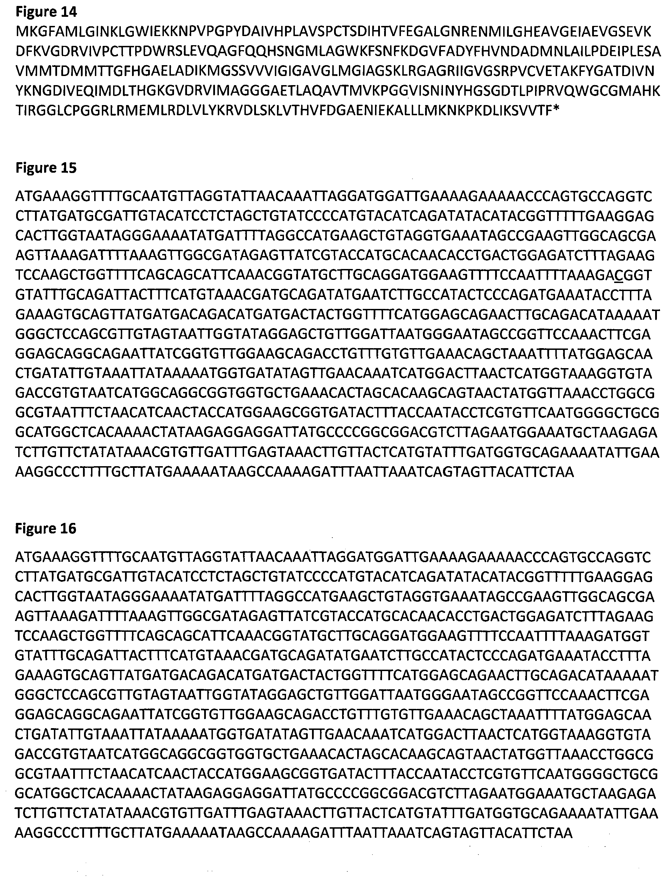

Fig. 14 shows SEQ_ID NO 1: Amino acid sequence of novel alcohol dehydrogenase from *C. autoethanogeum, C. ljungdahlii* and *C. ragsdalei.*
Fig. 15 shows SEQ_ID NO 2: Nucleic acid sequence of novel alcohol dehydrogenase gene from *C. autoethanogeum.*
Fig. 16 shows SEQ_ID NO 3: Nucleic acid sequence of novel alcohol dehydrogenase gene from *C. ljungdahlii.*

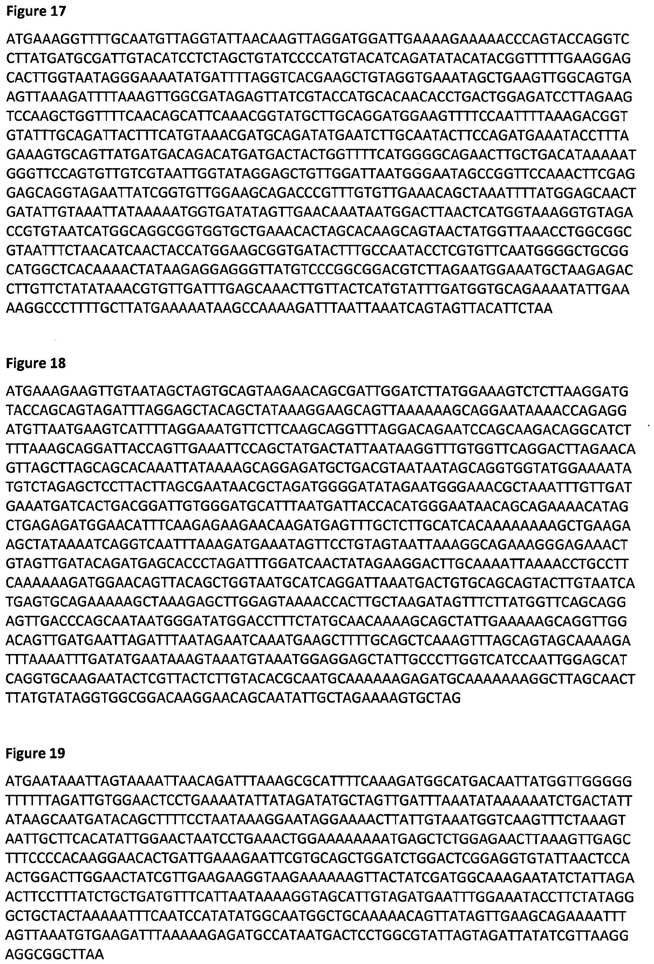

Fig. 17 shows SEQ_ID NO 4: Nucleic acid sequence of novel alcohol dehydrogenase gene from *C. ragsdalei.*
Fig. 18 shows SEQ_ID NO 18: Nucleic acid sequence of Thiolase gene (*thIA*) from *C. acetobutylicum* ATCC824.
Fig. 19 shows SEQ_ID NO 19: Nucleic acid sequence of Acetoacetyl-CoA:acetate Coenzyme A transferase A (*ctfA*) gene from *C. beijerinckii* NCIMB8052.

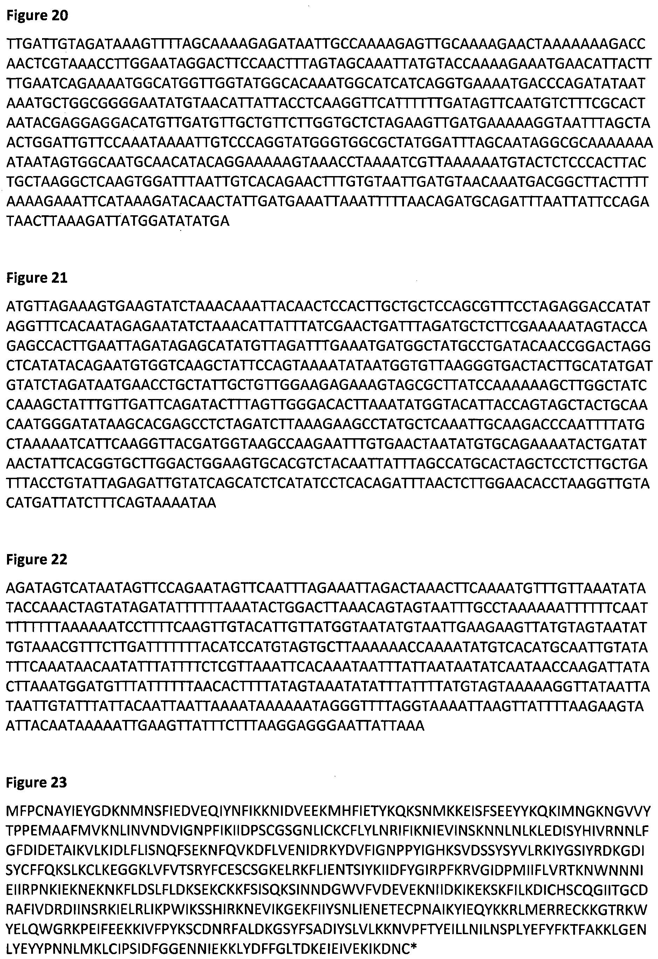

Fig. 20 shows SEQ_ID NO 20: Nucleic acid sequence of Acetoacetyl-CoA:acetate Coenzyme A transferase B (*ctfB*) gene from *C. beijerinckii* NCIMB8052.
Fig. 21 shows SEQ_ID NO 21: Nucleic acid sequence of Acetoacetate decarboxylase (adc) gene from *C. beijerinckii* NCIMB8052.
Fig. 22 shows SEQ_ID NO 22: Nucleic acid sequence of Wood-Ljungdahl cluster promoter (P_{WL}) from *C*. *autoethanogenum.*
Fig. 23 shows SEQ_ID NO 34: Amino acid sequence of designed Type II methyltransferase gene.

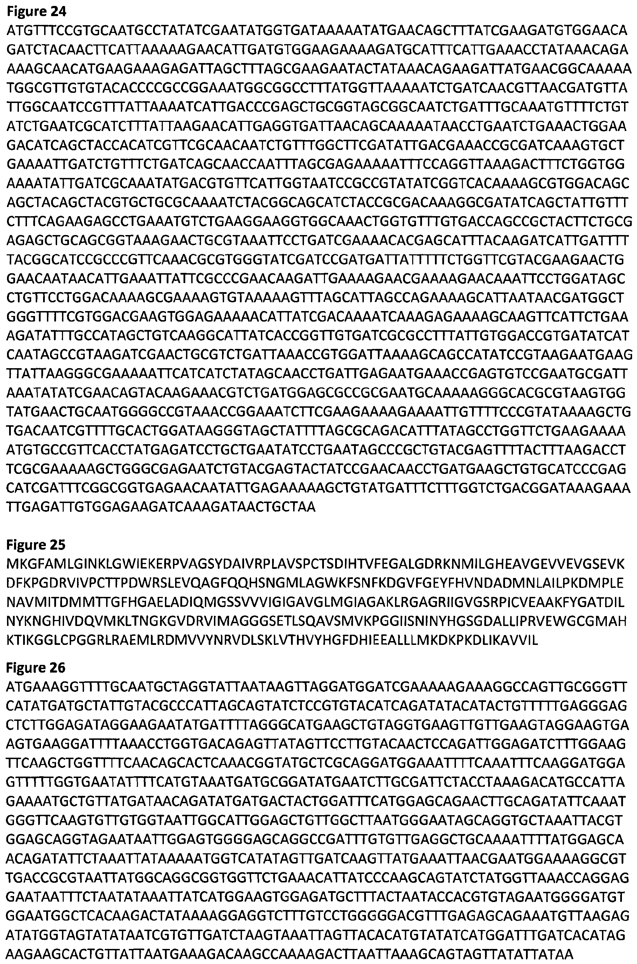

Fig. 24 shows SEQ_ID NO 35: Nucleic acid sequence of designed Type II methyltransferase gene.
Fig. 25 shows SEQ_ID NO 38: Amino acid sequence of NADP-dependent alcohol dehydrogenase from *Clostridium beijerinckii* NRRL B-593.
Fig. 26 shows SEQ_ID NO 39: Nucleic acid sequence of NADP-dependent alcohol dehydrogenase from *Clostridium beijerinckii* NRRL B-593.

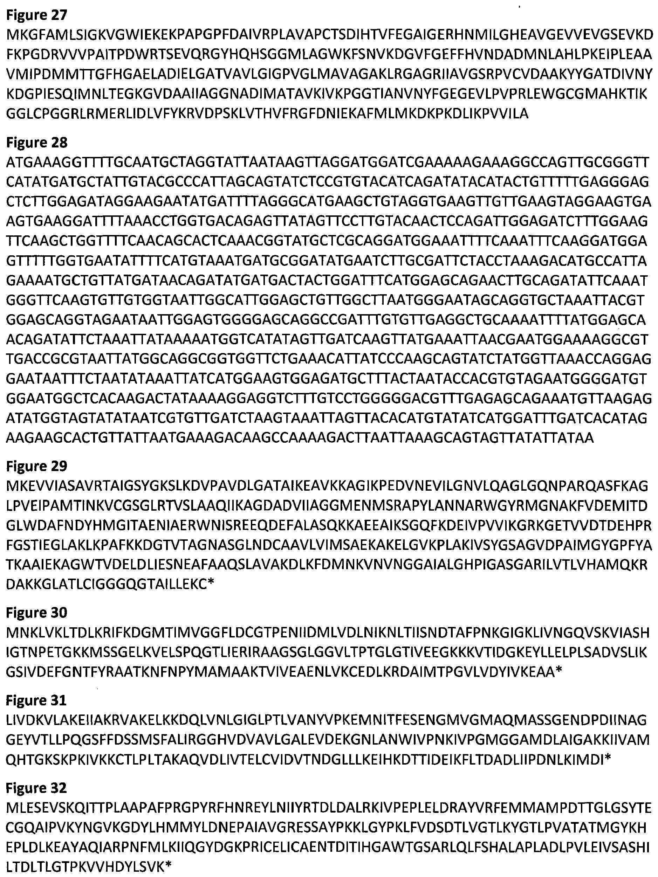

Fig. 27 shows SEQ_ID NO 40: Amino acid sequence of NADP-dependent alcohol dehydrogenase from *Thermoanaerobacter brockii* ATCC 53556.
Fig. 28 shows SEQ_ID NO 41: Nucleic acid sequence of alcohol dehydrogenase from *Thermoanaerobacter brockii.*
Fig. 29 shows SEQ_ID NO 42: Amino acid sequence of Thiolase ThIA from *C. acetobutylicum* ATCC824.
Fig. 30 shows SEQ_ID NO 43: Amino acid sequence of Acetoacetyl-CoA:acetate Coenzyme A transferase A CtfA from *C. beijerinckii* NCIMB8052.
Fig. 31 shows SEQ_ID NO 44: Amino acid sequence of Acetoacetyl-CoA:acetate Coenzyme A transferase A CtfB from *C. beijerinckii* NCIMB8052.
Fig. 32 shows SEQ_ID NO 45: Amino acid sequence of Acetoacetate decarboxylase Adc from *C. beijerinckii* NCIMB8052.

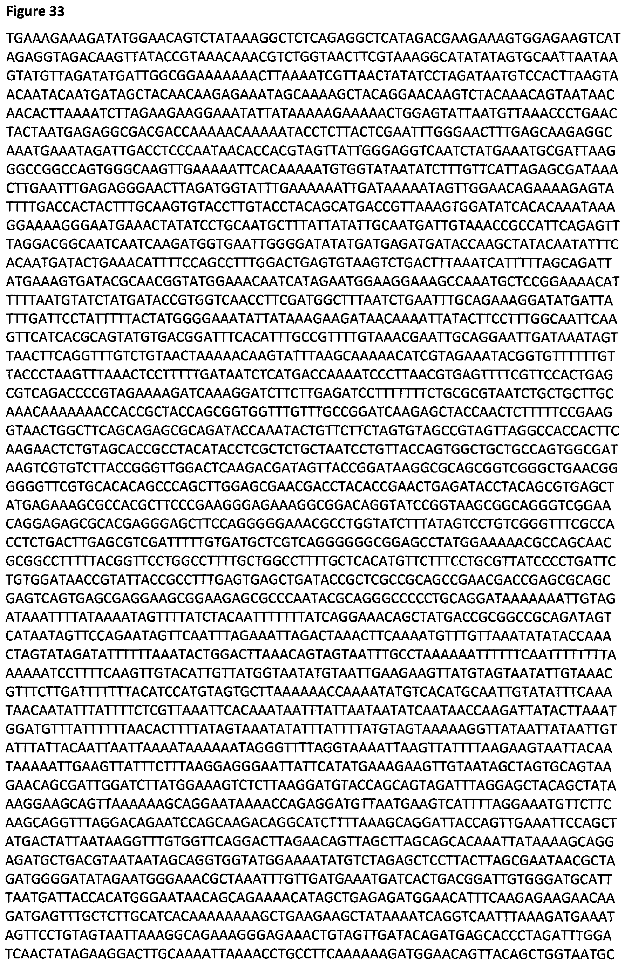

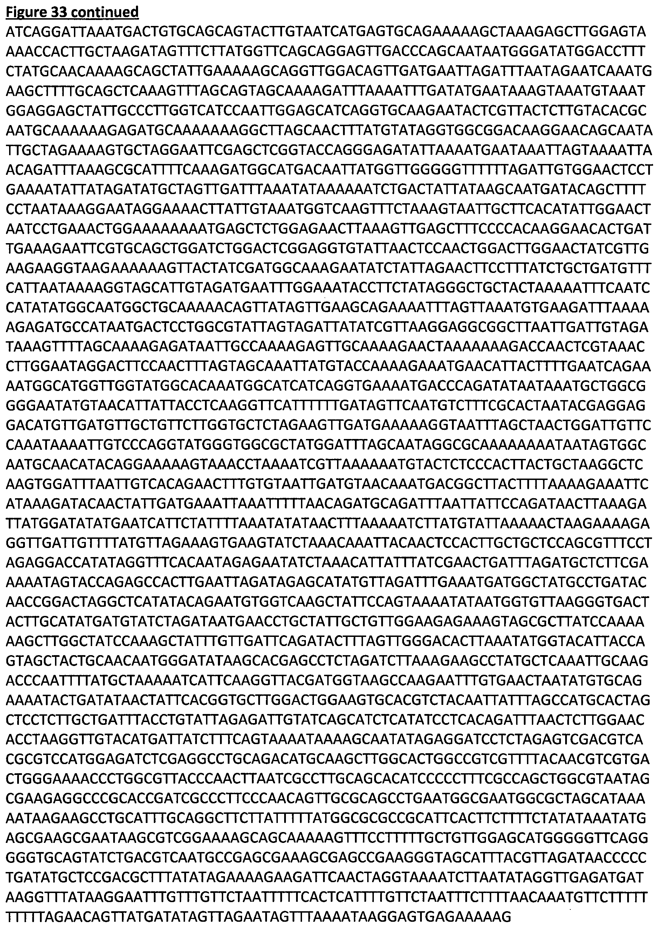

Fig. 33 shows SEQ_ID NO 46: Nucleic acid sequence of expression plasmid containing novel alcohol dehydrogenase pMTL85147-thlA-ctfAB-adc.

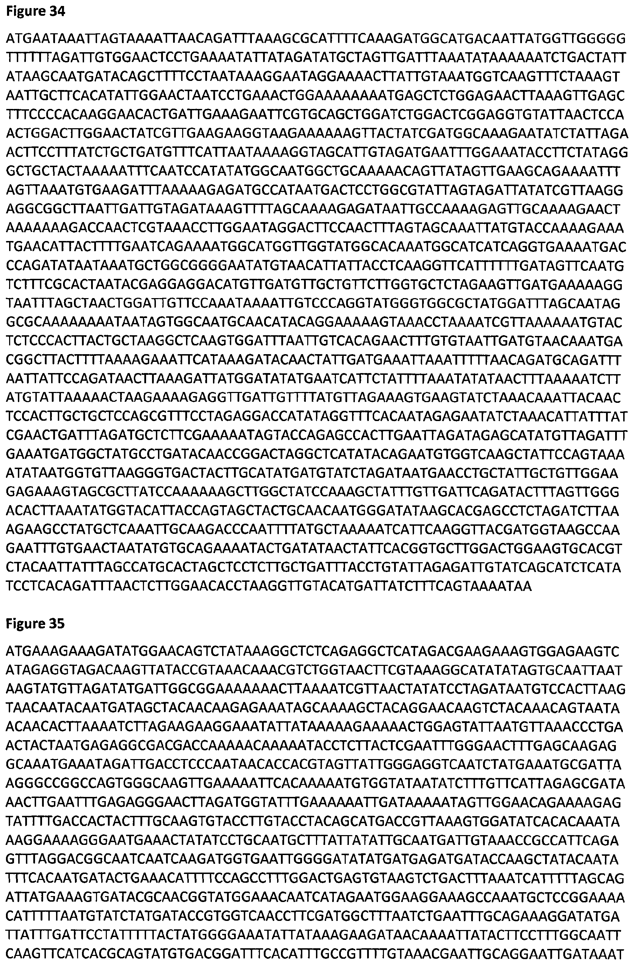

Fig. 34 shows SEQ_ID NO 47: Nucleic acid sequence of Acetoacetyl-CoA:acetate Coenzyme A transferase A (*ctfA*), acetoacetyl-CoA:acetate Coenzyme A transferase B (*ctfB*), and acetoacetate decarboxylase (*adc*) operon of C. beijerinckii.

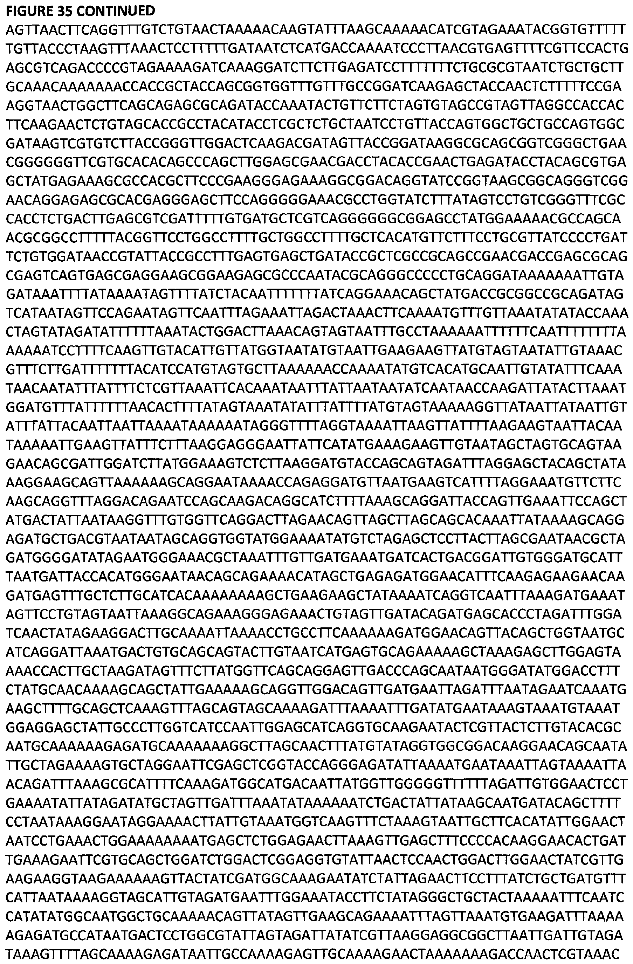

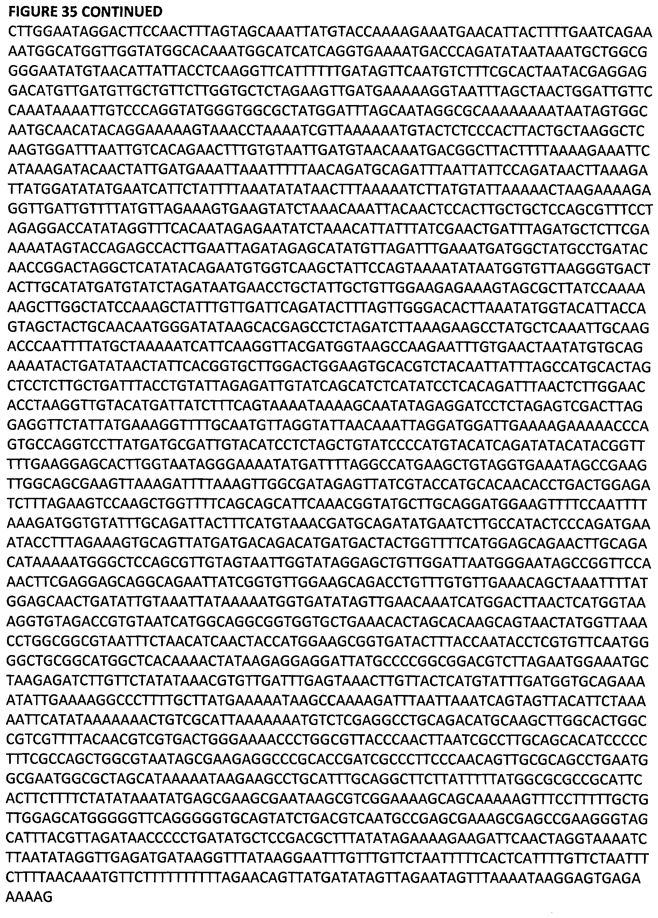

Fig. 35 shows SEQ_ID NO 48: Nucleic acid sequence of expression plasmid containing novel alcohol dehydrogenase pMTL85147-thlA-ctfAB-adc-adh.

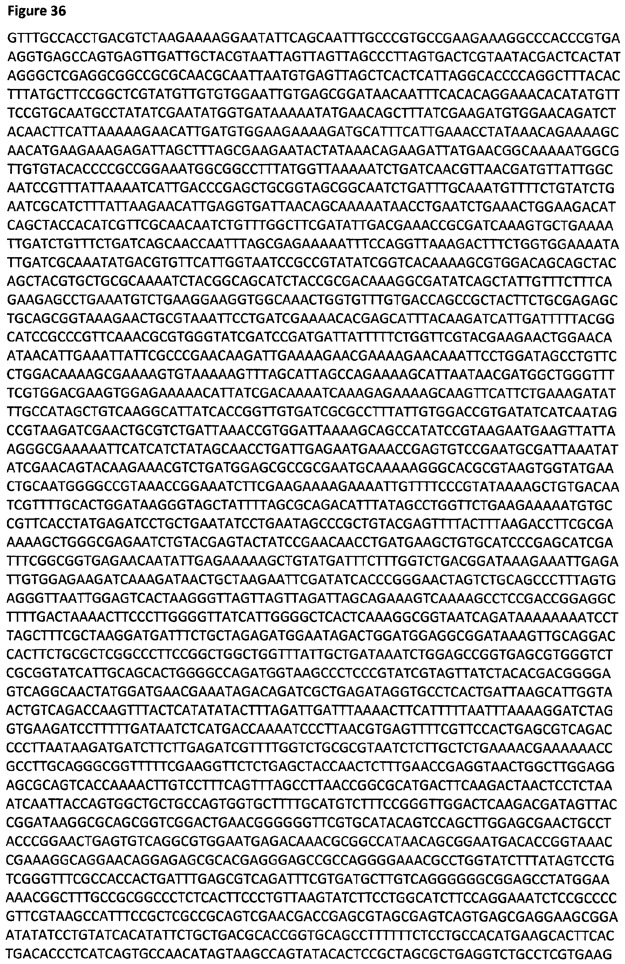

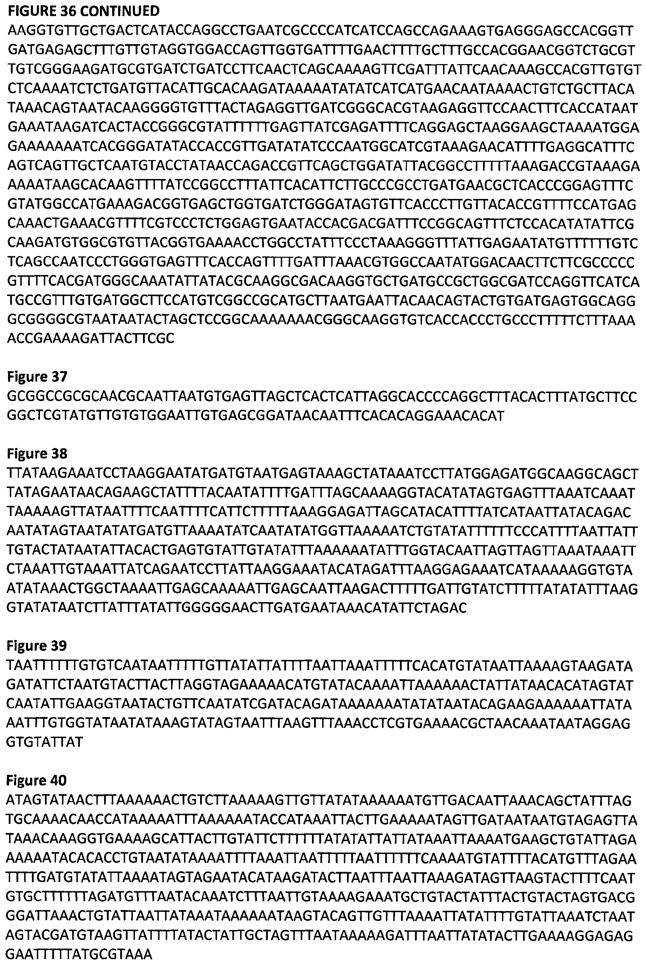

Fig. 36 shows SEQ_ID NO 49: Nucleic acid sequence of designed methylation plasmid.
Fig. 37 shows SEQ_ID NO 50: Nucleic acid sequence of *lac* promoter.
Fig. 38 shows SEQ_ID NO 51: Nucleic acid sequence of *Clostridium autoethanogenum* F₁F_{O} ATPase operon promoter region.
Fig. 39 shows SEQ_ID NO 52: Nucleic acid sequence of *Clostridium autoethanogenum* Rnf complex operon promoter region.
Fig. 40 shows SEQ_ID NO 53: Nucleic acid sequence of *Clostridium autoethanogenum* Pyruvate:ferredoxin oxidoreductase promoter region.
Fig. 41 shows the sequencing results of expression plasmid containing novel alcohol dehydrogenase pMTL85147-thlA-ctfA-ctfB-adc-adh.
Fig. 42 shows the results of acetone and isopropanol production with E. coli XL-1 Blue MRF' Kan carrying control plasmid (pMTL85147), acetone expression plasmid (pMTL85147-thlA-ctfA-ctfB-adc), and acetone expression plasmid including the novel alcohol dehydrogaenase (pMTL85147-thlA-ctfA-ctfB-adc-adh).
Fig. 43 shows expression plasmid containing novel alcohol dehydrogenase pMTL85147-thlA-ctfA-ctfB-adc-adh.
Fig. 44 shows SEQ_ID NO 56: Nucleic acid sequence of Wood-Ljungdahl cluster promoter (P_{WL}) from *C*. *ljungdahlii.*
Fig. 45 shows SEQ_ID NO 57: Nucleic acid sequence of Wood-Ljungdahl cluster promoter (P_{WL}) from *C. ragsdalei.*
Fig. 46 shows SEQ_ID NO 58: Nucleic acid sequence of *Clostridium ljungdahlii* F₁F_{O} ATPase operon promoter region.

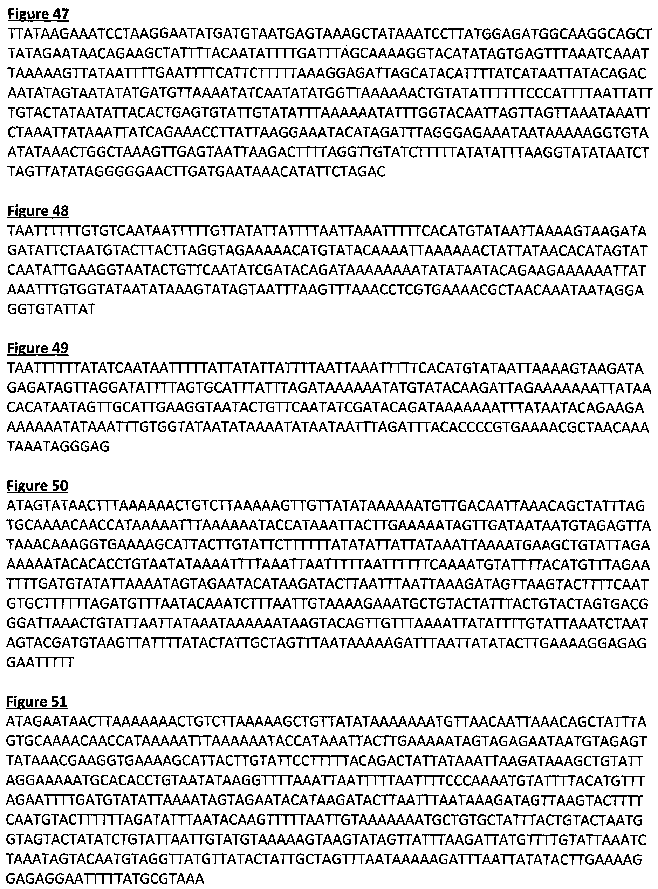

Fig. 47 shows SEQ_ID NO 59: Nucleic acid sequence of *Clostridium ragsdalei* F₁F_{O} ATPase operon promoter region.
Fig. 48 shows SEQ_ID NO 60: Nucleic acid sequence of *Clostridium ljungdahlii* Rnf complex operon promoter region.
Fig. 49 shows SEQ_ID NO 61: Nucleic acid sequence of *Clostridium ragsdalei* Rnf complex operon promoter region.
Fig. 50 shows SEQ_ID NO 62: Nucleic acid sequence of *Clostridium ljungdahlii* Pyruvate:ferredoxin oxidoreductase promoter region.
Fig. 51 shows SEQ_ID NO 63: Nucleic acid sequence of *Clostridium ragsdalei* Pyruvate:ferredoxin oxidoreductase promoter region.
Fig. 52 shows qRT-PCR amplification plot confirming amplification of probes for heterologous genes *thlA, ctfA, ctfB,* and *adc* in *Clostridium autoethanogenum* harbouring plasmid pMTL85147-thlA-ctfAB-adc
Fig. 53 shows qRT-PCR amplification plot confirming amplification of probes for heterologous genes *thlA, ctfA, ctfB,* and *adc* in *Clostridium ljungdahlii* harbouring plasmid pMTL85147-thlA-ctfAB-adc

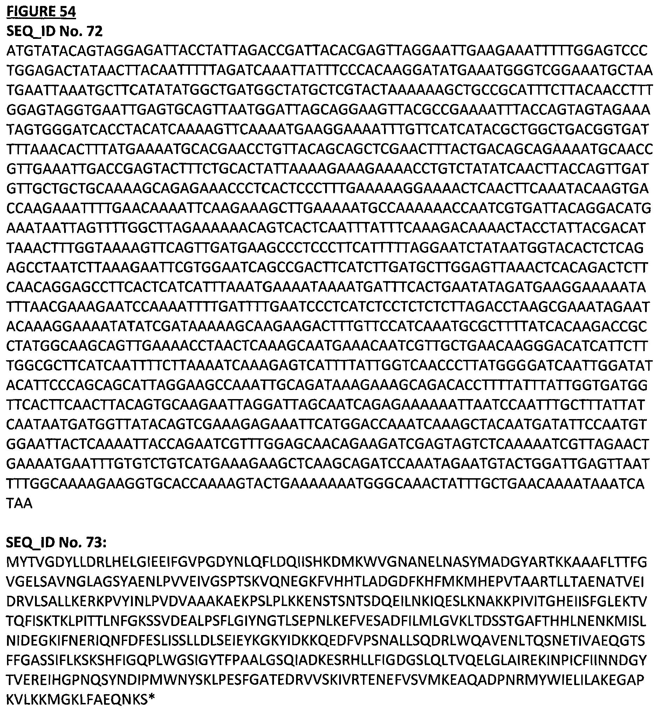

Fig. 54 shows SEQ_ID No. 73: Amino acid sequence of alpha-ketoisovalerate decarboxylase KivD from Lactococcus lactis KF147 and SEQ_ID No. 72 Nucleic acid sequence of Alpha-ketoacid decarboxylase (kivd).

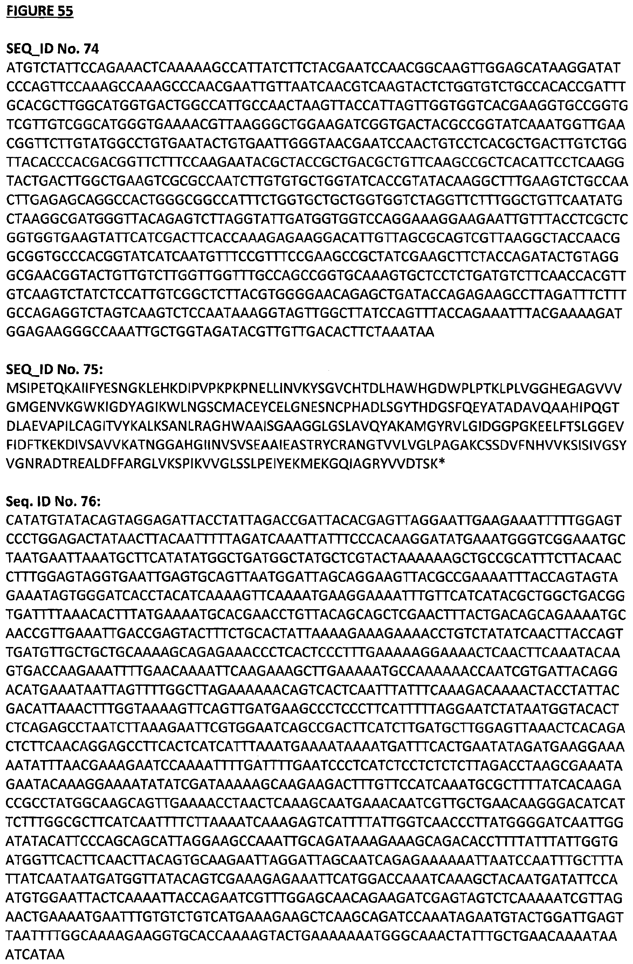

Fig 55 shows Seq. ID No. 76: Codon optimized sequence of Alpha-ketoacid decarboxylase (kivd), SEQ_ID No. 75: Amino acid sequence of alcohol dehydrogenase Adh2 from Saccharomyces cerevisiae and SEQ_ID No. 74 Nucleic acid sequence of Alcohol dehydrogenase (adh2)

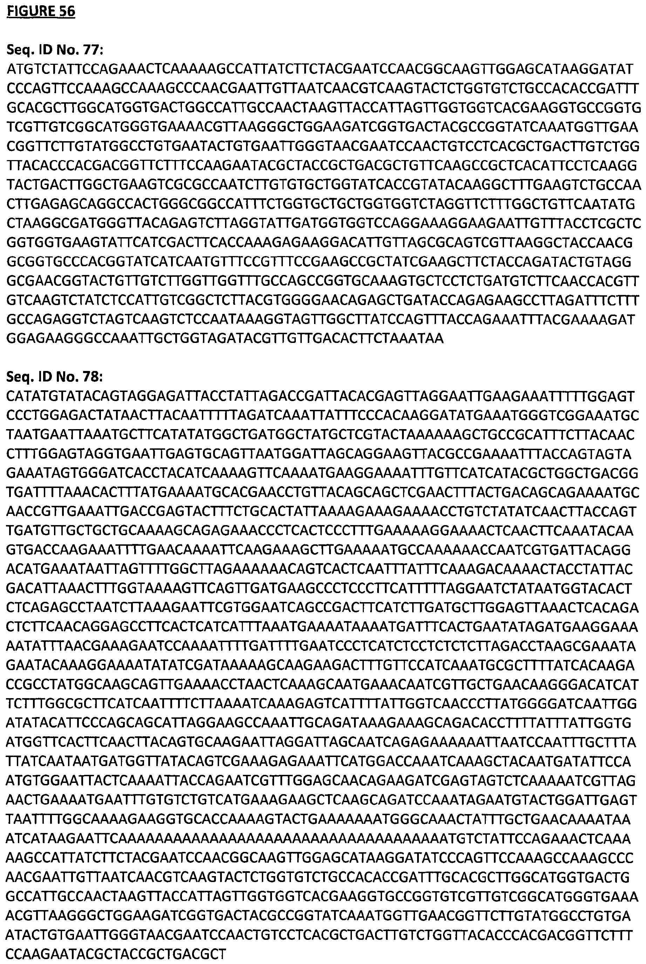

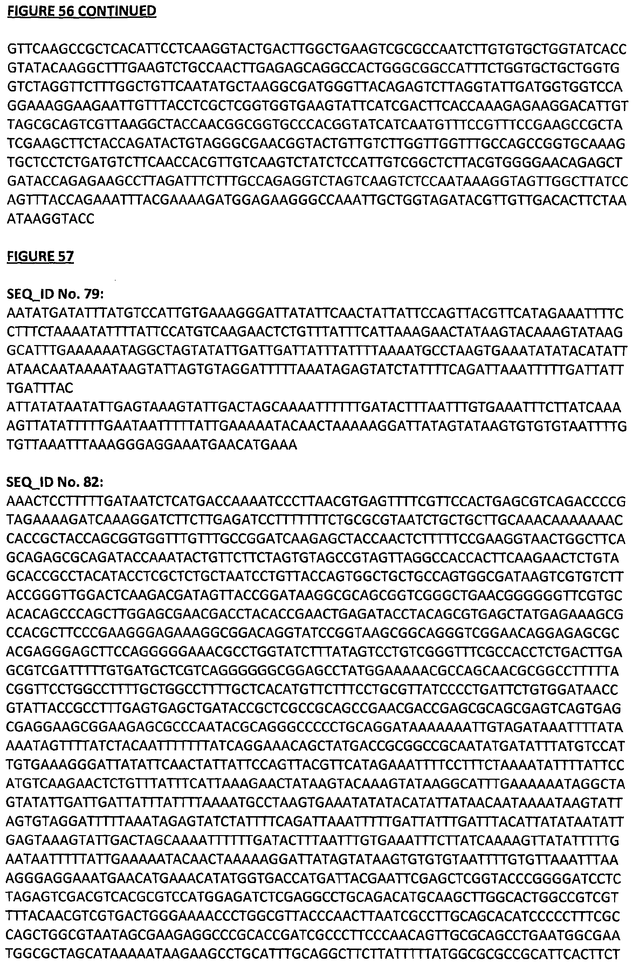

Fig 56 shows Seq. ID No. 78: Synthetic operon of codon optimized Alpha-ketoacid decarboxylase (kivd) and Alcohol dehydrogenase (Adh2) including spacer sequence with ribosomal binding site, flanked by Ndel and Kpnl and Seq. ID No. 77: Codon optimized sequence of Alcohol dehydrogenase (Adh2).

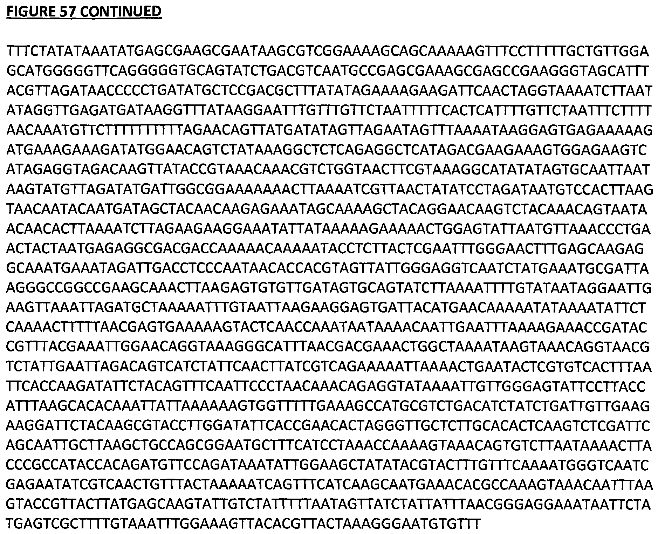

Fig 57 shows SEQ_ID No. 82: Nucleic acid sequence of *E. coli-Clostridium* shuttle vector pMTL 85245 and SEQ_ID No. 79: Nucleic acid sequence of Phosphotransacetylase Acetate kinase promoter from *C. autoethanogenum,*

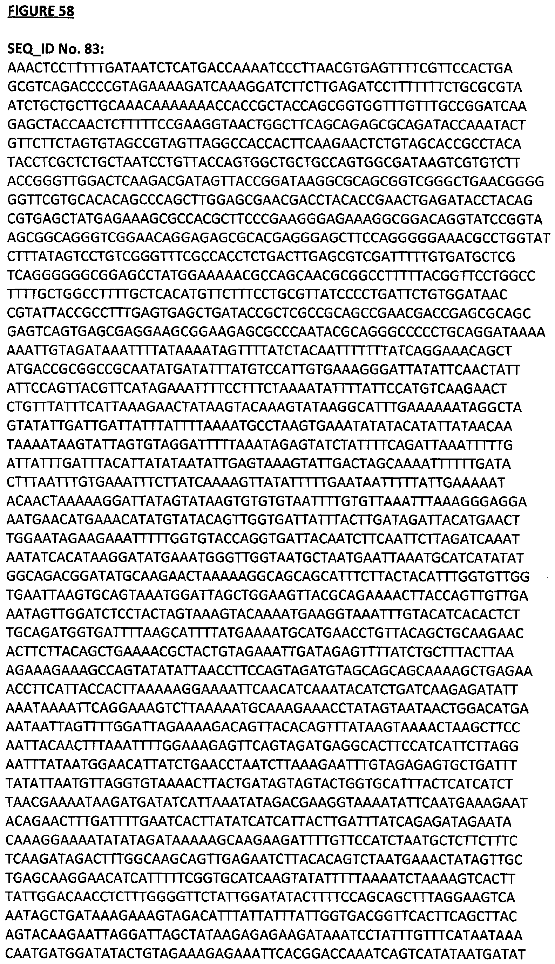

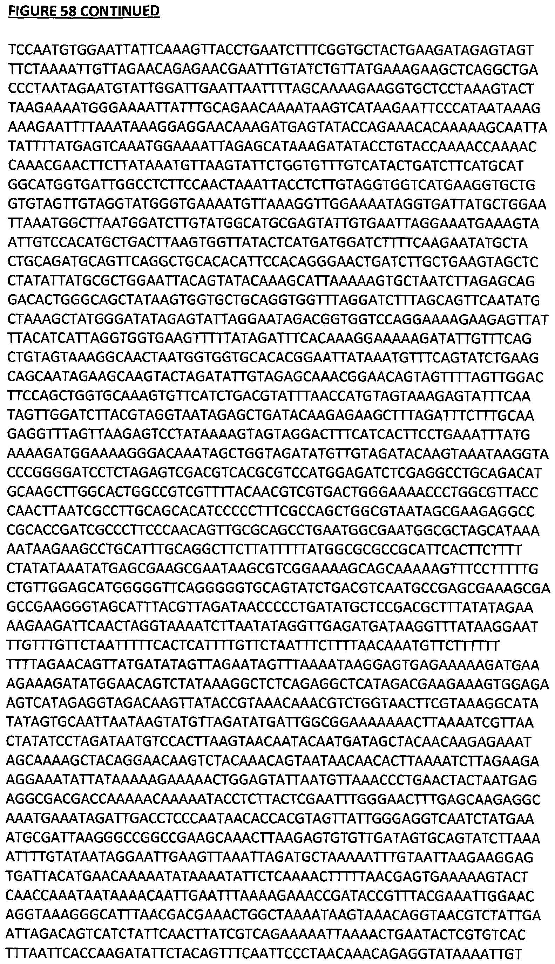

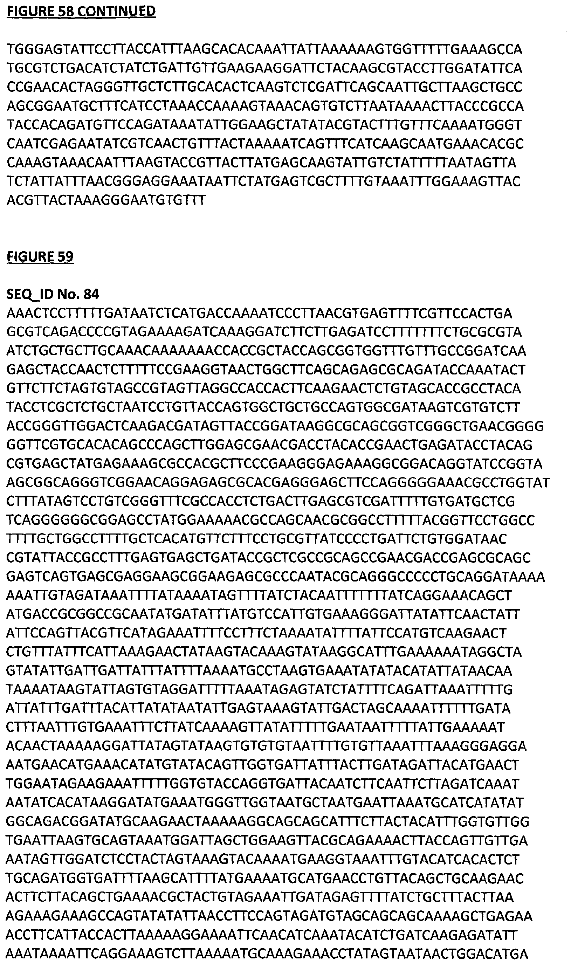

Fig 58 shows SEQ_ID No. 83: Nucleic acid sequence of expression plasmid pMTL85245-kivd-adh2

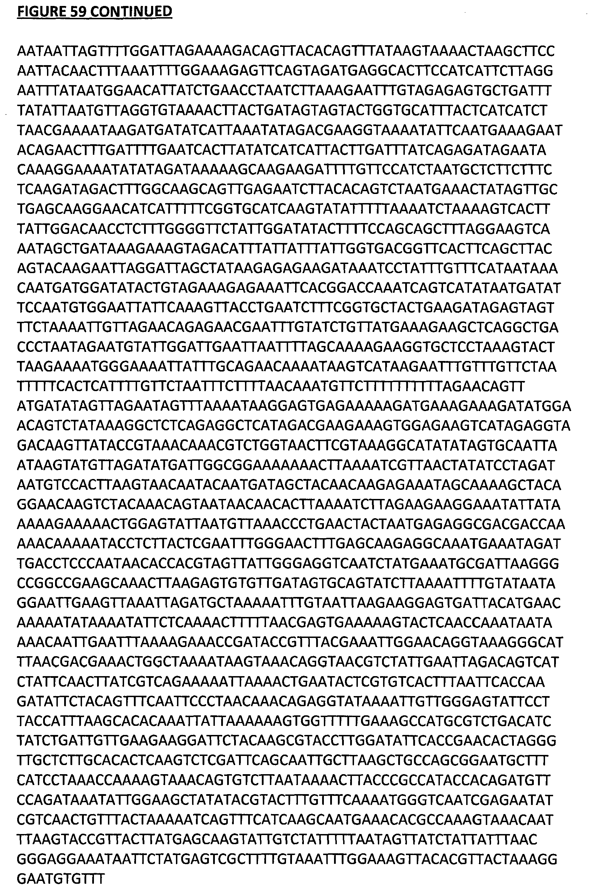

Fig 59 shows SEQ_ID No. 84 Nucleic acid sequence of expression plasmid pMTL85245-kivd

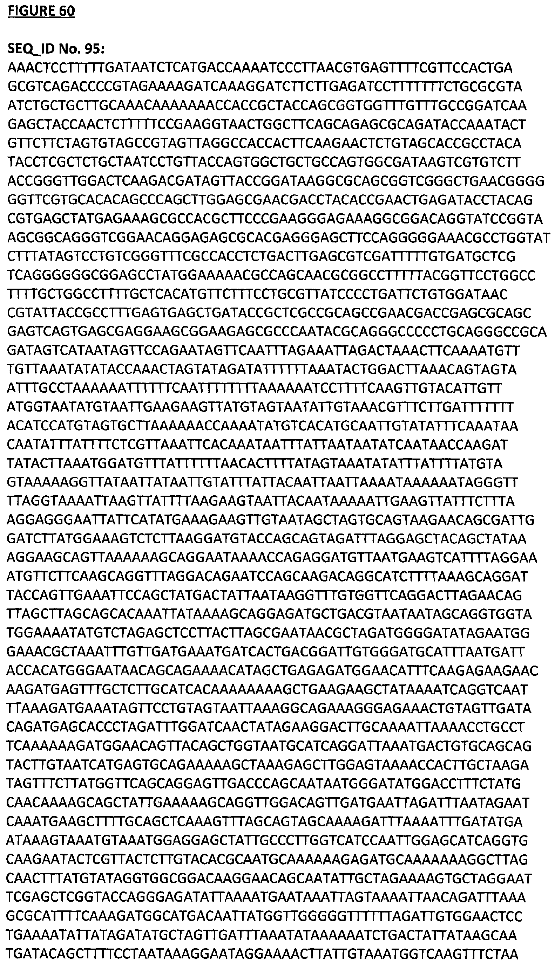

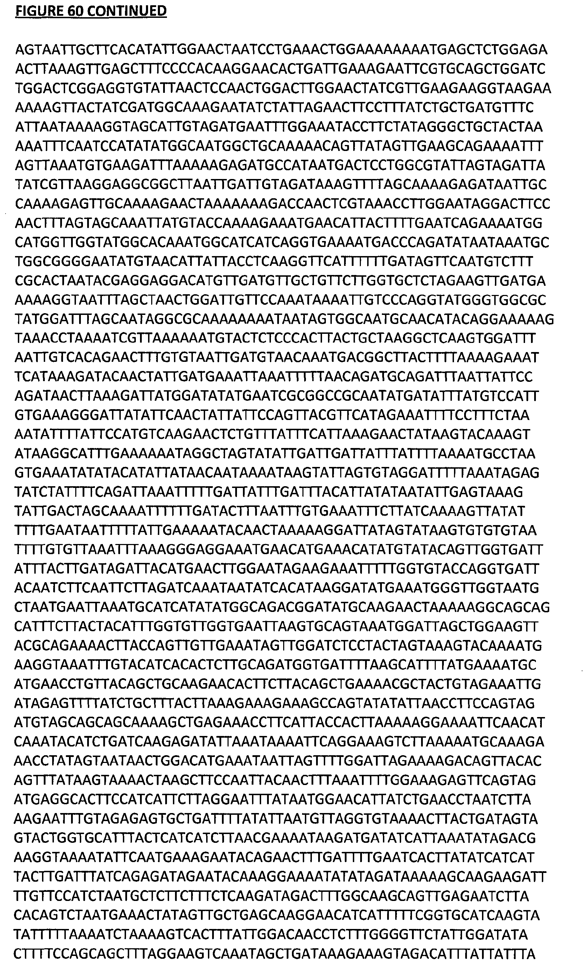

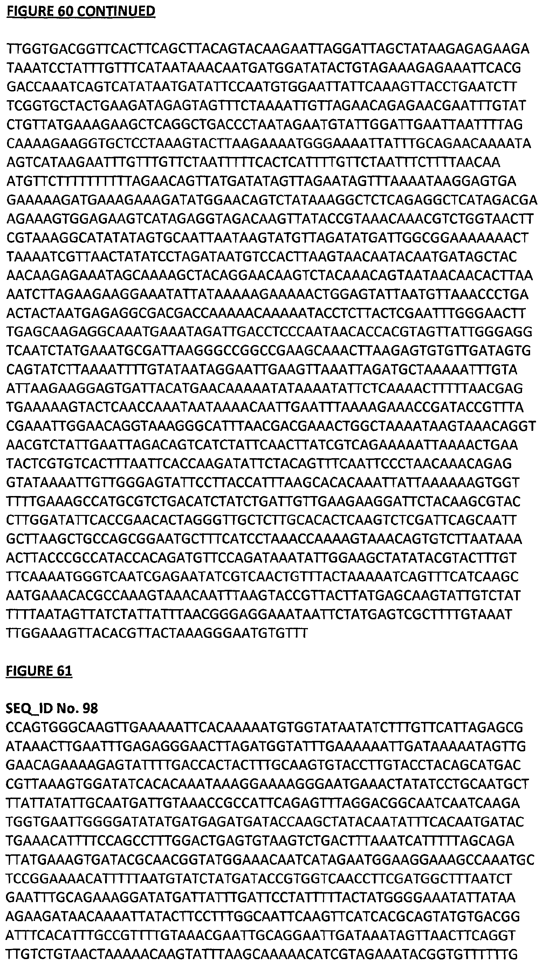

Fig 60 shows SEQ_ID No. 93: Nucleic acid sequence of expression plasmid pMTL85245-P-thl-ctfAB-P-kivd

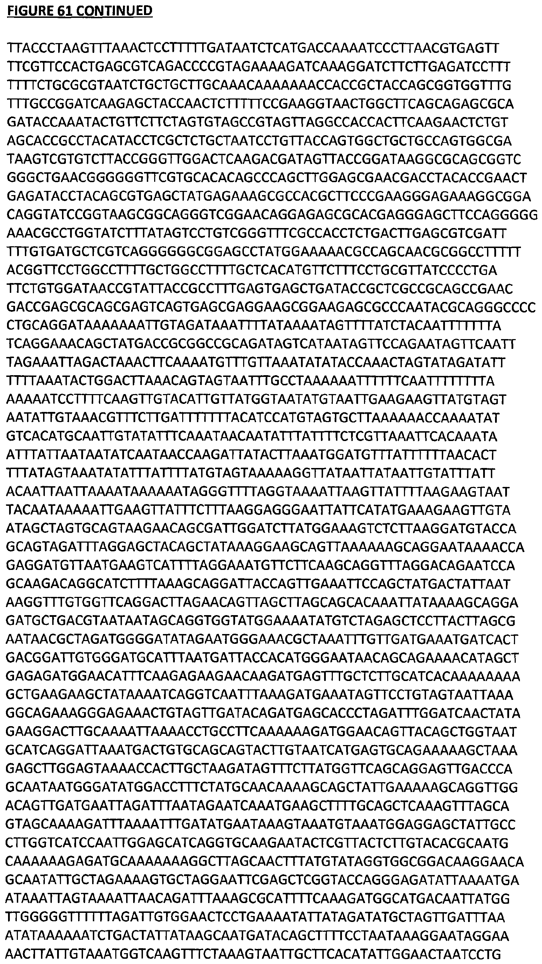

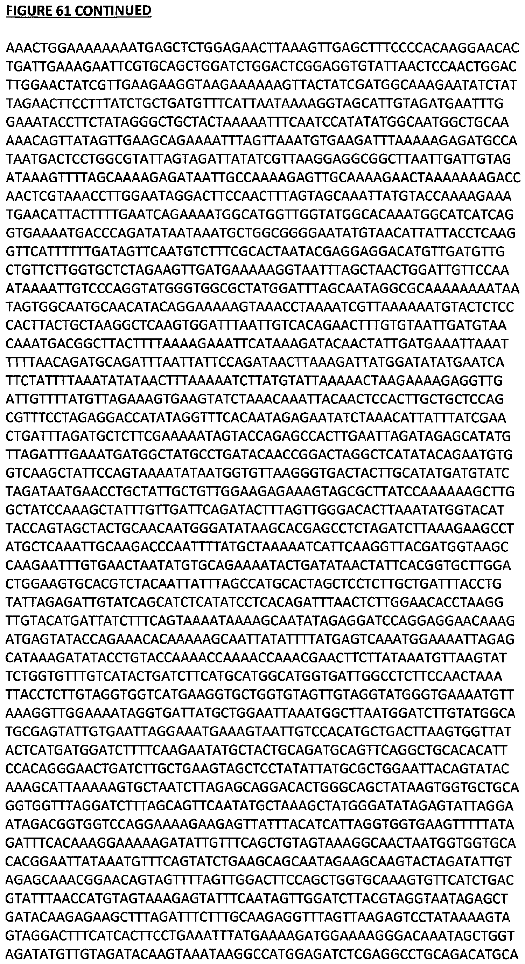

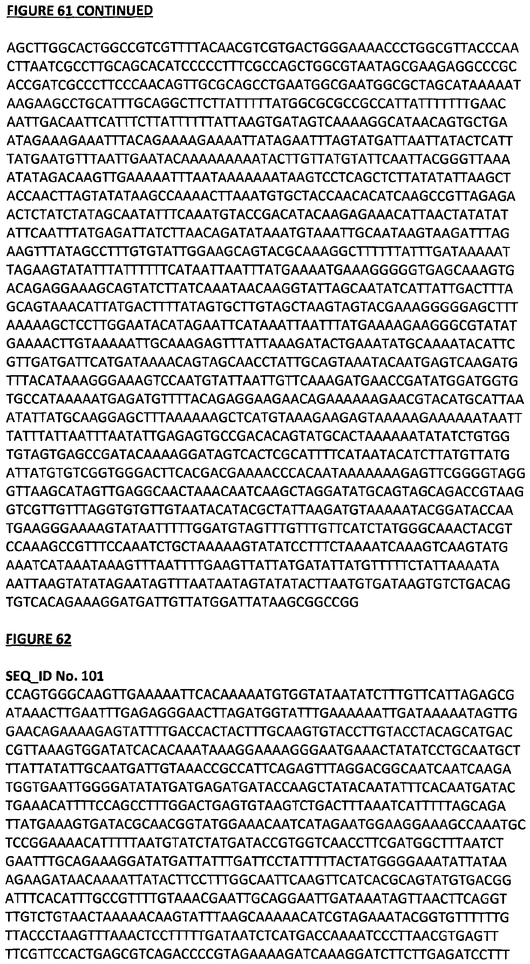

Fig 61 shows SEQ_ID No. 98 Nucleic acid sequence of expression plasmid pMTL83147-thlA-ctfAB-adc-adh2.

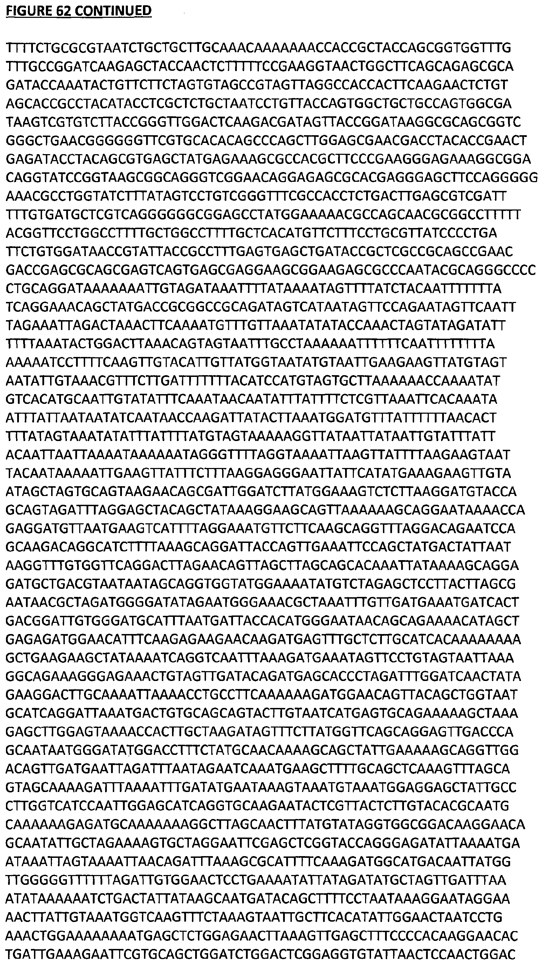

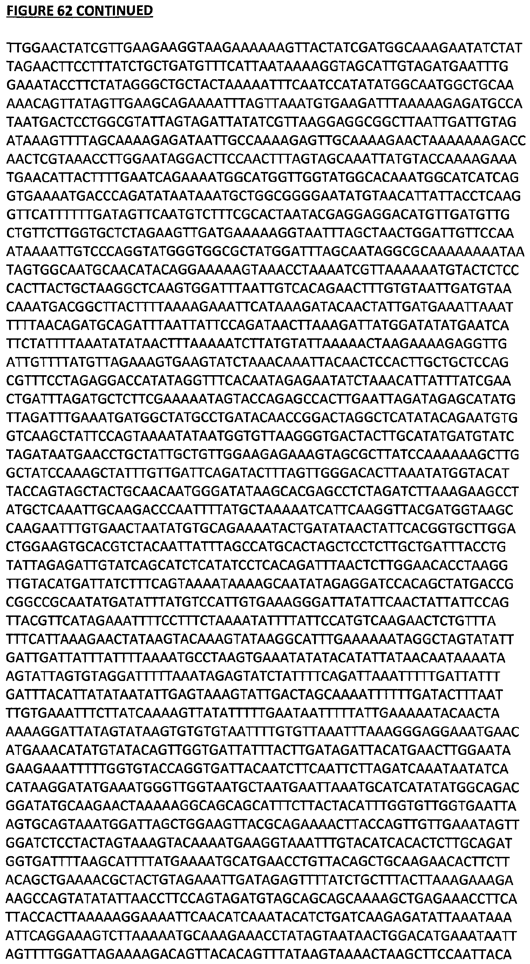

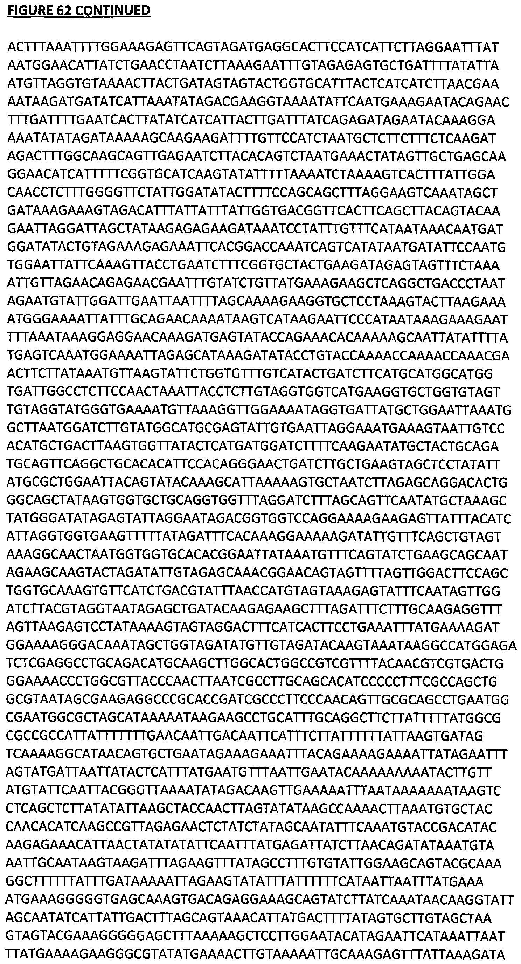

Fig 62 shows SEQ_ID No. 101 Nucleic acid sequence of expression plasmid pMTL83147-thlA-ctfAB-adc-P-kivd -adh2.
Fig 63 shows acetone expression plasmid pMTL85245-kivd-adh2
Fig 64 shows acetone expression plasmid pMTL85245-kivd
Fig 65 shows the GC result confirming acetone and isopropanol production with C. *autoethanogenum* DSM23693 as a control strain (top) and *C. autoethanogenum* DSM23693 + pMTL85245-kivd-adh2 (bottom) from CO-containing steel mill gas.
Fig 66 shows the GC result confirming acetone and isopropanol production with *C. autoethanogenum* DSM23693 + pMTL85245-kivd from CO-containing steel mill gas.
Fig 67 shows acetone expression plasmid pMTL85147-thlA-ctfA-ctfB-adc-P-kivd
Fig 68 shows acetone expression plasmid pMTL83147-thlA-ctfA-ctfB-adc-adh Fig 69 shows acetone expression plasmid pMTL83147-thlA-ctfA-ctfB-adc-P-kivd-adh
Fig 70 shows the GC result confirming acetone and isopropanol production with *C. autoethanogenum* DSM23693 (top) and *C*. *autoethanogenum* DSM23693 + pMTL85245-Pwl-thlA-ctfAB-kivd from CO-containing steel mill gas.
Fig 71 shows shows the GC result confirming acetone and isopropanol production with *C. autoethanogenum* DSM23693 + pMTL83147-thlA-ctfAB-adc-adh2 from CO-containing steel mill gas.
Fig 72 shows the GC result confirming acetone and isopropanol production with *C. autoethanogenum* DSM23693 + pMTL83147-thlA-ctfAB-adc-P-kivd-adh2 from CO-containing steel mill gas.
Fig 73 shows tested gene combinations of Clostridial pathway genes and codon-optimized Alpha-ketoacid decarboxylase Kivd from *L lactis* and Alcohol dehydrogenase Adh2 from *S. cerevisiae* heterologously expressed in *E. coli* and *C. autoethanogenum.*
Fig 74 shows complete conversion of acetone to isopropanol at high concentrations and rates when fed into a stable continuous culture of *C. autoethanogenum* DSM23693 with CO-containing steel mill gas as substrate.

### DETAILED DESCRIPTION OF THE INVENTION

The following is a description of the present invention, including preferred embodiments thereof, given in general terms. The invention is further elucidated from the disclosure given under the heading "Examples" herein below, which provides experimental data supporting the invention, specific examples of various aspects of the invention, and means of performing the invention.

The production of acetone and/or isopropanol by microbial fermentation of substrates comprising CO has not previously been reported. The inventors of the present invention have now demonstrated (*inter alia*), through genetic modification, the production of acetone and isopropanol in species of carboxydotrophic acetogenic bacteria capable of using CO as a carbon and energy source. The inventors have also surprisingly been able to demonstrate the natural enzymatic conversion of acetone to isopropanol in presence of CO-containing gases by closely related carboxydotrophic acetogenic species *C. autoethanogenum, C. Ijungdahlii,* and *C. ragsdalei.* A novel alcohol dehydrogenase was identified, which was shown to be expressed constitutively at a high level during a normal fermentation run with *C*. *autoethanogenum* and is able to convert acetone to isopropanol at high concentrations and ratios. The inventors have also found two genes that surprisingly confer activity towards acetone and isopropanol in *C. autoethanogenum.* These genes, an alpha-ketoacid decarboxylase (Kivd) from *Lactococcus lactis* and an alcohol dehydrogenase (Adh2) from *Saccharomyces cerevisiae* haven't been reported to confer activity towards acetone or isopropanol or any of it's precursors, rather converting amino acid precursors into branched chain alcohols. The inventors demonstrated production of acetone and isopropanol from CO in *C*. *autoethanogenum* using several different gene and enzyme combinations.

Accordingly, the invention provides, for example, methods for the production of acetone, isopropanol and/or precursors of acetone and/or isopropanol by microbial fermentation of substrates comprising CO, genetically modified microorganisms of use in such methods, nucleic acids suitable for preparation of genetically modified microorganisms and novel alcohol dehydrogenases and nucleic acids encoding same.

As referred to herein, a "fermentation broth" is a culture medium comprising at least a nutrient media and bacterial cells.

As referred to herein, a shuttle microorganism is a microorganism in which a methyltransferase enzyme is expressed and is distinct from the destination microorganism.

As referred to herein, a destination microorganism is a microorganism in which the genes included on an expression construct/vector are expressed and is distinct from the shuttle microorganism.

The term "main fermentation product" is intended to mean the one fermentation product which is produced in the highest concentration and/or yield.

The terms "increasing the efficiency", "increased efficiency" and the like, when used in relation to a fermentation process, include, but are not limited to, increasing one or more of the rate of growth of microorganisms catalysing the fermentation, the growth and/or product production rate at elevated acetone and/or isopropanol concentrations, the volume of desired product produced per volume of substrate consumed, the rate of production or level of production of the desired product, and the relative proportion of the desired product produced compared with other by-products of the fermentation.

The phrase "substrate comprising carbon monoxide" and like terms should be understood to include any substrate in which carbon monoxide is available to one or more strains of bacteria for growth and/or fermentation, for example.

The phrase "gaseous substrate comprising carbon monoxide" and like phrases and terms includes any gas which contains a level of carbon monoxide. In certain embodiments the substrate contains from 20% to 70% CO by volume, from 30% to 60% CO by volume, and from 40% to 55% CO by volume. In particular embodiments, the substrate comprises about 25%, or about 30%, or about 35%, or about 40%, or about 45%, or about 50% CO, or about 55% CO, or about 60% CO by volume.

While it is not necessary for the substrate to contain any hydrogen, the presence of H₂ should not be detrimental to product formation. In particular disclosures, the presence of hydrogen results in an improved overall efficiency of alcohol production. The substrate may comprise an approx 2:1, or 1:1, or 1:2 ratio of H₂:CO. In one disclosure the substrate comprises about 30% or less H₂ by volume, 20% or less H₂ by volume, about 15% or less H₂ by volume or about 10% or less H₂ by volume. In other diosclosures, the substrate stream comprises low concentrations of H₂, for example, less than 5%, or less than 4%, or less than 3%, or less than 2%, or less than 1%, or is substantially hydrogen free. The substrate may also contain some CO₂ for example, such as about 1% to about 80% CO₂ by volume, or 1% to about 30% CO₂ by volume. In one disclosure the substrate comprises less than or equal to about 20% CO₂ by volume. In particular disclosures the substrate comprises less than or equal to about 15% CO₂ by volume, less than or equal to about 10% CO₂ by volume, less than or equal to about 5% CO₂ by volume or substantially no CO₂.

In the description which follows, embodiments of the invention are described in terms of delivering and fermenting a "gaseous substrate containing CO". However, it should be appreciated that the gaseous substrate may be provided in alternative forms. For example, the gaseous substrate containing CO may be provided dissolved in a liquid. Essentially, a liquid is saturated with a carbon monoxide containing gas and then that liquid is added to the bioreactor. This may be achieved using standard methodology. By way of example, a microbubble dispersion generator (Hensirisak et. al. Scale-up of microbubble dispersion generator for aerobic fermentation; Applied Biochemistry and Biotechnology Volume 101, Number 3 / October, 2002) could be used. By way of further example, the gaseous substrate containing CO may be adsorbed onto a solid support. Such alternative methods are encompassed by use of the term "substrate containing CO" and the like.

In particular embodiments of the invention, the CO-containing gaseous substrate is an industrial off or waste gas. "Industrial waste or off gases" should be taken broadly to include any gases comprising CO produced by an industrial process and include gases produced as a result of ferrous metal products manufacturing, non-ferrous products manufacturing, petroleum refining processes, gasification of coal, gasification of biomass, electric power production, carbon black production, and coke manufacturing. Further examples may be provided elsewhere herein.

Unless the context requires otherwise, the phrases "fermenting", "fermentation process" or "fermentation reaction" and the like, as used herein, are intended to encompass both the growth phase and product biosynthesis phase of the process. As will be described further herein, the bioreactor may comprise a first growth reactor and a second fermentation reactor. As such, the addition of metals or compositions to a fermentation reaction should be understood to include addition to either or both of these reactors.

The term "bioreactor" includes a fermentation device consisting of one or more vessels and/or towers or piping arrangement, which includes the Continuous Stirred Tank Reactor (CSTR), Immobilized Cell Reactor (ICR), Trickle Bed Reactor (TBR), Bubble Column, Gas Lift Fermenter, Static Mixer, or other vessel or other device suitable for gas-liquid contact. The bioreactor may comprise a first growth reactor and a second fermentation reactor. As such, when referring to the addition of substrate to the bioreactor or fermentation reaction it should be understood to include addition to either or both of these reactors where appropriate.

"Exogenous nucleic acids" are nucleic acids which originate outside of the microorganism to which they are introduced. Exogenous nucleic acids may be derived from any appropriate source, including, but not limited to, the microorganism to which they are to be introduced, strains or species of microorganisms which differ from the organism to which they are to be introduced, or they may be artificially or recombinantly created. In one embodiment, the exogenous nucleic acids represent nucleic acid sequences naturally present within the microorganism to which they are to be introduced, and they are introduced to increase expression of or over-express a particular gene (for example, by increasing the copy number of the sequence (for example a gene), or introducing a strong or constitutive promoter to increase expression). In another embodiment, the exogenous nucleic acids represent nucleic acid sequences not naturally present within the microorganism to which they are to be introduced and allow for the expression of a product not naturally present within the microorganism or increased expression of a gene native to the microorganism (for example in the case of introduction of a regulatory element such as a promoter). The exogenous nucleic acid may be adapted to integrate into the genome of the microorganism to which it is to be introduced or to remain in an extra-chromosomal state.

It should be appreciated that the invention may be practised using nucleic acids whose sequence varies from the sequences specifically exemplified herein provided they perform substantially the same function. For nucleic acid sequences that encode a protein or peptide this means that the encoded protein or peptide has substantially the same function. For nucleic acid sequences that represent promoter sequences, the variant sequence will have the ability to promote expression of one or more genes. Such nucleic acids may be referred to herein as "functionally equivalent variants". By way of example, functionally equivalent variants of a nucleic acid include allelic variants, fragments of a gene, genes which include mutations (deletion, insertion, nucleotide substitutions and the like) and/or polymorphisms and the like. Homologous genes from other microorganisms may also be considered as examples of functionally equivalent variants of the sequences specifically exemplified herein. These include homologous genes in species such as *Clostridium acetobutylicum, Clostridium beijerinckii, C. saccharobutylicum and C. saccharoperbutylacetonicum,* details of which are publicly available on websites such as Genbank or NCBI. In the case of genes derived from *Sacchromyces cerevisiae* and *Lactococcus lactics,* homologous genes may be found, for example, in *Staphylococcus epidermidis* (for example, NP_765765.1, EGG67352.1, ZP_04826144.1, ZP_04797999.1), *Bacillus cereus* (for example, ZP_04273468.1, ZP_04317620.1) and *Bacillus thuringiensis* (for example, YP_003664720.1). The phrase "functionally equivalent variants" should also be taken to include nucleic acids whose sequence varies as a result of codon optimisation for a particular organism. "Functionally equivalent variants" of a nucleic acid herein will preferably have at least approximately 70%, preferably approximately 80%, more preferably approximately 85%, preferably approximately 90%, preferably approximately 95% or greater nucleic acid sequence identity with the nucleic acid identified.

It should also be appreciated that the invention may be practised using polypeptides whose sequence varies from the amino acid sequences specifically exemplified herein. These variants may be referred to herein as "functionally equivalent variants". A functionally equivalent variant of a protein or a peptide includes those proteins or peptides that share at least 40%, preferably 50%, preferably 60%, preferably 70%, preferably 75%, preferably 80%, preferably 85%, preferably 90%, preferably 95% or greater amino acid identity with the protein or peptide identified and has substantially the same function as the peptide or protein of interest. Such variants include within their scope fragments of a protein or peptide wherein the fragment comprises a truncated form of the polypeptide wherein deletions may be from 1 to 5, to 10, to 15, to 20, to 25 amino acids, and may extend from residue 1 through 25 at either terminus of the polypeptide, and wherein deletions may be of any length within the region; or may be at an internal location. Functionally equivalent variants of the specific polypeptides herein should also be taken to include polypeptides expressed by homologous genes in other species of bacteria, for example as exemplified in the previous paragraph.

"Substantially the same function" as used herein is intended to mean that the nucleic acid or polypeptide is able to perform the function of the nucleic acid or polypeptide of which it is a variant. For example, a variant of an enzyme of the invention will be able to catalyse the same reaction as that enzyme. However, it should not be taken to mean that the variant has the same level of activity as the polypeptide or nucleic acid of which it is a variant.

One may assess whether a functionally equivalent variant has substantially the same function as the nucleic acid or polypeptide of which it is a variant using any number of known methods. However, by way of example, the methods outlined in Wiesenborn et al [Thiolase from Clostridium acetobutylicum ATCC 824 and Its Role in the Synthesis of Acids and Solvents. Appl Environ Microbiol. 1988, 54: 2717-2722], Wiesenborn et al [Coenzyme A transferase from Clostridium acetobutylicum ATCC 824 and its role in the uptake of acids. Appl Environ Microbiol. 1989, 55:323-9.], Peterson and Bennet [Purification of acetoacetate decarboxylase from Clostridium acetobutylicum ATCC 824 and cloning of the acetoacetate decarboxylase gene in Escherichia coli. Appl Environ Microbiol. 1990 56: 3491-3498], Ismail et al. [Purification and characterization of a primary-secondary alcohol dehydrogenase from two strains of Clostridium beijerinckii. J Bacteriol 1993, 175: 5097-5105], de la Plaza et al [Biochemical and molecular characterization of a-ketoisovalerate decarboxylase, an enzyme involved in the formation of aldehydes from amino acids by Lactococcus lactis. FEMS Microbiol Lett. 2004 238: 367-374] or Khorkin et al [NADP-dependent bacterial alcohol dehydrogenases: crystal structure, cofactor-binding and cofactor specificity of the ADHs of Clostridium beijerinckii and Thermoanaerobacter brockii. J Mol Biol. 1998, 22: 278(5): 967-981] may be used to assess enzyme activity.

"Over-express", "over expression" and like terms and phrases when used in relation to the invention should be taken broadly to include any increase in expression of one or more protein as compared to the expression level of the protein of a parental microorganism under the same conditions. It should not be taken to mean that the protein is expressed at any particular level.

A "parental microorganism" is a microorganism used to generate a recombinant microorganism of the invention. The parental microorganism may be one that occurs in nature (ie a wild type microorganism) or one that has been previously modified but which does not express or over-express one or more of the enzymes the subject of the present invention. Accordingly, the recombinant microorganisms of the invention have been modified to express or over-express one or more enzymes that were not expressed or over-expressed in the parental microorganism.

The terms nucleic acid "constructs" or "vectors" and like terms should be taken broadly to include any nucleic acid (including DNA and RNA) suitable for use as a vehicle to transfer genetic material into a cell. The terms should be taken to include plasmids, viruses (including bacteriophage), cosmids and artificial chromosomes. Constructs or vectors may include one or more regulatory elements, an origin of replication, a multicloning site and/or a selectable marker. In one particular disclosure, the constructs or vectors are adapted to allow expression of one or more genes encoded by the construct or vector. Nucleic acid constructs or vectors include naked nucleic acids as well as nucleic acids formulated with one or more agents to facilitate delivery to a cell (for example, liposome-conjugated nucleic acid, an organism in which the nucleic acid is contained).

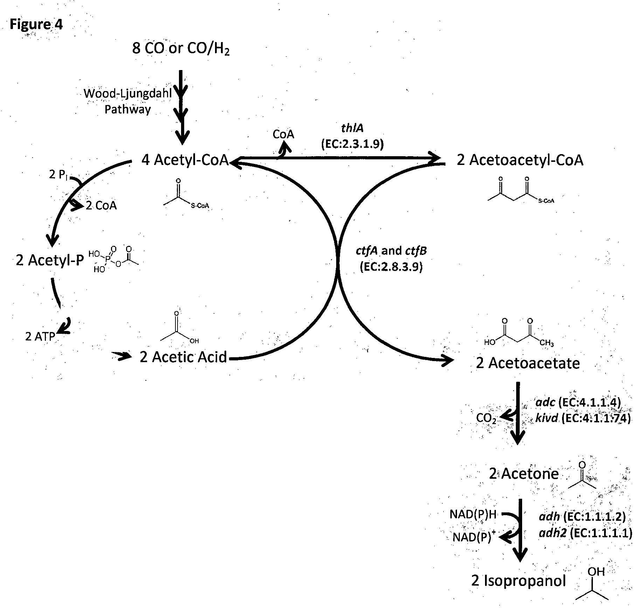

The "isopropanol biosynthesis pathway" is the enzymatic pathway allowing for metabolism of CO or CO/H₂ to isopropanol, as outlined, for example, in Figure 4.

The "acetone biosynthesis pathway" is the enzymatic pathway allowing for metabolism of CO or CO/H₂ to acetone, as outlined, for example, in Figure 4.

A "precursor" of acetone includes Acetyl-CoA, Acetoacetyl-CoA, Acetoacetate, Acetyl-Phosphate and Acetic Acid.

A "precursor" of isopropanol includes Acetyl-CoA, Acetoacetyl-CoA, Acetoacetate, Acetone, Acetyl-Phosphate and Acetic Acid.

Reference to "alcohol dehydrogenases" should be taken to include alcohol dehydrogenases which are capable of catalysing the conversion of ketones (such as acetone) to secondary alcohols (such as isopropanol), or vice versa. Such alcohol dehydrogenases include secondary alcohol dehydrogenases and primary alcohol dehydrogenases. A "secondary alcohol dehydrogenase" is one which can convert ketones (such as acetone) to secondary alcohols (such as isopropanol), or vice versa. A "primary alcohol dehydrogenase" is one which can convert aldehydes to primary alcohols, or vice versa; however, a number of primary alcohol dehydrogenases are also capable of catalysing the conversion of ketones to secondary alcohols, or vice versa. These alcohol dehydrogenases may also be referred to as "primary-secondary alcohol dehydrogenases".

As discussed herein before, the invention provides a method comprising the use of a recombinant microorganism capable of producing acetone, isopropanol and/or a precursor of acetone and/or isopropanol by fermentation of a substrate comprising CO. References the microorganisms and enzymes below relate to the use of the microorganisms or enzymes in the method of the invention.

In one particular embodiment, the microorganism is adapted to express one or more enzymes in the isopropanol biosynthesis pathway which are not naturally present in the parental microorganism. In another embodiment, the microorganism is adapted to over-express one or more enzymes in the isopropanol biosynthesis pathway which are naturally present in the parental microorganism.

In one particular embodiment, the microorganism is adapted to express one or more enzymes in the acetone biosynthesis pathway which are not naturally present in the parental microorganism. In another embodiment, the microorganism is adapted to over-express one or more enzymes in the acetone biosynthesis pathway which are naturally present in the parental microorganism.

In one particular embodiment, the microorganism is adapted to express one or more enzymes involved in the conversion of acetone to isopropanol which are not naturally present in the parental microorganism. In another embodiment, the microorganism is adapted to over-express one or more enzymes involved in the conversion of acetone to isopropanol which are naturally present in the parental microorganism.

In one embodiment, the parental microorganism is capable of fermenting a substrate comprising CO to produce acetone but not of converting acetone to isopropanol and the recombinant microorganism is adapted to express one or more enzymes involved in the conversion of acetone to isopropanol.

In another embodiment, the parental microorganism is capable of converting acetone to isopropanol but is not capable of fermenting a substrate comprising CO to produce acetone and the recombinant microorganism is adapted to express one or more enzymes in the acetone biosynthesis pathway.

In one embodiment, the parental microorganism is not capable of fermenting a substrate comprising CO to produce acetone and isopropanol and the recombinant microorganism is adapted to express one or more enzymes in the acetone biosynthesis pathway and one or more enzymes involved in the conversion of acetone to isopropanol.

The microorganism may be adapted to express or over-express the one or more enzymes by any number of recombinant methods including, for example, increasing expression of native genes within the microorganism (for example, by introducing a stronger or constitutive promoter to drive expression of a gene), increasing the copy number of a gene encoding a particular enzyme by introducing exogenous nucleic acids encoding and adapted to express the enzyme, introducing an exogenous nucleic acid encoding and adapted to express an enzyme not naturally present within the parental microorganism.

In certain embodiments, the parental microorganism may be transformed to provide a combination of increased or over-expression of one or more genes native to the parental microorganism and introduction of one or more genes not native to the parental microorganism. For example, one or more genes encoding an enzyme in the acetone biosynthesis pathway may be native to the parental microorganism but it may not include one or more gene encoding an enzyme involved in the conversion of acetone to isopropanol, or vice versa. The microorganism could be engineered to over-express the one or more native genes encoding an enzyme in the acetone biosynthesis pathway and to introduce a gene encoding an enzyme involved in conversion of acetone to isopropanol, or vice versa. Similarly, the microorganism could be engineered to over-express one or more enzymes in the acetone biosynthesis pathway (and/or the conversion of acetone to isopropanol) and to introduce one or more genes encoding an enzyme involved in the same pathway.

In one embodiment the one or more enzymes in the acetone biosynthesis pathway are chosen from the group consisting:
Acetyl-Coenzyme A acetyltransferase (Thiolase; ThIA; E.C. 2.3.1.9);
Acetoacetyl-CoA:Acetate Coenzyme A transferase A (CoA transferase; CtfA; EC 2.8.3.9);
Acetoacetyl-CoA:Acetate Coenzyme A transferase B (CoA transferase; CtfB; EC 2.8.3.9);
Acetoacetate decarboxylase (Adc; EC 4.1.1.4);
Alpha-ketoisovalerate decarboxylase (decarboxylase; KivD; EC4.1.1.74); and,
A functionally equivalent variant of any one or more thereof.

By way of example only, sequence information for each of the peptides in provided in table 6 or table 18 herein after.

The enzymes used in the microorganisms of the invention may be derived from any appropriate source, including different genera and species of bacteria, or other organisms. However, in one embodiment, the Acetyl-Coenzyme A acetyltransferase (Thiolase; ThlA) is that derived from *C. acetobutylicum.* In one embodiment, the Acetyl-Coenzyme A acetyltransferase has the amino acid sequence exemplified in table 6 herein after, or it is a functionally equivalent variant thereof.

In one embodiment, the enzymes Acetoacetyl-CoA:Acetate Coenzyme A transferase A (CoA transferase; CtfA), Acetoacetyl-CoA:Acetate Coenzyme A transferase B (CoA transferase; CtfB) and Acetoacetate decarboxylase (Adc) are derived from *C. Beijerinckii.*

In one embodiment, the enzymes alpha-ketoisovalerate decarboxylase (decarboxylase; KivD) is that derived from *L. lactis.*

In one embodiment, each enzyme has the amino acid sequence exemplified in table 6 or 18 herein after, or it is a functionally equivalent variant thereof.

In one embodiment, the one or more enzyme involved in the conversion of acetone to isopropanol are chosen from the group consisting:
Alcohol Dehydrogenase (Adh; EC 1.1.1.2);
Alcohol dehydrogenase (Adh2; EC 1.1.1.1); and,
A functionally equivalent variant thereof.

Again, the alcohol dehydrogenase enzyme used in the invention may be derived from any appropriate source, including different genera and species of bacteria (for example, the species of bacteria exemplified in table 13 herein after. However, in one particular embodiment, the Alcohol Dehydrogenase (Adh) is derived *from C. autoethanogenum, C. ljungdahlii,* and/or *C. ragsdalei.* In one disclosure, the alcohol dehydrogenase has the amino acid sequence of SEQ_ID NO. 1 or it is a functionally equivalent variant thereof. In one disclosure, the functionally equivalent variant has at least approximately 88%, 89%, 90%, 91%, 92%, 93%, 94%, 95%, 96%, 97%, 98% or 99% sequence identity to SEQ_ID NO. 1.

In one embodiment, the Alcohol Dehydrogenase (Adh2) is derived *from S. cerevisiae.*

In one embodiment, the microorganism comprises one or more exogenous nucleic acids encoding and adapted to express one or more of the enzymes referred to herein before. In one embodiment, the microorganisms comprise one or more exogenous nucleic acid encoding and adapted to express at least two of the enzymes. In other embodiments, the microorganism comprises one or more exogenous nucleic acid encoding and adapted to express 3, 4, 5, or 6 of the enzymes.

In one particular embodiment, the microorganism comprises one or more exogenous nucleic acids encoding each of Acetyl-Coenzyme A acetyltransferase (Thiolase; ThIA; E.C. 2.3.1.9), Acetoacetyl-CoA:Acetate Coenzyme A transferase A (CoA transferase; CtfA; EC 2.8.3.9), Acetoacetyl-CoA:Acetate Coenzyme A transferase B (CoA transferase; CtfB; EC 2.8.3.9), and Acetoacetate decarboxylase (Adc; EC 4.1.1.4) or a functionally equivalent variant of any one or more thereof.

In one particular embodiment, the microorganism comprises one or more exognenous nucleic acids encoding Alcohol Dehydrogenase (Adh; EC 1.1.1.2) or a functionally equivalent variant thereof.

In one particular embodiment, the microorganism comprises one or more exogenous nucleic acids encoding each of Acetyl-Coenzyme A acetyltransferase (Thiolase; ThlA; E.C. 2.3.1.9), Acetoacetyl-CoA:Acetate Coenzyme A transferase A (CoA transferase; CtfA; EC 2.8.3.9), Acetoacetyl-CoA:Acetate Coenzyme A transferase B (CoA transferase; CtfB; EC 2.8.3.9), Acetoacetate decarboxylase (Adc; EC 4.1.1.4), and Alcohol Dehydrogenase (Adh; EC 1.1.1.2), or a functionally equivalent variant of any one or more thereof.

In one particular embodiment, the microorganism comprises one or more exogenous nucleic acids encoding each of Alpha-ketoisovalerate decarboxylase (decarboxylase; KivD; EC4.1.1.74), and Alcohol dehydrogenase (Adh2; EC 1.1.1.1), or a functionally equivalent variant of any one or more thereof.

In one particular embodiment, the microorganism comprises one or more exogenous nucleic acids encoding each of Acetyl-Coenzyme A acetyltransferase (Thiolase; ThlA; E.C. 2.3.1.9), Acetoacetyl-CoA:Acetate Coenzyme A transferase A (CoA transferase; CtfA; EC 2.8.3.9), Acetoacetyl-CoA:Acetate Coenzyme A transferase B (CoA transferase; CtfB; EC 2.8.3.9), and Alpha-ketoisovalerate decarboxylase (decarboxylase; KivD; EC4.1.1.74), or a functionally equivalent variant of any one or more thereof.

In one particular embodiment, the microorganism comprises one or more exogenous nucleic acids encoding Alpha-ketoisovalerate decarboxylase (decarboxylase; KivD; EC4.1.1.74), or a functionally equivalent variant of any one or more thereof.

In one particular embodiment, the microorganism comprises one or more exogenous nucleic acids encoding each of Acetyl-Coenzyme A acetyltransferase (Thiolase; ThlA; E.C. 2.3.1.9), Acetoacetyl-CoA:Acetate Coenzyme A transferase A (CoA transferase; CtfA; EC 2.8.3.9), Acetoacetyl-CoA:Acetate Coenzyme A transferase B (CoA transferase; CtfB; EC 2.8.3.9), Acetoacetate decarboxylase (Adc; EC 4.1.1.4), and Alcohol dehydrogenase (Adh2; EC 1.1.1.1), or a functionally equivalent variant of any one or more thereof.

In another particular embodiment, the microorganism comprises one or more exogenous nucleic acids encoding each of Acetyl-Coenzyme A acetyltransferase (Thiolase; ThlA; E.C. 2.3.1.9), Acetoacetyl-CoA:Acetate Coenzyme A transferase A (CoA transferase; CtfA; EC 2.8.3.9), Acetoacetyl-CoA:Acetate Coenzyme A transferase B (CoA transferase; CtfB; EC 2.8.3.9), Acetoacetate decarboxylase (Adc; EC 4.1.1.4), Alpha-ketoisovalerate decarboxylase (decarboxylase; KivD; EC4.1.1.74), and Alcohol dehydrogenase (Adh2; EC 1.1.1.1), or a functionally equivalent variant of any one or more thereof.

In one embodiment, Acetyl-Coenzyme A acetyltransferase (Thiolase; ThIA) is encoded by a nucleic acid comprising SEQ_ID NO. 18, or a functionally equivalent variant thereof. In one embodiment, the Acetoacetyl-CoA:Acetate Coenzyme A transferase A (CoA transferase; CtfA) is encoded by a nucleic acid comprising SEQ_ID NO. 19, or a functionally equivalent variant thereof. In one embodiment, Acetoacetyl-CoA:Acetate Coenzyme A transferase B (CoA transferase; CtfB) is encoded by a nucleic acid comprising SEQ_ID NO. 20, or a functionally equivalent variant thereof. In one embodiment, Acetoacetate decarboxylase (Adc) is encoded by a nucleic acid comprising SEQ_ID NO. 21, or a functionally equivalent variant thereof. In one embodiment, the Alcohol Dehydrogenase (Adh) is encoded by a nucleic acid comprising SEQ_ID NO. 2, SEQ_ID NO. 3, or SEQ_ID NO. 4, or a functionally equivalent variant of any one thereof.

The microorganism may comprise one or more exogenous nucleic acids. Where it is desirable to transform the parental microorganism with two or more genetic elements (such as genes or regulatory elements (for example a promoter)) they may be contained on one or more exogenous nucleic acids.

In one embodiment, the one or more exogenous nucleic acid is a nucleic acid construct or vector, in one particular embodiment a plasmid, encoding one or more of the enzymes referred to hereinbefore in any combination. In one particular embodiment, the construct encodes each of ThlA, CtfA, CtfB, and Adc and optionally, Adh. In another embodiment, the one or more exogenous nucleic acids is a nucleic acid construct or vector, in one particular embodiment a plasmid, encoding Adh, and optionally ThlA, CtfA, CtfB, and/or Adc. In one particular embodiment, the construct encodes all of ThlA, CtfA, CtfB, Adc and Adh. The vector may also comprise other combinations of nucleic acids encoding alternative enzyme combinations, as is apparent from the desription elsewhere in this document. In one particular embodiment, the vector comprises 1, 2, 3 or 4 of the nucleic acid sequences SEQ_ID NO. 19, 20, 21 and 22 or a functionally equivalent variant of any one thereof, in any order. In another embodiment, the vector comprises SEQ_ID_NO. 2, 3 and/or 4, or a functionally equivalent variant of any one thereof, in any order. In one embodiment, the vector comprises 1, 2, 3, or 4 of sequences SEQ_ID NO. 19, 20, 21 and 22 or a functionally equivalent variant of any one thereof and SEQ_ID_NO. 2, 3 or 4, or a functionally equivalent variant of any one thereof, in any order.

The exogenous nucleic acids may remain extra-chromosomal upon transformation of the parent microorganism or may intergrate into the genome of the parent microorganism. Accordingly, they may include additional nucleotide sequences adapted to assist integration (for example, a region which allows for homologous recombination and targeted integration into the host genome) or expression and replication of an extrachromosomal construct (for example, origin of replication, promoter and other regulatory elements or sequences).

In one embodiment, the parental microorganism is selected from the group of carboxydotrophic acetogenic bacteria. In certain embodiments the microorganism is selected from the group comprising *Clostridium autoethanogenum, Clostridium ljungdahlii, Clostridium ragsdalei, Clostridium carboxidivorans, Clostridium drakei, Clostridium scatologenes, Clostridium coskatii, Butyribacterium limosum, Butyribacterium methylotrophicum, Acetobacterium woodii, Alkalibaculum bacchii, Blautia producta, Eubacterium limosum, Moorella thermoacetica, Moorella thermautotrophica, Oxobacter pfennigii,* and *Thermoanaerobacter kiuvi.*

In one particular embodiment, the parental microorganism is selected from the cluster of ethanologenic, acetogenic Clostridia comprising the species *C*. *autoethanogenum, C. ljungdahlii,* and *C*. *ragsdalei* and related isolates. These include but are not limited to strains C. *autoethanogenum* JAI-1^{T} (DSM10061) [Abrini J, Naveau H, Nyns E-J: Clostridium autoethanogenum, sp. nov., an anaerobic bacterium that produces ethanol from carbon monoxide. Arch Microbiol 1994, 4: 345-351], *C*. *autoethanogenum* LBS1560 (DSM19630) [Simpson SD, Forster RL, Tran PT, Rowe MJ, Warner IL: Novel bacteria and methods thereof. International patent 2009, WO/2009/064200], C. *autoethanogenum* LBS1561 (DSM23693), C. *ljungdahlii* PETC^{T} (DSM13528 = ATCC 55383) [Tanner RS, Miller LM, Yang D: Clostridium ljungdahlii sp. nov., an Acetogenic Species in Clostridial rRNA Homology Group I. Int J Syst Bacteriol 1993, 43: 232-236], *C*. *ljungdahlii* ERI-2 (ATCC 55380) [Gaddy JL: Clostridium stain which produces acetic acid from waste gases. US patent 1997, 5,593,886], *C*. *ljungdahlii* C-01 (ATCC 55988) [Gaddy JL, Clausen EC, Ko C-W: Microbial process for the preparation of acetic acid as well as solvent for its extraction from the fermentation broth. US patent, 2002, 6,368,819], *C*. *ljungdahlii* O-52 (ATCC 55989) [Gaddy JL, Clausen EC, Ko C-W: Microbial process for the preparation of acetic acid as well as solvent for its extraction from the fermentation broth. US patent, 2002, 6,368,819], *C. ragsdalei* P11^{T} (ATCC BAA-622) [Huhnke RL, Lewis RS, Tanner RS: Isolation and Characterization of novel Clostridial Species. International patent 2008, WO 2008/028055], related isolates such as *"C. coskatii"* [Zahn *et al* - Novel ethanologenic species Clostridium coskatii (US Patent Application number US20110229947)], or mutated strains such as *C*. *Ijungdahlii* OTA-1 (Tirado-Acevedo O. Production of Bioethanol from Synthesis Gas Using *Clostridium ljungdahlii.* PhD thesis, North Carolina State University, 2010). These strains form a subcluster within the Clostridial rRNA cluster I , and their 16S rRNA gene is more than 99% identical with a similar low GC content of around 30%. However, DNA-DNA reassociation and DNA fingerprinting experiments showed that these strains belong to distinct species [Huhnke RL, Lewis RS, Tanner RS: Isolation and Characterization of novel Clostridial Species. International patent 2008, WO 2008/028055].

All species of this cluster have a similar morphology and size (logarithmic growing cells are between 0.5-0.7 x 3-5 µm), are mesophilic (optimal growth temperature between 30-37 °C) and strictly anaerobe [Tanner RS, Miller LM, Yang D: Clostridium ljungdahlii sp. nov., an Acetogenic Species in Clostridial rRNA Homology Group I. Int J Syst Bacteriol 1993, 43: 232-236; Abrini J, Naveau H, Nyns E-J: Clostridium autoethanogenum, sp. nov., an anaerobic bacterium that produces ethanol from carbon monoxide. Arch Microbiol 1994, 4: 345-351; Huhnke RL, Lewis RS, Tanner RS: Isolation and Characterization of novel Clostridial Species. International patent 2008, WO 2008/028055]. Moreover, they all share the same major phylogenetic traits, such as same pH range (pH 4-7.5, with an optimal initial pH of 5.5-6), strong autotrophic growth on CO containing gases with similar growth rates, and a similar metabolic profile with ethanol and acetic acid as main fermentation end product, and small amounts of 2,3-butanediol and lactic acid formed under certain conditions. [Tanner RS, Miller LM, Yang D: Clostridium ljungdahlii sp. nov., an Acetogenic Species in Clostridial rRNA Homology Group I. Int J Syst Bacteriol 1993, 43: 232-236; Abrini J, Naveau H, Nyns E-J: Clostridium autoethanogenum, sp. nov., an anaerobic bacterium that produces ethanol from carbon monoxide. Arch Microbiol 1994, 4: 345-351; Huhnke RL, Lewis RS, Tanner RS: Isolation and Characterization of novel Clostridial Species. International patent 2008, WO 2008/028055]. Indole production was observed with all three species as well. However, the species differentiate in substrate utilization of various sugars (e.g. rhamnose, arabinose), acids (e.g. gluconate, citrate), amino acids (e.g. arginine, histidine), or other substrates (e.g. betaine, butanol). Moreover some of the species were found to be auxotroph to certain vitamins (e.g. thiamine, biotin) while others were not.

In one embodiment, the parental strain uses CO as its sole carbon and energy source.

In one embodiment the parental microorganism is *Clostridium autoethanogenum* or *Clostridium ljungdahlii.* In one particular embodiment, the microorganism is *Clostridium autoethanogenum* DSM23693. In another particular embodiment, the microorganism is *Clostridium ljungdahlii* DSM13528 (or ATCC55383).

In one embodiment, the parental microorganism lacks one or more genes encoding ThIA, CtfA, CtfB, Adc, KivD, Adh and Adh2. In one particular embodiment, the parental microorganism lacks a gene encoding Adh. In another particular embodiment, the parental microorganism lacks each of the genes encoding ThIA, CtfA, CtfB, Adc, and KivD.

The inventors have identified a novel Adh protein. Disclosed is an Alcohol Dehydrogenase (Adh) having the amino acid sequence of SEQ_ID NO. 1, or a functionally equivalent variant of any one thereof. In one disclosure, the functionally equivalent variant of Alcohol Dehydrogenase (Adh) has at least approximately 88%, 89%, 90%, 91%, 92%, 93%, 94%, 95%, 96%, 97%, 98% or 99% sequence identity to SEQ_ID NO. 1.

Disclosed is a nucleic acid encoding Adh of SEQ_ID NO. 1 or a functionally equivalent variant thereof. Skilled persons will readily appreciate such nucleic acids, having regard to the amino acid sequence provided herein and the genetic code and the degeneracy therein. However, by way of example, nucleic acids encoding Adh of SEQ_ID NO. 1 include the nucleic acids of SEQ_ID NO. 2, 3 or 4, or functionally equivalent variants thereof. In one particular disclosure, a functionally equivalent variant of SEQ_ID NO. 2, 3 or 4 is a nucleic acid having at least approximately 83%, 84%, 85%, 86%, 87%, 88%, 89%, 90%, 91%, 92%, 93%, 94%, 95%, 96%, 97%, 98% or 99% sequence identity to SEQ_ID NO. 2, 3 or 4.

Also disclosed are nucleic acids which are capable of hybridising to at least a portion of the nucleic acid SEQ_ID NO. 2, 3 or 4, a nucleic acid complementary to any one thereof, or a functionally equivalent variant of any one thereof. Such nucleic acids will preferably hybridise to the nucleic acid of SEQ_ID NO. 2, 3 or 4, a nucleic acid complementary to any one thereof, or a functionally equivalent variant of any one thereof, under stringent hybridisation conditions. "Stringent hybridisation conditions" means that the nucleic acid is capable of hybridising to a target template under standard hybridisation conditions such as those described in Sambrook et al, Molecular Cloning: A Laboratory Manual (1989), Cold Spring Harbor Laboratory Press, New York, USA. It will be appreciated that the minimal size of such nucleic acids is a size which is capable of forming a stable hybrid between a given nucleic acid and the complementary sequence to which it is designed to hybridise. Accordingly, the size is dependent on the nucleic acid composition and percent homology between the nucleic acid and its complementary sequence, as well as the hybridisation conditions which are utilised (for example, temperature and salt concentrations). In one disclosure, the nucleic acid is at least 10 nucleotides in length, at least 15 nucleotides in length, at least, 20 nucleotides in length, at least 25 nucleotides in length, or at least 30 nucleotides in length.

The inventor's have also identified a number of novel nucleic acids useful as probes and primers, as detailed herein after in the examples section. For example, SEQ_ID NO. 5; SEQ_ID NO. 6; SEQ_ID NO. 7; SEQ_ID NO. 8; SEQ_ID NO. 9; SEQ_ID NO. 10; SEQ_ID NO. 11; SEQ_ID NO. 12; SEQ_ID NO. 13; SEQ_ID NO. 14; SEQ_ID NO. 15; SEQ_ID NO. 16; SEQ_ID NO. 17; SEQ_ID NO. 18; SEQ_ID NO. 23; SEQ_ID NO. 24; SEQ_ID NO. 25; SEQ_ID NO. 26; SEQ_ID NO. 27; SEQ_ID NO. 28; SEQ_ID NO. 29; SEQ_ID NO. 30; SEQ_ID NO. 31; SEQ_ID NO. 32; SEQ_ID NO. 33; SEQ_ID NO. 64; SEQ_ID NO. 65; SEQ_ID NO. 66; SEQ_ID NO. 67; SEQ_ID NO. 68; SEQ_ID NO. 69; SEQ_ID NO. 70; SEQ_ID NO. 71; SEQ_ID NO. 85; SEQ_ID NO. 86; SEQ_ID NO. 87; SEQ_ID NO. 88; SEQ_ID NO. 89; SEQ_ID NO. 90; SEQ_ID NO. 91; SEQ_ID NO. 92; SEQ_ID NO. 93; SEQ_ID NO. 94; SEQ_ID NO. 96; SEQ_ID NO. 97; SEQ_ID NO. 99; SEQ_ID NO. 100.

The invention also provides nucleic acids and nucleic acid constructs of use in generating a recombinant microorganism of the invention.

In one embodiment, the nucleic acids comprises sequences encoding one or more of the enzymes which when expressed in a microorganism allows the microorganism to produce acetone, isopropanol and/or a precursor of acetone and/or isopropanol by fermentation of substrate comprising CO. In one particular embodiment, the invention provides a nucleic acid encoding two or more enzymes which when expressed in a microorganism allows the microorganism to produce acetone, isopropanol and/or a precursor of acetone and/or isopropanol by fermentation of substrate comprising CO. In one embodiment, the nucleic acids of the invention encode 3, 4, 5 or 6 such enzymes.

In one particular embodiment, the enzymes are chosen from Acetyl-Coenzyme A acetyltransferase (Thiolase; ThlA), Acetoacetyl-CoA:Acetate Coenzyme A transferase A (CoA transferase; CtfA), Acetoacetyl-CoA:Acetate Coenzyme A transferase B (CoA transferase; CtfB), Acetoacetate decarboxylase (Adc), ketoisovalerate decarboxylase (decarboxylase; KivD), Alcohol Dehydrogenase (Adh), Alcohol Dehydrogenase (Adh2), and a functionally equivalent variant of any one or more thereof.

In one embodiment, a nucleic acid of the invention comprises nucleic acid sequences encoding each of Acetyl-Coenzyme A acetyltransferase (Thiolase; ThlA), Acetoacetyl-CoA:Acetate Coenzyme A transferase A (CoA transferase; CtfA), Acetoacetyl-CoA:Acetate Coenzyme A transferase B (CoA transferase; CtfB), and Acetoacetate decarboxylase (Adc) or a functionally equivalent variant of any one or more thereof, in any order

In one embodiment, a nucleic acid of the invention comprises nucleic acid sequences encoding each of Acetyl-Coenzyme A acetyltransferase (Thiolase; ThlA), Acetoacetyl-CoA:Acetate Coenzyme A transferase A (CoA transferase; CtfA), Acetoacetyl-CoA:Acetate Coenzyme A transferase B (CoA transferase; CtfB), Acetoacetate decarboxylase (Adc), and Alcohol Dehydrogenase (Adh) or a functionally equivalent variant of any one or more thereof, in any order.

In one embodiment, a nucleic acid of the invention comprises nucleic acid sequences encoding each of Alpha-ketoisovalerate decarboxylase (decarboxylase; KivD), and Alcohol dehydrogenase (Adh2), or a functionally equivalent variant of any one or more thereof, in any order.

In one embodiment, a nucleic acid of the invention comprises nucleic acids encoding each of Acetyl-Coenzyme A acetyltransferase (Thiolase; ThlA), Acetoacetyl-CoA:Acetate Coenzyme A transferase A (CoA transferase; CtfA), Acetoacetyl-CoA:Acetate Coenzyme A transferase B (CoA transferase; CtfB), and Alpha-ketoisovalerate decarboxylase (decarboxylase; KivD), or a functionally equivalent variant of any one or more thereof, in any order.

In one embodiment, a nucleic acid of the invention comprises nucleic acids encoding Alpha-ketoisovalerate decarboxylase (decarboxylase; KivD), or a functionally equivalent variant thereof. In one one embodiment, a nucleic acid of the invention comprises nucleic acids encoding each of Acetyl-Coenzyme A acetyltransferase (Thiolase; ThlA), Acetoacetyl-CoA:Acetate Coenzyme A transferase A (CoA transferase; CtfA), Acetoacetyl-CoA:Acetate Coenzyme A transferase B (CoA transferase; CtfB), Acetoacetate decarboxylase (Adc), and Alcohol dehydrogenase (Adh2), or a functionally equivalent variant of any one or more thereof, in any order.

In another embodiment, a nucleic acid of the invention comprises nucleic acids encoding each of Acetyl-Coenzyme A acetyltransferase (Thiolase; ThlA), Acetoacetyl-CoA:Acetate Coenzyme A transferase A (CoA transferase), Acetoacetyl-CoA:Acetate Coenzyme A transferase B (CoA transferase; CtfB), Acetoacetate decarboxylase (Adc), Alpha-ketoisovalerate decarboxylase (decarboxylase; KivD), and Alcohol dehydrogenase (Adh2), or a functionally equivalent variant of any one or more thereof, in any order.

Exemplary amino acid sequences and nucleic acid sequence encoding each of the above enzymes are provided in GenBank as described elsewhere herein (see, in particular, the examples provided in tables 6 and 18 herein after). However, skilled persons will readily appreciate alternative nucleic acids sequences encoding the enzymes or functionally equivalent variants thereof, having regard to the information contained herein, in GenBank and other databases, and the genetic code.

In one embodiment, Alcohol Dehydrogenase (Adh2) has the sequence of SEQ_ID NO 75, or a functionally equivalent variant thereof.

In one embodiment, the nucleic acid sequence encoding Acetyl-Coenzyme A acetyltransferase (Thiolase; ThlA) comprises SEQ_ID NO. 18, or is a functionally equivalent variant thereof. In one embodiment, the nucleic acid sequence encoding Acetoacetyl-CoA:Acetate Coenzyme A transferase A (CoA transferase; CtfA) comprises SEQ_ID NO. 19, or is a functionally equivalent variant thereof. In one embodiment, the nucleic acid sequence encoding Acetoacetyl-CoA:Acetate Coenzyme A transferase B (CoA transferase; CtfB) comprises SEQ_ID NO. 20, or is a functionally equivalent variant thereof. In one embodiment, the nucleic acid sequence encoding Acetoacetate decarboxylase (Adc) comprises SEQ_ID NO. 21, or is a functionally equivalent variant thereof. In one particular embodiment, the nucleic acid sequence encoding Alcohol Dehydrogenase (Adh) comprises SEQ_ID NO. 2, SEQ_ID NO. 3, or SEQ_ID NO. 4, or is a functionally equivalent variant of any one thereof. In one disclosure, the functionally equivalent variant of SEQ_ID NO. 2, SEQ_ID NO. 3, or SEQ_ID NO. 4 has at least approximately 83%, 84%, 85%, 86%, 87%, 88%, 89%, 90%, 91%, 92%, 93%, 94%, 95%, 96%, 97%, 98% or 99% sequence identity to SEQ_ID NO. 2, 3 or 4.

The nucleic acids of the invention may remain extra-chromosomal upon transformation of a parental microorganism or may be adapted for intergration into the genome of the microorganism. Accordingly, nucleic acids of the invention may include additional nucleotide sequences adapted to assist integration (for example, a region which allows for homologous recombination and targeted integration into the host genome) or stable expression and replication of an extrachromosomal construct (for example, origin of replication, promoter and other regulatory sequences).

In one disclosure, the nucleic acid is nucleic acid construct or vector. In one particular disclosure, the nucleic acid construct or vector is an expression construct or vector.

It will be appreciated that an expression construct/vector may contain any number of regulatory elements in addition to the promoter as well as additional genes suitable for expression of further proteins if desired. In one disclosure the expression construct/vector includes one promoter. In another disclosure, the expression construct/vector includes two or more promoters. In one particular embodiment, the expression construct/vector includes one promoter for each gene to be expressed. In one disclosure, the expression construct/vector includes one or more ribosomal binding sites, preferably a ribosomal binding site for each gene to be expressed.

It will be appreciated by those of skill in the art that the nucleic acid sequences and construct/vector sequences described herein may contain standard linker nucleotides such as those required for ribosome binding sites and/or restriction sites. Such linker sequences should not be interpreted as being required and do not provide a limitation on the sequences defined.

Nucleic acids and nucleic acid constructs, including expression constructs/vectors may be constructed using any number of techniques standard in the art. For example, chemical synthesis or recombinant techniques may be used. Such techniques are described, for example, in Sambrook et al (Molecular Cloning: A laboratory manual, Cold Spring Harbor Laboratory Press, Cold Spring Harbor, NY, 1989). Further exemplary techniques are described in the Examples section herein after. Essentially, the individual genes and regulatory elements will be operably linked to one another such that the genes can be expressed to form the desired proteins. Suitable vectors for use in the invention will be appreciated by those of ordinary skill in the art. However, by way of example, the following vectors may be suitable: pMTL80000 vectors, pIMP1, pJIR750, and the plasmids exemplified in the Examples section herein after.

It should be appreciated that nucleic acids of the invention may be in any appropriate form, including RNA, DNA, or cDNA.

Also disclosed are host organisms, particularly microorganisms, and including viruses, bacteria, and yeast, comprising any one or more of the nucleic acids described herein.

The one or more exogenous nucleic acids may be delivered to a parental microorganism as naked nucleic acids or may be formulated with one or more agents to facilitate the tranformation process (for example, liposome-conjugated nucleic acid, an organism in which the nucleic acid is contained). The one or more nucleic acids may be DNA, RNA, or combinations thereof, as is appropriate. Restriction inhibitors may be used; see, for example Murray, N.E. et al. (2000) Microbial. Molec. Biol. Rev. 64, 412.)

The microorganisms of the invention may be prepared from a parental microorganism and one or more exogenous nucleic acids using any number of techniques known in the art for producing recombinant microorganisms. By way of example only, transformation (including transduction or transfection) may be achieved by electroporation, ultrasonication, polyethylene glycol-mediated transformation, chemical or natural competence, or conjugation. Suitable transformation techniques are described for example in, Sambrook J, Fritsch EF, Maniatis T: Molecular Cloning: A laboratory Manual, Cold Spring Harbour Labrotary Press, Cold Spring Harbour, 1989.

Due to the restriction systems which are active in the microorganism to be transformed, may be necessary to methylate the nucleic acid to be introduced into the microorganism. This can be done using a variety of techniques, including those described below, and further exemplified in the Examples section herein after.

By way of example, a recombinant microorganism is produced by a method comprises the following steps:
introduction into a shuttle microorganism of (i) of an expression construct/vector as described herein and (ii) a methylation construct/vector comprising a methyltransferase gene;
expression of the methyltransferase gene;
isolation of one or more constructs/vectors from the shuttle microorganism; and,
introduction of the one or more construct/vector into a destination microorganism.

Once the expression construct/vector and the methylation construct/vector are introduced into the shuttle microorganism, the methyltransferase gene present on the methylation construct/vector is induced. Induction may be by any suitable promoter system. Suitable promoters include the ara, tet, lac promoter, or T7 system.

In a particular disclosure, the methylation construct/vector has an origin of replication specific to the identity of the shuttle microorganism so that any genes present on the methylation construct/vector are expressed in the shuttle microorganism. Preferably, the expression construct/vector has an origin of replication specific to the identity of the destination microorganism so that any genes present on the expression construct/vector are expressed in the destination microorganism.

Expression of the methyltransferase enzyme results in methylation of the genes present on the expression construct/vector. The expression construct/vector may then be isolated from the shuttle microorganism according to any one of a number of known methods. By way of example only, the methodology described in the Examples section described hereinafter may be used to isolate the expression construct/vector.

The expression construct/vector may be introduced into the destination microorganism using any number of known methods. However, by way of example, the methodology described in the Examples section hereinafter may be used. Since the expression construct/vector is methylated, the nucleic acid sequences present on the expression construct/vector are able to be incorporated into the destination microorganism and successfully expressed.

It is envisaged that a methyltransferase gene may be introduced into a shuttle microorganism and over-expressed. Thus, in one disclosure, the resulting methyltransferase enzyme may be collected using known methods and used *in vitro* to methylate an expression plasmid. The expression construct/vector may then be introduced into the destination microorganism for expression. In another disclosure, the methyltransferase gene is introduced into the genome of the shuttle microorganism followed by introduction of the expression construct/vector into the shuttle microorganism, isolation of one or more constructs/vectors from the shuttle microorganism and then introduction of the expression construct/vector into the destination microorganism.

It is envisaged that the expression construct/vector and the methylation construct/vector as defined above may be combined to provide a composition of matter. Such a composition has particular utility in circumventing restriction barrier mechanisms to produce the recombinant microorganisms of the invention.

In one particular disclosure, the expression construct/vector and/or the methylation construct/vector are plasmids.

Persons of ordinary skill in the art will appreciate a number of suitable methyltransferases of use in producing the microorganisms of the invention. However, by way of example the *Bacillus subtilis* phage ΦT1 methyltransferase and the methyltransferase described in the Examples herein after may be used.

Any number of constructs/vectors adapted to allow expression of a methyltransferase gene may be used to generate the methylation construct/vector. However, by way of example, the plasmid described in the Examples section hereinafter may be used.

The invention provides a method for the production of one or more desirable products (acetone, isopropanol, and/or or a precursor of acetone and/or isopropanol) by microbial fermentation comprising fermenting a substrate comprising CO using a recombinant microorganism of the invention. The methods of the invention may be used to reduce the total atmospheric carbon emissions from an industrial process.

Preferably, the fermentation comprises the steps of anaerobically fermenting a substrate in a bioreactor to produce the one or more products using a recombinant microorganism of the invention.

In one disclosure the method comprises the steps of:
(a) providing a substrate comprising CO to a bioreactor containing a culture of one or more microorganism of the invention; and
(b) anaerobically fermenting the culture in the bioreactor to produce the one or more products.

In one embodiment the method comprises the steps of:
(a) capturing CO-containing gas produced as a result of the industrial process, before the gas is released into the atmosphere;
(b) the anaerobic fermentation of the CO-containing gas to produce the one or more products by a culture containing one or more microorganism of the invention.

In an embodiment of the invention, the gaseous substrate fermented by the microorganism is a gaseous substrate containing CO. The gaseous substrate may be a CO-containing waste gas obtained as a by-product of an industrial process, or from some other source such as from automobile exhaust fumes. In certain embodiments, the industrial process is selected from the group consisting of ferrous metal products manufacturing, such as a steel mill, non-ferrous products manufacturing, petroleum refining processes, gasification of coal, electric power production, carbon black production, ammonia production, methanol production and coke manufacturing. In these embodiments, the CO-containing gas may be captured from the industrial process before it is emitted into the atmosphere, using any convenient method. The CO may be a component of syngas (gas comprising carbon monoxide and hydrogen). The CO produced from industrial processes is normally flared off to produce CO₂ and therefore the invention has particular utility in reducing CO₂ greenhouse gas emissions and producing butanol for use as a biofuel. Depending on the composition of the gaseous CO -containing substrate, it may also be desirable to treat it to remove any undesired impurities, such as dust particles before introducing it to the fermentation. For example, the gaseous substrate may be filtered or scrubbed using known methods.

It will be appreciated that for growth of the bacteria and CO-to-the one or more product(s) to occur, in addition to the CO-containing substrate gas, a suitable liquid nutrient medium will need to be fed to the bioreactor. The substrate and media may be fed to the bioreactor in a continuous, batch or batch fed fashion. A nutrient medium will contain vitamins and minerals sufficient to permit growth of the micro-organism used. Anaerobic media suitable for fermentation to produce butanol using CO are known in the art. For example, suitable media are described Biebel (2001). In one disclosure the media is as described in the Examples section herein after.

The fermentation should desirably be carried out under appropriate conditions for the CO-to-the one or more product(s) fermentation to occur. Reaction conditions that should be considered include pressure, temperature, gas flow rate, liquid flow rate, media pH, media redox potential, agitation rate (if using a continuous stirred tank reactor), inoculum level, maximum gas substrate concentrations to ensure that CO in the liquid phase does not become limiting, and maximum product concentrations to avoid product inhibition.

In addition, it is often desirable to increase the CO concentration of a substrate stream (or CO partial pressure in a gaseous substrate) and thus increase the efficiency of fermentation reactions where CO is a substrate. Operating at increased pressures allows a significant increase in the rate of CO transfer from the gas phase to the liquid phase where it can be taken up by the micro-organism as a carbon source for the production of the one or more products. This in turn means that the retention time (defined as the liquid volume in the bioreactor divided by the input gas flow rate) can be reduced when bioreactors are maintained at elevated pressure rather than atmospheric pressure. The optimum reaction conditions will depend partly on the particular micro-organism of the invention used. However, in general, it is preferred that the fermentation be performed at pressure higher than ambient pressure. Also, since a given CO-to-the one or more product(s) conversion rate is in part a function of the substrate retention time, and achieving a desired retention time in turn dictates the required volume of a bioreactor, the use of pressurized systems can greatly reduce the volume of the bioreactor required, and consequently the capital cost of the fermentation equipment. According to examples given in US patent no. 5,593,886, reactor volume can be reduced in linear proportion to increases in reactor operating pressure, i.e. bioreactors operated at 10 atmospheres of pressure need only be one tenth the volume of those operated at 1 atmosphere of pressure.

By way of example, the benefits of conducting a gas-to-ethanol fermentation at elevated pressures has been described. For example, WO 02/08438 describes gas-to-ethanol fermentations performed under pressures of 30 psig and 75 psig, giving ethanol productivities of 150 g/l/day and 369 g/l/day respectively. However, example fermentations performed using similar media and input gas compositions at atmospheric pressure were found to produce between 10 and 20 times less ethanol per litre per day.

It is also desirable that the rate of introduction of the CO-containing gaseous substrate is such as to ensure that the concentration of CO in the liquid phase does not become limiting. This is because a consequence of CO-limited conditions may be that the ethanol product is consumed by the culture.

The composition of gas streams used to feed a fermentation reaction can have a significant impact on the efficiency and/or costs of that reaction. For example, O2 may reduce the efficiency of an anaerobic fermentation process. Processing of unwanted or unnecessary gases in stages of a fermentation process before or after fermentation can increase the burden on such stages (e.g. where the gas stream is compressed before entering a bioreactor, unnecessary energy may be used to compress gases that are not needed in the fermentation). Accordingly, it may be desirable to treat substrate streams, particularly substrate streams derived from industrial sources, to remove unwanted components and increase the concentration of desirable components.

In certain disclosures a culture of a bacterium of the invention is maintained in an aqueous culture medium. Preferably the aqueous culture medium is a minimal anaerobic microbial growth medium. Suitable media are known in the art and described for example in US patent no.s 5,173,429 and 5,593,886 and WO 02/08438, and as described in the Examples section herein after.

Acetone, isopropanol, or a mixed stream containing acetone and/or isopropanol and/or one or more other products, may be recovered from the fermentation broth by methods known in the art, such as fractional distillation or evaporation, pervaporation, gas stripping and extractive fermentation, including for example, liquid-liquid extraction.

In certain disclosures, the one or more products are recovered from the fermentation broth by continuously removing a portion of the broth from the bioreactor, separating microbial cells from the broth (conveniently by filtration), and recovering one or more products from the broth. Alcohols may conveniently be recovered for example by distillation. Acetone may be recovered for example by distillation.. Any acids produced may be recovered for example by adsorption on activated charcoal. The separated microbial cells are preferably returned to the fermentation bioreactor. The cell free permeate remaining after any alcohol(s) and acid(s) have been removed is also preferably returned to the fermentation bioreactor. Additional nutrients (such as B vitamins) may be added to the cell free permeate to replenish the nutrient medium before it is returned to the bioreactor.

Also, if the pH of the broth was adjusted as described above to enhance adsorption of acetic acid to the activated charcoal, the pH should be re-adjusted to a similar pH to that of the broth in the fermentation bioreactor, before being returned to the bioreactor.

### Examples:

The invention will now be described in more detail with reference to the following non-limiting examples.

### Microorganisms and Growth conditions

*Acetobacterium woodii* DSM1030, *Clostridium aceticum* DSM1496, *C*. *autoethanogenum* DSM23693, *C*. *carboxidivorans* DSM15243, and *C*. *Ijungdahlii* DSM13528 were sourced from DSMZ (The German Collection of Microorganisms and Cell Cultures, Inhoffenstraße 7 B, 38124 Braunschweig, Germany). *C. autoethanogenum* DSM23693 is a derivate of C. *autoethanogenum* DSM10061.

*C. ragsdalei* ATCC BAA-622 were sourced from the American Type Culture Collection, Manassas, VA 20108, USA.

*C. acetobutylicum* ATCC824, C. *beijerinckii* NRRL-B593, and *C. beijerinckii* NCIMB8052 were obtained from Prof. David Jones (University of Otago) and can also be obtained from public strain collections DSMZ and ATCC under accession numbers ATCC824/DSM792, DSM6423, and ATCC51743 respectively.

*Escherichia coli* DH5α-T1^{R} was sourced from Invitrogen, Carlsbad, CA 92008, USA and *Escherichia coli* XL1-Blue MRF' Kan and ABLE K from Stratagene (Santa Clara, CA 95051-7201, USA). *Escherichia coli* JW3350-2 was sourced from The Coli Genetic Stock Center (CGSC), New Haven, CT 06520-8103.

*E. coli* was cultivated under both aerobic and anaerobic conditions, while all other strains were grown strictly anaerobically in a volume of 50 ml liquid media in serum bottles with fructose (heterotrophic growth) or 30 psi CO-containing steel mill gas (collected from New Zealand Steel site in Glenbrook, NZ; composition: 44% CO, 32% N₂, 22% CO₂, 2% H₂) in the headspace (autotrophic growth).

Media was prepared using standard anaerobic techniques [Hungate RE: A roll tube method for cultivation of strict anaerobes, in Norris JR and Ribbons DW (eds.), Methods in Microbiology, vol. 3B. Academic Press, New York, 1969: 117-132; Wolfe RS: Microbial formation of methane. Adv Microb Physiol 1971, 6: 107-146] according to formulations are given in Tab. 2-4. For solid media, 1.2 % Bacto agar (BD, Frankton Lakes, NJ 07417, USA) was added.

All strains were grown at 37 °C, except for *A. woodii, C. aceticum,* and *C. ragsdalei* which were grown at 30 °C.

**Tab. 2: PETC medium (A. woodii, pH8.2; C. aceticum, pH7.4; C. autoethanogenum, C. carboxidivorans, C. ljungdahlii, and C. ragsdalei, pH5.6)**

| **Media component** | **Concentration per 1.0L of media** |
|---|---|
| NH₄Cl | 1 g |
| KCl | 0.1 g |
| MgSO₄.7H₂O | 0.2 g |
| NaCl | 0.8 g |
| KH₂PO₄ | 0.1 g |
| CaCl₂ | 0.02 g |
| Trace metal solution (see below) | 10 ml |
| Wolfe's vitamin solution (see below) | 10 ml |
| Yeast Extract (optional) | 1 g |
| Resazurin (2 g/L stock) | 0.5 ml |
| NaHCO₃ | 2g |
| Reducing agent | 0.006-0.008 % (v/v) |
| Fructose (for heterotrophic growth) | 5 g |

| **Trace metal solution** | **per L of stock** |
|---|---|
| **Nitrilotriacetic Acid** | **2 g** |
| MnSO₄.H₂O | 1 g |
| Fe (SO₄)₂(NH₄)₂.6H₂O | 0.8 g |
| CoCl₂.6H₂O | 0.2 g |
| ZnSO₄.7H₂O | 0.2 mg |
| CuCl₂.2H₂O | 0.02 g |
| NaMoO₄.2H₂O | 0.02 g |
| Na₂SeO₃ | 0.02 g |
| NiCl₂.6H₂O | 0.02 g |
| Na₂WO₄.2H₂O | 0.02 g |

| **Reducing agent stock** | **per 100 mL of stock** |
|---|---|
| NaOH | 0.9 g |
| Cystein.HCl | 4 g |
| Na₂S | 4g |

**Tab. 3: Reinforced Clostridial Medium RCM (C. acetobutylicum, C. beijerinckii)**

| **Media component** | **Concentration per 1.0L of media** |
|---|---|
| Pancreatic Digest of Casein | 5 g |
| Proteose Peptone No. 3 | 5 g |
| Beef Extract | 10 g |
| Yeast Extract | 3 g |
| Dextrose | 5 g |
| NaCl | 5 g |
| Soluble starch | 1 g |
| Cystein.HCl | 0.5 g |
| Sodium Acetate | 3 g |

**Tab. 4: Luria Bertani medium LB (E. coli)**

| **Media component** | **Concentration per 1.0L of media** |
|---|---|
| Tryptone | 10 g |
| Yeast Extract | 5 g |
| NaCl | 10 g |

**Tab. 5: SD-8 minimal media (E. coli)**

| **Media component** | **Concentration per 1.0L of media** |
|---|---|
| NH₄Cl | 7 g |
| Na₂HPO₄ | 7.5 g |
| K₂SO₄ | 0.85 g |
| MgSO₄.7H₂O | 0.17 g |
| KH₂PO₄ | 7.5 g |
| Trace metal solution (see below) | 0.8 ml |
| Yeast Extract | 5 g |
| Glucose | 20 g |

| **Trace metal solution** | **per 100L of stock** |
|---|---|
| MnSO₄.H₂O | 1 g |
| FeSO₄.7H₂O | 4 g |
| COCl₂.6H₂O | 0.4 g |
| ZnSO₄.7H₂O | 0.2 g |
| CuCl₂.2H₂O | 0.1 g |
| Na₂MoO₄.2H₂O | 0.2 g |
| Al₂(SO₄)₃ | 2.83 g |
| H₃BO₄ | 0.5 g |

Fermentations with *C*. *autoethanogenum* DSM23693 were carried out in 1.5L bioreactors at 37°C and CO-containing steel mill gas as sole energy and carbon source as described below. A defined medium was used containing per litre: MgCl, CaCl₂ (0.5mM), KCl (2mM), H₃PO₄ (5mM), Fe (100µM), Ni, Zn (5µM), Mn, B, W, Mo, Se(2 µM) was prepared for culture growth. The media was transferred into the bioreactor and autoclaved at 121°C for 45 minutes. After autoclaving, the medium was supplemented with Thiamine, Pantothenate (0.05mg), Biotin (0.02mg) and reduced with 3mM Cysteine-HCl. To achieve anaerobicity the reactor vessel was sparged with nitrogen through a 0.2 µm filter. Prior to inoculation, the gas was switched to CO-containing steel mill gas, feeding coniniously to the reactor. The gas flow was initially set at 80 ml/min, increasing to 200 ml/min during mid exponential phase, while the agitation was increased from 200 rpm to 350. Na₂S was dosed into the bioreactor at 0.25 ml/hr. Once the OD600 reached 0.5, the bioreactor was switched to a continuous mode at a rate of 1.0 ml/min (Dilution rate 0.96 d⁻¹). Media samples were taken to measure the biomass and metabolites and a headspace analysis of the in- and outflowing gas was performed on regular basis.

### Analysis of metabolites

HPLC analysis of acetone, isopropanol and other metabolites was performed using an Agilent 1100 Series HPLC system equipped with a RID operated at 35 °C (Refractive Index Detector) and an Alltech IOA-2000 Organic acid column (150 x 6.5 mm, particle size 5 µm) kept at 60 °C. Slightly acidified water was used (0.005 M H₂SO₄) as mobile phase with a flow rate of 0.7 ml/min. To remove proteins and other cell residues, 400 µl samples were mixed with 100 µl of a 2 % (w/v) 5-Sulfosalicylic acid and centrifuged at 14,000 x g for 3 min to separate precipitated residues. 10 µl of the supernatant were then injected into the HPLC for analyses.

GC analysis of acetone, isopropanol and other metabolites was performed using an Agilent 6890N headspace GC equipped with a Supelco PDMS 100 1cm fiber, an Alltech EC-1000 (30m x 0.25mm x 0.25 µm) column, and a flame ionization detector (FID). 5 ml samples were transferred into a Hungate tube, heated to 40 °C in a water bath and exposed to the fiber for exactly 5 min. The injector was kept at 250 °C and helium with a constant flow of 1 ml/min was used as carrier gas. The oven program was 40 °C for 5 min, followed by an increase of 10 °C/min up to 200 °C. The temperature was then further increased to 220 °C with a rate of 50 °C/min followed by a 5 min hold this temperature, before the temperature was decreased to 40 °C with a rate of 50 °C/min and a final 1 min hold. The FID was kept at 250 °C with 40 ml/min hydrogen, 450 ml/min air and 15 ml/min nitrogen as make up gas.

### Headspace Analysis

Measurements were carried out on a Varian CP-4900 micro GC with two installed channels. Channel 1 was a 10m Mol-sieve column running at 70°C, 200kPa argon and a backflush time of 4.2s, while channel 2 was a 10m PPQ column running at 90°C, 150kPa helium and no backflush. The injector temperature for both channels was 70°C. Runtimes were set to 120s, but all peaks of interest would usually elute before 100s.

### Genetic modification of C. autoethanogenum and C. ljungdahlii for acetone production using Clostridial pathway

*C. autoethanogenum* and *C*. *ljungdahlii* are naturally not able to produce acetone, therefore the acetone biosynthesis pathway occurring in other Clostridial species was introduced into both organisms (Fig. 4). The first step in the Clsotridial acetone biosynthesis pathway from acetyl-CoA to acetoacetyl-CoA is catalysed by a acetyl-Coenzyme A acetyltransferase or thiolase. The conversion of acetoacetyl-CoA to acetone is then catalysed by a specialized set of enzymes acetate/butyrate-acetoacetate CoA-transferase complex and acetoacetate decarboxylase, which can be found in few organisms like C. *acetobutylicum* and C. *beijerinckii* (Tab. 6).

**Tab. 6: Accession numbers of genes and enzymes involved in acetone and isopropanol formation.**

| **Description** | ***C. acetobutylicum*** | | ***C. beijerinckii*** | |
|---|---|---|---|---|
| | **nucleic acid** | **amino acid** | **nucleic acid** | **Amino acid** |
| **Thiolase (ThlA)** | NC_003030.1; GI: 1119056 | NP_349476.1 | NC_009617; GI: 5294796 | YP_001310706.1 |
| **Acetate/Butyrate-acetoacetate CoA-transferase subunit A (CtfA)** | NC_001988.2; GI: 1116168 | NP_149326.1 | NC_009617; GI: 5294994 | YP_001310904.1 |
| **Acetate/Butyrate-acetoacetate CoA-transferase subunit A CtfB** | NC_001988.2; GI: 1116169 | NP_149327.1 | NC_009617; GI: 5294995 | YP_001310905.1 |
| **Acetoacetate decarboxylase (Adc)** | NC_001988.2; GI: 1116170 | NP_149328.1 | NC_009617; GI: 5294996 | YP_001310906.1 |

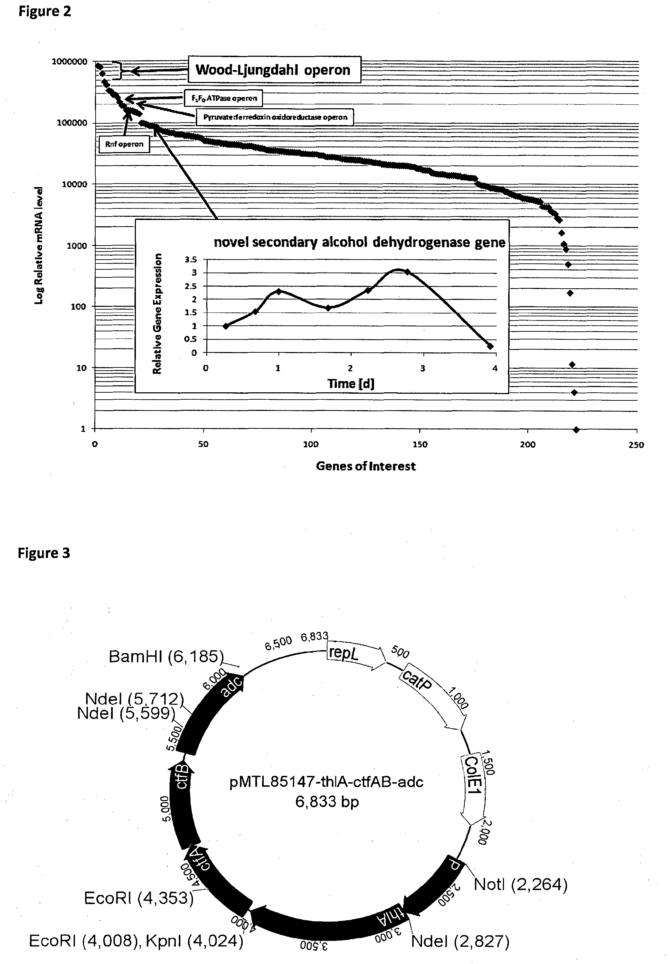

Whereas the genes of *C. acetobutylicum* encoding the respective enzymes are split into 2 operons, the genes of *C. beijerinckii* form a common operon, which the inventor(s) believe offers an advantage. The genes encoding a thiolase from *C. acetobutylicum* and the operon coding for enzymes acetate/butyrate-acetoacetate CoA-transferase subunit A, acetate/butyrate-acetoacetate CoA-transferase subunit B and acetoacetate decarboxylase were assembled into a synthetic operon under control of a strong, native *C. autoethanogenum* promoter (Fig. 3). This construct was used to genetically engineer both organism for acetone production. In order to create a recombinant strain, a novel methyltransferase was used to methylate the construct, which was then transformed and expressed in *C. autoethanogenum* DSM23693 and *C. Ijungdahlii* DSM13528 (described herein after). Production of acetone was shown on different industrial gas streams (steel mill waste gas, syngas).

### Construction of expression plasmid with Clostridial acetone pathway genes:

Standard Recombinant DNA and molecular cloning techniques were used in this invention [Sambrook J, Fritsch EF, Maniatis T: Molecular Cloning: A laboratory Manual, Cold Spring Harbour Labrotary Press, Cold Spring Harbour, 1989; Ausubel FM, Brent R, Kingston RE, Moore DD, Seidman JG, Smith JA, Struhl K: Current protocols in molecular biology. John Wiley & Sons, Ltd., Hoboken, 1987]. DNA sequences of acetone biosynthetic genes are shown in Tab. 7. The Wood-Ljungdahl cluster promoter of *C. autoethanogenum* (upstream of CO dehydrogenase gene *acsA*) was used for expression of target genes (Tab. 7).

**Tab. 7: Sequences used for Clostridial acetone expression plasmid**

| **Description** | **Source** | **SEQ_ID NO.** |
|---|---|---|
| Thiolase (*thlA*) | *Clostridium acetobutylicum* ATCC 824; NC_003030.1; GI: 1119056 | 18 |
| Acetoa cetyl-CoA: acetate Coenzyme A transferase A *(ctfA),* acetoacetyl-CoA:acetate Coenzyme A transferase B (*ctfB*), and acetoacetate decarboxylase (*adc*) operon | *Clostridium beijerinckii* NCIMB 8052; NC_009617; region: 4,400,524-4,402,656; including GI: 5294994, GI: 5294995, and GI: 5294996 | 47 |
| Wood-Ljungdahl cluster promoter (*P_{wL}*) | *Clostridium autoethanogenum* DSM10061 | 22 |

Genomic DNA from *Clostridium acetobutylicum* ATCC824, *C. beijerinckii* NCIMB8052 and *C. autoethanogenum* DSM10061 was isolated using a modified method by Bertram and Dürre (Conjugal transfer and expression of streptococcal transposons in Clostridium acetobutylicum. Arch Microbiol 1989, 151: 551-557). A 100-ml overnight culture was harvested (6,000 x g, 15 min, 4 °C), washed with potassium phosphate buffer (10 mM, pH 7.5) and suspended in 1.9 ml STE buffer (50 mM Tris-HCl, 1 mM EDTA, 200 mM sucrose; pH 8.0). 300 µl lysozyme (∼100,000 U) were added and the mixture was incubated at 37 °C for 30 min, followed by addition of 280 µl of a 10 % (w/v) SDS solution and another incubation for 10 min. RNA was digested at room temperature by addition of 240 µl of an EDTA solution (0.5 M, pH 8), 20 µl Tris-HCl (1 M, pH 7.5), and 10 µl RNase A. Then, 100 µl Proteinase K (0.5 U) were added and proteolysis took place for 1-3 h at 37 °C. Finally, 600 µl of sodium perchlorate (5 M) were added, followed by a phenolchloroform extraction and an isopropanol precipitation. DNA quantity and quality was inspected spectrophotometrically.

Acetone biosynthetic genes and the Wood-Ljungdahl cluster promoter were amplified by PCR with oligonucleotides in Tab. 8 using iProof High Fidelity DNA Polymerase (Bio-Rad Labratories, Hercules, CA 94547, USA) and the following program: initial denaturation at 98 °C for 30 seconds, followed by 32 cycles of denaturation (98 °C for 10 seconds), annealing (50-62 °C for 30-120 seconds) and elongation (72 °C for 45 seconds), before a final extension step (72 °C for 10 minutes).

**Tab. 8: Oligonucleotides used for amplification of acetone biosynthesis genes and promoter region**

| **Description** | **Oligonucleotide Name** | **DNA Sequence (5' to 3')** | **SEQ_ID NO.** |
|---|---|---|---|
| ThlA | ThlA-Cac-NdeI-F | GTTCATATGAAAGAAGTTGTAATAGC | 23 |
| | ThlA-Cac-EcoRI-R | CAAGAATTCCTAGCACTTTTCTAGC | 24 |
| CtfA, CtfB, Adc operon | Ctf-adc-cbei-KpnI-F | CTAGGTACCAGGGAGATATTAAAATG | 25 |
| | Ctf-adc-cbei-BamH1-R | CGTGGATCCTCTATATTGCTTTTATT | 26 |
| P_{WL} | Pwoodlj-NotI-F | AAGCGGCCGCAGATAGTCATAATAGTTCC | 27 |
| | Pwoodlj-NdeI-R | TTCCATATGAATAATTCCCTCCTTAAAGC | 28 |

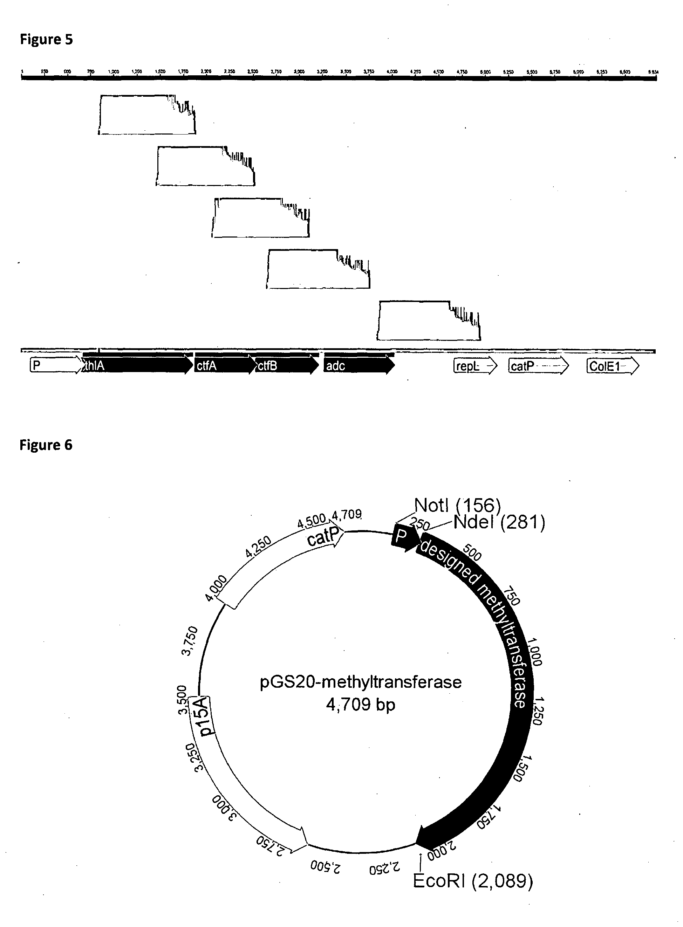

The amplified 573 bp promoter region of the Wood-Ljungdahl cluster (P*_{WL}*) was cloned into the *E*. *coli-Clostridium* shuttle vector pMTL 85141 (FJ797651.1; Nigel Minton, University of Nottingham, UK) [Heap JT, Pennington OJ, Cartman ST, Minton NP. A modular system for Clostridium shuttle plasmids. J Microbiol Methods. 2009, 78: 79-85] using *Not*I and *Nde*I restriction sites and strain DH5α-T1^{R}. The created plasmid pMTL85147 and the 1,194 bp PCR product of the thiolase gene were both cut with *Nde*I and *EcoR*I*.* A ligation was transformed into *E. coli* XL1-Blue MRF' Kan resulting in plasmid pMTL85147-thlA. Subsequently, the amplified 2,177 bp PCR fragment of the *ctfA-ctfB-adc* operon from *C. beijerinckii* NCIMB 8052 was cloned into this vector using *Kpn*I and *Bam*HI and *E. coli* ABLE K, creating plasmid pMTL85147-thlA-ctfA-ctfB-adc. The insert of the resulting plasmid pMTL85147-thlA-ctfAB-adc was completely sequenced using oligonucleotides given in Tab. 9 and results confirmed that the acetone biosynthesis genes and promoter region were free of mutations (Fig. 5).

**Tab. 9: Oligonucleotides used for sequencing**

| **Oligonucleotide Name** | **DNA Sequence (5' to 3')** | **SEQ_ID NO.** |
|---|---|---|
| Seq-ThlA-CtfAB-Adh 3539-4139 | CAGAGGATGTTAATGAAGTC | 29 |
| Seq-ThlA-CtfAB-Adh 4140-4740 | CTGTGCAGCAGTACTTGT | 30 |
| Seq-ThlA-CtfAB-Adh 4741-5341 | GCAATGATACAGCTT | 31 |
| Seq-ThlA-CtfAB-Adh 5342-5942 | AACCTTGGAATAGGACTTC | 32 |
| Seq-ThlA-CtfAB-Adh 6544-7144 | TGTGAACTAATATGTGCAGA | 33 |
| M13 Forward | GTAAAACGACGGCCAG | 56 |
| M13 Reverse | CAGGAAACAGCTATGAC | 57 |

### Acetone production in E. coli with Clostridial acetone pathway genes:

To confirm the functionality of the constructed plasmid, a metabolic profile from a 5 ml overnight culture of *E. coli* ABLE K harbouring plasmid pMTL85147-thlA-ctfA-ctfB-adc were obtained using GC and HPLC, confirming acetone production.

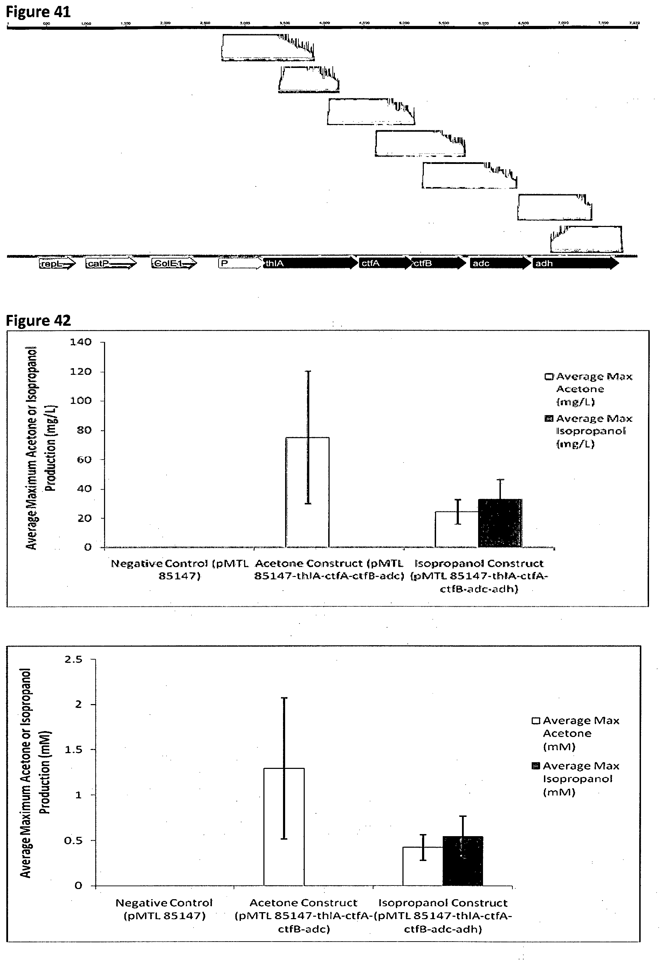

To investigate this further, detailed growth experiments were carried out in triplicates with SD-8 minimal media containing 25 µg/ml chloramphenicol and *E. coli* XL-1 Blue MRF' Kan carrying either plasmid pMTL 85147 (negative control) or expression plasmid pMTL 85147-thlA-ctfA-ctfB-adc (Fig. 42). While no acetone production could be observed in the negative control, an average maximum acetone production of 75.05 mg/L with an average dry biomass of 1.44 g/L was measured for the strain carrying the acetone plasmid.

### Methylation of expression plasmid with Clostridial acetone pathway genes:

Methylation of the acetone expression plasmid pMTL85147-thlA-ctfA-ctfB-adc was performed *in vivo* in *E. coli* using a synthesized hybrid Type II methyltransferase gene (SEQ_ID NO 35) designed from methyltransferase genes from *C*. *autoethanogenum, C. ragsdalei* and *C*. *ljungdahlii.* The methyltransferase (SEQ_ID NO 34) was synthesized and fused with an inducible *lac* promoter in vector pGS20 (ATG:biosynthetics GmbH, Merzhausen, Germany) (Fig. 6; SEQ_ID NO 49).

Both expression plasmid and methylation plasmid were transformed into same cells of restriction negative E. coli XL1-Blue MRF' Kan, which is possible due to their compatible Gram-(-) origins of replication (high copy ColE1 in expression plasmid and low copy p15A in methylation plasmid). In *vivo* methylation was induced by addition of 1 mM IPTG, and methylated plasmids were isolated using QIAGEN Plasmid Midi Kit (QIAGEN GmbH, Hilden, Germany). The resulting mix was used for transformation experiments with *C*. *autoethanogenum* DSM23693 and *C*. *ljungdahlii* DSM 13528, but only the abundant (high-copy) expression plasmid has a Gram-(+) replication origin *(repL)* allowing to replicate in Clostridia.

### Transformation of methylated acetone expression plasmid in C. autoethanogenum and C. ljungdahlii:

To make competent cells of *C*. *autoethanogenum* DSM23693 and *C*. *ljungdahlii* DSM 13528, a 50 ml culture (PETC media (Tab. 2) with steel mill gas and fructose as carbon source; 37 °C) was subcultured to fresh media for 3 consecutive days. These cells were used to inoculate 50 ml PETC media containing 40 mM DL-threonine at an OD₆₀₀ₙₘ of 0.05. When the culture reached an OD₆₀₀ₙₘ of 0.4, the cells were transferred into an anaerobic chamber and harvested at 4,700 x g and 4 °C. The culture was twice washed with ice-cold electroporation buffer (270 mM sucrose, 1 mM MgCl2, 7 mM sodium phosphate, pH 7.4) and finally suspended in a volume of 500 µl fresh electroporation buffer. This mixture was transferred into a pre-cooled electroporation cuvette with a 0.4 cm electrode gap containing ∼1 µg of the methylated plasmid mix. Since an additional Type I restriction system was identified in the genome of *C. ljungdahlii* compared to *C. autoethanogenum,* 5 µl of a Type I restriction inhibitor (EPICENTRE Biotechnologies, Madison, WI 53713, USA) were added to the plasmid mix, which increased the transformation efficiency of *C. ljungdahlii* by 2-10 fold. The cells were mixed with plasmid and restriction inhibitor and immediately pulsed using a Gene pulser Xcell electroporation system (Bio-Rad Labratories, Hercules, CA 94547, USA) with the following settings: 2.5 kV, 600 Ω, and 25 µF. Time constants were between 3.7-5.1 ms. For regeneration, the culture was transferred in 5 ml special regeneration media (Tab. 10), which increased recovery of the cells, which was monitored at a wavelength of 600 nm using a Spectronic Helios Epsilon Spectrophotometer (Thermo Fisher Scientific Inc., Waltham MA 02454, USA) equipped with a tube holder. Once growth was observed (one doubling) the cells were harvested, suspended in 200 µl fresh media and plated on selective PETC plates with 15 µg/ml thiamphenicol (dissolved in 100 % (v/v) dimethylfuran (DMF)) and 30 psi steel mill gas in the headspace. 50-200 colonies were visible after 4-6 days, which were used to inoculate 2 ml PETC media containing 15 µg/ml thiamphenicol (in DMF) and fructose and 30 psi steel mill gas as carbon source. When growth occurred, the culture was up-scaled into 5 ml and later 50 ml PETC media containing each 15 µg/ml thiamphenicol (in DMF) and 30 psi steel mill gas in the headspace as sole carbon source.

**Tab. 10: Regeneration media**

| **Media component** | **Concentration per 1.0L of media** |
|---|---|
| NH₄Cl | 1 g |
| KCl | 0.1 g |
| MgSO₄.7H₂O | 0.2 g |
| KH₂PO₄ | 0.2 g |
| CaCl₂ | 0.02 g |
| Trace metal solution (see Tab. 2) | 10 ml |
| Wolfe's vitamin solution (see Tab. 2) | 10 ml |
| Yeast Extract | 2 g |
| Resazurin (2 g/L stock) | 0.5 ml |
| 2-(*N*-morpholino)ethanesulfonic acid (MES) | 20 g |
| Reducing agent | 0.006-0.008 % (v/v) |
| Fructose | 5 g |
| Sodium acetate | 0.25 g |
| Fe (SO₄)₂(NH₄)₂.6H₂O | 0.05 g |
| Nitriolotriacetic Acid | 0.05 g |
| pH 5.7 | Adjusted with NaOH |

### Confirmation of successful transformation of C. autoethanogenum and C. ljungdahlii with acetone plasmid with Clostridial acetone pathway genes:

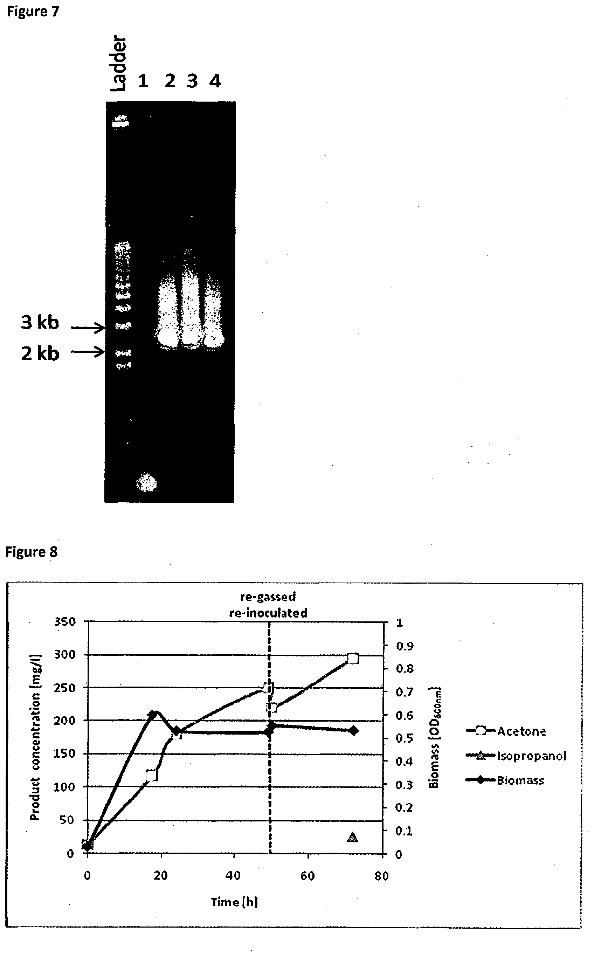

To verify the DNA transfer, a plasmid mini prep was performed from 10 ml culture volume using Zyppy plasmid miniprep kit (Zymo Research, Irvine, CA 92614, USA). Since the quality of the isolated plasmid wasn't sufficient for a restriction digest due to Clostridial exonuclease activity [Burchhardt G and Dürre P, Isolation and characterization of DNase-deficient mutants of Clostridium acetobutylicum. Curr Microbiol 1990, 21: 307-311] with the isolated plasmid as template using primers ctf-adc-cbei-KpnI-F (Seq_ID no 25) and ctf-adc-cbei-BamH1-R (SEQ_ID NO 26) to confirm the presence of the plasmid (Fig. 7). PCR was carried out using iNtRON Maximise Premix PCR kit (Intron Bio Technologies) with the following conditions: initial denaturation at 94 °C for 2 minutes, followed by 35 cycles of denaturation (94 °C for 20 seconds), annealing (55 °C for 20 seconds) and elongation (72 °C for 135 seconds), before a final extension step (72 °C for 5 minutes).

To confirm the identity of the clones, genomic DNA was isolated using the protocol given above from 50 ml cultures of each *C*. *autoethanogenum* DSM23693 and *C*. *ljungdahlii* DSM13528. A PCR was performed against the 16s rRNA gene using oligonucleotides fD1 (SEQ_ID NO 36: CCGAATTCGTCGACAACAGAGTTTGATCCTGGCTCAG) and rP2 (SEQ_ID NO 37: CCCGGGATCCAAGCTTACGGCTACCTTGTTACGACTT) [Weisberg WG, Barns SM, Pelletier BA and Lane DJ, 16S ribosomal DNA amplification for phylogenetic study. J Bacteriol 1990, 173: 697-703] and iNtRON Maximise Premix PCR kit (Intron Bio Technologies, Sangdaewon Joongwon Seognam Kyunggi, Korea) with the following conditions: initial denaturation at 94 °C for 2 minutes, followed by 35 cycles of denaturation (94 °C for 20 seconds), annealing (55 °C for 20 seconds) and elongation (72 °C for 60 seconds), before a final extension step (72 °C for 5 minutes). All sequences obtained had >99.9 % identity against the 16s rRNA gene (*rrsA*) of C. *autoethanogenum* (Y18178, GI:7271109) and respectively *C*. *ljungdahlii* (CP001666.1; GI:300433347).

### Acetone production from CO and CO₂/H₂ with with Clostridial acetone pathway genes in C. autoethanogenum and C. jungdahlii:

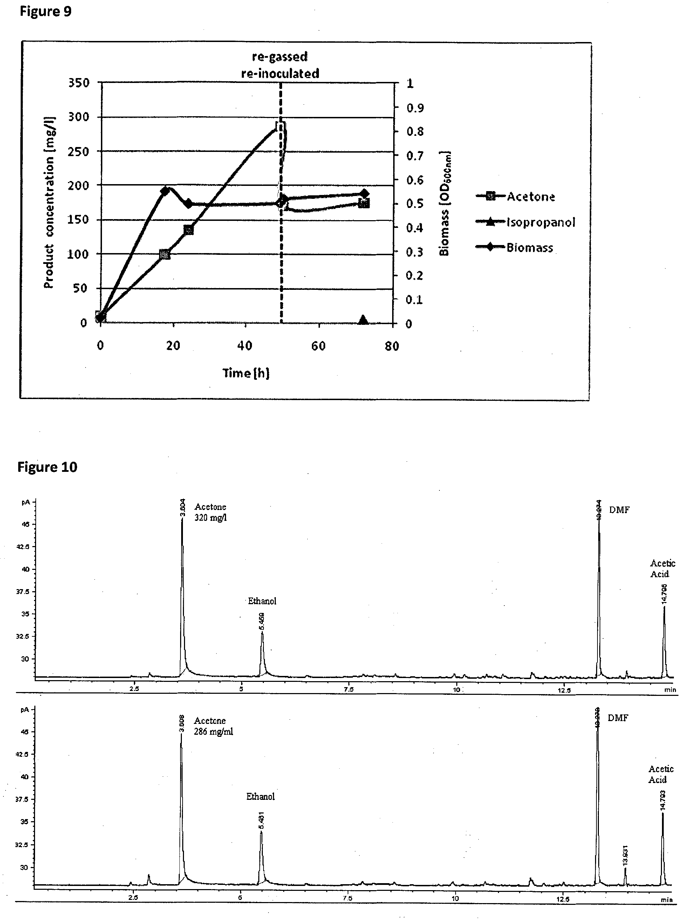

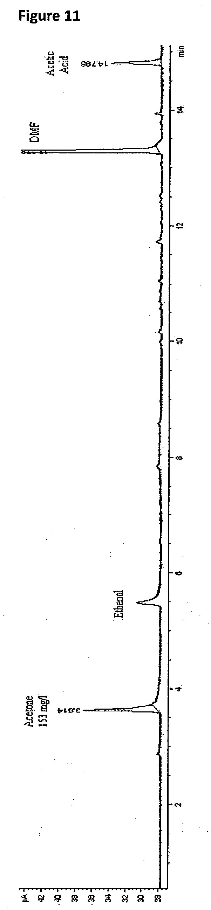

Growth experiments were carried out with transformed *C*. *autoethanogenum* DSM23693 and *C*. *Ijungdahlii* DSM 13528 carrying plasmid pMTL85147-thlA-ctfAB-adc in 250 ml PETC media (Tab. 2; without fructose and yeast extract) in 1 I Schott bottles with rubber stoppers and 30 psi steel mill gas (collected from New Zealand Steel site in Glenbrook, NZ; composition: 44% CO, 32% N₂, 22% CO₂, 2% H₂) in the headspace as sole energy and carbon source. Acetone production was confirmed with both strains using HPLC and GC analysis. In Schott bottles acetone concentrations of around 0.3 g/l (6.5 mM) after 48 hours were achieved with both, *C. autoethanogenum* DSM23693 (Fig. 8 and 10) and *C. ljungdahlii* DSM 13528 (Fig. 9 and 10). Using appropriate conditions, the produced acetone can then be further converted to isopropanol. Acetone production of 153 mg/ml was also demonstrated on 30 psi biomass syngas (Range Fuels Inc., Broomfield, CO; composition: 29 % CO, 45 % H₂, 13 % CH₄, 12 % CO₂, 1 % N₂) as sole energy and carbon source in 50 ml PETC media (Tab. 2; without fructose and yeast extract) in serum bottles with *C*. *autoethanogenum* DSM23693 (Fig. 11).

### Experssion of heterologous with Clostridial acetone pathway genes in C. autoethanogenum:

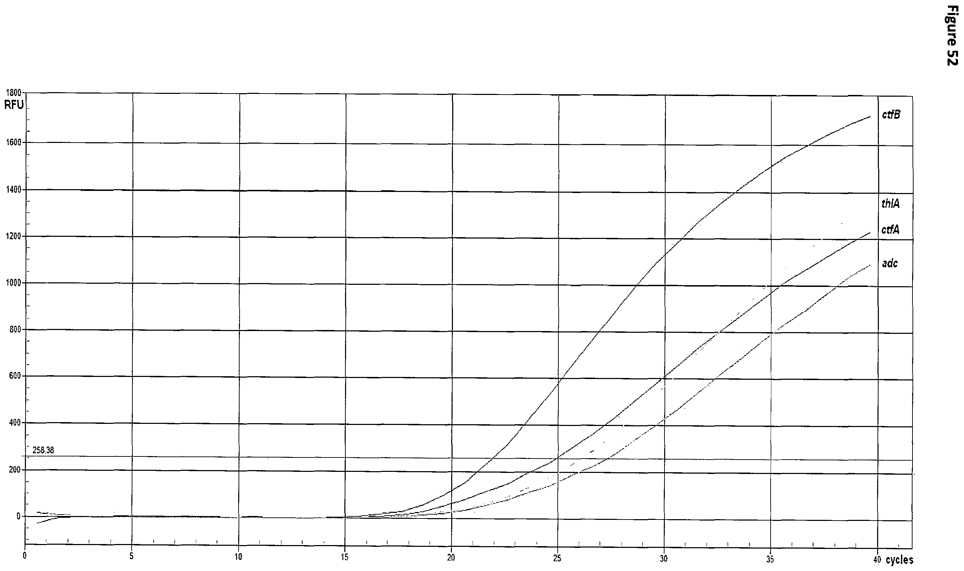

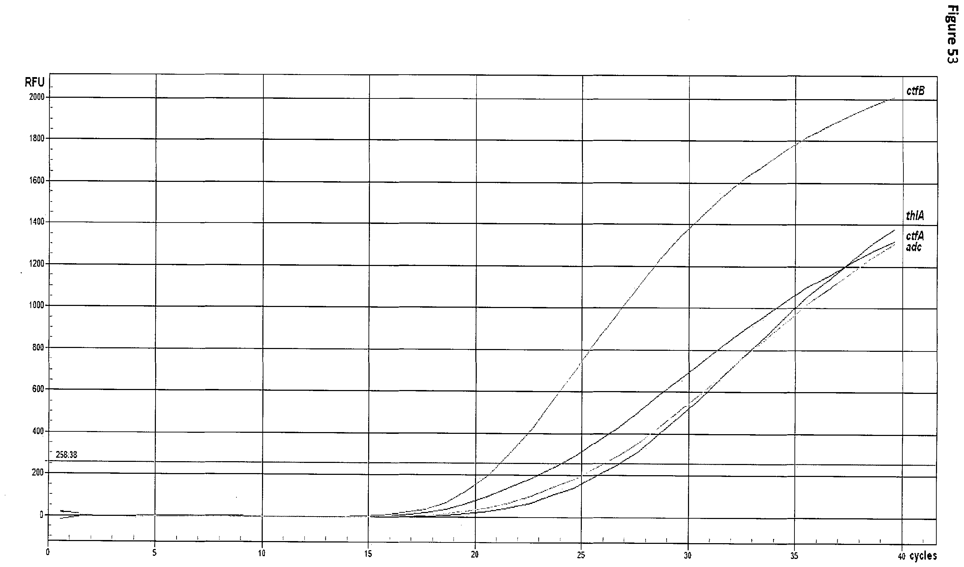

qRT-PCR experiments were performed to confirm successful expression of introduced genes *thlA, ctfA, ctfB,* and *adc* leading to acetone production in *C*. *autoethanogenum* and *C*. *ljungdahlii.* Signals for all genes could successfully be detected (Fig. 52 and 53).

A 50-ml culture of each *C*. *autoethanogenum* and *C*. *ljungdahlii* harbouring plasmid pMTL85147-harvested by centrifugation (6,000 x g, 5 min, 4 °C), snap frozen in liquid nitrogen and stored at - 80 °C until RNA extraction. Total RNA was isolated using PureLink™ RNA Mini Kit (Invitrogen, Carlsbad, CA, USA) and eluted in 100 µL of RNase-free water. After DNase I treatment (Roche Applied Science, Indianapolis, IN, USA), the reverse transcription step was then carried out using SuperScript III Reverse Transcriptase Kit (Invitrogen, Carlsbad, CA, USA). RNA was checked using an Agilent Bioanalyzer 2100 (Agilent Technologies, Santa Clara, CA, USA), Qubit Fluorometer (Invitrogen, Carlsbad, CA, USA) and by gel electrophoresis. A non-RT control was performed for every primer pair. All qRT-PCR reactions were performed in duplicates using a MyiQ Single Colour Detection System (Bio-Rad Labratories, Carlsbad, CA, USA) in a total reaction volume of 15 µL with 25 ng of cDNA template, 67 nM of each primer (Table 17), and 1x iQ SYBR Green Supermix (Bio-Rad Labratories, Carlsbad, CA, USA). The reaction conditions were 95 °C for 3 min, followed by 40 cycles of 95 °C for 15 s, 55 °C for 15 s and 72 °C for 30 s. For detection of primer dimerisation or other artifacts of amplification, a melting-curve analysis was performed immediately after completion of the qPCR (38 cycles of 58 °C to 95 °C at 1 °C/s). Two housekeeping genes (Guanylate kinase and formate tetrahydrofolate ligase) were included for each cDNA sample for normalization. Derivation of relative gene expression was conducted using Relative Expression Software Tool (REST©) 2008 V2.0.7 (38). Dilution series of cDNA spanning 4 log units were used to generate standard curves and the resulting amplification efficiencies to calculate concentration of mRNA.

**Table 17: Oligonucleotides for qRT-PCR**

| **Target** | **Oligonucleotid e Name** | **DNA Sequence (5' to 3')** | **SEQ_I D NO.** |
|---|---|---|---|
| Guanylate kinase (*gnk*) | GnK-F | TCAGGACCTTCTGGAACTGG | 5 |
| | GnK-R | ACCTCCCCTTTTCTTGGAGA | 6 |
| Formate tetrahydrofolate ligase (FoT4L) | FoT4L-F | CAGGTTTCGGTGCTGACCTA | 7 |
| | FoT4L-F | AACTCCGCCGTTGTATTTCA | 8 |
| | thIA-RT-F | TTGATGAAATGATCACTGACGGATT | 64 |
| Thiolase A | thIA-RT-R | GAAATGTTCCATCTCTCAGCTATGT | 65 |
| | ctfB-RT-F | CTAATACGAGGAGGACATGTTGATG | 66 |
| Acetoacetyl-CoA:Acetate CoA-transferase B | ctfB-RT-R | CACCCATACCTGGGACAATTTTATT | 67 |
| | ctfA-RT-F | GGGCTGCTACTAAAAATTTCAATCC | 68 |
| Acetoacetyl-CoA:Acetate CoA-transferase A | ctfA-RT-R | CAGGAGTCATTATGGCATCTCTTTT | 69 |
| | adc-RT-F | TAGTACCAGAGCCACTTGAATTAGA | 70 |
| Acetoacetate decarboxylase | adc-RT-R | GGAATAGCTTGACCACATTCTGTAT | 71 |

### Conversion of acetone to isopropanol by C. autoethanogenum, C. ljungdahlii, and C. ragsdalei:

Acetone can be further converted to isopropanol by action of an alcohol dehydrogenase. However, only few microorganisms such as *C. beijerinckii* NRRL-B593 are described to produce isopropanol, and acetone-to-isopropanol converting enzymes are very rare in nature. So far only two secondary alcohol dehydrogenases have been identified and described to date, from *C. beijerinckii* NRRL-B593 [Ismaiel AA, Zhu CX, Colby GD, Chen JS: Purification and characterization of a primary-secondary alcohol dehydrogenase from two strains of Clostridium beijerinckii. J Bacteriol 1993, 175: 5097-5105] (SEQ_ID NO 38-39) and *Thermoanaerobacter brockii* [Peretz M and Burstein Y: Amino acid sequence of alcohol dehydrogenase from the thermophilic bacterium Thermoanaerobium brockii. Biochemistry. 1989, 28:6549-6555] (SEQ_ID NO 40-41).

Therefore, a collection of microorganisms - acetogenic bacteria, acetone and isopropanol producing Clostridia and *E. coli* - were tested for their ability to convert acetone to isopropanol (Tab. 11).

**Tab. 11: Addition of acetone to growing cultures of various microorganisms.**

| **Organism**/**Sample** | **Description** | **Media** | **Directly after acetone addition** | | **End of growth** | |
|---|---|---|---|---|---|---|
| | | | **Acetone [g/l]** | **Isopropanol [g/l]** | **Acetone [g/l]** | **Isopropanol [g/l]** |
| *Acetobacterium woodii* DSM1030 | Acetogenic species | PETC (pH 8.2) | 10.81 | 0 | 10.83 | 0 |
| *Clostridium aceticum* DSM1496 | | PETC (pH 7.4) | 10.07 | 0 | 10.09 | 0 |
| *C. autoethanogenum* DSM23693 | | PETC (pH 5.9) | 9.25 | 0 | 1.13 | 8.03 |
| *C. carboxidivorans* DSM15243 | | | 10.43 | 0 | 10.34 | 0 |
| *C. ljungdahlii* DSM13528 | | | 10.23 | 0 | 3.73 | 6.54 |
| *C. ragsdalei* ATCC BAA-622 | | | 11.25 | 0 | 9.94 | 1.34 |
| *C. beijerinckii* NRRL-B593 | Isopopanol producing species | RCM | 9.96 | 0 | 7.65 | 2.54 |
| *C. beijerinckii* NCIMB8052 | Acetone producing species | | 10.49 | 0 | 10.59 | 0 |
| *C*. *acetobutylicum* ATCC824 | | | 10.80 | 0 | 10.91 | 0 |
| *Escherichia coli* DH5 (Invitrogen) | | LB + glucose | 11.67 | 0 | 11.71 | 0 |
| Blank media | Control | PETC | 10.51 | 0 | 10.55 | 0 |

All cultures were inoculated to an OD₆₀₀ₙₘ of 0.1 in 50 ml appropriate media containing a heterotrophic carbon source and 30 psi steel mill gas. The cultures were allowed to double (OD₆₀₀ₙₘ = 0.2) before acetone was added. A sample was taken and analyzed by HPLC and GC immediately after acetone addition and again at the end of growth (which were followed by measuring the optical density). Results are summarized in Tab. 11. Blank media was used as negative control.

As expected, isopropanol producing strain *C. beijerinckii* NRRL-B593 [George HA, Johnson JL, Moore WEC, Holdeman LV, Chen JS: Acetone, isopropanol, and butanol production by Clostridium beijerinckii (syn. Clostridium butylicum) and Clostridium aurantibutyricum. Appl Environ Microbiol 45: 1160-1163] had the ability to reduce externally added acetone to isopropanol by action of its alcohol dehydrogenase. A different strain of *C. beijerinckii,* NRCIMB8052, which lacks this enzyme wasn't able to convert acetone to isopropanol, as the acetone producing *C. acetobutylicum* ATCC-824. The same is also true for *E. coli.*

Surprisingly, three carboxydotrophic acetogenic bacetia *C*. *autoethanogenum, C. ljungdahlii,* and *C*. *ragsdalei,* which form a subcluster within the Clostridial rRNA Homology Group I, were found to be able to convert acetone to isopropanol as well, while all other acetogenic bacteria tested couldn't utilize acetone (Tab. 11). Conversion of different amounts of acetone to isopropanol by *C*. *autoethanogenum* was then tested using different concentrations (Tab. 12).

**Tab. 12: Conversion of different concentrations of acetone to isopropanol by cultures of C. autoethanogenum DSM23693.**

| **Acetone [g/l] added** | **Acetone [g/l] left at end of growth** | **Isopropanol [g/l] left at end of growth** |
|---|---|---|
| 0 | 0 | 0 |
| 1.66 | 0.22 | 1.48 |
| 9.25 | 1.13 | 8.03 |
| 26.13 | 17.82 | 8.39 |
| 50.01 | 43.30 | 6.95 |

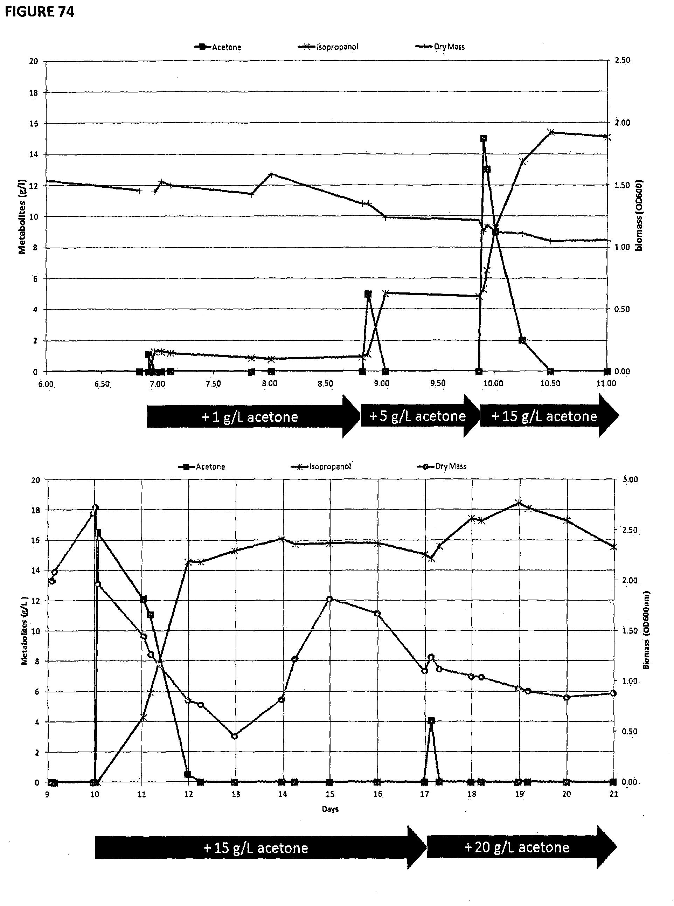

A reactor study with *C*. *autoethanogenum* DSM23693 was performed to demonstrate effective conversion of acetone to isopropanol at high rates. The reactor was set-up as described above. Once in continuous mode with stable biomass and metabolite production, acetone was added to both the bioreactor and the feed medium. Acetone was spiked into the reactor to a certain level, which was then obtained by continuous feeding. Initially, 1 g/L acetone was added, once the metabolite concentrations had stabilised, the concentration was increased to 5 g/L, 15 g/l, and in a second experiment to 20 g/L. Even at high concentrations of 20 g/L the culture converted all acetone to isopropanol at high rate demonstrating that the identified primary:secondary alcohol dehydrogenase is highly effective (Fig. 74).

### Identification of a novel alcohol dehydrogenase in C. autoethanogenum, C. Ijungdahlii, and C. ragsdalei:

To confirm that the conversion of acetone to isopropanol by *C*. *autoethanogenum* is driven enzymatically, enzyme assays were carried out with crude extract of *C*. *autoethanogenum* 23693, *C. beijerinckii* NRRL-B593, and *C*. *carboxidivorans* DSM15243 according to Ismaiel et al [Ismaiel AA, Zhu CX, Colby GD, Chen JS: Purification and characterization of a primary-secondary alcohol dehydrogenase from two strains of Clostridium beijerinckii. J Bacteriol 1993, 175: 5097-5105]. Crude extracts were obtained by sonication and lysozyme treatment (100,000 U/ml) of late exponential cultures. Cell debris was removed by centrifugation and protein concentrations was determined using the Pierce BCA protein assay - reducing agent compatible (Thermo Fisher Scientific Inc., Waltham MA 02454, USA). The assay mixture (1 ml) contained 50 mM Tris buffer (pH 7.5), 1 mM dithiothreitol (DTT), and 0.2 mM NAD(P)H. The reaction was started by adding 10 mM of the substrate acetone (from a 10-fold dilution in water) and followed spectrophotometrically with a Spectramax M2 (Molecular Devices, Inc., Sunnyvale, CA 94089-1136, USA) at a wavelength of 365 nm. H₂O was used as negative control instead of crude extract and respectively acetone. Enzyme activity could be detected with crude extracts of both *C. beijerinckii* and *C*. *autoethanogenum* and NADPH (not with NADH), but not with crude extracts of *C. carboxidivorans* DSM15243 or H₂O (with both NADPH and NADH). This demonstrates that the conversion of acetone to isopropanol by *C*. *autoethanogenum* is driven enzymatically, and as no activity was detected with NADH, the enzyme appears to be NADPH-dependent.

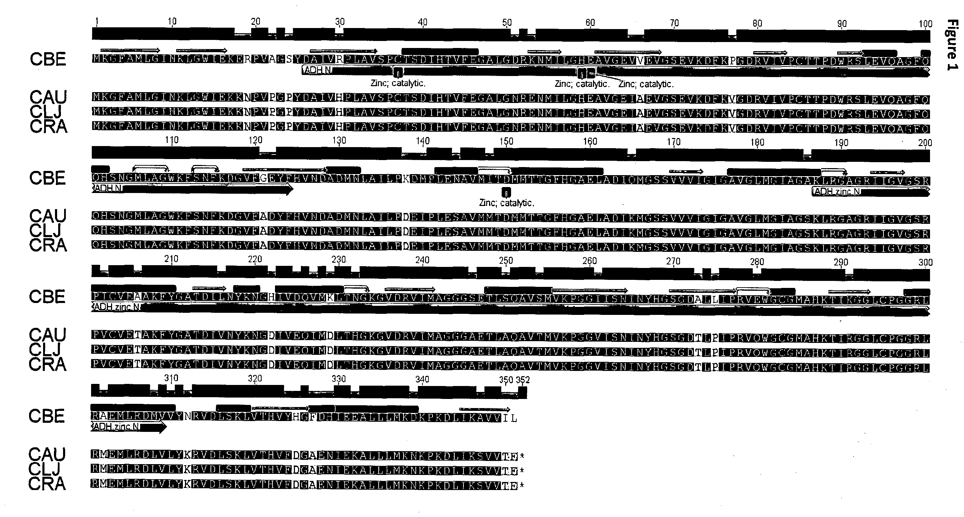

By sequencing and careful analysis, a novel alcohol dehydrogenase gene/enzyme was identified in all three strain, *C*. *autoethanogenum, C. Ijungdahlii,* and *C*. *ragsdalei* (Fig. 1; SEQ_ID NO. 1-4). The amino acid sequence was found to be identical in all three species and share some homology to the primary-secondary alcohol dehydrogenase of *C. beijerinckii* NRRL-B593 (87 %) and *T. brockii* ATCC 53556 (76 %) (Tab. 13). Compared to the well-described secondary alcohol dehydrogenase of *C. beijerinckii* NRRL-B593 [Ismaiel AA, Zhu CX, Colby GD, Chen JS: Purification and characterization of a primary-secondary alcohol dehydrogenase from two strains of Clostridium beijerinckii. J Bacteriol 1993, 175: 5097-5105], a total of 49 amino acids exchanges were found. 4 amino acids of the catalytic centre of the protein are conserved, however, other amino acids in the catalytic domain are not (Fig. 1). A motif search predicted the novel alcohol dehydrogenase gene/enzyme to be zinc and NAD(P)H dependent. The respective genes coding for the novel alcohol dehydrogenase was found to be 98 % identical within the 3 species *C*. *autoethanogenum, C. Ijungdahlii,* and *C*. *ragsdalei,* but only 82 % identical to the one from *C. beijerinckii* and 72 % identical to the one from *T. brockii* (Tab. 14).

**Tab. 13: Comparison of amino acid sequences of novel alcohol dehydrogenase and known secondary alcohol dehydrogenases**

| **Organism** | **Description** | **Seq ID** | **Accession number** | **Reference** | **Score** | **e-Value** | **Identity** |
|---|---|---|---|---|---|---|---|
| *C*. *autoethanogenum* | - | SEQ_ID NO.1 | - | - | 717 bits (1852) | 0 | 351/351 (100%) |
| *C. ljungdahlii* | zinc-containing alcohol dehydrogenase | SEQ_ID NO.1 | YP_003780646. 1 | - | 717 bits (1852) | 0 | 351/351 (100 %) |
| *C. ragsdalei* | - | SEQ_ID NO.1 | - | - | 717 bits (1852) | 0 | 351/351 (100 %) |
| *C. beijerinckii* NRRL B-593 | NADP-dependent *alcohol* dehydrogenase | SEQ_ID NO. 38 | P25984.2 | Ismaiel et al., 1993 | 630 bits (1626) | 7E-179 | 302/351 (87%) |
| *T. brockii* ATCC 53556 | NADP-dependent *alcohol* dehydrogenase | SEQ_ID NO. 40 | P14941.1 | Peretz and Burstein, 1989 | 557 bits (1436) | 7E-157 | 264/351 (76%) |

**Tab. 14: Comparison of nucleic acid sequences of novel alcohol dehydrogenase and known secondary alcohol dehydrogenases**

| **Organism** | **Description** | **Seq ID** | **Accession number** | **Reference** | **Score** | **e-Value** | **Identity** |
|---|---|---|---|---|---|---|---|
| *C*. *autoethanogenum* | | SEQ_ID NO.2 | - | - | 1905 bits (2112) | 0 | 1056/1056 (100%) |
| *C. ljungdahlii* | zinc-containing alcohol dehydrogenase | SEQ_{_}ID NO.3 | CP001666.1 | - | 1900 bits (2106) | 0 | 1055/1056 (99%) |
| *C. ragsdalei* | | SEQ_ID NO.4 | - | - | 1803 bits (1998) | 0 | 1033/1056 (98%) |
| *C. beijerinckii* NRRL B-593 | NADP-dependent alcohol dehydrogenase | SEQ_ID NO. 39 | AF157307.2 | - | 558 bits (618) | 0 | 861/1056 (82%) |
| *T. brockii* | alcohol dehydrogenase | SEQ_{_}ID NO. 41 | X64841.1 | - | 562 bits (622) | 3.00E-155 | 757/1053 (72%) |

### Experssion studies of the novel alcohol dehydrogenase from C. autoethanogenum

To identify, if the gene encoding the novel alcohol dehydrogenase is active during a normal fermentation with *C*. *autoethanogenum,* as well as identifying potential promoter regions for gene-overxpression, a qRT-PCR study with a over 250 genes was performed.

Samples were taken from a typical 1.5 l fed-batch fermentation run as described above over the whole growth (4 days). The samples were harvested by centrifugation (6,000 x g, 5 min, 4 °C) and the cell pellet snap frozen in liquid nitrogen and stored at -80 °C until use. RNA was isolated by thawing the cell pellet on ice and suspending it in 100 µL of lysozyme solution (50,000 U lysozyme, 0.5 µL 10% SDS, 10 mM Tris-HCl, 0.1 mM EDTA; pH 8). After 5 min, 350 µL of lysis buffer (containing 10 µL of 2-mercaptoethanol) was added. The cell suspension was mechanistically disrupted by passing five times through an 18-21 gauge needle. RNA was then isolated using PureLink™ RNA Mini Kit (Invitrogen, Carlsbad, CA 92008, USA) and eluted in 100 µL of RNase-free water. The RNA was checked via PCR and gel electrophoresis and quantified spectrophotometrically, and treated with DNase I (Roche) if necessary. The reverse transcription step was carried out using SuperScript III Reverse Transcriptase Kit (Invitrogen, Carlsbad, CA 92008, USA). RT-PCR reactions were performed in MyiQ Single Colour Real-Time PCR Detection System (Bio-Rad Labratories, Hercules, CA 94547, USA) in a reaction volume of 15 µL with 25 ng of cDNA template, 67 nM of each primer (Tab. 15), and 1x iQ SYBR Green Supermix (Bio-Rad Labratories, Hercules, CA 94547, USA). Guanylate kinase (GnK) and formate tetrahydrofolate ligase (FoT4L) were used as housekeeping gene and non-template controls were included. The reaction conditions were 95 °C for 3 min, followed by 40 cycles of 95 °C for 15 s, 55 °C for 15 s and 72 °C for 30 s. A melting-curve analysis was performed immediately after completion of the qRT PCR (38 cycles of 58 °C to 95 °C at 1 °C/s), for detection of primer dimerisation or other artifacts of amplification. Data on the expression level was computed in the form of threshold cycle (Cₜ) values based on PCR base line subtracted curve fit method as calculated by the Biorad iQ5 2.0 software. The raw Cₜ values were further analyzed using Relative Expression Software Tool (REST©) 2008 V2.0.7.

**Tab. 15: Oligonucleotides for RT-PCR**

| **Target** | **Oligonucleotide Name** | **DNA Sequence (5' to 3')** | **SEQ_I D NO .** |
|---|---|---|---|
| Guanylate kinase (*gnk*) | GnK-F | TCAGGACCTTCTGGAACTGG | 5 |
| | GnK-R | ACCTCCCCTTTTCTTGGAGA | 6 |
| Formate tetrahydrofolate ligase (FoT4L) | FoT4L-F | CAGGTTTCGGTGCTGACCTA | 7 |
| | FoT4L-F | AACTCCGCCGTTGTATTTCA | 8 |
| CO dehydrogenase (*acsA*) | acsA-F | ACAAGA TGGGGTCGAAACAGTTTGG | 9 |
| | acsA-R | TGGCACTGGACTTACTCTACATGGG | 10 |
| Formyl-THF synthase (*fhs*) | fhs-F | | 11 |
| | fhs-R | TCCAGCAGGTGTTGGGTTTATAGC | 12 |
| Formimido-THF | fchA-F | AGCTGCAACTCCTGGTGGAGGC | 13 |
| cyclodeaminase (*fchA*) | fchA-R | GCCTTTTACCTTTTCGTCATACTGTGC | 14 |
| Methylene-THF dehydrogenase | folD-F | | 15 |
| formyl-THF cyclohydrolase (*folD*) | folD-R | | 16 |
| alcohol dehydrogenase (*adh*) | Adh-F | GGTCCTTATGATGCGATTGTACATCC | 17 |
| | Adh-R | GCTATTTCACCTACAGCTTCATGGCC | 18 |

The result of the qRT-PCR study showed, that the gene for the novel alcohol dehydrogenase is expressed over the whole growth on a relatively constant level and only ceases at end of growth (Fig. 2). Compared to over 200 genes chosen from every part of the metabolism, the alcohol dehydrogenase gene belongs to the top 50 expressed genes. The highest gene expression of all genes analyzed showed the genes of the Wood-Ljungdahl operon, with an mRNA level of more than 10-fold higher than the alcohol dehydrogenase gene (Fig. 2). The respective promoter (SEQ_ID NO 22) region is therefore ideal to over-express genes, such as the genes for acetone biosynthesis enzymes and an alcohol dehydrogenase gene, although in the case of over-expression of an alcohol dehydrogenase gene native to the micro-organisms it may require additional genetic modification to ensure sufficient co-factor availability. This could include, for example, (over-)expression of further genes to increase the NADPH pool such as transhydrogenase, elimination of competing NADPH consuming reactions, or protein engineering to change the co-factor requirement to NADH. Other useful promoter regions identified for gene over-expression include the promoter region of F₁F_{O}-ATPase operon (SEQ_ID NO 51), Rnf complex operon (SEQ_ID NO 52), and Pyruvate:ferredoxin oxidoreductase (SEQ_ID NO 53).

### Isopropanol production from CO and CO₂/H₂ by C. autoethanogenum and C. ljungdahlii with expression plasmid containing Clostridial acetone genes

The 250 ml Schott bottle cultures of recombinant strains of *C*. *autoethanogenum* DSM23693 and *C. ljungdahlii* DSM 13528 carrying acetone expression plasmid pMTL85147-thlA-ctfAB-adc were shown to produce acetone, but no isopropanol could be detected (Fig. 8 + 9). This might be due to the lack of reducing power at end of growth, due to the given static conditions in Schott bottles, where CO gets depleted from the headspace and is not constantly fed like in a fed-batch or continuous fermentation process. Reducing equivalents such as NAD(P)H or ferredoxin gets generated from CO, but are also consumed for ethanol production, which already occurs during exponential and early stationary growth. At this point is the concentration of produced acetone, which is needed as precursor for isopropanol production, is still relatively low.

Therefore, both cultures were re-gassed with 30 psi fresh steel-mill gas after 48 h of growth and also re-inoculated. While biomass didn't increase much further, some of the produced acetone got converted into isopropanol within 24 hours (Tab. 16).

**Tab. 16: Conversion of acetone to isopropanol by cultures of C. autoethanogenum DSM23693 and C. ljungdahlii DSM 13528**

| **Organism** | **Acetone [mg/l]** | | **Isopropanol [mg/l]** | |
|---|---|---|---|---|
| | **After 48 h** | **After 72 hours** | **After 48 h** | **After 72 hours** |
| *C. autoethanogenum* + pMTL85147-thlA-ctfAB-adc | 220 | 295 | 0 | 25 |
| *C. ljungdahlii* + pMTL85147-thlA-ctfAB-adc | 171 | 175 | 0 | 5 |

In a fermentation system with constant supply of CO, sufficient reducing power is present for continuous production of isopropanol from CO or CO/H₂ and both acetone and isopropanol were produced in a respective fermentation run with *C. autoethanogenum* DSM23693 carrying acetone expression plasmid pMTL85147-thlA-ctfAB-adc.

### Cloning of novel alcohol dehydrogenase

The novel alcohol dehydrogenase was cloned into the acetone expression plasmid and put under control of the Wood-Ljungdahl promoter for gene over-expression and test of functionality in *E. coli.*

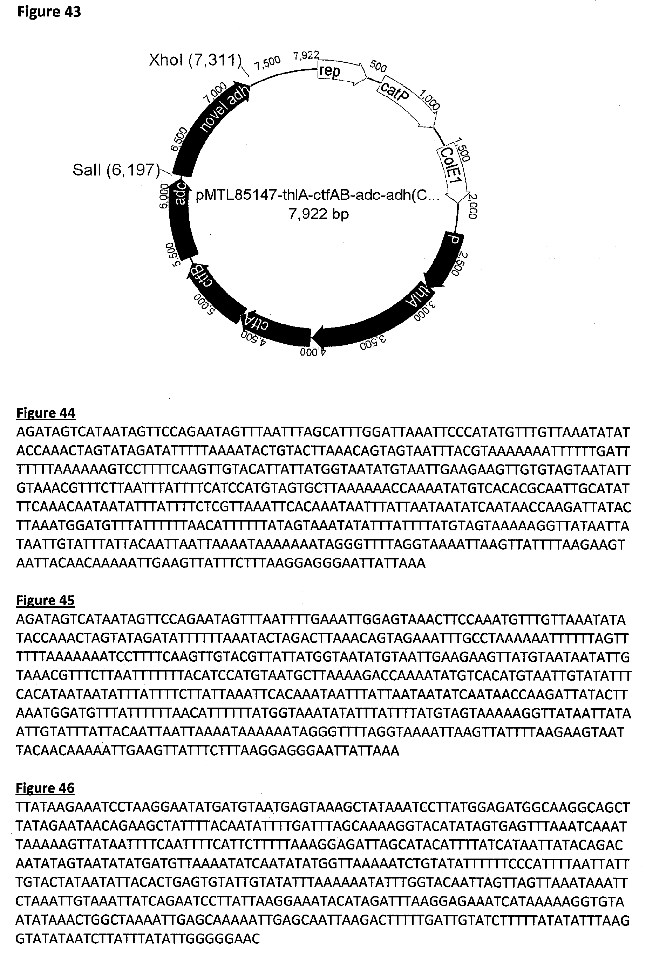

Alcohol dehydrogenase was amplified from isolated *C*. *autoethanogenum* DSM10061 chromosomal DNA using oligonucleotides SecAdh-Sall-F (SEQ_ID NO 54: TATTTGTCGACTTAGGAGGTTCTATTATGAAAGG) and SecAdh-Xhol-R (SEQ_ID NO 55: AAAACTCGAGACATTTTTTTAATGCGACAG). The 1129 bp PCR fragment was cloned into plasmid pMTL85147-thlA-ctfAB-adc using *SalI* and *XhoI* and *E. coli* XL-1 Blue MRF' Kan. The resulting plasmid pMTL85147-thlA-ctfA-ctfB-adc-adh (SEQ_ID NO 48; Fig. 43) was completely sequenced using oligonucleotides given in Tab. 9 and results confirmed that the isopropanol biosynthesis genes and promoter region were free of mutations (Fig. 41).

### Production of isopropanol with novel alcohol dehydrogenase from C. autoethanogenum in E. coli

To further test the functionality of the novel alcohol dehydrogenase from *C*. *autoethanogenum,* growth experiments were carried out using *E. coli* XL-1 Blue MRF' Kan expressing only the acetone biosynthesis genes (carrying plasmid pMTL 85147-thlA-ctfA-ctfB-adc) and expressing the acetone biosynthesis genes plus the novel alcohol dehydrogenase (carrying plasmid pMTL85147-thlA-ctfA-ctfB-adc-adh) in 100 ml SD-8 minimal media with chloramphenicol (Fig. 42).

While no isopropanol could be detected with the strain carrying the acetone plasmid, an average maximum of 32.7 mg/L isopropanol was measured with the strain additionally expressing the novel alcohol dehydrogenase from *C*. *autoethanogenum.*

### Identification of genes from Lactococcus lactis and Saccharomyces cerevisiae that confer novel activity towards acetone or isopropanol in C. autoethanogenum

In addition to the Clostridial acetone and isopropanol pathway, two enzymes an Alpha-ketoisovalerate decarboxylase (KivD) from *Lactococcus lactis* and an Alcohol dehydrogenase (Adh2) from *Saccharomyces cerevisiae* (Tab. 18) were identified that confer activity towards acetone and isopropanol production in *C*. *autoethanogenum.* Those two enzymes haven't been reported to be involved in acetone or isopropanol production or have catalytic functions on any of the precursors in the Clostridial acetone and isopropanol pathway. Heterologous expression of these proteins in *E. coli* (Atsumi et al., 2008. Non-fermentative pathways for synthesis of branched-chain higher alcohols as biofuels. Nature, 451: 86-90) or other organisms like *Corynebacterium glutamicum* (Blombach et al., 2011. Corynebacterium glutamicum tailored for efficient Isobutanol production. Appl. Environ. Microbiol. 77: 3300-10) or *Clostridium cellulolyticum* (Higashide W., et al. 2011. Metabolic Engineering of Clostridium cellulolyticum for Production of Isobutanol from Cellulose. Appl. Environ. Microbiol. 77: 2727-33) led to production of branched-chain higher alcohols like isobutanol, 1-butanol, 2-methyl-1-butanol, 3-methyl-1-butanol and 2-phenylethanolfrom amino acid precursors, but neither acetone nor isobutanol was reported. Expression of codon-optimized Alpha-ketoisovalerate decarboxylase (KivD) from *Lactococcus lactis* alone or a combination of codon optimized Alpha-ketoisovalerate decarboxylase (KivD) from *Lactococcus lactis* and an Alcohol dehydrogenase (Adh2) from *Saccharomyces cerevisiae* in *C*. *autoethanogenum* however, led suprisingly to production of acetone and isopropanol.

**Tab. 18: Sequences from Lactococcus lactis and Saccharomyces cerevisiae that confer novel activity towards acetone or isopropanol in C. autoethanogenum**

| **Description** | | |
|---|---|---|
| | ***L. lactis*** | |
| | **nucleic acid** | **amino acid** |
| **Alpha-ketoisovalerate decarboxylase (KivD)** | SEQ_ID No. 72 | SEQ_ID No. 73; |
| | AJ746364 | YP_003353820.1 |

| | ***S. cerevisiae*** | |
|---|---|---|
| | **nucleic acid** | **amino acid** |
| **Alcohol dehydrogenase (Adh2)** | SEQ_ID No. 74 | SEQ_ID No. 75; |
| | NC_001145.2, GenelD: 855349 | AAA34408.1 |

### Construction of expression plasmid with Alpha-ketoisovalerate decarboxylase (KivD) from Lactococcus lactis and Alcohol dehydrogenase (Adh2) from Saccharomyces cerevisiae

The Alpha-ketoisovalerate decarboxylase (decarboxylase; KivD) from *L. lactis,* and Alcohol dehydrogenase (Adh2) from *S*. *cerevisiae* (Tab. 18) were codon-optimised by ATG:Biosynthetics GmbH (Merzhausen, Germany) and flanked by *Nde*I and *Kpn*I restriction sites for further subcloning. The Phosphiotransacetylase/Acetate kinase operon promoter of *C*. *autoethanogenum* was used for expression of target genes. All DNA sequences used are given in Tab. 19.

**Tab. 19: Sequences used for expression plasmid with Alpha-ketoisovalerate decarboxylase (KivD) from Lactococcus lactis and Alcohol dehydrogenase (Adh2) from Saccharomyces cerevisiae**

| **Description** | **Source** | **SEQ_ID NO.** |
|---|---|---|
| Alpha-ketoisovalerate decarboxylase (KivD) and Alcohol dehydrogenase (Adh2) | *Codon optimized* | 76-78 |
| Phosphotransacetylase/Acetate kinase operon promoter region | *Clostridium autoethanogenum* DSM10061 | 79 |

The promoter region of the phosphotransacetylase-acetate kinase operon (P*_{pta-ack}*) was amplified using primers Ppta-ack-NotI-F (Seq. ID. No. 80: GAGCGGCCGCAATATGATATTTATGTCC) and Ppta-ack-Ndel-R (Seq. ID. No. 81: TTCCATATGTTTCATGTTCATTTCCTCC) and cloned into the *E. coli-Clostridium* shuttle vector pMTL 85141 (FJ797651.1; Nigel Minton, University of Nottingham, UK) [Heap JT, Pennington OJ, Cartman ST, Minton NP. A modular system for Clostridium shuttle plasmids. J Microbiol Methods. 2009, 78: 79-85] using *Not*I and *Nde*I restriction sites and strain XL1-Blue MRF' Kan.

The antibiotic resistance gene in the created plasmid pMTL85145 was subsequently replaced with an erythromycin resistance gene from pMTL 82254 (FJ797646.1; Nigel Minton, University of Nottingham, UK) [Heap JT, Pennington OJ, Cartman ST, Minton NP. A modular system for Clostridium shuttle plasmids. J Microbiol Methods. 2009, 78: 79-85] using *FseI* and *PmeI* restriction sites and strain XL1-Blue MRF' Kan.

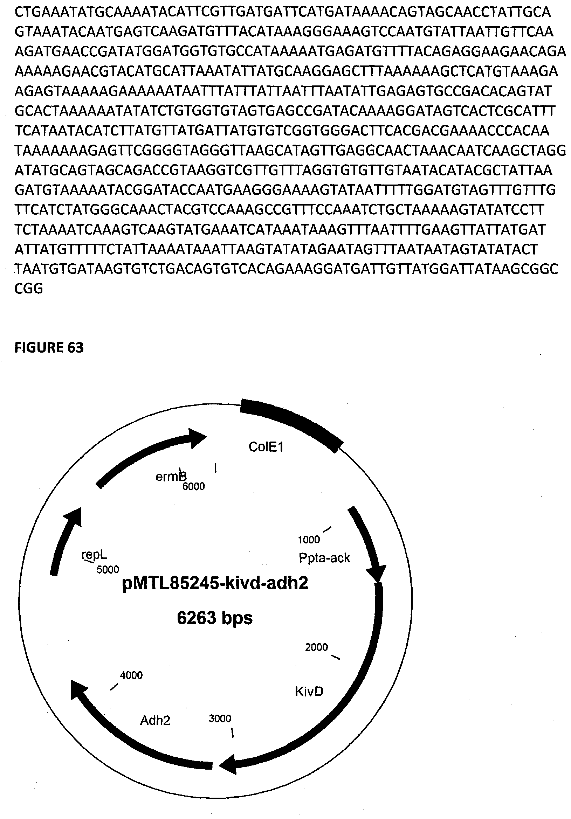

The created plasmid pMTL85245 (Seq. ID. No. 80) and the 2746 bp codon-optimised product of the decarboxylase and alcohol dehydrogenase (Adh2) gene cluster were both cut with *Nde*I and *Kpn*I*.* A ligation was transformed into *E. coli* XL1-Blue MRF' Kan resulting in plasmid pMTL85245-kivd-adh2 (Seq. ID. No. 83; Fig. 63). The insert of the resulting plasmid pMTL85245-kivd-adh was completely sequenced using oligonucleotides given in Tab. 20 and results confirmed that genes and promoter region were free of mutations.

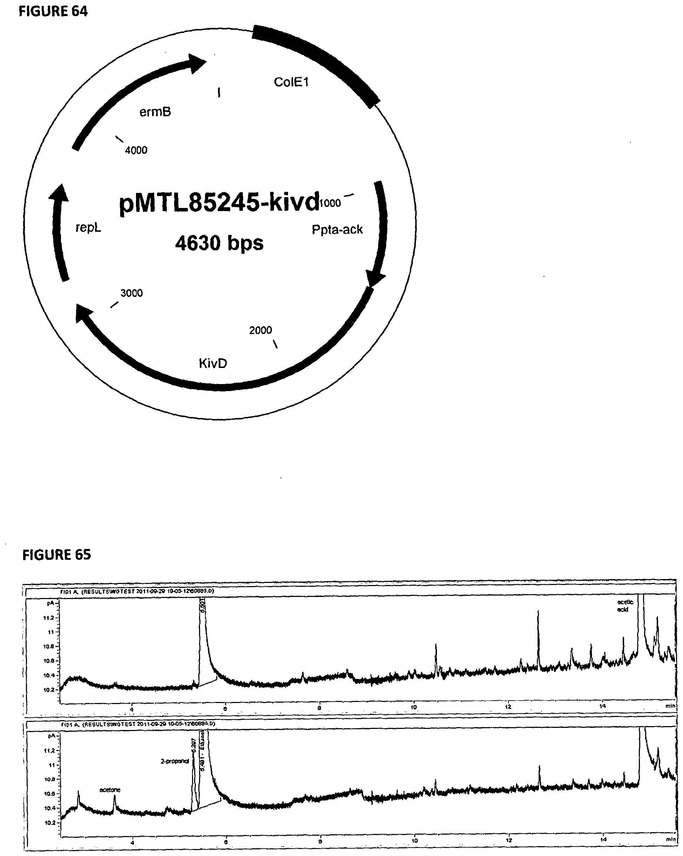

The *kivD* gene alone was amplified using primer pair M13 Reverse (Seq. ID. 57: CAGGAAACAGCTATGAC) and Adh_seqR1 (Seq. ID. 85; Tab. 16). The 2635 bp PCR fragment of KivD was cloned into the *E. coli-Clostridium* shuttle vector pMTL 85245 using *Nde*I and *EcoR*I restriction sites and strain *E. coli* XL1-Blue MRF' Kan, creating plasmid pMTL85245-kivd (Seq. ID No. 84; Fig. 64). The insert of the resulting plasmid pMTL85245-kivd was completely sequenced using oligonucleotides given in Tab. 20 and results confirmed that the acetone biosynthesis gene was free of mutations.

**Tab. 20: Oligonucleotides used for sequencing**

| | | |
|---|---|---|
| Adh_seqR1 | TCAGTTCCCTGTGGAATGTGTGC | Seq. ID. No. 85 |
| Kivd_seqR2 | TCAGTAGCACCGAAAGATTCAG | Seq. ID. No. 86 |
| Kivd_seqR3 | AGTGCCTCATCTACTGAACTC | Seq. ID. No. 87 |
| -ori_F | ATTAGTTTAAACACGCCAGCAACGCGGCCTTTTTAC | Seq. ID. No. 88 |
| ctfAB_seqR1 | TCCTATTCCAAGGTTTACGAGTTGGTC | Seq. ID. No. 89 |
| ctfAB_seqR2 | ACCCCCAACCATAATTGTCATGCCATC | Seq. ID. No. 90 |
| ctfAB_seqR3 | TGCAAGAGCAAACTCATCTTGTTCTTC | Seq. ID. No. 91 |
| P-thl-ctfAB_R2 | AGGGTGCGGCCGCGATTCATATATCCATAATCTTTAAGTTATC | Seq. ID. No. 92 |

### Expression of codon-optimized genes for Alpha-ketoisovalerate decarboxylase (KivD) from Lactococcus lactis and Alcohol dehydrogenase (Adh2) from Saccharomyces cerevisiae in C. autoethanogenum for production of acetone and isopropanol

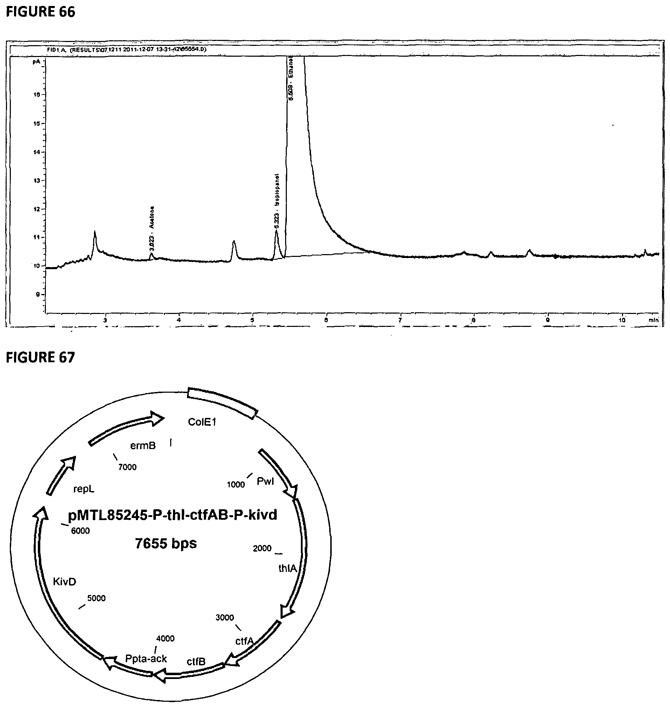

Constructed expression plasmids pMTL85245-kivd-adh2 and pMTL85245-kivd were transformed into *E. coli* strain JW3350-2 and prepared for transformation in *C. autoethanogenum* DSM23693, which was performed as described above. While in *E. coli* harbouring the two plasmids, neither acetone nor isopropanol could be detected (but higher branched-chain alcohols such as isobutanol as described in the literature), in *C*. *autoethanogenum,* both acetone and isopropanol could be detected. In serum bottle experiments, highest isopropanol concentrations from CO-containing steel mill gas were 0.050-0.064 g/L for both expression plasmids (Fig. 65 and 66).

### Production of acetone and isopropanol with a combination of Clostridial pathway genes and Alpha-ketoisovalerate decarboxylase (KivD) from Lactococcus lactis and Alcohol dehydrogenase (Adh2) from Saccharomyces cerevisiae

Without wanting to be bound by any particular theory, the inventors believe that the codon-optimized alpha-ketoacid decarboxylase Kivd from *Lactococcus lactis* has activity converting acetoacetate to acetone, as the Clostridial acetoacetate decarboxylase, while the codon-optimized alcohol dehydrogenase Adh2 from *Saccharomyces cerevisiae* have activity converting acetone to isopropanol as the novel primary:secondary alcohol dehydrogenase identified or the primary:secondary alcohol dehydrogease from *Clostridium beijkerickii.* To test this hypothesis several combinations of Clostridial acetone/isopropanol pathway genes and the alpha-ketoacid decarboxylase Kivd from *Lactococcus lactis* and alcohol dehydrogenase Adh2 from *Saccharomyces cerevisiae* have been created and tested within *E. coli* and *C*. *autoethanogenum* demonstrating production of acetone and isopropanol.

### Construction of expression plasmids with different gene combinations

Based on the constructed expression plasmids pMTL85147-thlA-ctfA-ctfB-adc, pMTL85245-kivd-adh2 and pMTL85245-kivd, new combinations were constructed.

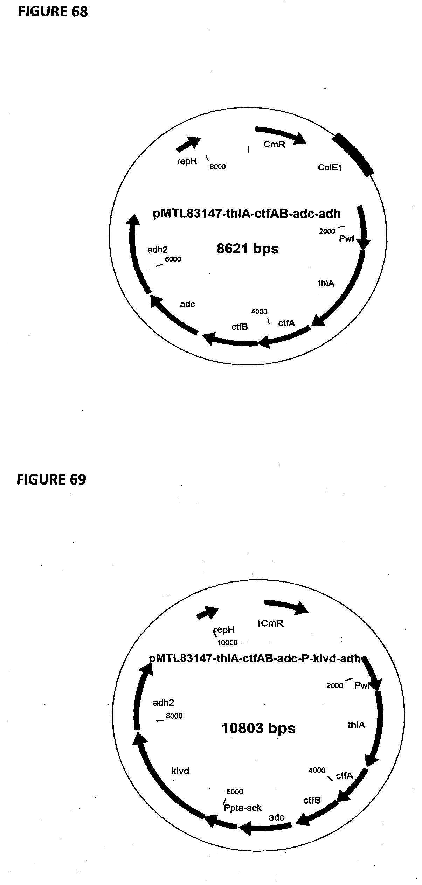

A 3122bp P_{WL}-thlA-ctfAB fragment was amplified from plasmid pMTL85147-thlA-ctfA-ctfB-adc using oligonucleotides P-thl-ctfAB_F2 (Seq. ID. No. 93: ATCTTCTGCAGGGCCGCAGATAGTCATAATAGTTCCAG) and P-thl-ctfAB_R2 (Seq. ID. No. 94: AGGGTGCGGCCGCGATTCATATATCCATAATCTTTAAGTTATC). The amplified fragment was cloned into plasmid pMTL 85245-kivd using *Pst*I and *Not*I restriction sites and strain *E. coli* XL1-Blue MRF' Kan, creating plasmid pMTL85245-P_{WL}-thlA-ctfAB-kivd (Seq. ID. No. 95; Fig. 67). The insert of the resulting plasmid pMTL85245-P_{WL}-thlA-ctfAB-kivd was completely sequenced using oligonucleotides given in Tab. 9 and 20 and confirmed that the plasmid was free of mutations. The Adh2 gene was amplified from plasmid pMTL85245-kivd-adh2 using primer pair adh_F (Seq. ID. No. 96: ACGTTGGATCCAGGAGGAACAAAGATGAGTATACC) and P-kivd-adh_R (Seq. ID. No. 97: AGCGTCCATGGCCTTATTTACTTGTATCTACAACATATC). The 1084 bp PCR fragment was cloned into the plasmid pMTL85147-thlA-ctfAB-adc using *BamH*I and *Nco*I restriction sites and strain *E. coli* XL1-Blue MRF' Kan, creating plasmid pMTL85147-thlA-ctfAB-adc-adh2 (Seq. ID. No. 98; Fig. 68). The created plasmid pMTL85147-thlA-ctfAB-adc-adh2 and a 1625 bp fragment of the repL gene from pMTL83151 (FJ797647.1; Nigel Minton, University of Nottingham, UK) [Heap JT, Pennington OJ, Cartman ST, Minton NP. A modular system for Clostridium shuttle plasmids. J Microbiol Methods. 2009, 78: 79-85] were both cut with *Fse*I and *Asc*I. A ligation was performed resulting in plasmid pMTL83147-thlA-ctfAB-adc-adh2. The insert of the resulting plasmid pMTL83147-thlA-ctfAB-adc-adh2 was completely sequenced using oligonucleotides given in Tab. 9 and 20 and results confirmed that the fragment was mutation free.

Oligonucleotides P-kivd-adh_F (Seq. ID. No. 99: ATATTGGATCCACAGCTATGACCGCGGCCGCAATATG) and P-kivd-adh_R (Seq. ID. No. 100: AGCGTCCATGGCCTTATTTACTTGTATCTACAACATATC) were used to amplify a 3266 bp PCR fragment of P_{pta-ack}-kivd-adh2 from plasmid pMTL85245-kivd-adh2, which was then cloned into the plasmid pMTL85147-thlA-ctfAB-adc using *BamH*I and *Nco*I restriction sites and strain *E. coli* XL1-Blue MRF' Kan, creating plasmid pMTL83147-thlA-ctfAB-adc-P_{pta-ack}-kivd-adh2 (Seq. ID. 101; Fig. 69). The created plasmid pMTL83147-thlA-ctfAB-adc-P_{pta-ack}-kivd-adh2 and a 1625 bp fragment of the repL gene from pMTL83151 (FJ797647.1; Nigel Minton, University of Nottingham, UK) [Heap JT, Pennington OJ, Cartman ST, Minton NP. A modular system for Clostridium shuttle plasmids. J Microbiol Methods. 2009, 78: 79-85] were both cut with *Fse*I and AscI. A ligation was performed resulting in plasmid pMTL83147-thlA-ctfAB-adc-P_{pta-ack}-kivd-adh2. The insert of the resulting plasmid pMTL83147-thlA-ctfAB-adc-P_{pta-ack}-kivd-adh2 was completely sequenced using oligonucleotides given in Tab. 9 and results confirmed that the plasmid was free of mutations.

### Production of acetone and isopropanol in C. autoethanogenum using different gene combinations

Methylation of the newly constructed expression plasmids pMTL85147-thlA-ctfA-ctfB-adc, pMTL83147-thlA-ctfAB-adc-adh2 and pMTL83147-thlA-ctfAB-adc-P_{pta-ack}-kivd-adh2 were performed *in vivo* in *E. coli* using a synthesized hybrid Type II methyltransferase gene (SEQ_ID NO 35) designed from methyltransferase genes from *C*. *autoethanogenum, C. ragsdalei* and *C*. *ljungdahlii* and transformed into C. autoethanogenum DSM23693 as described above.

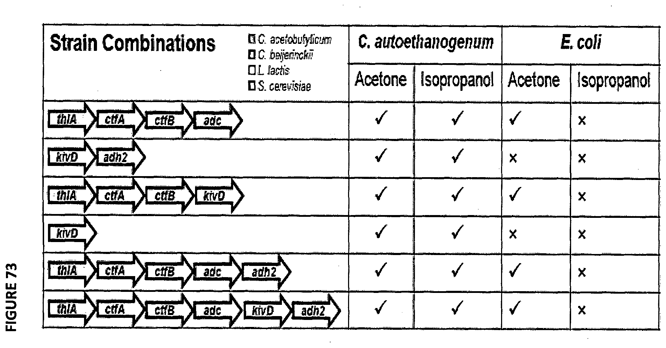

All plasmid construct were tested in *E. coli* and *C. autoethanogenum* DSM23693 using serum bottle experiments with sugar (*E. coli*) or CO-containing steel mill gas (*C*. *autoethanogenum*) as sole substrate. With all combinations tested, acetone and isopropanol production was measured when expressed heterologously in *C*. *autoethanogenum,* while in *E. coli* acetone production only occured with few combinations and an alcohol dehydrogenase gene was needed for isopropanol production (Tab. 21). The results presented show that both, in *E. coli* as well as *C*. *autoethanogenum,* the codon-optimized Alpha-ketoacid decarboxylase Kivd from *Lactococcus lactis* is able to replace the Clostridial acetoacetate decarboxylase and catalyse the conversion of acetoacetate to acetone (Fig. 4). In *C*. *autoethanogenum,* acetone and isopropanol production even occurred with expressing the decarboxylase as only heterologous gene, indicating CoA-transferase activity. Figure 4 illustrates the proposed pathway and Tab. 21 of acetone and isopropanol formation from CO and Figure 73 gives an overview of combinations of Clostridial pathway genes and codon-optimized genes for Alpha-ketoacid decarboxylase Kivd from *L lactis* and Alcohol dehydrogenase Adh2 from *S. cerevisiae* tested in *E. coli* and *C*. *autoethanogenum.*

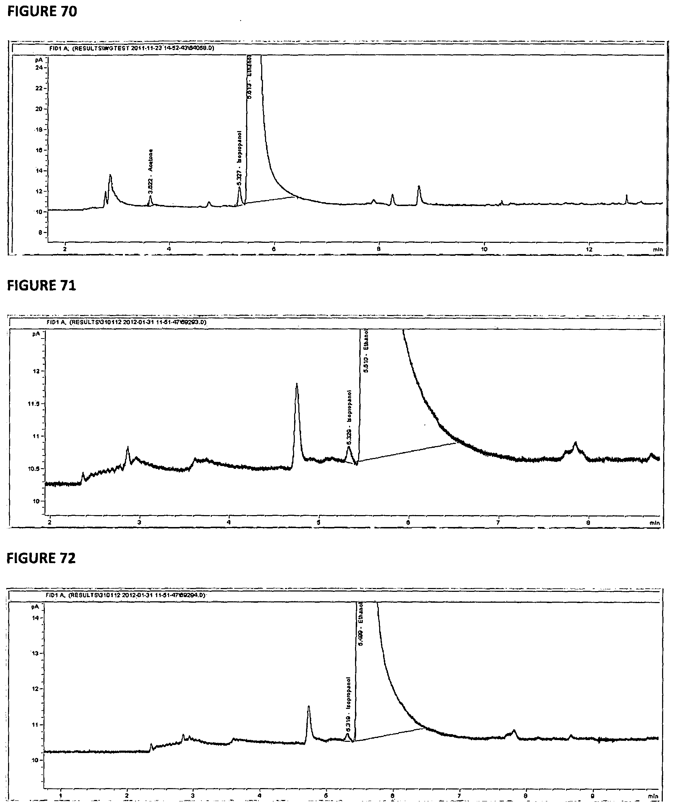

Production of acetone and isopropanol with *C. autoethanogenum* DSM23693 and plasmids pMTL85245-P_{WL}-thlA-ctfAB-kivd, pMTL83147-thlA-ctfAB-adc-adh2 and pMTL83147-thlA-ctfAB-adc-P_{pta-ack}-kivd-adh2 from CO-containing steel mill gas is shown in Fig. 70, 71, and 72 respectively.

**Tab. 21: Acetone and isopropanol produced from various combinations of genes**

| **Clostridia genes** | **Organism** | **Substrate** | **Acetone (g/L)** | **Isopropanol (g/L)** |
|---|---|---|---|---|
| **pMTL85147-thlA-ctfAB-adc** | *E. coli* | Sugar | 0.200 | N/A |
| | *C. autoethanogenum* | CO | 0.300 | 0.025 |
| | *C. ljungdahlii* | CO | 0.180 | 0.005 |
| **pMTL85147-thlA-ctfAB-adc-sadh (*C. beijerinckii*)** | *E. coli* | Sugar | 0.080 | 0.070 |
| **pMTL85147-thlA-ctfAB-adc-sadh (*C. autoethanogenum*)** | *E. coli* | Sugar | 0.060 | 0.080 |
| | | | | |

| **Novel genes** | **Organism** | **Substrate** | **Acetone (g/L)** | **Isopropanol (g/L)** |
|---|---|---|---|---|
| **pMTL85245-kivd-adh2** | *E. coli* | Sugar | N/A | N/A |
| | *C. autoethanogenum* | CO | Detected by GC qualitatively | 0.050 |
| **pMTL85245-kivd** | *E. coli* | Sugar | N/A | N/A |
| | *C. autoethanogenum* | CO | Detected by GC qualitatively | 0.064 |
| | | | | |
| **Combination of Clostridia and novel genes** | **Organism** | **Substrate** | **Acetone (g/L)** | **Isopropanol (g/L)** |
| **pMTL85147-thlA-ctfAB-adc-kivd** | *E. coli* | Sugar | Detected by GC qualitatively | N/A |
| | *C. autoethanogenum* | CO | Detected by GC qualitatively | 0.091 |
| **pMTL83147-thlA-ctfAB-adc-adh2** | *E. coli* | Sugar | 0.040 | N/A |
| | *C. autoethanogenum* | CO | Detected by GC qualitatively | 0.648 |
| **pMTL83147-thlA-ctfAB-adc-P-kivd-adh2** | *E. coli* | Sugar | 0.076 | N/A |
| | *C. autoethanogenum* | CO | Detected by GC qualitatively | 0.043 |

### Tolerance to acetone and isopropanol and detoxification of acetate in acetogens

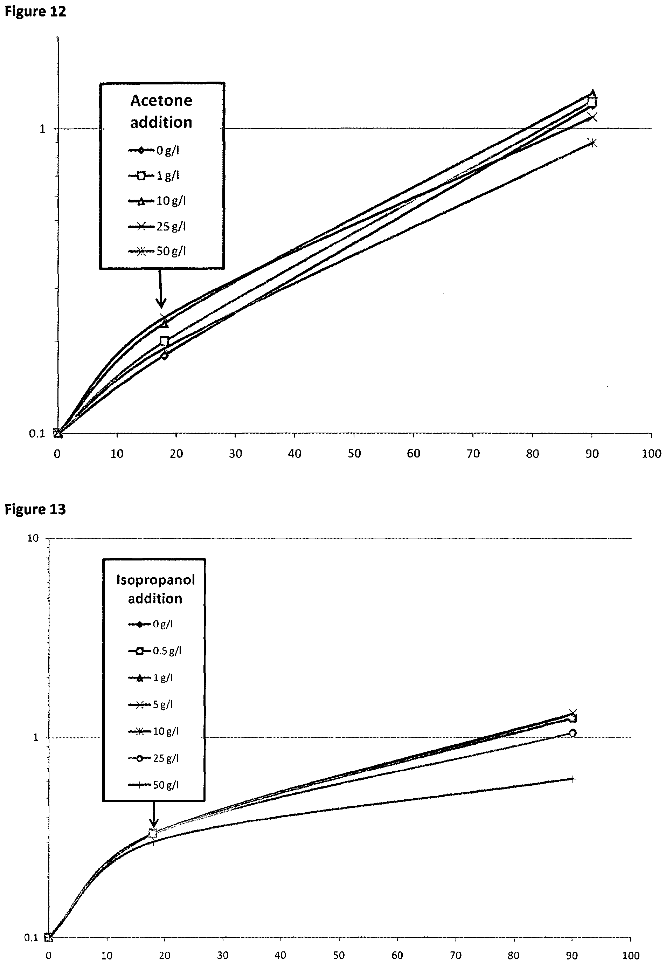

Several metabolites such as alcohols (ethanol and butanol) or acids (acetic acid and butyric acid) are known to be toxic for bacteria in high concentrations and thus limit their biotechnological production [Alsaker KV, Parades C, Papoutsakis ET: Metabolite stress and tolerance in the production of biofuels and chemicals - systems analysis of butanol, butyrate, and Acetate Stresses in the Anaerobe Clostridium acetobutylicum. Biotechnol Bioeng, 2009, 105: 1131-1147]. To see if acetone and isopropanol have a toxic effect on cultures, growth experiments were carried out in 50 ml PETC media (Tab. 2) in serum bottles, adding different concentrations of acetone (Fig. 12) and isopropanol (Fig. 13) to growing cultures of *Clostridium autoethanogenum* DSM23693. Cell growth was visible in presence of concentrations as high as 5 % acetone or isopropanol (with only slight inhibition of growth rate).

A high concentration of free or undissociated acetic acid on the other hand is known to be detrimental for most anaerobic bacteria (including acetogenic bacteria) due to the deleterious effect on the membrane gradient [Warnecke T, Gill RT: Organic acid toxicity, tolerance, and production in Escherichia coli biorefining applications. Microb Cell Fact, 2005, 4: 25; Köpke M, Dürre P: Biochemical production of biobutanol, in Luque R, Campelo J, Clark JH (Eds.): Handbook of biofuel production - Processes and technologies, Woodhead Publishing, Camebridge, 2010: 221-257]. Acetogenic bacteria however, need to produce acetic acid to gain ATP from substrate level phosphorylation [Drake HL, Küsel K, Matthies C: Acetogenic Prokaryotes. In Dworkin M, Falkow S, Rosenberg E, Schleifer KH, Stackebrandt E (eds.): The Prokaryotes, 3rd Edition, Volume 2, Springer, New York, 2006: 354-420] and thus all known acetogenic species produce acetic acid [Drake HL, Küsel K, Matthies C: Acetogenic Prokaryotes. In Dworkin M, Falkow S, Rosenberg E, Schleifer KH, Stackebrandt E (eds.): The Prokaryotes, 3rd Edition, Volume 2, Springer, New York, 2006: 354-420]. Conversion of acetic acid to other products such as ethanol via aldehyde ferredoxin oxidoreductase (AOR) or back to acetyl-CoA via phosphotransacetylase/acetate kinase (Pta/Ack) or AMP-dependent acetyl-CoA synthase (Acs) is unfavourable, since it requires energy in the form of reduced ferredoxin or ATP [Wolfe AJ: The acetate switch. Microbiol Mol Biol Rev, 2005, 69: 12-50]. This invention presents a novel mode of acetic acid detoxification in acetogenic bacteria, which is free of energy requirement. Acetic acid can get recycled back to acetyl-CoA via a Acetoacetyl-CoA:Acetate/Butyrate Coenzyme A transferase system consisting of Acetyl-Coenzyme A acetyltransferase, Acetoacetyl-CoA:Acetate/Butyrate Coenzyme A transferase A, Acetoacetyl-CoA:Acetate/Butyrate Coenzyme A transferase B. This reaction drives the conversion of Acetoacetyl-CoA to Acetoacetate, which can then get decarboxylated to acetone and reduced to isopropanol (Fig. 4).

The invention has been described herein, with reference to certain preferred embodiments, in order to enable the reader to practice the invention without undue experimentation. Titles, headings, or the like are provided to enhance the reader's comprehension of this document, and should not be read as limiting the scope of the present invention.

The reference to any applications, patents and publications in this specification is not, and should not be taken as, an acknowledgment or any form of suggestion that they constitute valid prior art or form part of the common general knowledge in any country in the world.

Throughout this specification and any claims which follow, unless the context requires otherwise, the words "comprise", "comprising" and the like, are to be construed in an inclusive sense as opposed to an exclusive sense, that is to say, in the sense of "including, but not limited to".

## Claims

1. A method for the production of isopropanol by microbial fermentation, comprising fermenting a substrate comprising CO using a recombinant carboxydotrophic acetogenic microorganism capable of producing isopropanol by fermentation of CO,
wherein the microorganism expresses the enzymes: acetyl-coenzyme A acetyltransferase (thiolase; ThlA; E.C. 2.3.1.9); acetoacetyl-CoA:acetate coenzyme A transferase A (CoA transferase; CtfA; EC 2.8.3.9); and acetoacetyl-CoA:acetate coenzyme A transferase B (CoA transferase; CtfB; EC 2.8.3.9); and
wherein the microorganism further expresses the enzyme:
a. acetoacetate decarboxylase (Adc; EC 4.1.1.4); or
b. alpha-ketoisovalerate decarboxylase (decarboxylase; KivD; EC4.1.1.74);
wherein the enzymes are not naturally present in a parental microorganism from which the recombinant microorganism is derived.

2. The method of claim 1a, wherein the microorganism further expresses the enzyme alcohol dehydrogenase (Adh; EC 1.1.1.2).

3. The method of claim 2, wherein Adh is exogenously expressed.

4. The method of claim la, wherein the microorganism further expresses the enzyme alcohol dehydrogenase (Adh2; EC 1.1.1.1).

5. The method of claim 4, wherein Adh2 is exogenously expressed.

6. The method of claim 1a, wherein the microorganism further expresses the enzymes alpha-ketoisovalerate decarboxylase (decarboxylase; KivD; EC4.1.1.74) and alcohol dehydrogenase (Adh2; EC 1.1.1.1).

7. The method of claim 6, wherein Adh2 is exogenously expressed.

8. The method of claim 1, wherein the parental microorganism is selected from the group of carboxydotrophic acetogenic bacteria comprising: *Clostridium autoethanogenum, Clostridium ljungdahlii, Clostridium ragsdalei, Clostridium carboxidivorans, Clostridium drakei, Clostridium scatologenes, Butyribacterium limosum, Butyribacterium methylotrophicum, Acetobacterium woodii, Alkalibaculum bacchii, Blautia producta, Eubacterium limosum, Moorella thermoacetica, Moorella thermautotrophica, Oxobacter pfennigii,* and *Thermoanaerobacter kiuvi.*

9. The method of claim 6, wherein the parental microorganism is *Clostridium autoethanogenum* DSM23693 or *Clostridium ljungdahlii* DSM13528 (or ATCC55383).

10. The method of any one of the previous claims, wherein the enzyme:
a. ThlA is encoded by a nucleotide comprising the sequence of SEQ ID NO. 18; and
b. CtfA is encoded by a nucleotide comprising the sequence of SEQ ID NO. 19; and
c. CtfB is encoded by a nucleotide comprising the sequence of SEQ ID NO. 20 and/or
d. Adc is encoded by a nucleotide comprising the sequence of SEQ ID NO. 21; and/or
e. KivD is encoded by a nucleotide comprising the sequence of SEQ ID NO. 71; and/or
f. Adh is encoded by a nucleotide comprising the sequence of SEQ ID NO. 2 3 or 4; and/or
g. Adh2 is encoded by a nucleotide comprising the sequence of SEQ ID NO. 75.

11. The method of any one of the previous claims, wherein:
a. the gaseous substrate comprising CO is produced by an industrial process selected from the group consisting of ferrous metal products manufacturing, non-ferrous products manufacturing, petroleum refining processes, gasification of coal, gasification of biomass, electric power production, carbon black production, and coke manufacturing; or
b. the gaseous substrate comprises 20% to 70% CO by volume.

## Patentansprüche

1. Verfahren zur Herstellung von Isopropanol durch Mikroorganismenfermentation, umfassend das Fermentieren eines Substrats, das CO umfasst, unter Verwendung eines rekombinanten carboxydotrophischen acetogenen Mikroorganismus, der fähig ist Isopropanol durch Fermentation von CO zu erzeugen;
wobei der Mikroorganismus die Enzyme exprimiert: Acetyl-Coenzym A-Acetyltransferase (Thiolase; ThIA; E.C. 2.3.1.9); Acetoacetyl-CoA: Acetat-Coenzym A-Transferase A (COA-Transferase; CtfA; EC 2.8.3.9); und Acetoacetyl-CoA:Acetat-Coenzym A-Transferase B (COA-Transferase; CtfB; EC 2.8.3.9); und
wobei der Mikroorganismus weiter das Enzym exprimiert:
a. Acetoacetat-Decarboxylase (Adc; EC 4.1.1.4); oder
b. Alpha-Ketoisovalerat-Decarboxylase (Decarboxylase; KivD; EC4.1.1.74);
wobei die Enzyme in einem elterlichen Mikroorganismus, von dem der rekombinante Mikroorganismus abgeleitet ist, nicht natürlich vorhanden sind.

2. Verfahren nach Anspruch la, wobei der Mikroorganismus ferner das Enzym Alkoholdehydrogenase (Adh; EC 1.1.1.2) exprimiert.

3. Verfahren nach Anspruch 2, wobei Adh exogen exprimiert wird.

4. Verfahren nach Anspruch la, wobei der Mikroorganismus ferner das Enzym Alkoholdehydrogenase (Adh2; EC 1.1.1.1) exprimiert.

5. Verfahren nach Anspruch 4, wobei Adh2 exogen exprimiert wird.

6. Verfahren nach Anspruch 1a, wobei der Mikroorganismus ferner die Enzyme Alpha-Ketoisovalerat-Decarboxylase (Decarboxylase; KivD; EC4.1.1.74) und Alkoholdehydrogenase (Adh2; EC 1.1.1.1) exprimiert.

7. Verfahren nach Anspruch 6, wobei Adh2 exogen exprimiert wird.

8. Verfahren nach Anspruch 1, wobei der elterliche Mikroorganismus ausgewählt ist aus der Gruppe von carboxydotrophischen Säurebakterien, die umfasst: *Clostridium autoethanogenum, Clostridium jungdahlii, Clostridium ragsdalei, Clostridium carboxidivorans, Clostridium drakei, Clostridium scatologenes, Butyribacterium limosum, Butyribacterium methylotrophicum, Acetobacterium woodii, Alkalibaculum bacchii, Blautia producta, Eubacterium limosum, Moorella thermoacetica, Moorella thermautotrophica, Oxobacterpfennigii* und *Thermoanaerobacter kiuvi.*

9. Verfahren nach Anspruch 6, wobei der elterliche Mikroorganismus *Clostridium autoethanogenum* DSM23693 oder *Clostridium jungdahlii* DSM13528 (oder ATCC55383) ist.

10. Verfahren nach einem der vorstehenden Ansprüche, wobei das Enzym:
a. ThlA durch ein Nukleotid codiert ist, das die Sequenz von SEQ ID NO. 18 umfasst; und
b. CtfA durch ein Nukleotid codiert ist, das die Sequenz von SEQ ID NO. 19 umfasst; und
c. CtfB durch ein Nukleotid codiert ist, das die Sequenz von SEQ ID NO. 20 umfasst, und/oder
d. Adc durch ein Nukleotid codiert ist, das die Sequenz von SEQ ID NO. 21 umfasst, und/oder
e. KivD durch ein Nukleotid codiert ist, das die Sequenz von SEQ ID NO. 71 umfasst; und/oder
f. Adh durch ein Nukleotid codiert ist, das die Sequenz von SEQ ID NO. 2, 3 oder 4 umfasst; und/oder
g. Adh2 durch ein Nukleotid codiert ist, das die Sequenz von SEQ ID NO. 75 umfasst.

11. Verfahren nach einem der vorstehenden Ansprüche, wobei:
a. das gasförmige Substrat, das CO umfasst, durch einen technischen Prozess hergestellt wird, der ausgewählt ist aus der Gruppe bestehend aus Eisenmetallprodukteherstellung, Nichteisenprodukteherstellung, Erdölraffinationsprozesse, Kohlevergasung, Biomassenvergasung, Erzeugung von elektrischem Strom, Rußerzeugung und Koksherstellung; oder
b. das gasförmige Substrat 20 bis 70 Vol.-% CO umfasst.

## Revendications

1. Procédé pour la production d'isopropanol par fermentation microbienne, comprenant la fermentation d'un substrat comprenant du CO à l'aide d'un microorganisme acétogène carboxydotrophique recombinant capable de produire de l'isopropanol par fermentation de CO,
le microorganisme exprimant les enzymes : acétyl-coenzyme A acétyltransférase (thiolase ; ThlA ; E.C. 2.3.1.9) ; acétoacétyl-CoA:acétate coenzyme A transférase A (CoA transférase ; CtfA ; EC 2.8.3.9) ; et acétoacétyl-COA:acétate coenzyme A transférase B (CoA transférase ; CtfB ; EC 2.8.3.9) ; et
le microorganisme exprimant en outre l'enzyme :
a. acétoacétate décarboxylase (Adc ; EC 4.1.1.4) ; ou
b. alpha-cétoisovalérate décarboxylase (décarboxylase ; KivD ; EC4.1.1.74) ;
les enzymes n'étant pas naturellement présentes dans un microorganisme parent dont le microorganisme recombinant est dérivé.

2. Procédé de la revendication la, dans lequel le microorganisme exprime en outre l'enzyme alcool déshydrogénase (Adh ; EC 1.1.1.2).

3. Procédé de la revendication 2, dans lequel Adh est exprimée de manière exogène.

4. Procédé de la revendication la, dans lequel le microorganisme exprime en outre l'enzyme alcool déshydrogénase (Adh2 ; EC 1.1.1.1).

5. Procédé de la revendication 4, dans lequel Adh2 est exprimée de manière exogène.

6. Procédé de la revendication la, dans lequel le microorganisme exprime en outre les enzymes alpha-cétoisovalérate décarboxylase (décarboxylase ; KivD ; EC4.1.1.74) et alcool déshydrogénase (Adh2 ; EC 1.1.1.1).

7. Procédé de la revendication 6, dans lequel Adh2 est exprimé de manière exogène.

8. Procédé de la revendication 1, dans lequel le microorganisme parent est choisi dans le groupe des bactéries acétogènes carboxydotrophiques comprenant : *Clostridium autoethanogenum, Clostridium ljungdahlii, Clostridium ragsdalei, Clostridium carboxidivorans, Clostridium drakei, Clostridium scatologenes, Butyribacterium limosum, Butyribacterium methylotrophicum, Acetobacterium woodii, Alkalibaculum bacchii, Blautia producta, Eubacterium limosum, Moorella thermoacetica, Moorella thermautotrophica, Oxobacter pfennigii,* et *Thermoanaerobacter kiuvi.*

9. Procédé de la revendication 6, dans lequel le microorganisme parent est *Clostridium autoethanogenum* DSM23693 ou *Clostridium ljungdahlii* DSM13528 (ou ATCC55383).

10. Procédé de l'une quelconque des revendications précédentes, dans lequel l'enzyme :
a. ThlA est codée par un nucléotide comprenant la séquence de SEQ ID NO : 18 ; et
b. CtfA est codée par un nucléotide comprenant la séquence de SEQ ID NO : 19 ; et
c. CtfB est codée par un nucléotide comprenant la séquence de SEQ ID NO : 20 et/ou
d. Adc est codée par un nucléotide comprenant la séquence de SEQ ID NO : 21 ; et/ou
e. KivD est codée par un nucléotide comprenant la séquence de SEQ ID NO : 71 ; et/ou
f. Adh est codée par un nucléotide comprenant la séquence de SEQ ID NO : 2, 3 ou 4, et/ou
g. Adh2 est codée par un nucléotide comprenant la séquence de SEQ ID NO : 75.

11. Procédé de l'une quelconque des revendications précédentes, dans lequel :
a. le substrat gazeux comprenant du CO est produit par un processus industriel choisi dans le groupe constitué par la fabrication de produits métalliques ferreux, la fabrication de produits métalliques non ferreux, les processus de raffinage du pétrole, la gazéification du charbon, la gazéification de la biomasse, la production d'énergie électrique, la production de noir de charbon et la fabrication de coke ; ou
b. le substrat gazeux comprend 20 % à 70 % de CO en volume.
